# EUROPÄISCHE PATENTANMELDUNG

(11) **EP 4 458 804 A1**
(43) Veröffentlichungstag der Anmeldung: **06.11.2024**
(21) Anmeldenummer: 23171456.9
(22) Anmeldetag: 04.05.2023
(51) Int. Cl.: C07C 205/34, C07C 217/22, C07C 217/24, C07C 225/18, C07C 233/36, C07C 233/31, C07C 271/22, C07C 309/25, C07D 213/00, C07C 309/44, C07C 317/22, C07J 1/00, A01N 33/00, C08K 5/00, C09D 183/00

(54) **VERFAHREN ZUR HERSTELLUNG EINES GEGENSTANDES, PHENALEN-1-ON-VERBINDUNG SOWIE DEREN VERWENDUNG**

(71) Anmelder: TriOptoTec GmbH, 93053 Regensburg (DE)
(72) Erfinder: SPÄTH, Andreas, 93055 Regensburg (DE); KOLLER, Leonie, 93080 Pentling (DE); EICHNER, Anja, 93053 Regensburg (DE)
(74) Vertreter: Louis Pöhlau Lohrentz

(57) **Zusammenfassung**

Die vorliegende Erfindung betrifft ein Verfahren zur Herstellung eines Gegenstandes, eine Photosensibilisator-haltige Zusammensetzung, ein Phenalen-1-on Derivat der allgemeinen Formel (1), einen photokatalytisch aktiven Gegenstand sowie deren Verwendung.

## Beschreibung

Die vorliegende Erfindung betrifft ein Verfahren zur Herstellung eines Gegenstandes, eine Photosensibilisator-haltige Zusammensetzung, ein Phenalen-1-on Derivat der allgemeinen Formel (1), einen photokatalytisch aktiven Gegenstand sowie deren Verwendung.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erregerbedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkt zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen. Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen. Daher können gesunde, körpereigene Zellen eine Schädigung durch reaktive Sauerstoffspezies (ROS), beispielsweise Radikale oder Singulett-Sauerstoff, entgegenwirken.

Die WO 00/78854 A1 betrifft ein Verfahren zum Bereitstellen einer antimikrobiellen Oberfläche, wobei das Verfahren das Kombinieren von einem oder mehreren Polymeren mit einem oder mehreren Photosensibilisatoren umfasst, um eine Oberfläche zu bilden, die eine gehärtete Polymerzusammensetzung aufweist, die ein oder mehrere Polymere und einen oder mehrere nicht-kovalent und nicht-ionisch gebundene Photosensibilisatoren aufweist, wobei mindestens einer davon ein Xanthen-Photosensibilisator ist.

Nachteilig dabei ist, dass der Photosensibilisator aus der Polymermatrix ausläuft und somit die antimikrobielle Wirksamkeit bei längerer Lagerung abnimmt.

Die US 5,830,526 A offenbart ein Substrat, an das ein lichtaktivierbarer Farbstoff allein oder in Kombination mit zusätzlichen herkömmlichen antimikrobiellen und/oder antiviralen Mitteln gebunden ist. Das Substrat wird mit einem lichtaktivierbaren Farbstoff mit antimikrobiellen und/oder antiviralen Eigenschaften imprägniert, wobei ein kationisches oder anionisches wasserlösliches Polymer den Farbstoff an das Substrat bindet. Nachteilig ist jedoch, dass das in der US 5,830,526 A verwendete Polymer ausgewaschen werden kann und der verwendete lichtaktivierbaren Farbstoff nicht mehr am Substrat haftet.

Die WO 2018/167264 A1 offenbart eine Photosensibilisatorzusammensetzung umfassend (a) wenigstens eine Phenalen-1-on-Verbindung und (b) wenigstens eine polymere Komponente und/oder Vorläufer davon sowie einen Gegenstand, beispielsweise in Form einer Beschichtung, umfassend (a) die wenigstens eine Phenalen-1-on-Verbindung und (b) wenigstens eine ausgehärtete polymere Komponente, wobei die wenigstens eine Phenalen-1-on-Verbindung kovalent und/oder elektrostatisch an die wenigstens eine ausgehärtete polymere Komponente gebunden ist. Nachteilig bei den in der WO 2018/167264 A1 offenbarten Beschichtungen ist jedoch, dass die den Photosensibilisator enthaltende Beschichtung, beispielsweise durch mechanische Beanspruchung, von dem Substrat gelöst werden kann und somit die antimikrobielle Wirksamkeit reduziert wird.

Aufgabe der vorliegenden Erfindung ist es, eine Oberfläche bereitzustellen, die zumindest in wenigstens einem Teilbereich einen oder mehrere Photosensibilisatoren aufweist und somit nach Belichtung mit elektromagnetischer Strahlung geeigneter Wellenlänge und Intensität reaktive Sauerstoffspezies (ROS) bilden kann, um beispielsweise eine lokale antimikrobielle Wirkung gegenüber verschiedensten Mikroorganismen zu entfalten.

Die Fähigkeit, nach Belichtung mit elektromagnetischer Strahlung geeigneter Wellenlänge und Intensität reaktive Sauerstoffspezies (ROS) bilden zu können, soll dabei über ein möglichst einfaches und kostengünstiges Verfahren erfolgen, dass insbesondere auch eine nachträgliche Ausstattung bereits bestehender Oberflächen oder Teilbereiche davon ermöglicht. Dabei sollen vorzugsweise verschiedene Arten von Oberflächen mit der Fähigkeit ausgestattet werden können, nach Belichtung mit elektromagnetischer Strahlung geeigneter Wellenlänge und Intensität reaktive Sauerstoffspezies (ROS) zu bilden, wobei insbesondere zugleich eine gute antimikrobielle Wirksamkeit nach Belichtung mit elektromagnetischer Strahlung geeigneter Wellenlänge und Intensität gewährleistet werden soll.

Weiterhin soll das verwendete Verfahren eine behandelte Oberfläche bereitstellen, die die vorgenannten Nachteile einer aufgebrachten Beschichtung vermeidet, beispielsweise eine teilweise oder vollständige Trennung einer Photosensibilisator-haltigen Beschichtung unter mechanischer Beanspruchung.

Darüber hinaus soll ein Ausbluten des Photosensibilisators aus der behandelten Oberfläche vorzugsweise vermieden werden.

Das verwendete Verfahren soll vorzugsweise die mechanischen und/oder chemischen Eigenschaften, beispielsweise Reißfestigkeit, Elastizität und/oder Beständigkeit, beispielsweise gegenüber Umwelteinflüssen, nicht wesentlich verändern.

Die Aufgabe der vorliegenden Erfindung wird gelöst durch die Bereitstellung eines Verfahrens nach Anspruch 1 zur Herstellung eines Gegenstandes umfassend wenigstens einen photokatalytisch aktiven, vorzugsweise photodynamisch aktiven, Teilbereich wenigstens einer Oberfläche, wobei das Verfahren folgende Schritte umfasst
(a) Einbringen wenigstens eines Photosensibilisators in wenigstens einen Teilbereich wenigstens einer Oberfläche des Gegenstandes, wobei zumindest der Teilbereich wenigstens ein thermoplastisches Polymer, wenigstens ein elastomeres Polymer, Copolymere davon oder Mischungen davon umfasst, durch Kontaktieren des wenigstens eines Teilbereichs mit einer Photosensibilisator-haltigen Zusammensetzung umfassend:
   - Wasser, vorzugsweise in einem Anteil von mindestens 50 Gew.-%, bezogen auf das Gesamtgewicht der Photosensibilisator-haltigen Zusammensetzung,
   - wenigstens einen Photosensibilisator, der vorzugsweise aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on Derivaten, besteht,
   - wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, und
   - optional, wenigstens einen Alkohol, der vorzugsweise 2 bis 10 C-Atome, die gradkettig oder verzweigt sein können, und 1 bis 4 OH-Gruppen, umfasst oder daraus besteht,
   bei einer Temperatur aus einem Bereich von 5°C bis 95°C, vorzugsweise für einen Zeitraum von wenigstens 0,1 Minuten, unter Erhalt wenigstens eines behandelten Teilbereichs, und
(b) Trocknen zumindest des in Schritt (a) erhaltenen behandelten Teilbereichs der Oberfläche,
wobei zwischen Schritt (a) und Schritt (b) optional der wenigstens eine behandelte Teilbereich der wenigstens einen Oberfläche gespült wird, vorzugsweise mit Wasser.

Bevorzugte Ausführungsformen des erfindungsgemäßen Verfahrens sind in den Ansprüchen 2 bis 5, 15 und 17 angegeben.

Die Aufgabe der vorliegenden Erfindung wird weiterhin gelöst durch die Bereitstellung einer Photosensibilisator-haltigen Zusammensetzung gemäß Anspruch 6, vorzugsweise zur Verwendung in einem Verfahren nach einem der Ansprüche 1 - 5, umfassend:
- wenigstens einen Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivate, besteht,
- wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, und,
- optional, wenigstens ein Alkohol, der vorzugsweise 2 bis 10 C-Atome, die gradkettig oder verzweigt sein können, und 1 bis 4 OH-Gruppen aufweist oder daraus besteht.

Bevorzugte Ausführungsformen der erfindungsgemäßen Photosensibilisator-haltigen Zusammensetzung sind in den Ansprüchen 7 bis 11 und 14 angegeben.

Die Aufgabe der vorliegenden Erfindung wird ebenfalls gelöst durch die Bereitstellung eines erfindungsgemäßen Phenalen-1-on Derivats der allgemeinen Formel (1) gemäß Anspruch 12: 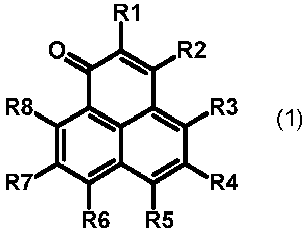
wobei die Reste R1 bis R8, die jeweils unabhängig voneinander gleich oder voneinander verschieden sein können, jeweils Wasserstoff, Chlor, Brom, lod, Alkyl mit 1 bis 4 C-Atomen, vorzugsweise Methyl, Ethyl, n-Propyl oder iso-Propyl, *-O-Alkyl mit 1 bis 4 C-Atomen, vorzugsweise Methoxy oder Ethoxy, oder einen organischen Rest W1a, der ein ungeladener organischer Rest ist, bedeuten,
mit der Maßgabe, dass wenigstens einer der Reste R1 bis R8, vorzugsweise wenigstens einer der Reste R1 bis R6, vorzugsweise wenigstens einer der Reste R1, R2, R4, R6 oder R8, weiter bevorzugt wenigstens einer der Reste R1, R2, R4 oder R6, weiter bevorzugt wenigstens einer der Reste R1 oder R2, weiter bevorzugt der Rest R1, ein organischer Rest W1a bedeutet,
wobei der Rest W1a ein Rest mit der allgemeinen Formel (10a) bis (12b), (13b), (14b), oder (15b) bis (16b) vorzugsweise ein Rest mit der allgemeinen Formel (10b) bis (12b), (13b), (14b), oder (15b) bis (16b), weiter bevorzugt ein Rest mit der allgemeinen Formel (10b), (11a), (12a), (14b) oder (16a), bedeutet:

   * - (C(D)(E))ₕ - T (10a)

   * - Q - (C(D)(E))ₕ - T (10b)

   * - [(C(D)(E))_{b} - A]ₐ - (C(D)(E))_{g} - T (11a)

   * - Q - [(C(D)(E))_{b} -A]ₐ - (C(D)(E))_{g} - T (11b)

   * - [(C(D)(E))_{b} - A]ₐ - (C(D)(E))_{y} - Ar - (C(D)(E))_{g} - T (12a)

   * - Q - [(C(D)(E))_{b} - A]ₐ - (C(D)(E))_{y} - Aᵣ - (C(D)(E))_{g} - T (12b)

   * - Q - (C(D)(E))ₕ - Ar - (C(D)(E))_{g} - T (13b)

   * - Q - (C(D)(E))_{y} - Ar - B - (C(D)(E))_{g} - T (14b)

   * - Q - (C(D)(E))_{y} - Aᵣ - B - [(C(D)(E))_{b} - A]_{c} - (C(D)(E))_{y} - T (15b)

   * - [(C(D)(E))_{b} - A]ₐ - (C(D)(E)_{y} - Ar - B - [(C(D)(E))_{b} - A]_{c} - (C(D)(E))_{y} - T (16a)

   * - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - C(=O)O - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * -NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei zwei oder mehr der Reste D und/oder E jeweils verbrückt sein können und jeweils einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, vorzugsweise Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei wenigstens einer der Reste D und/oder E mit wenigstens einem Ringatom des Rests Ar verbrückt sein kann und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei C-Atome der Cycloalkylreste, Cycloalkenylreste, Cycloalkanonreste, und Cycloalkanonreste jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 29, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 5, vorzugsweise 0 bis 3, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei in Rest (11a) Index g eine ganze Zahl von 2 bis 35 bedeutet, wenn Rest A ein Rest der Formel * - OC(=O) - ** und Rest T ein Rest der allgemeinen Formel * - C(D)(E)H bedeutet, und
wobei in Rest (15b) Index c eine ganze Zahl von 1 bis 35 bedeutet, wenn Rest B Sauerstoff und Rest T ein Rest der allgemeinen Formel * - C(D)(E)H bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet.

Die Aufgabe der vorliegenden Erfindung wird ebenfalls gelöst durch die Bereitstellung eines Curcumin-Derivats der allgemeinen Formel (2a) gemäß Anspruch 13: 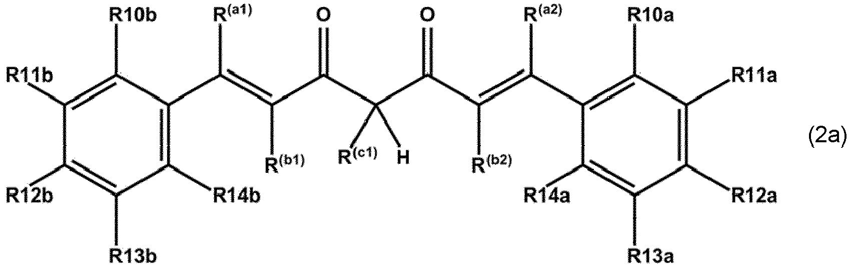
wobei die Reste R^{(a1)}, R^{(a2)}, R^{(b1)} und R^{(b2)}, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können,
wobei der Rest R^{(c1)} jeweils unabhängig voneinander Wasserstoff, Chlor, Brom, lod, Alkyl, das gradkettig oder verzweigt sein kann, mit 1 bis 4 C-Atomen, vorzugsweise Methyl, Ethyl, n-Propyl oder iso-Propyl, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
und wobei die Reste R10a bis R14b, die jeweils unabhängig voneinander gleich oder voneinander verschieden sein können, jeweils Wasserstoff, Halogen, vorzugsweise Chlor, Brom oder lod, Alkyl, das gradkettig oder verzweigt sein kann, mit 1 bis 8 C-Atomen, vorzugsweise Methyl, Ethyl, n-Propyl oder iso-Propyl, *-O-Alkyl, das gradkettig oder verzweigt sein kann, mit 1 bis 8 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, oder einen organischen Rest W1b bedeuten,
wobei in Variante b1) wenigstens einer der Reste R10a bis R14a und wenigstens einer der Reste R10b bis R14b, vorzugsweise die Reste R10a und R10b oder die Reste R11a und R11b oder die Reste R12a und R12b oder die Reste R13a und R13b oder die Reste R14a und R14b, ein organischer Rest W1b bedeutet und wobei optional wenigstens einer der Reste R10a bis R14a und wenigstens einer der Reste R10b bis R14b, vorzugsweise mindestens 2, weiter bevorzugt 2 oder 4, der Reste R10a und R10b oder R11a und R11b oder R12a und R12b oder R13a und R13b oder R14a und R14b, die nicht ein Rest W1b bedeuten, weiterhin Chlor, Brom oder lod bedeutet,
wobei der Rest W1b in Variante b1) ein Rest mit der allgemeinen Formel (10a) bis (11b) bedeutet:

   * - (C(D)(E))ₕ - T (10a)

   * - Q - (C(D)(E))ₕ - T (10b)

   * - [(C(D)(E))_{b} - A]ₐ - (C(D)(E))_{g} - T (11a)

   * - Q - [(C(D)(E))_{b} - A]ₐ - (C(D)(E))_{g} - T (11b)
wobei der Rest Q jeweils unabhängig voneinander Sauerstoff, ein Rest der Formel * - C(=O)O - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet, und
wobei der Rest T jeweils unabhängig voneinander ein Rest der allgemeinen Formel * - (C(D)(E)H, oder * - Ph, vorzugsweise ein Rest der allgemeinen Formel * - (C(D)(E)H, bedeutet
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, oder ein Rest der allgemeinen Formel * - C(=O)O - R⁽⁴⁾ bedeuten,
wobei der Rest R⁽¹⁾, jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeutet, und
wobei der Reste R⁽⁴⁾ jeweils unabhängig voneinander Alkyl, das gradkettig oder verzweigt sein kann, mit 1 bis 4 Kohlenstoffatomen bedeutet,
wobei der Index h jeweils unabhängig voneinander eine ganze Zahl von 1 bis 30, vorzugsweise von 4 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet
   oder
wobei in Variante b3) wenigstens einer der Reste R10a bis R14a und wenigstens einer der Reste R10b bis R14b, vorzugsweise die Reste R10a und R10b oder die Reste R11a und R11b oder die Reste R12a und R12b oder die Reste R13a und R13b oder die Reste R14a und R14b, ein organischer Rest W1b bedeutet und wobei optional wenigstens einer der Reste R10a bis R14a und wenigstens einer der Reste R10b bis R14b, vorzugsweise mindestens 2, weiter bevorzugt 2 oder 4, der Reste R10a und R10b oder R11a und R11b oder R12a und R12b oder R13a und R13b oder R14a und R14b, die nicht ein Rest W1b bedeuten, weiterhin Chlor, Brom oder lod bedeuten,
wobei der Rest W1b in Variante b3) ein Rest mit der allgemeinen Formel (30) bis (31b) bedeutet:

   - Z (30)

   * - [(C(D)(E))_{b} - A]_{c} - (C(D)(E))_{g} - Z (31a)

   * - Q - [(C(D)(E))_{b} - A]_{c} - (C(D)(E))_{g} - Z (31b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, oder ein Rest der allgemeinen Formel * - C(=O)O - R⁽⁴⁾ bedeuten, und
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽⁴⁾ jeweils unabhängig voneinander Alkyl, das gradkettig oder verzweigt sein kann, mit 1 bis 4 Kohlenstoffatomen bedeutet, und wobei der Rest Z jeweils unabhängig voneinander ein Säure-Rest ist, der aus der Gruppe, die aus Carboxygruppe und Sulfonsäuregruppe besteht, ausgewählt wird,
wobei der Index c jeweils unabhängig voneinander eine ganze Zahl von 0 bis 10, vorzugsweise von 0 bis 6, vorzugsweise 0 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet.

Die Aufgabe der vorliegenden Erfindung wird weiterhin gelöst durch die Bereitstellung einer Mehrkomponentenzusammensetzung gemäße Anspruch 16 zur gemeinsamen Verwendung in einem Verfahren nach einem der Ansprüche 1 bis 5 oder 15, umfassend
A) eine erste Zusammensetzung, umfassend Wasser, und wenigstens ein Co-Solvens, das aus der Gruppe, die aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon besteht, ausgewählt ist, sowie optional wenigstens einen aliphatischen Alkohol, der vorzugsweise 2 bis 10 C-Atome, die gradkettig oder verzweigt sein können, und 1 bis 4 OH-Gruppen aufweist oder daraus besteht, und/oder wenigstens einen Hilfsstoff, und
B) eine zweite Zusammensetzung, umfassend wenigstens einen Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht, wenigstens ein Co-Solvens, das aus der Gruppe, die aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon besteht, ausgewählt ist, sowie optional Wasser und/oder wenigstens einen Hilfsstoff.

Die Aufgabe der vorliegenden Erfindung wird ebenfalls gelöst durch die Bereitstellung eines photokatalytisch-aktiven Gegenstands gemäß Anspruch 18, vorzugsweise erhältlich nach einem Verfahren gemäß einem der Ansprüche 1 bis 5, 15 oder 17, wobei der photokatalytisch-aktiver Gegenstand dadurch gekennzeichnet ist, dass in wenigstens einem Teilbereich wenigstens einer Oberfläche des Gegenstandes, umfassend wenigstens ein thermoplastisches Polymer, und/oder wenigstens ein elastomeres Polymer, Copolymere davon und Mischungen davon, wenigstens ein Photosensibilisator, der vorzugsweise aus der Gruppe ausgewählt ist, die aus Phenalen-1-on-Derivaten, Curcumin-Derivaten, Porphycen-Derivaten und Mischungen davon besteht, eingebracht ist.

Bevorzugte Ausführungsformen der erfindungsgemäßen photokatalytisch-aktiven Gegenstands sind in Anspruch 19 angegeben.

Unter dem Begriff "photokatalytisch aktiv" bzw. "photokatalytisch aktiver Teilbereich" wird vorzugsweise eine Erzeugung von reaktiven Sauerstoffspezies (ROS), vorzugsweise freien Radikalen und/oder Singulett-Sauerstoff, aus Triplett-Sauerstoff nach Bestrahlung mit elektromagnetischer Strahlung, vorzugsweise sichtbares Licht und/oder UV-Licht, in Gegenwart von Sauerstoff und/oder einer Sauerstoff-abgebenden Verbindung, verstanden. Bestrahlung eines "photokatalytisch aktiven Teilbereichs" mit elektromagnetischer Strahlung, vorzugsweise sichtbares Licht und/oder UV-Licht, in Gegenwart von Sauerstoff und/oder einer Sauerstoff-abgebenden Verbindung führt somit zur, vorzugsweise lokalen, Erzeugung von reaktiven Sauerstoffspezies (ROS), vorzugsweise freien Radikalen und/oder Singulett-Sauerstoff, aus Triplett-Sauerstoff.

Unter dem Begriff "photodynamisch-aktiv" bzw. "photodynamisch-aktiver Teilbereich" wird vorzugsweise die lichtinduzierte Inaktivierung von Zellen oder Mikroorganismen und/oder einem Biofilm verstanden davon verstanden. Bestrahlung eines "photodynamisch-aktiven Teilbereichs" mit elektromagnetischer Strahlung, vorzugsweise sichtbares Licht und/oder UV-Licht, in Gegenwart von Sauerstoff und/oder einer Sauerstoff-abgebenden Verbindung führt dabei zur, vorzugsweise lokalen, Erzeugung von reaktiven Sauerstoffspezies (ROS), vorzugsweise freien Radikalen und/oder Singulett-Sauerstoff, aus Triplett-Sauerstoff, welche nachfolgend zu einer Photooxidation von Biomolekülen, insbesondere Lipiden und Proteinen, die sich in direkter Nachbarschaft zu den reaktiven Sauerstoffspezies (ROS) befinden, bewirken. Beispielsweise bewirkt eine Photooxidation von Lipiden der Zellmembran die Zerstörung der Zellmembran, wodurch es vorzugsweise wiederum zur Inaktivierung der betreffenden Mikroorganismen kommt.

Unter dem Begriff "Biofilm" wird vorzugsweise eine Matrix aus extrazellulärer polymerer Substanzen (EPS), die vorzugsweise von Mikroorganismen gebildet und von ihnen in ihre unmittelbare, der Zelle angrenzenden, Umgebung abgegeben werden, verstanden, in der Mikroorganismen, vorzugsweise Bakterien, Algen, Pilze, Protozoen oder Kombinationen davon, zumindest teilweise angeordnet oder eingebettet sind.

Nach Bestrahlung erzeugte reaktiven Sauerstoffspezies (ROS), vorzugsweise freien Radikalen und/oder Singulett-Sauerstoff, können ebenfalls oxidierbare organische oder anorganische Moleküle, beispielsweise verschiedenste Geruchsstoffe, abgebaut werden.

Als "Photosensibilisator" werden vorzugsweise Verbindungen verstanden, die elektromagnetische Strahlung, vorzugsweise sichtbares Licht und/oder UV-Licht, absorbieren und sodann reaktive Sauerstoffspezies (ROS), vorzugsweise freie Radikale und/oder Singulett-Sauerstoff, aus Triplett-Sauerstoff erzeugen. Erfindungsgemäße Photosensibilisatoren werden vorzugsweise aus der Gruppe ausgewählt, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht.

Ein photokatalytisch-aktiver, vorzugsweise photodynamisch-aktiver, Teilbereich, der mit dem erfindungsgemäßen Verfahren hergestellt wurde, weist Photosensibilisator-Moleküle auf, die zumindest oberflächennah und vorzugsweise bis zu einer bestimmten Tiefe im Material, das den Teilbereich der Oberfläche bildet, angeordnet sind.

Nach Bestrahlung des photokatalytisch-aktiven, vorzugsweise photodynamisch-aktiven, Teilbereichs mit elektromagnetischer Strahlung, vorzugsweise sichtbares Licht und/oder UV-Licht, in Gegenwart von Sauerstoff und/oder einer Sauerstoff-abgebenden Verbindung katalysieren die im Teilbereich eingebrachten Photosensibilisator-Moleküle die Bildung von reaktive Sauerstoffspezies (ROS), vorzugsweise freie Radikale und/oder Singulett-Sauerstoff, aus Triplett-Sauerstoff. Gebildete reaktive Sauerstoffspezies (ROS), vorzugsweise freie Radikale und/oder Singulett-Sauerstoff, diffundieren vorzugsweise aus dem wenigstens einen mit dem erfindungsgemäßen Verfahren behandelten Teilbereich der Oberfläche heraus und bewirken nachfolgend eine Inaktivierung von Zellen oder Mikroorganismen und/oder einem Biofilm davon und/oder einen Abbau von sonstigen oxidierbaren organischen oder anorganischen Molekülen, beispielsweise verschiedensten oxidierbaren Geruchsstoffen.

Unter dem Begriff "Geruchsstoff" werden vorzugsweise Moleküle verstanden, die olfaktorisch wahrgenommen einen Geruch entwickeln können. Geeignete Moleküle sind vorzugsweise organische oder anorganische Moleküle, die eine Molekülmasse von vorzugsweise kleiner als 350 Da aufweisen. Weiter bevorzugt ist ein Geruchsstoff ein oxidierbarer Geruchsstoff, der weiter bevorzugt wenigstens eine oxidierbare funktionelle Gruppe, die weiter bevorzugt ausgewählt ist aus OH-Gruppe, SH-Gruppe, -SAlkyl-Reste, exocyclische Doppelbindungen, elektronenreiche Amine, Polyphenole, Metallsulfide, organische Disulfide und Kombinationen davon, aufweist.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäßen Verbindung mit der Formel (1) oder (2a) und/oder wenigstens einer erfindungsgemäß verwendeten Verbindung mit der Formel (1), (2a), (2b) oder (3) und/oder wenigstens eines, mit dem erfindungsgemäßen Verfahren hergestellten Gegenstandes bestimmt werden.

Vorzugsweise kann eine Reduzierung der Lebensfähigkeit oder die Zerstörung von Mikroorganismen eines Biofilms, vorzugsweise dessen Zerstörung, eine Abgabe von extrazellulären polymeren Substanzen, die die Matrix eines Biofilms bilden, verringern bzw. verhindern. Dadurch wird vorzugsweise die Bildung eines Biofilms verlangsamt oder unterdrückt.

Erfindungsgemäß wird unter einer Reduzierung der Lebensfähigkeit verstanden, dass die Anzahl der Mikroorganismen, beispielsweise in einem Biofilm, um wenigstens 90,0 %, vorzugsweise wenigstens 95,0 %, vorzugsweise wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 % verringert wird.

Vorzugsweise wird die Reduzierung der Anzahl der Mikroorganismen gemäß Boyce, J. M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.lnfect.Control 30 (8), 2002, Seite 1 - 46) als log10-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens erfindungsgemäßen Verbindung mit der Formel (1) oder (2a) und/oder wenigstens einer erfindungsgemäß verwendeten Verbindung mit der Formel (1), (2a), (2b) oder (3) und/oder wenigstens eines, mit dem erfindungsgemäßen Verfahren hergestellten Gegenstandes verstanden. Beispielsweise können die Mikroorganismen in einem Biofilm enthalten sein.

Geeignete Methoden zur Bestimmung des log₁₀-Reduktionsfaktors sind beispielsweise in der DIN EN 14885:2007-01 "Chemische Desinfektionsmittel und Antiseptika -Anwendung Europäischer Normen für chemische Desinfektionsmittel und Antiseptika" oder in Rabenau, H. F. und Schwebke, I. ("Leitlinie der Deutschen Vereinigung zur Bekämpfung der Viruskrankheiten (DVV) e. V. und der Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen und/oder eines Biofilms davon mit elektromagnetischer Strahlung in Gegenwart wenigstens einer erfindungsgemäßen Verbindung mit der Formel (1) oder (2a) und/oder wenigstens einer erfindungsgemäß verwendeten Verbindung mit der Formel (1), (2a), (2b) oder (3) und/oder wenigstens eines, mit dem erfindungsgemäßen Verfahren hergestellten Gegenstandes mindestens 1 log₁₀, vorzugsweise mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀.

Vorzugsweise wird unter einem Halogen jeweils unabhängig voneinander Fluor, Chlor, Brom oder lod verstanden. Vorzugsweise wird unter einem Halogenid jeweils unabhängig voneinander Chlorid, Bromid oder lodid verstanden.

Vorzugsweise wird unter dem Begriff "Ringatom" ein Atom eines organischen Restes verstanden, der Bestandteil eines oder mehrerer Ringe ist.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1), (2a), (2b) und/oder (3), sowohl die reinen Verbindungen als auch die Isomerengemische in jedem Verhältnis.

Falls nichts anderes angegeben, bezeichnet ein * in einer Formel einen Verknüpfungspunkt zwischen zwei Resten.

Das erfindungsgemäße Verfahren zur Herstellung eines Gegenstandes umfassend wenigstens einen photokatalytisch aktiven, vorzugsweise photodynamisch aktiven, Teilbereich wenigstens einer Oberfläche ist dadurch gekennzeichnet, dass das Verfahren folgende Schritte umfasst
(a) Einbringen wenigstens eines Photosensibilisators in wenigstens einen Teilbereich wenigstens einer Oberfläche des Gegenstandes, wobei zumindest der Teilbereich wenigstens ein thermoplastisches Polymer, wenigstens ein elastomeres Polymer, Copolymere davon oder Mischungen davon umfasst oder daraus besteht, durch Kontaktieren des wenigstens eines Teilbereichs mit einer Photosensibilisator-haltigen Zusammensetzung umfassend:
   - Wasser, vorzugsweise in einem Anteil von mindestens 50 Gew.-%, bezogen auf das Gesamtgewicht der Photosensibilisator-haltigen Zusammensetzung,
   - wenigstens einen Photosensibilisator, der vorzugsweise aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on Derivaten, besteht,
   - wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, und
   - optional, wenigstens einen Alkohol, der vorzugsweise 2 bis 10 C-Atome, die gradkettig oder verzweigt sein können, und 1 bis 4 OH-Gruppen, umfasst oder daraus besteht,
   bei einer Temperatur aus einem Bereich von 5°C bis 95°C, vorzugsweise für einen Zeitraum von wenigstens 0,1 Minuten, unter Erhalt wenigstens eines behandelten Teilbereichs, und
(b) Trocknen zumindest des in Schritt (a) erhaltenen behandelten Teilbereichs der Oberfläche, vorzugsweise bei einer Temperatur von weniger als 150°C, vorzugsweise aus einem Bereich von 5°C bis 120°C,
   wobei zwischen Schritt (a) und Schritt (b) optional der wenigstens eine behandelte Teilbereich der wenigstens einen Oberfläche gespült wird, vorzugsweise mit Wasser.

Die Erfinder haben festgestellt, dass das erfindungsgemäße Verfahren ein einfaches und kostengünstiges Einbringen eines Photosensibilisators, der vorzugsweise aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht, in wenigstens einen Teilbereich wenigstens einer Oberfläche eines Gegenstandes ermöglicht.

Eine vorzugsweise Durchführung des erfindungsgemäßen Verfahrens bei einer Temperatur von weniger als 150°C hat weiterhin den Vorteil, dass eine thermische Inaktivierung des verwendeten Photosensibilisators vorzugsweise vermieden wird. Die Erfinder haben weiterhin festgestellt, dass hohe Verarbeitungstemperaturen, die beispielsweise bei einer Co-Extrusion von geeigneten Polymeren und Photosensibilisatoren auftreten, im erfindungsgemäßen Verfahren nicht erforderlich sind, um eine ausreichende Anzahl von Photosensibilisator-Molekülen in wenigstens einen Teilbereich wenigstens einer Oberfläche eines Gegenstandes einzubringen.

Die Struktur von organischen Photosensibilisator-Molekülen, insbesondere Phenalen-1-on-Derivaten, Curcumin-Derivaten, Porphycen-Derivaten und Mischungen davon, können bei Temperaturen von größer als 180°C geschädigt oder abgebaut werden, wodurch diese ihre photokatalytische Wirksamkeit verlieren. Weiterhin können durch eine thermische Schädigung und/oder einen thermischen Abbau gebildete Abbauprodukte des verwendeten Photosensibilisators zu einer unerwünschten Verfärbung des behandelten polymeren Materials, insbesondere wenigstens eines Teilbereichs einer Oberfläche, führen. Eine vorzugsweise Durchführung des erfindungsgemäßen Verfahrens bei einer Temperatur von höchstens 150°C verhindert weiter bevorzugt eine Bildung von entsprechenden Abbauprodukten, und somit weiter bevorzugt eine Verfärbung des behandelten Oberflächenteilbereichs.

Während des Kontaktierens des Teilbereichs, der wenigstens ein thermoplastisches Polymer, wenigstens ein elastomeres Polymer, Copolymere davon oder Mischungen davon umfasst oder daraus besteht, in Schritt (a) des erfindungsgemäßen Verfahrens mit einer in Anspruch 1 spezifizierten Photosensibilisator-haltigen Zusammensetzung, vorzugsweise einer erfindungsgemäßen Photosensibilisator-haltigen Zusammensetzung, bei einer Temperatur aus einem Bereich von 5°C bis 95°C quillt vorzugsweise der Teilbereich der Oberfläche zumindest oberflächennah teilweise auf. Dadurch kommt es vorzugsweise zu einer Aufweitung des polymeren Netzwerkes des behandelten Teilbereichs, wobei vorzugsweise Poren und/oder Spalten im behandelten Teilbereich entstehen können, in die der Photosensibilisator der in Schritt (a) verwendeten Zusammensetzung eindringen kann.

In Schritt (b) des erfindungsgemäßen Verfahrens wird zumindest der in Schritt (a) erhaltene behandelte Teilbereich getrocknet, vorzugsweise bei einer Temperatur von höchstens 150°C, vorzugsweise bei einer Temperatur aus einem Bereich von 5°C bis 120°C, vorzugsweise 10°C bis 80°C, weiter bevorzugt 15°C bis 70°C.

Vorzugsweise werden in Schritt (b) flüssige Bestandteile, beispielsweise Lösungsmittelrückstände, vom zumindest teilweise gequollenen Oberflächenteilbereich entfernt wodurch weiter bevorzugt entstandene Poren und/oder Spalten im behandelten Oberflächenteilbereich verschlossen werden. Der in den behandelten Teilbereich der Oberfläche eingebrachte Photosensibilisator wird vorzugsweise zumindest teilweise in der Oberfläche oder in oberflächennahen Bereichen fixiert.

Der mit dem erfindungsgemäßen Verfahren eingebrachte Photosensibilisator ist dabei nicht kovalent an die in den behandelten Teilbereich enthaltenen Polymermoleküle gebunden sondern vorzugsweise sterisch eingeschlossen.

Weiterhin kann der eingebrachte wenigstens eine Photosensibilisator, der vorzugsweise aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht, elektromagnetische Strahlung, vorzugsweise sichtbares Licht und/oder UV-Licht, absorbieren.

Darüber hinaus haben die Erfinder festgestellt, dass Sauerstoff zumindest teilweise in die Oberfläche, insbesondere den behandelten Teilbereich, eindringt. Der zumindest im behandelten Teilbereich eingebrachte wenigstens eine Photosensibilisator kann somit nach Absorption von eingestrahlter elektromagnetischer Strahlung, vorzugsweise sichtbares Licht und/oder UV-Licht, vorzugsweise sodann reaktive Sauerstoffspezies (ROS), vorzugsweise freie Radikale und/oder Singulett-Sauerstoff, aus Triplett-Sauerstoff erzeugen.

Weiterhin haben die Erfinder festgestellt, dass die nach Bestrahlung erzeugten reaktiven Sauerstoffspezies (ROS), vorzugsweise freie Radikale und/oder Singulett-Sauerstoff, ebenso aus der behandelten Oberflächenteilbereich diffundieren und nachfolgend eine Oxidation von Bestandteilen von Zellen oder Mikroorganismen und/oder einem Biofilm davon und/oder sonstigen oxidierbaren organischen oder anorganischen Molekülen, beispielsweise verschiedensten oxidierbaren Geruchsstoffen, bewirken.

Die Erfinder haben weiterhin festgestellt, dass bereits ein sehr geringer Zeitraum der Kontaktierung in Schritt (a) genügt, um eine ausreichend hohe Inaktivierung von Zellen oder Mikroorganismen und/oder einem Biofilm davon und/oder sonstigen oxidierbaren organischen oder anorganischen Molekülen, beispielsweise verschiedensten oxidierbaren Geruchsstoffen, nach Bestrahlung zu bewirken.

Vorzugsweise erfolgt das Kontaktieren in Schritt (a) des erfindungsgemäßen Verfahrens bei einer Temperatur aus einem Bereich von 5°C bis 95°C, vorzugsweise aus einem Bereich von 15°C bis 85°C, vorzugsweise aus einem Bereich von 20°C bis 80°C, weiter bevorzugt aus einem Bereich von 35°C bis 70°C, vorzugsweise für einen Zeitraum von wenigstens 0,1 Minuten, vorzugsweise für einen Zeitraum von 0,5 Minuten bis höchstens 360 Minuten, weiter bevorzugt für einen Zeitraum von 0,5 Minuten bis 180 Minuten, weiter bevorzugt von 1 Minuten bis 60 Minuten, erfolgt, weiter bevorzugt 2 Minuten bis 30 Minuten.

Vorzugsweise kann der Verlauf während des Kontaktierens in Schritt (a) das Einbringen des wenigstens einen Photosensibilisators in den behandelten Teilbereich der Oberfläche durch UV/VIS Spektroskopie kontrolliert werden, vorzugsweise durch Messung der Extinktion von eingestrahlter elektromagnetischer Strahlung mit aus dem Stand der Technik bekannte Verfahren, beispielsweise unter Verwendung eines UV/VIS-Spektrometers bei einer Wellenlänge, die vorzugsweise dem Hauptabsorptionsmaximums des verwendeten wenigstens einen Photosensibilisators entspricht, beispielsweise bei einer Wellenlänge von 400 nm. Die gemessene Extinktion eines behandelten Teilbereichs kann beispielsweise mit der Extinktion eines unbehandelten Teilbereichs der Oberfläche, die bei derselben Wellenlänge gemessen wurde, verglichen werden.

Vorzugsweise wird zwischen Schritt (a) und Schritt (b) optional der wenigstens eine behandelte Teilbereich der wenigstens einen Oberfläche gespült, vorzugsweise mit Wasser.

Das erfindungsgemäße Verfahren kann vorzugsweise diskontinuierlich oder kontinuierlich durchgeführt werden.

Beispielsweise kann bei einer diskontinuierlichen Durchführung der wenigstens ein Teilbereich einer Oberfläche in Schritt (a) in einem Behälter mit der verwendeten Photosensibilisator-haltigen Zusammensetzung bei einer Temperatur aus einem Bereich von 5°C bis 95°C für einen Zeitraum von vorzugsweise wenigstens 0,1 Minuten kontaktiert werden, beispielsweise durch Eintauchen in die verwendete Photosensibilisator-haltigen Zusammensetzung. Nach Abschluss der Kontaktierung wird weiter bevorzugt der wenigstens eine behandelte Teilbereich aus dem verwendeten Behälter entfernt, optional gewaschen, vorzugsweise mit Wasser, und anschließen in Schritt (b) getrocknet.

Bei einer kontinuierlichen Durchführung kann in Schritt (a) der wenigstens ein Teilbereich einer Oberfläche durch einen Behälter, der mit der verwendeten Photosensibilisator-haltigen Zusammensetzung gefüllt ist, bei einer Temperatur aus einem Bereich von 5°C bis 95°C für einen Zeitraum von vorzugsweise wenigstens 0,1 Minuten geführt werden, beispielsweise unter Verwendung von Walzen, Rollen oder einem Transportband. Nach Abschluss der Kontaktierung wird weiter bevorzugt der wenigstens eine behandelte Teilbereich optional gewaschen, vorzugsweise mit Wasser, und anschließen in Schritt (b) getrocknet.

Vorzugsweise umfasst oder besteht zumindest der wenigstens eine Teilbereich der wenigstens einen Oberfläche aus wenigstens ein thermoplastisches Polymer, das aus der Gruppe ausgewählt ist, die aus thermoplastischen aliphatischen Polyurethanen (TPU), thermoplastischen aromatischen Polyurethanen (TPU), thermoplastischen aliphatischen Polyamiden (PA), thermoplastischen aromatischen Polyamiden (PA), Polyimide (PI), Polyvinylchlorid (PVC), Polyethylenterephthalaten (PET), Polylactiden (PLA), Polymethacrylaten und Estern davon, Polyacrylaten und Estern davon, Copolymere von Polystyrol mit Ethylen, Propylen, Acrylnitril und/oder Vinylacetat, Copolymere aus Ethylen und Methacrylsäure, und Mischungen davon besteht, und/oder wenigstens ein elastomeres Polymer, das aus der Gruppe ausgewählt ist, die aus Kautschuk, Acrylnitril-Butadien-Kautschuk, Poly(organo)siloxanen, Styren-Ethylen-Propylen-Styren-Copolymere (SEPS), Ethylen-Vinylacetat-Copolymere (EVA), und Mischungen davon, besteht. Weiter bevorzugt umfasst oder besteht zumindest der wenigstens eine Teilbereich der wenigstens einen Oberfläche aus wenigstens einem Polymer, das aus der Gruppe, die aus thermoplastischen aliphatischen Polyurethanen (TPU), thermoplastischen aromatischen Polyurethanen (TPU), Polyimiden (Pl), Polyvinylchloriden (PVC), Polyethylenterephthalaten (PET), Poly(organo)siloxanen, Copolymere davon und Mischungen davon besteht, ausgewählt ist.

Mit dem erfindungsgemäßen Verfahren kann insbesondere wenigstens ein Oberflächenteilbereich eines bereits bestehenden Gegenstandes mit einer photokatalytischen Aktivität, vorzugsweise photodynamischen Aktivität, versehen werden, ohne das vorzugsweise das gesamte Volumen des entsprechenden Gegenstandes mit dem verwendete Photosensibilisators dotiert werden muss.

Vorzugsweise liegt ein geeigneter Gegenstand für das erfinderische Verfahren in Form eines selbsttragenden Films, eines Verbunds aus Fasern oder eines Formgegenstandes, vorzugsweise eines selbsttragenden Films oder eines Verbunds aus Fasern, vor, weiter bevorzugt in Form einer Folie, weiter bevorzugt Schutzfolie, Schutzhülle, oder Vorhang, eines Transportbandes, einer flächigen Unterlage, eines Gewebes, eines Filters, eines Bezugs, eines Schlauchs, eines Beutels, eines Gefäßes, einer Schale, einer Displayabdeckung, eines Tasters, eines Griffs, eines Handlaufs, einer Tastatur, oder einer Computer-Maus.

Vorzugsweise liegt ein verwendetes Polymer nicht in Form eines Kunststoff-Granulats vor.

Das erfindungsgemäße Verfahren kann insbesondere verwendet werden, um eine Oberflächen von Gegenständen aus geeigneten Polymeren, die bei höherer Temperatur, beispielsweise einer Temperatur von > 180°C, verarbeitet werden, bei der der verwendete Photosensibilisator jedoch in Mitleidenschaft gezogen oder zerstört wird, mit einer photokatalytische Aktivität, vorzugsweise photodynamischen Aktivität, zu versehen.

Eine erfindungsgemäße Photosensibilisator-haltige Zusammensetzung, die vorzugsweise im erfindungsgemäßen Verfahren verwendet wird, umfasst:
- wenigstens einen Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht,
- wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, und,
- optional, wenigstens ein Alkohol, der vorzugsweise 2 bis 10 C-Atome, die gradkettig oder verzweigt sein können, und 1 bis 4 OH-Gruppen aufweist oder daraus besteht.

Die Erfinder haben festgestellt, dass die erfindungsgemäße Photosensibilisator-haltige Zusammensetzung stabil über mehrere Monate ist. Vorzugsweise kann eine erfindungsgemäße Photosensibilisator-haltige Zusammensetzung auf die Verwendung mit unterschiedlichen Polymeren angepasst werden, vorzugsweise durch Auswahl entsprechender Lösungsmittel und/oder geeigneter Photosensibilisatoren und/oder geeigneter Hifsstoffe

Die erfindungsgemäße Photosensibilisator-haltige Zusammensetzung kann weiterhin wiederverwendet werden oder durch Zugabe von entsprechenden Komponenten, insbesondere dem verwendeten Photosensibilisator, regeneriert werden.

Eine erfindungsgemäße Photosensibilisator-haltige Zusammensetzung umfasst vorzugsweise keine Bindemittel, keine hochsiedenden Polyglykole mit vorzugsweise mehr als 25 Glykoleinheiten, keine Titanate, keine Siloxane, keine Silane, keine Aluminate, keine Zirkonate, keine Silikonöle, keine Metalloxide, keine Metallpartikel, keine Azofarbstoffe, keine Pigmente, keine Reaktivfarbstoffe, keine Küpenfarbstoffe, keine Dispersionsfarbstoffe, keine aliphatischen oder aromatischen Kohlenwasserstoffe, wie beispielsweise Benzin, Petrolether, Cyclohexan, Ligroin, Paraffinöl, Toluen oder Xylen, keine Salze von Metallen der 3 bis 7 Hauptgruppe des Periodensystems der Elemente sowie von Metallen der Nebengruppen des Periodensystems der Elemente.

Die Erfinder haben festgestellt, dass Polyglykole mit vorzugsweise mehr als 25 Glykoleinheiten, beispielsweise Polyethylenglycole (PEG) mit einem mittleren Molekülgewicht von 1400 g/mol und größer, in einen behandelten Oberflächenteilbereich verbleiben und nachfolgend vorzugsweise ein Trocknen verhindern, wodurch weiter bevorzugt entstandene Poren und/oder Spalten im behandelten Oberflächenteilbereich nicht oder nur teilweise verschlossen werden.

Vorzugsweise umfasst eine erfindungsgemäße Photosensibilisator-haltige Zusammensetzung weiterhin Wasser, vorzugsweise in einem Anteil von wenigstens 10 Gew.-%, jeweils bezogen auf das Gesamtgewicht der Zusammensetzung.

Das wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, wird weiter bevorzugt ausgewählt aus der Gruppe aus Ethylacetat, Ethylpropionat, n-Propylacetat, Isopropylacetat, n-Butylacetat, Isobutylacetat, n-Amylacetat, Isoamylacetat, Methylisobutyrat, Ethylisobutyrat, Methylpentanoat, Methylisovalerat, Methylhexanoat, 1,2-Propylenglykoldiacetat, Triacetin, Aceton, Methylethylketon, 2-Pentanon, 3-Pentanon, 2-Hexanon, 3-Hexanon, 2-Heptanon, 3-Heptanon, 4-Heptanon, Methylisobutylketon, Acetylaceton, 3-Methyl-2,4-pentandion, 3,3-dimethylpentan-2,4-dion, Diacetonalkohol, Cyclohexanon, Cyclopentanon, Malonsäuredimethylester, Malonsäurediethylester, Acetessigsäuremethylester, Acetessigsäureethylester, Dimethylsuccinat, Diethylsuccinat, Dimethylglutarat, Dibasischer Ester, Methoxyethylacetat, Methoxypropylacetat, Ethyllactat, Dihydrolevoglucosenon oder Texanol.

Weiter bevorzugt ist das wenigstens eine Co-Solvens in der erfindungsgemäßen Photosensibilisator-haltige Zusammensetzung in einem Anteil von 0,5 Gew.-% bis 12 Gew.-%, vorzugsweise in einem Anteil von 1 Gew.-% bis 10 Gew.-%, weiter bevorzugt in einem Anteil von 1,5 Gew.-% bis 9 Gew.-%, weiter bevorzugt in einem Anteil von 2 Gew.-% bis 8 Gew.-%, jeweils bezogen auf das Gesamtgewicht der Zusammensetzung, enthalten.

Weiter bevorzugt ist das wenigstens eine Co-Solvens Ethylacetat, Ethylpropionat, n-Propylacetat, Isopropylacetat, n-Butylacetat, Isobutylacetat, n-Amylacetat, Isoamylacetat, Methylisobutyrat, Ethylisobutyrat, Methylpentanoat, Methylisovalerat, Methylhexanoat, oder 1,2-Propylenglykoldiacetat in einem Anteil von 1 Gew.-% bis 8 Gew.-%, weiter bevorzugt in einem Anteil von 2 Gew.-% bis 6 Gew.-%, jeweils bezogen auf das Gesamtgewicht der Zusammensetzung, oder Aceton, Methylethylketon, 2-Pentanon, 3-Pentanon, 2-Hexanon, 3-Hexanon, 2-Heptanon, 3-Heptanon, 4-Heptanon oder Diacetonalkohol in einem Anteil von 1 Gew.-% bis 10 Gew.-%, weiter bevorzugt in einem Anteil von 2 Gew.-% bis 8 Gew.-%, jeweils bezogen auf das Gesamtgewicht der Zusammensetzung, oder Methylisobutylketon, Acetylaceton, 3-Methyl-2,4-pentandion, 3,3-dimethylpentan-2,4-dion, Cyclohexanon oder Cyclopentanon in einem Anteil von 1 Gew.-% bis 5 Gew.-%, weiter bevorzugt in einem Anteil von 2 Gew.-% bis 4 Gew.-%, jeweils bezogen auf das Gesamtgewicht der Zusammensetzung, oder Malonsäuredimethylester, Malonsäurediethylester, Acetessigsäuremethylester, Acetessigsäureethylester, Dimethylsuccinat, Diethylsuccinat, Dimethylglutarat oder Dibasischer Ester in einem Anteil von 1 Gew.-% bis 12 Gew.-%, weiter bevorzugt in einem Anteil von 2 Gew.-% bis 10 Gew.-%, jeweils bezogen auf das Gesamtgewicht der Zusammensetzung, oder Methoxyethylacetat, Methoxypropylacetat, Ethyllactat, Dihydrolevoglucosenon, oder Texanol in einem Anteil von 1 Gew.-% bis 8 Gew.-%, weiter bevorzugt in einem Anteil von 2 Gew.-% bis 6 Gew.-%, jeweils bezogen auf das Gesamtgewicht der Zusammensetzung.

Eine erfindungsgemäße Photosensibilisator-haltige Zusammensetzung umfasst vorzugsweise weiterhin wenigstens ein Alkohol, der vorzugsweise 2 bis 10 C-Atome, die gradkettig oder verzweigt sein können, und 1 bis 4 OH-Gruppen aufweist oder daraus besteht.

Ein geeigneter Alkohol wird weiter bevorzugt aus der Gruppe, die aus Isopropanol, n-Propanol, 2-Methyl-1-propanol, n-Butanol, 2-Butanol, 2-Methyl-1-butanol, 3-Methyl-1-butanol, tert-Butanol, Neopentylalkohol, Isoamylalkohol (Isomerenmischung), 1-Phenyl-1-propanol, 1,2-Propandiol, 1,3-Propandiol, Glycerin, Diglycerin, Dipropylenglykol, 1,2-Butandiol, 1,4-Butandiol, 1,2-Pentandiol, 1,4-Pentandiol, 1,5-Pentandiol, 1-Methoxy-2-propanol, 1-Ethoxy-2-propanol, 3-Methoxy-1,2-propandiol, 2-Methyl-1-butanol, 2-Methyl-2-butanol, 3-Methyl-2-butanol, 3-Methoxybutanol, 3-Methoxy-3-methyl-butanol, 3-Methoxy-1-butanol, 3-Methyl-1-pentanol, 2-Ethylhexanol, O-Butylglycerin, O-Pentylglycerin, O-Ethylhexylglycerin, 2-Isopropoxyethanol, 2-Propoxyethanol, iso-Butylglykol, Butylglykol, Butyldiglykol, Cyclopentanol, Cyclohexanol, Solketal und Propylenglykolmonobutylether besteht, ausgewählt.

Weiter bevorzugt ist der optional enthaltene wenigstens eine Alkohol, der vorzugsweise 2 bis 10 C-Atome, die gradkettig oder verzweigt sein können, und 1 bis 4 OH-Gruppen aufweist oder daraus besteht, in der erfindungsgemäßen Photosensibilisator-haltige Zusammensetzung in einem Anteil von 0 Gew.-% bis 30 Gew.-%, vorzugsweise in einem Anteil von 0,5 Gew.-% bis 25 Gew.-%, vorzugsweise in einem Anteil von 1 Gew.-% bis 17 Gew.-%, weiter bevorzugt in einem Anteil von 1,5 Gew.-% bis 15 Gew.-%, weiter bevorzugt in einem Anteil von 2 Gew.-% bis 13 Gew.-%, weiter bevorzugt in einem Anteil von 3 Gew.-% bis 12 Gew.-%, jeweils bezogen auf das Gesamtgewicht der Zusammensetzung, enthalten.

Weiter bevorzugt ist der wenigstens eine Alkohol, der vorzugsweise 2 bis 10 C-Atome, die gradkettig oder verzweigt sein können, und 1 bis 4 OH-Gruppen aufweist oder daraus besteht, Isopropanol oder n-Propanol in einem Anteil von 0 Gew.-% bis 30 Gew.-%, weiter bevorzugt in einem Anteil von 2 Gew.-% bis 25 Gew.-%, noch weiter bevorzugt in einem Anteil von 1 Gew.-% bis 10 Gew.-%, jeweils bezogen auf das Gesamtgewicht der Zusammensetzung, oder 2-Methyl-1-propanol, n-Butanol oder 2-Butanol in einem Anteil von 0 Gew.-% bis 10 Gew.-%, weiter bevorzugt in einem Anteil von 0 Gew.-% bis 8 Gew.-%, jeweils bezogen auf das Gesamtgewicht der Zusammensetzung, oder 2-Methyl-1-butanol, 3-Methyl-1-butanol, tert-Butanol, Neopentylalkohol, Isoamylalkohol (Isomerenmischung), oder 1-Phenyl-1-propanol in einem Anteil von 0 Gew.-% bis 10 Gew.-%, weiter bevorzugt in einem Anteil von 1 Gew.-% bis 3 Gew.-%, jeweils bezogen auf das Gesamtgewicht der Zusammensetzung, oder 1,2-Propandiol, 1,3-Propandiol, Glycerin, Diglycerin oder Dipropylenglykol in einem Anteil von 0 Gew.-% bis 12 Gew.-%, weiter bevorzugt in einem Anteil von 0 Gew.-% bis 8 Gew.-%, jeweils bezogen auf das Gesamtgewicht der Zusammensetzung, oder 1,5-Pentandiol, 1,2-Butandiol, 1,4-Butandiol, 1,2-Pentandiol, 1,4-Pentandiol oder 1,5-Pentandiol in einem Anteil von 0 Gew.-% bis 12 Gew.-%, weiter bevorzugt in einem Anteil von 1 Gew.-% bis 10 Gew.-%, jeweils bezogen auf das Gesamtgewicht der Zusammensetzung, oder 1-Methoxy-2-propanol, 1-Ethoxy-2-propanol oder 3-Methoxy-1,2-propandiol in einem Anteil von 0 Gew.-% bis 8 Gew.-%, weiter bevorzugt in einem Anteil von 1 Gew.-% bis 6 Gew.-%, jeweils bezogen auf das Gesamtgewicht der Zusammensetzung, oder 2-Methyl-1-butanol, 2-Methyl-2-butanol, 3-Methyl-2-butanol, 3-Methoxybutanol, Methoxy-3-methyl-butanol, 3-Methoxy-1-butanol, 3-Methyl-1-pentanol, 2-Ethylhexanol, O-Butylglycerin, O-Pentylglycerin, oder O-Ethylhexylglycerin in einem Anteil von 0 Gew.-% bis 8 Gew.-%, weiter bevorzugt in einem Anteil von 0 Gew.-% bis 5 Gew.-%, jeweils bezogen auf das Gesamtgewicht der Zusammensetzung, oder 2-Isopropoxyethanol, 2-Propoxyethanol, Butylglykol, iso-Butylglykol, Butyldiglykol, Cyclopentanol, Cyclohexanol, Solketal oder Propylenglykolmonobutylether in einem Anteil von 0 Gew.-% bis 8 Gew.-%, weiter bevorzugt in einem Anteil von 0 Gew.-% bis 5 Gew.-%, jeweils bezogen auf das Gesamtgewicht der Zusammensetzung.

Weiterhin umfasst die Photosensibilisator-haltige Zusammensetzung Wasser, vorzugsweise in einem Anteil von wenigstens 10 Gew.-%, vorzugsweise in einem Anteil von wenigstens 50 Gew.-%, weiter bevorzugt in einem Anteil von 65 Gew.-% bis 98 Gew.-%, weiter bevorzugt in einem Anteil von 70 Gew.-% bis 93 Gew.-%, jeweils bezogen auf das Gesamtgewicht der Zusammensetzung.

Der Wassergehalt der im erfindungsgemäßen Verfahren verwendeten Photosensibilisator-haltigen Zusammensetzung kann vorzugsweise vor Schritt (a) durch Zugabe von Wasser auf einem Anteil von wenigstens 50 Gew.-%, weiter bevorzugt in einem Anteil von 65 Gew.-% bis 98 Gew.-%, weiter bevorzugt in einem Anteil von 70 Gew.-% bis 93 Gew.-%, jeweils bezogen auf das Gesamtgewicht der Zusammensetzung, eingestellt werden.

Weiter bevorzugt kann der Wassergehalt ebenfalls während Schritt (a) kontrolliert werden, ggf. durch geeignete dem Fachmann bekannte Maßnahmen, und bei unterschreiten eines Anteils von wenigstens 50 Gew.-%, jeweils bezogen auf das Gesamtgewicht der Zusammensetzung, durch Zugabe von Wasser wieder auf einen Anteil von wenigstens 50 Gew.-% eingestellt werden.

Eine erfindungsgemäße Photosensibilisator-haltige Zusammensetzung umfasst wenigstens eine Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht.

Vorzugsweise ist das wenigstens eine Phenalen-1-on-Derivat ein erfindungsgemäß zu verwendendes Phenalen-1-on-Derivat der allgemeinen Formel (1): 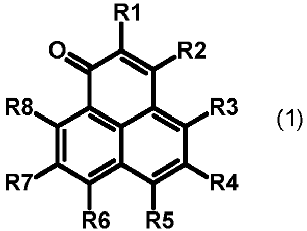
wobei die Reste R1 bis R8, die jeweils unabhängig voneinander gleich oder voneinander verschieden sein können, jeweils Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, oder einen organischen Rest W1a, der:
   a1) ein ungeladener organischer Rest, oder
   a2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder,
   a3) ein organischer Rest mit wenigstens einer Säuregruppe, vorzugsweise 1 oder 2 Säuregruppen, oder
   a4) ein amphoterer organischer Rest, der ein permanent positiv geladenes Stickstoffatom oder ein protonierbares Stickstoffatom, das jeweils nicht direkt am aromatischen Ring angeordnet ist, und eine Säuregruppe umfasst,
   vorzugsweise a2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder, a3) ein organischer Rest mit wenigstens einer Säuregruppe, vorzugsweise 1 oder 2 Säuregruppen, bedeutet,
   mit der Maßgabe, dass einer der Reste R1 bis R8, vorzugsweise einer der Reste R1 bis R6, vorzugsweise einer der Reste R1, R2, R4, R6 oder R8, weiter bevorzugt einer der Reste R1, R2, R4 oder R6, weiter bevorzugt einer der Reste R1 oder R2, weiter bevorzugt der Rest R1, ein organischer Rest W1a bedeutet,
wobei der Rest W1a in Variante a1) ein Rest mit der allgemeinen Formel (10a) bis (16b) bedeutet:

   * - (C(D)(E))ₕ - T (10a)

   * - Q - (C(D)(E))ₕ - T (10b)

   * - [(C(D)(E))_{b} - A]ₐ - (C(D)(E))_{g} - T (11a)

   * - Q - [(C(D)(E))_{b} - A]ₐ - (C(D)(E))_{g} - T (11b)

   * - [(C(D)(E))_{b} - A]ₐ - (C(D)(E))_{y} - Ar - (C(D)(E))_{g} - T (12a)

   * - Q - [(C(D)(E))_{b} - A]ₐ - (C(D)(E))_{y} - Ar - (C(D)(E))_{g} - T (12b)

   * - (C(D)(E))ₕ - Ar - (C(D)(E))_{g} - T (13a)

   * - Q - (C(D)(E))ₕ - Ar - (C(D)(E))_{g} - T (13b)

   * - (C(D)(E))_{y} - Ar - B - (C(D)(E))_{g} - T (14a)

   * - Q - (C(D)(E))_{y} - Ar - B - (C(D)(E))_{g} - T (14b)

   * - (C(D)(E))_{y} - Ar - B - [(C(D)(E))_{b} - A]_{c} - (C(D)(E))_{y} - T (15a)

   * - Q - (C(D)(E))_{y} - Aᵣ - B - [(C(D)(E))_{b} - A]_{c} - (C(D)(E))_{y} - T (15b)

   * - [(C(D)(E))_{b} - A]ₐ - (C(D)(E)_{y} - Ar - B - [(C(D)(E))_{b} - A]_{c} - (C(D)(E))_{y} - T (16a)

   * - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Hydroxyl, Nitro, Alkyl, das gradkettig oder verzweigt sein kann, mit 1 bis 15 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
und wobei zwei oder mehr der Reste D und/oder E jeweils verbrückt sein können und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkanonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei wenigstens einer der Reste D und/oder E mit wenigstens einem Ringatom des Rests Ar verbrückt sein kann und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei C-Atome des Cycloalkylrestes, cyklischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, Nitro, Nitril, einem Rest der allgemeinen Formel * - C(=O)NH₂ und Kombinationen davon, besteht, ausgewählt werden, substituiert ist, bedeutet, und
wobei der Rest W1a in Variante a2) ein Rest mit der allgemeinen Formel (21a) bis (24b) bedeutet:

   * - [(C(D)(E))_{b} - A]_{c} - (C(D)(E))_{g} - X (21a)

   * - Q - [(C(D)(E))_{b} - A]_{c} - (C(D)(E))_{g} - X (21b)

   * - [(C(D)(E))_{b} - A]ₐ - (C(D)(E))_{y} - Ar - (C(D)(E))_{f} - X (22a)

   * - Q - [(C(D)(E))_{b} - A]ₐ - (C(D)(E))_{y} - Aᵣ - (C(D)(E))_{f} - X (22b)

   * - (C(D)(E))_{d} - Ar - (C(D)(E))_{f} - X (23a)

   * - Q - (C(D)(E))_{d} - Ar - (C(D)(E))_{f} - X (23b)

   * - (C(D)(E))_{d} - Ar - [A - (C(D)(E))_{b}]ₐ - X (24a)

   * - Q - (C(D)(E))_{d} - Ar - [A - (C(D)(E))_{b}]ₐ - X (24b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise 1 bis 6, vorzugsweise von 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices d, und f jeweils unabhängig voneinander eine ganze Zahl von 1 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar einen unsubstituierten Phenylen-Rest oder einen substituierten Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert ist, bedeutet, und
wobei der Rest X jeweils unabhängig voneinander ein Rest der Formel (15a), (15b), (16), (17a), (17b), (17c), oder (17d), vorzugsweise ein Rest der Formel (15a), (15b), (17a) oder (17b), bedeutet: 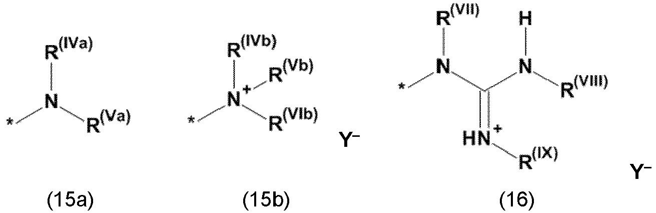 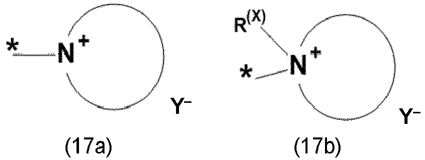 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)} und R^{(X)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (17a): 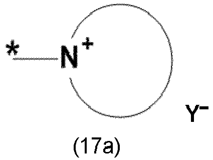 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, bedeutet, wobei der heterocyclische Rest mit 5 bis 10 Ringatomen unsubstituiert oder mit einem oder mehrerer Reste substituiert ist, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden, und
wobei der Rest mit der Formel (17b): 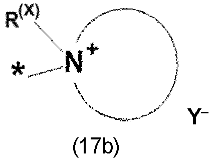 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung mit einem Ringatom, vorzugsweise Kohlenstoffatom, ausbildet, bedeuten, wobei der heterocyclisch Rest mit 5 bis 7 Ringatomen, unsubstituiert oder mit einem oder mehrerer Reste substituiert ist, die aus der Gruppe, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, und Mischungen davon besteht, ausgewählt ist, und
wobei der Rest W1a in Variante a3) ein Rest mit der allgemeinen Formel (30) bis (34b) bedeutet:

   - Z (30)

   * - [(C(D)(E))_{b} - A]_{c} - (C(D)(E))_{g} - Z (31a)

   * - Q - [(C(D)(E))_{b} - A]_{c} - (C(D)(E))_{g} - Z (31b)

   * - [(C(D)(E))_{b} - A]ₐ - (C(D)(E))_{y} - Ar - (C(D)(E))ₑ - Z (32a)

   * - Q - [(C(D)(E))_{b} - A]ₐ - (C(D)(E))_{y} - Ar - (C(D)(E))ₑ - Z (32b)

   * - (C(D)(E))_{d} - Ar - (C(D)(E))ₑ - Z (33a)

   * - Q - (C(D)(E))_{d} - Ar - (C(D)(E))ₑ - Z (33b)

   * - (C(D)(E))_{d} - Ar - [A - (C(D)(E))_{b}]ₐ - Z (34a)

   *-Q-(C(D)(E))_{d}-Ar-[A-(C(D)(E))_{b}]ₐ-Z (34b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise 1 bis 6, vorzugsweise von 2 bis 4, weiter bevorzugt 1 oder 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index d jeweils unabhängig voneinander eine ganze Zahl von 1 bis 10, vorzugsweise von 2 bis 4, bedeutet, und
wobei der Index e jeweils unabhängig voneinander eine ganze Zahl von 0 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Rest W1a in Variante a4) ein Rest mit der allgemeinen Formel (41a) oder (41b) bedeutet:

   *-(CH₂)_{g}-Het-(CH₂)ᵢ-Z (41a)

   *-(CH₂)_{g}-Het-[B-(CH₂)ₖ]ₗ-Z (41b)
wobei der Rest B jeweils unabhängig voneinander Sauerstoff, ein Rest der Formel * - C(=O) - *, ein Rest der Formel * - C(=O)O - *, ein Rest der Formel * - OC(=O) - *, oder ein Rest der Formel *-C(=O)N(R⁽⁶⁾)-* bedeutet, wobei der Rest R⁽⁶⁾ jeweils unabhängig voneinander Wasserstoff oder Alkyl, das gradkettig oder verzweigt sein kann, mit 1 bis 4 Kohlenstoffatomen bedeutet,
wobei der Rest Het jeweils einen heterocyclischen Rest mit 5 bis 10 Ringatomen, die mindestens 1 Kohlenstoffatom und mindestens 1 Stickstoffatom sowie optional 1 oder 2 Sauerstoffatome umfassen, bedeutet, der vorzugsweise ein Rest mit der Formel (17a) oder ein Rest mit der Formel (17c) ist: 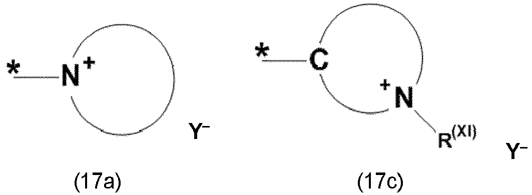
wobei der heterocyclisch Rest 5 bis 10 Ringatome aufweist, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, wobei der Rest R^{(XI)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices i, und k jeweils unabhängig voneinander eine ganze Zahl von 1 bis 4, vorzugsweise 2 bis 3, bedeutet, und
wobei die Index I jeweils unabhängig voneinander eine ganze Zahl von 0 bis 4, vorzugsweise 0 bis 3, bedeuten, und
wobei der Rest Z jeweils unabhängig voneinander ein Säure-Rest ist, der aus der Gruppe, die aus Carboxygruppe und Sulfonsäuregruppe besteht, ausgewählt wird.

Weiter bevorzugt ist das wenigstens eine Phenalen-1-on-Derivat ein erfindungsgemäß zu verwendendes Phenalen-1-on-Derivat der allgemeinen Formel (1), wobei einer der Reste R1 bis R8, vorzugsweise einer der Reste R1 bis R6, vorzugsweise einer der Reste R1, R2, R4, R6 oder R8, weiter bevorzugt einer der Reste R1, R2, R4 oder R6, weiter bevorzugt einer der Reste R1 oder R2, weiter bevorzugt der Rest R1, ein organischer Rest W1a bedeutet, wobei der organische Rest W1a ein Rest mit der Formel (11a), (11b), (12a), (12b), (21a), (21b), (22a), oder (22b) bedeutet, und wobei der Index a jeweils unabhängig voneinander eine ganze Zahl von 0 bis 2, vorzugsweise 1 oder 2, bedeutet, und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und wobei der Index b jeweils unabhängig voneinander eine ganze Zahl von 1 bis 3, vorzugsweise 2 bis 3, bedeutet, und wobei der Index y jeweils unabhängig voneinander eine ganze Zahl von 0 bis 2, und wobei der Index c jeweils unabhängig voneinander eine ganze Zahl von 0 bis 2, vorzugsweise 1 oder 2, bedeutet.

Bei einer weiter bevorzugten Ausführungsform ist das wenigstens eine Phenalen-1-on-Derivat ein erfindungsgemäß zu verwendendes Phenalen-1-on-Derivat der allgemeinen Formel (1), wobei einer der Reste R1 bis R8, vorzugsweise einer der Reste R1 bis R6, vorzugsweise einer der Reste R1, R2, R4, R6 oder R8, weiter bevorzugt einer der Reste R1, R2, R4 oder R6, weiter bevorzugt einer der Reste R1 oder R2, weiter bevorzugt der Rest R1, ein organischer Rest W1a bedeutet, der ausgewählt ist aus der Gruppe, die aus den Resten mit der Formel (400) bis (585) besteht:

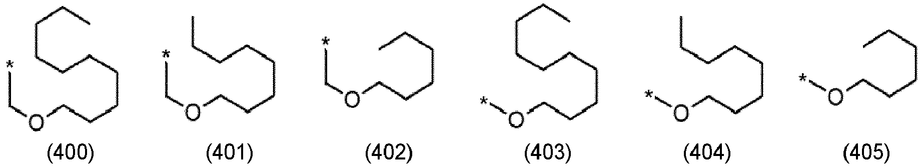

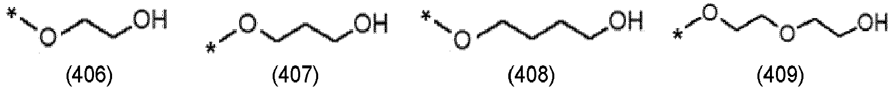

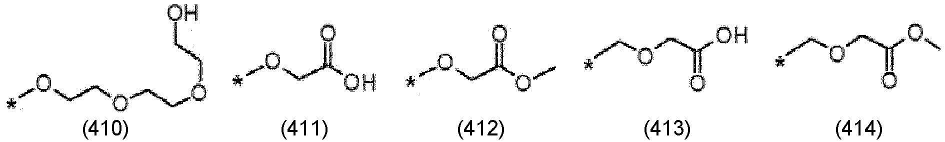

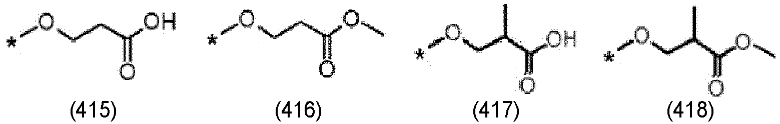

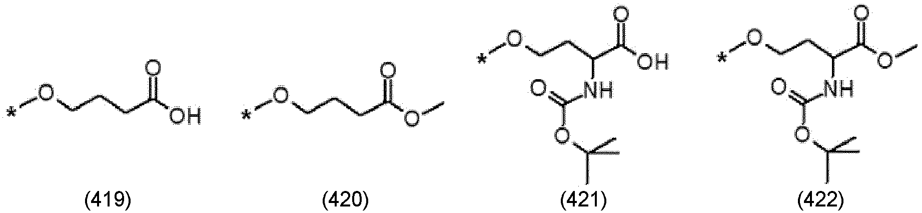

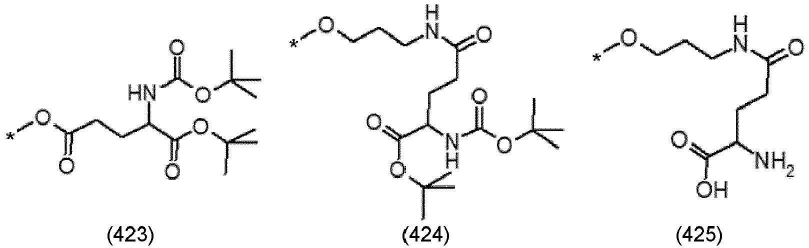

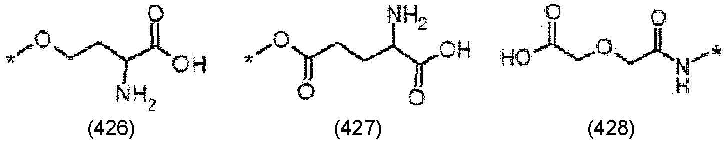

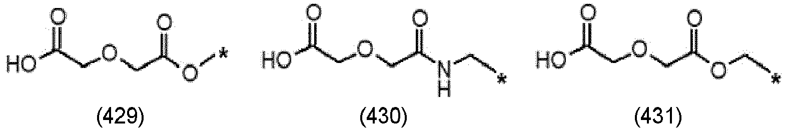

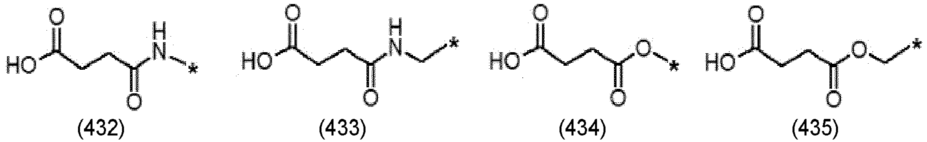

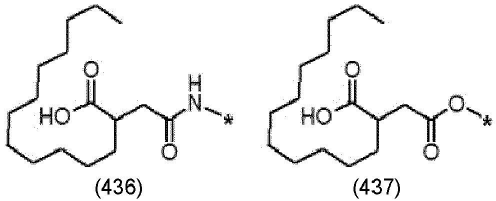

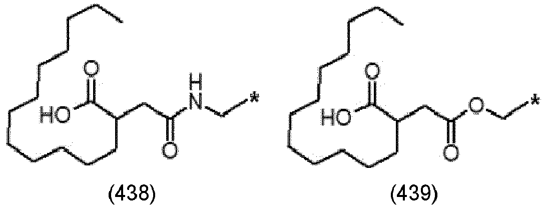

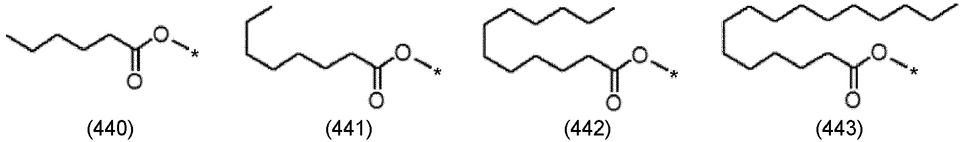

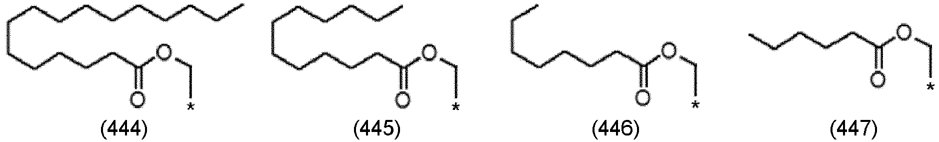

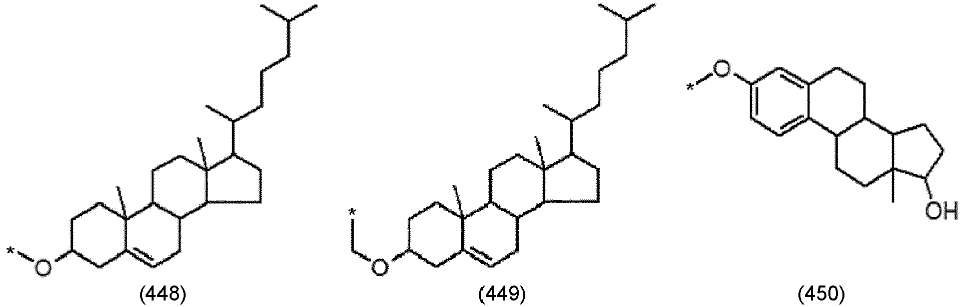

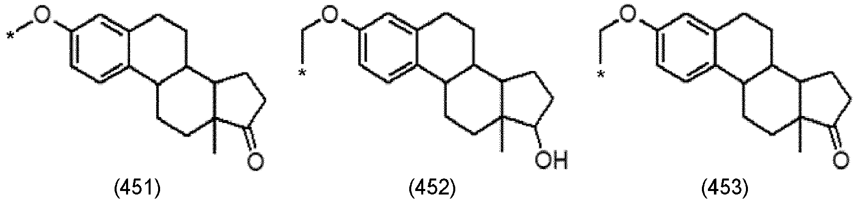

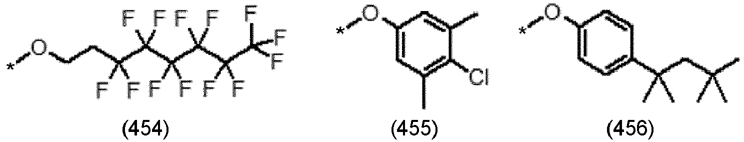

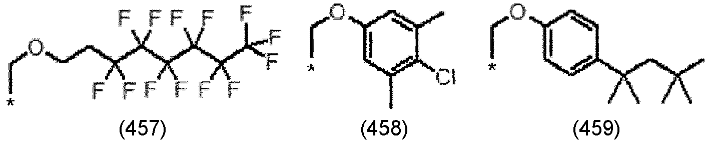

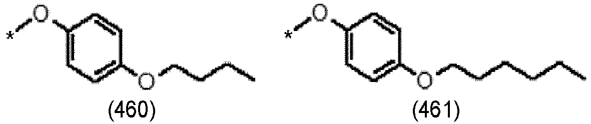

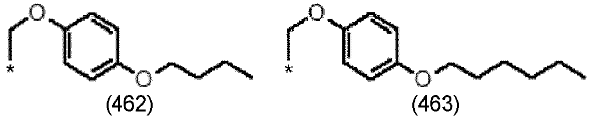

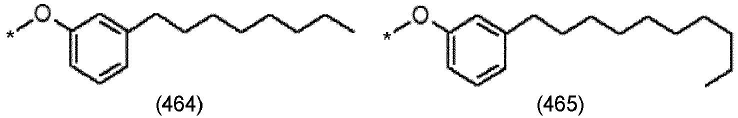

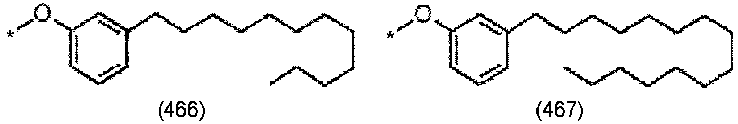

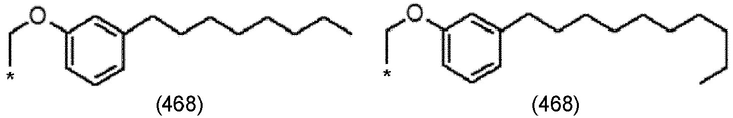

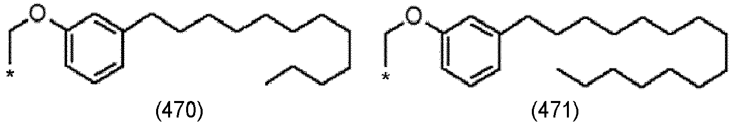

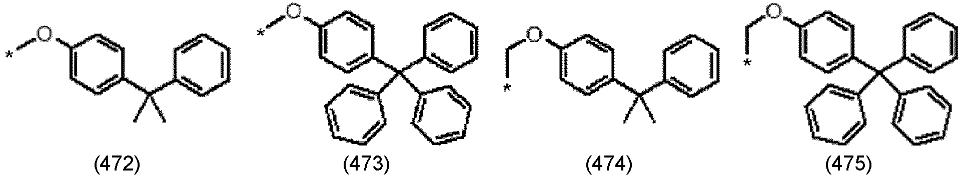

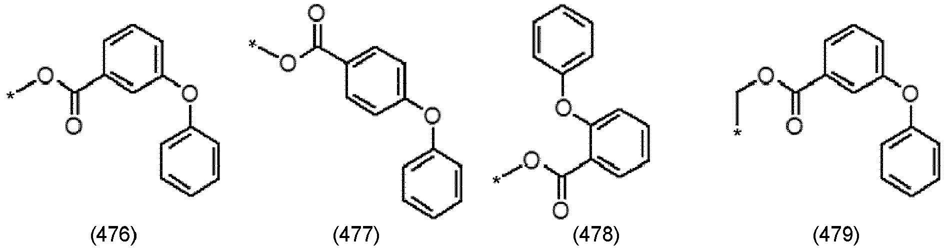

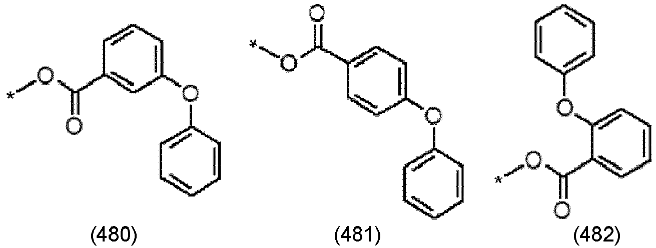

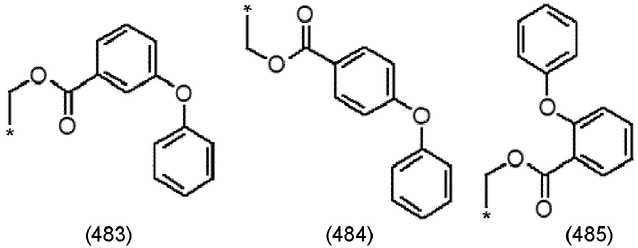

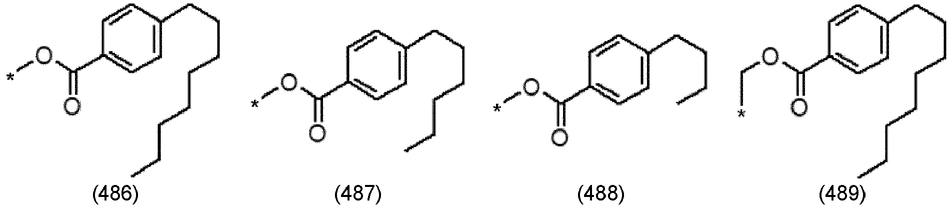

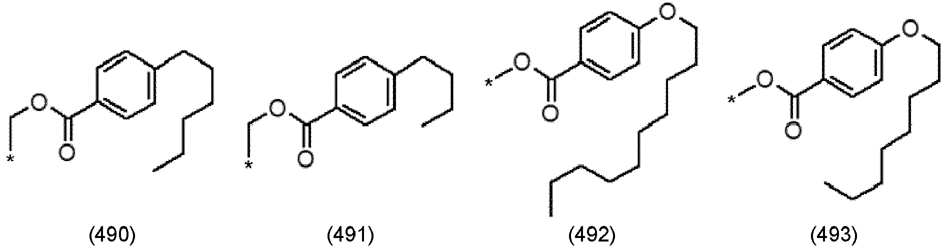

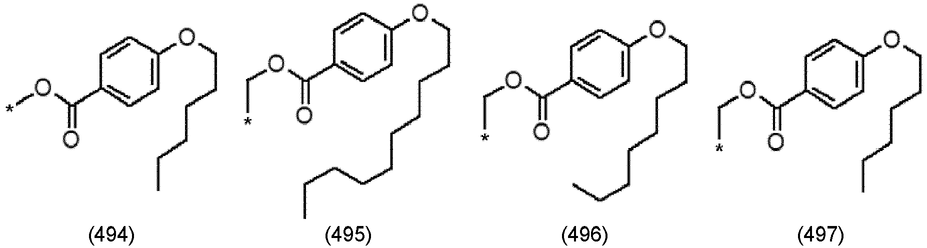

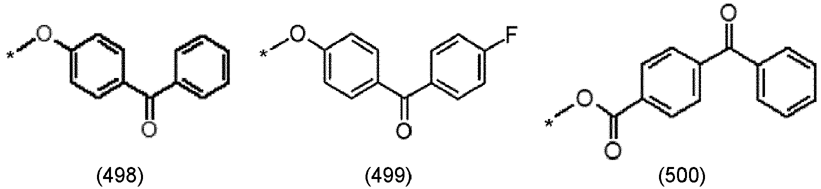

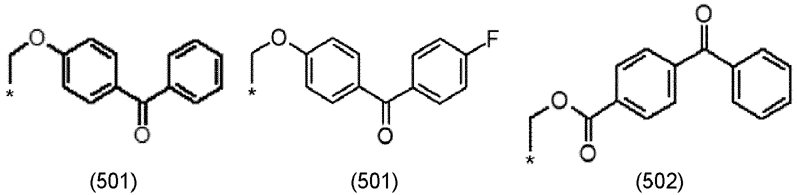

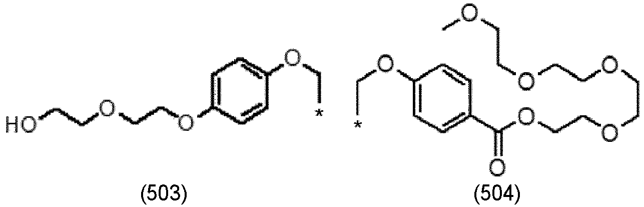

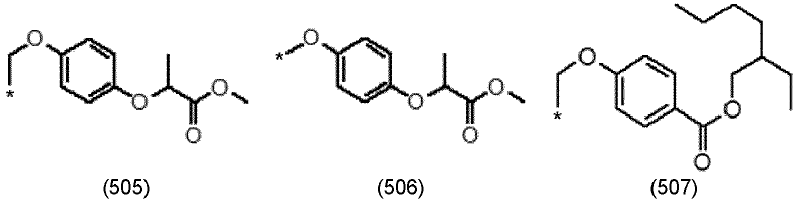

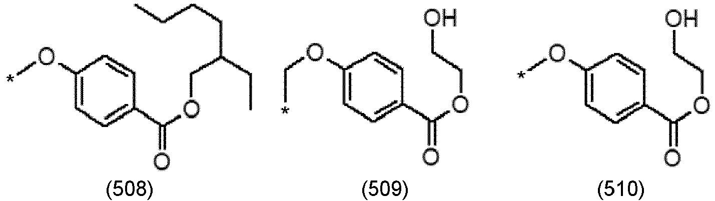

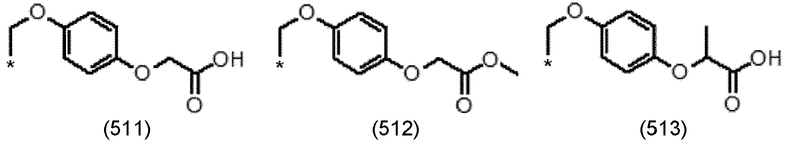

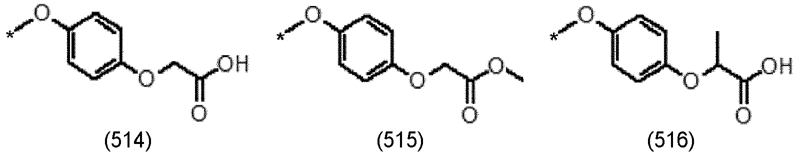

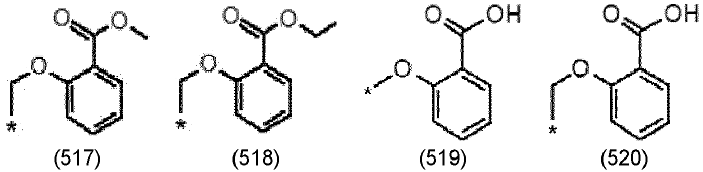

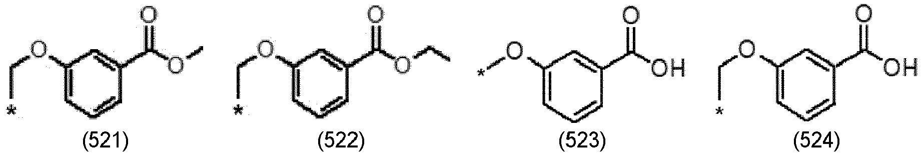

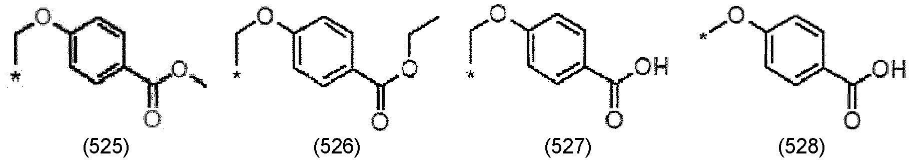

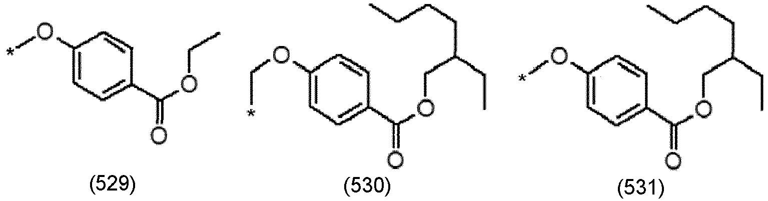

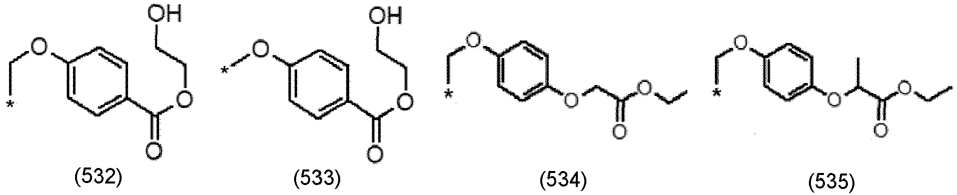

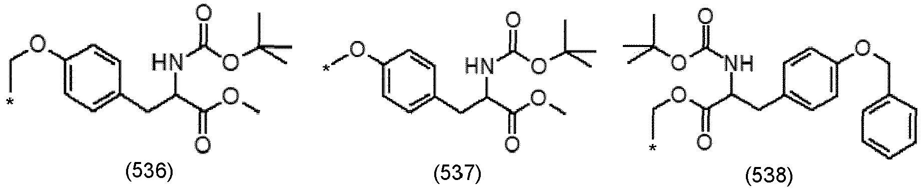

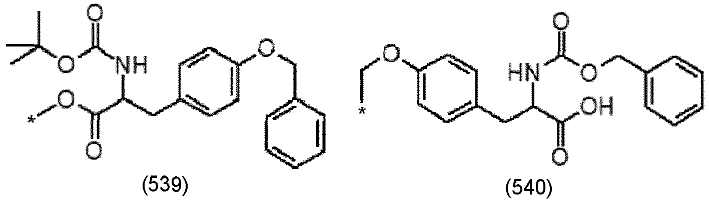

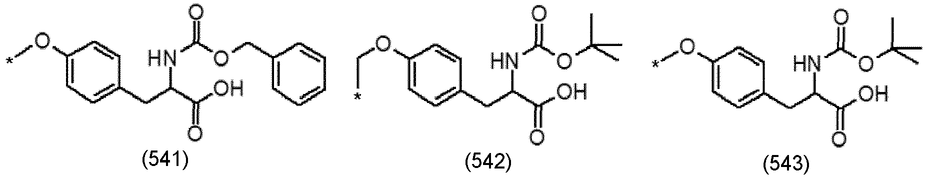

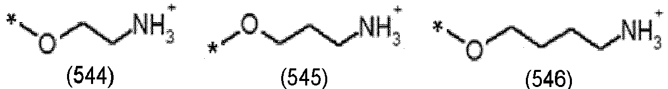

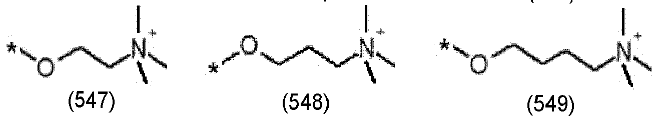

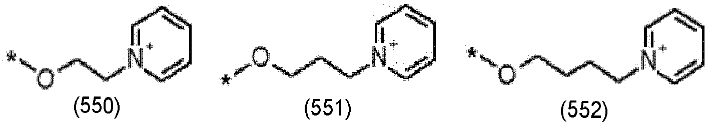

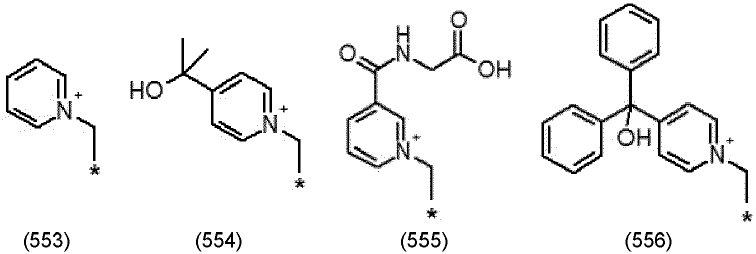

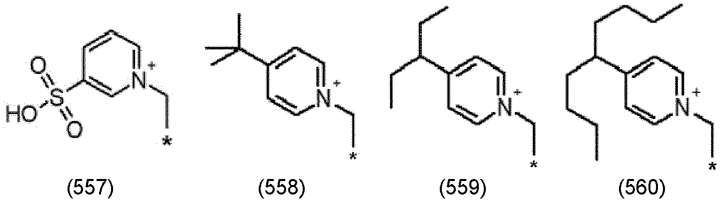

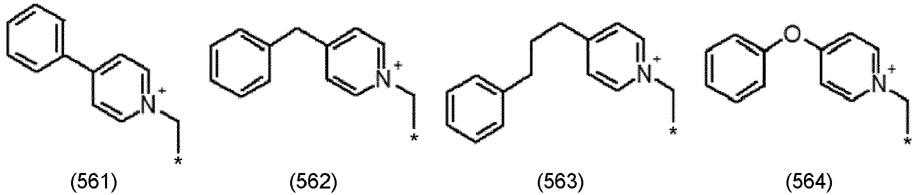

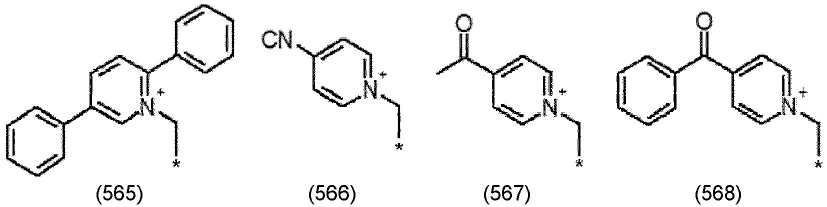

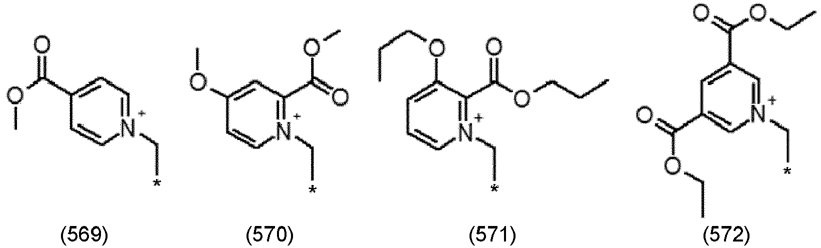

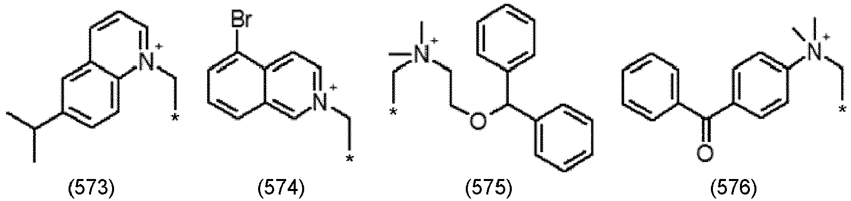

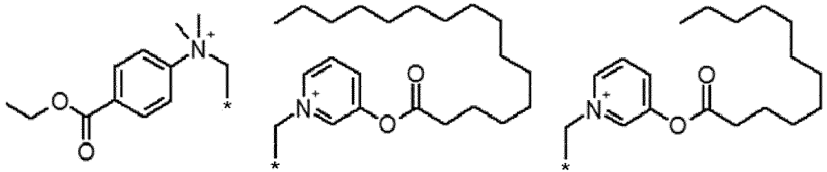

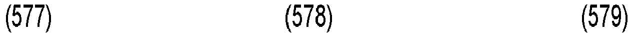

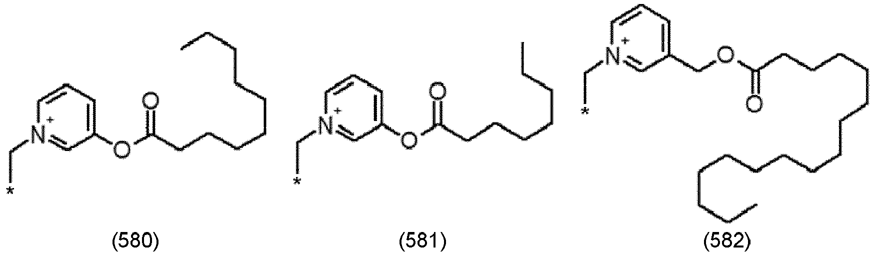

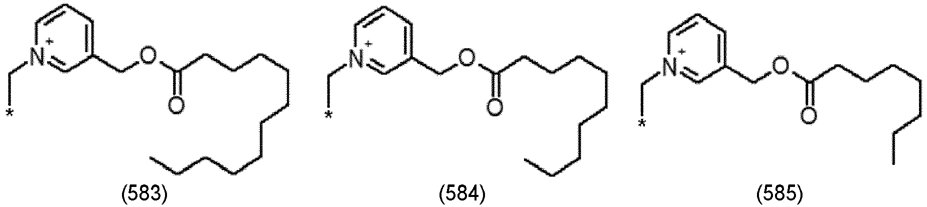

Weiter bevorzugt wird das wenigstens eine erfindungsgemäß zu verwendende Phenalen-1-on-Derivat der allgemeinen Formel (1) ausgewählt aus der Gruppe, die aus den Verbindungen mit der Formel (101a) bis (184) und Mischungen davon, vorzugsweise Verbindungen mit der Formel (101a) bis (164b) und Mischungen davon, weiter bevorzugt Verbindungen mit der Formel (101a), (101b), (102a), (107a) bis (107d), (108), (109), (110a), (110b), (111a), (119) bis (164b) und Mischungen davon, besteht:

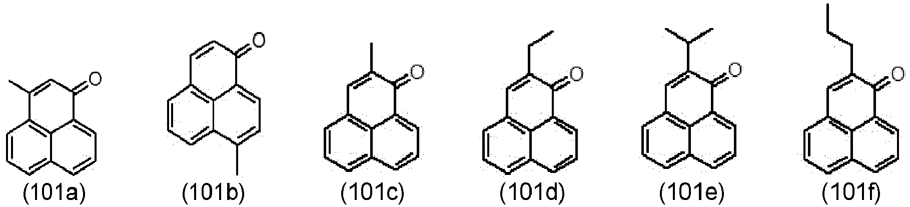

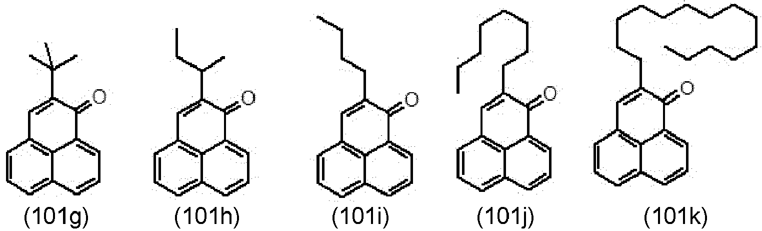

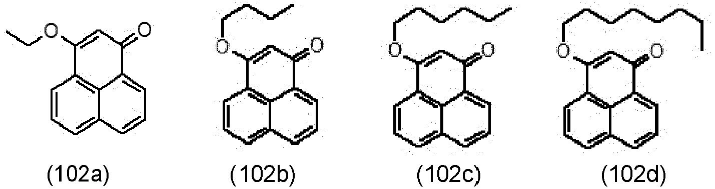

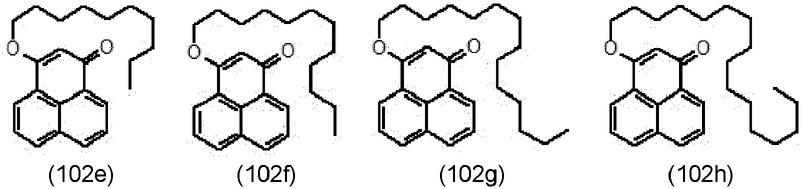

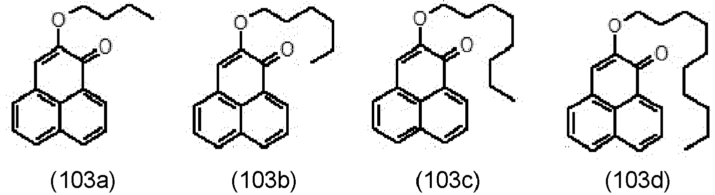

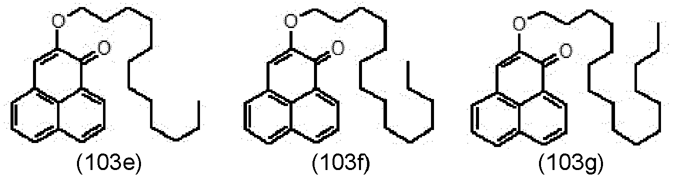

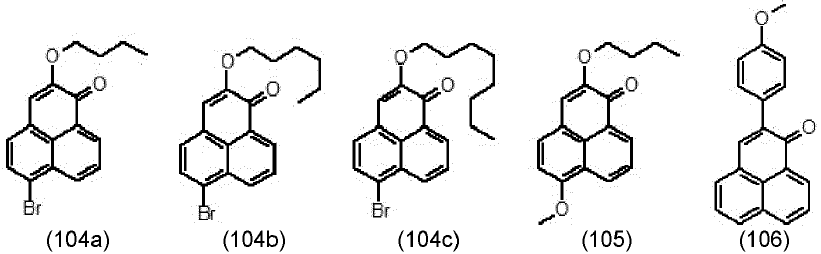

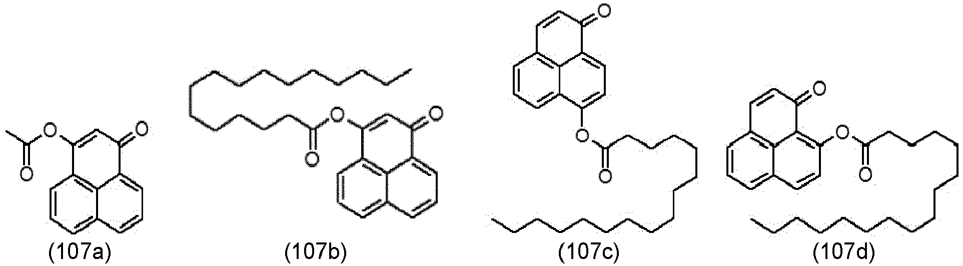

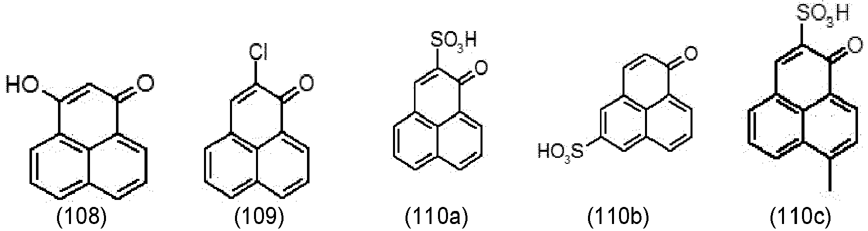

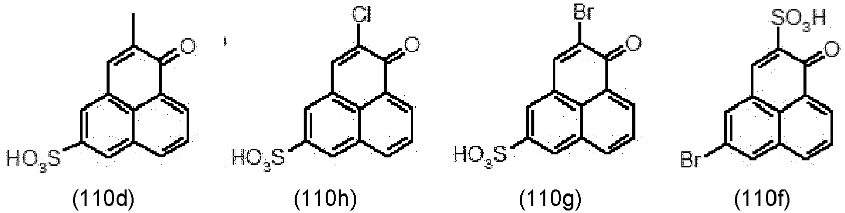

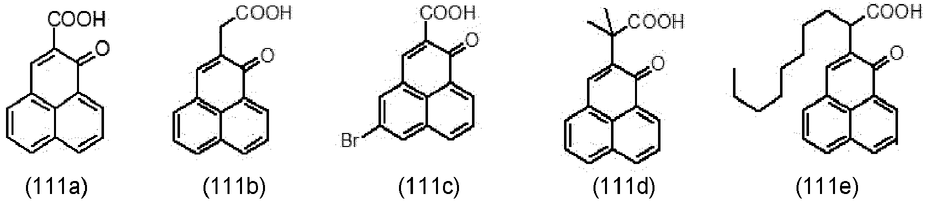

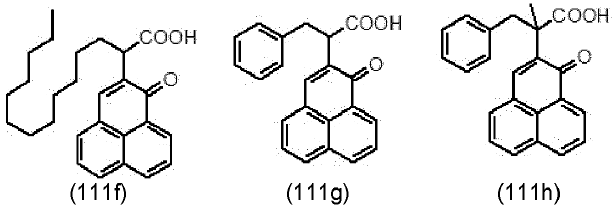

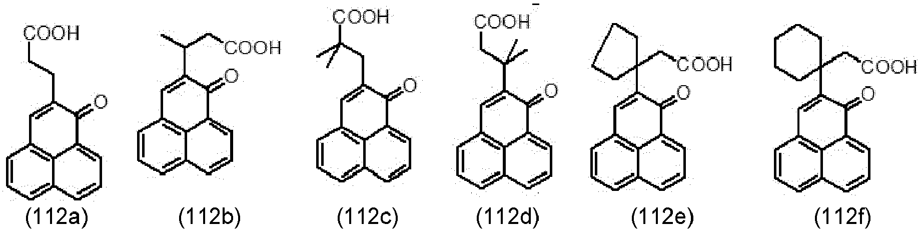

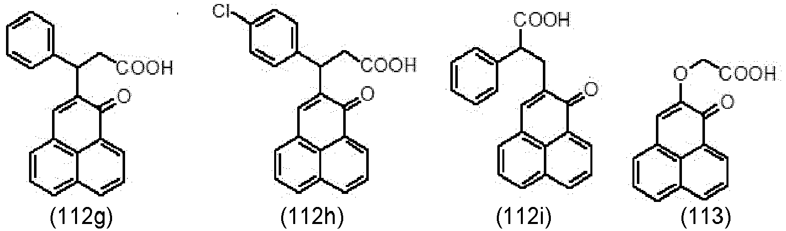

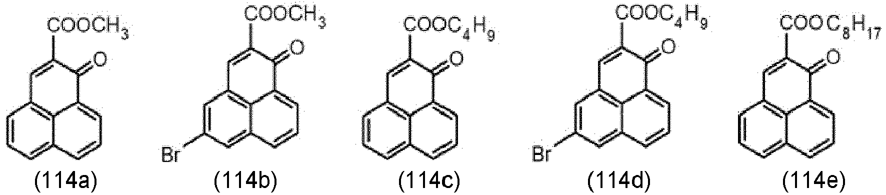

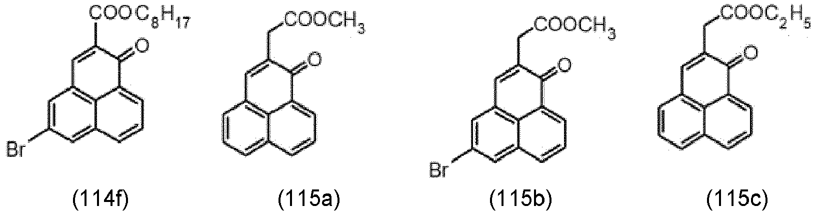

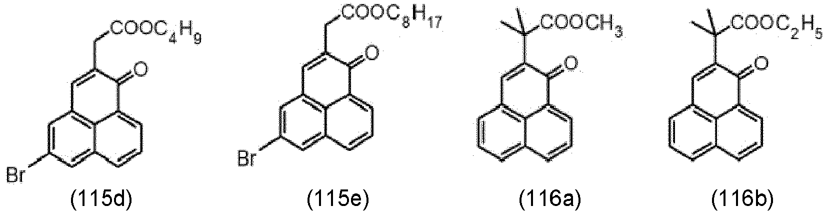

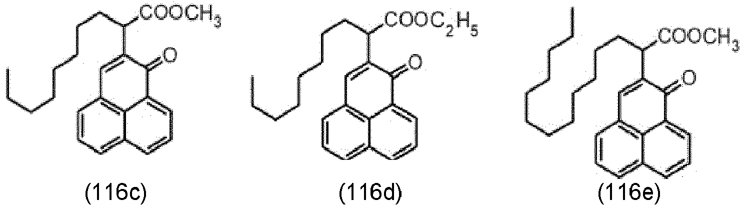

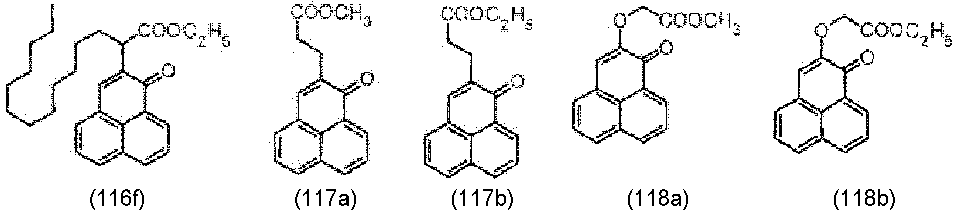

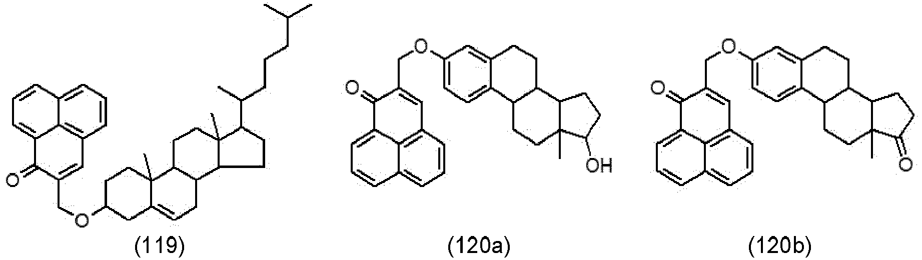

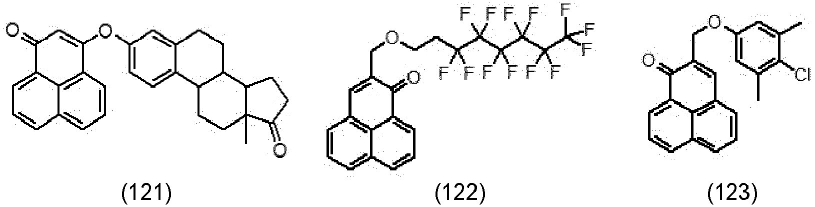

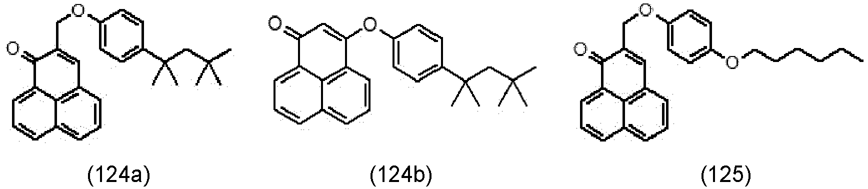

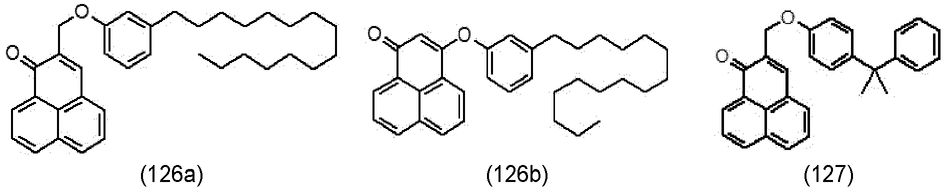

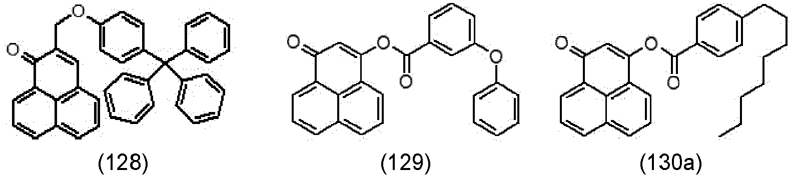

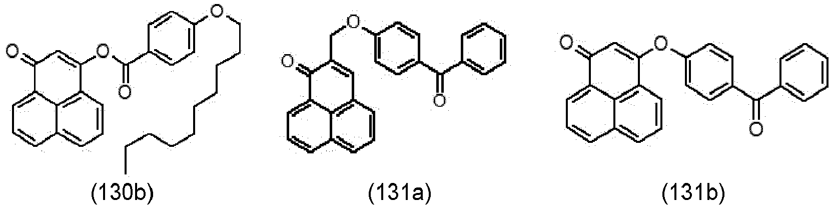

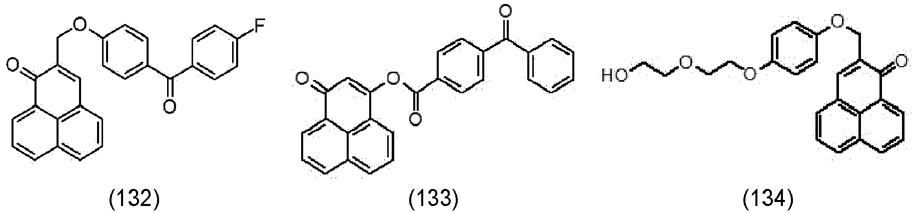

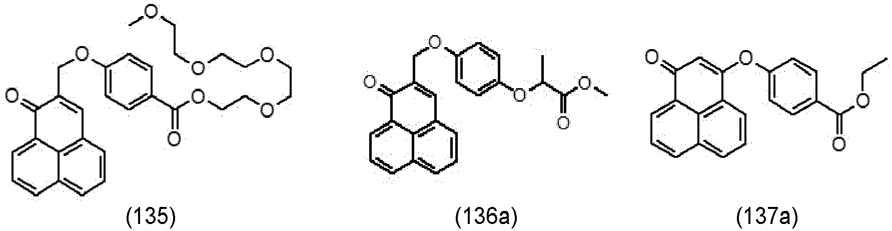

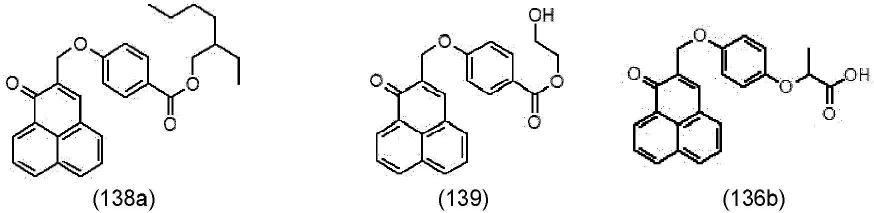

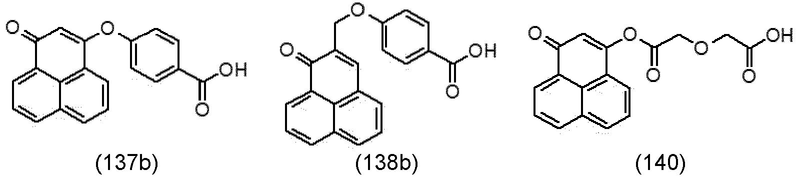

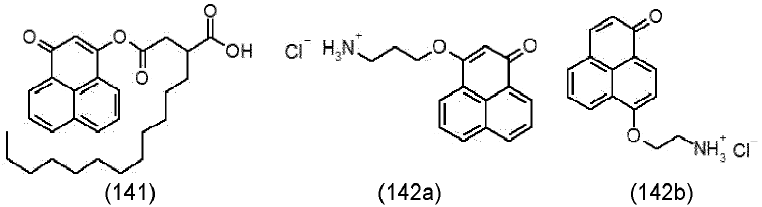

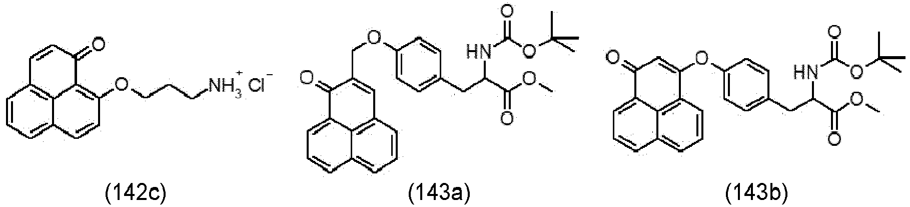

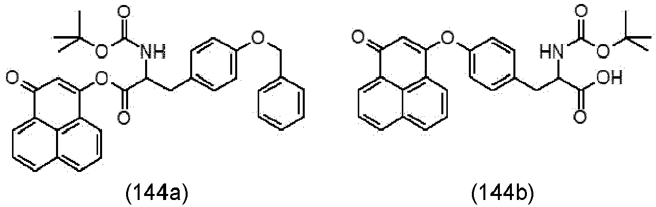

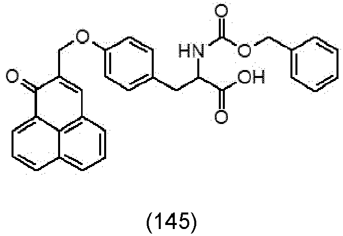

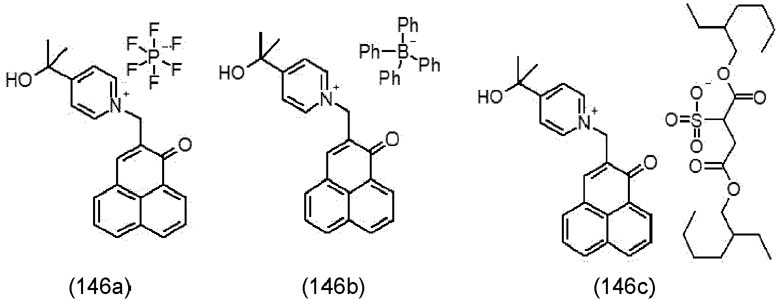

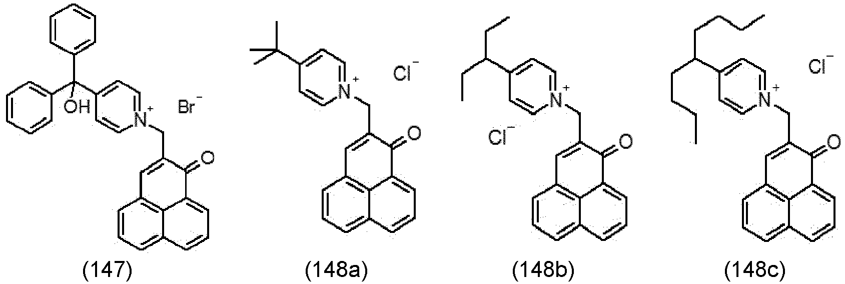

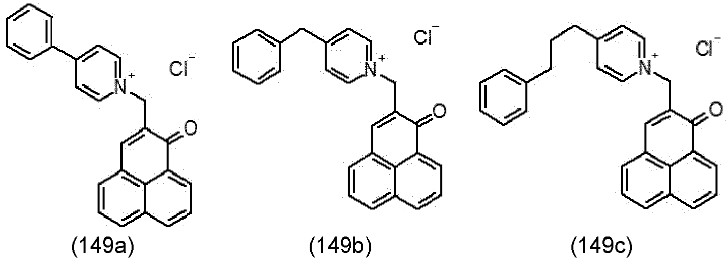

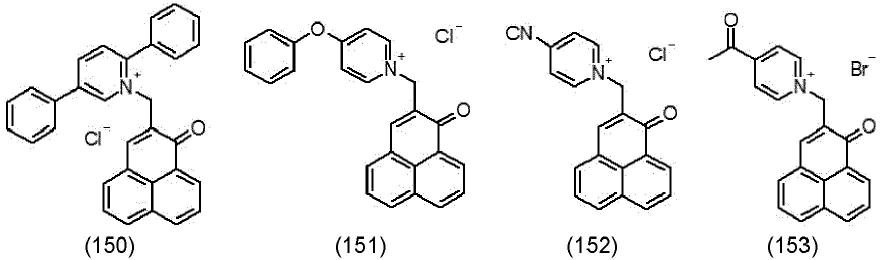

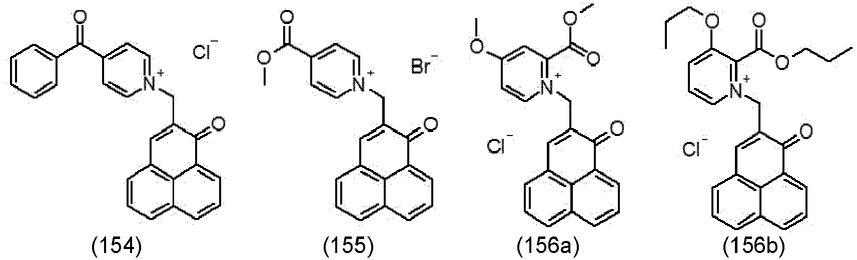

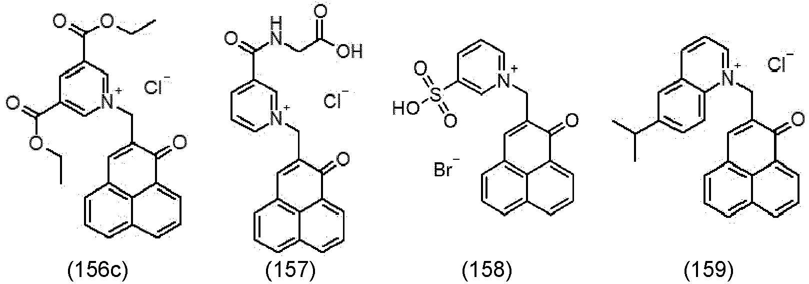

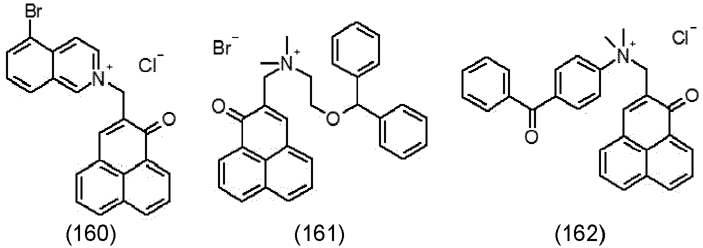

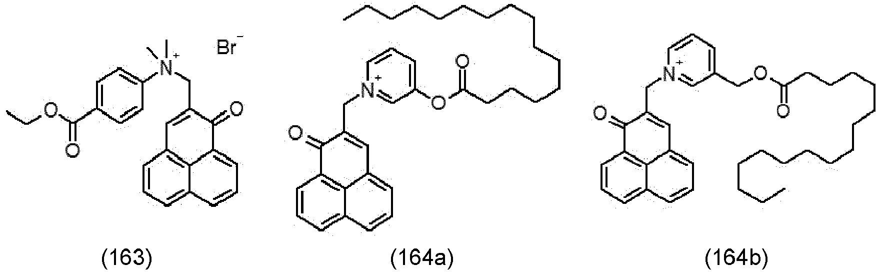

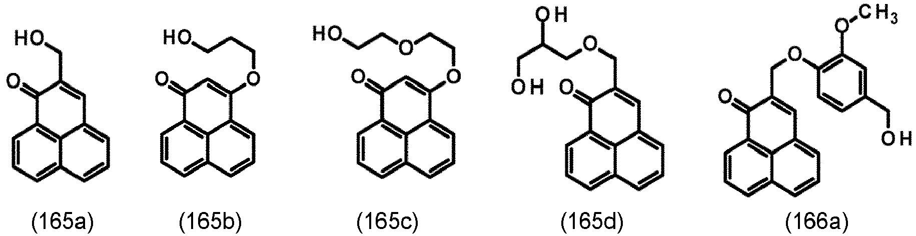

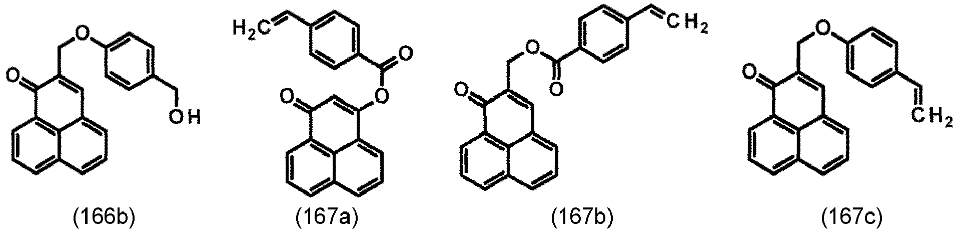

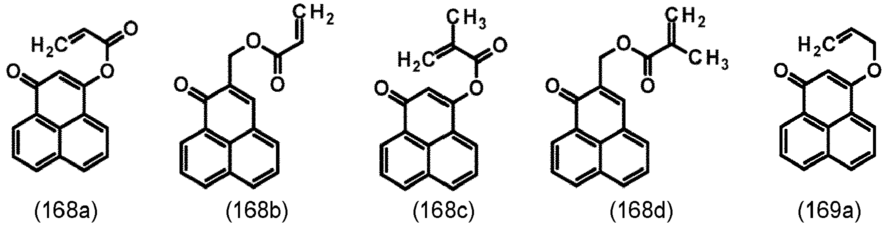

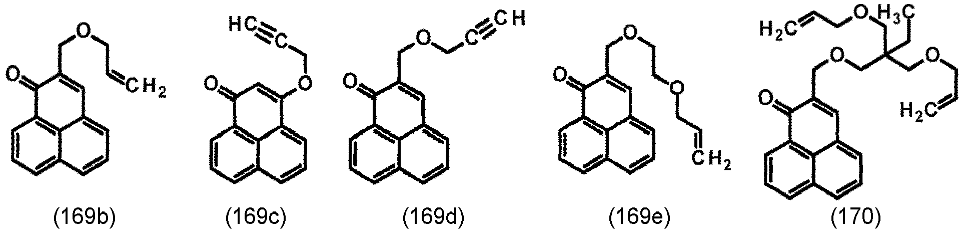

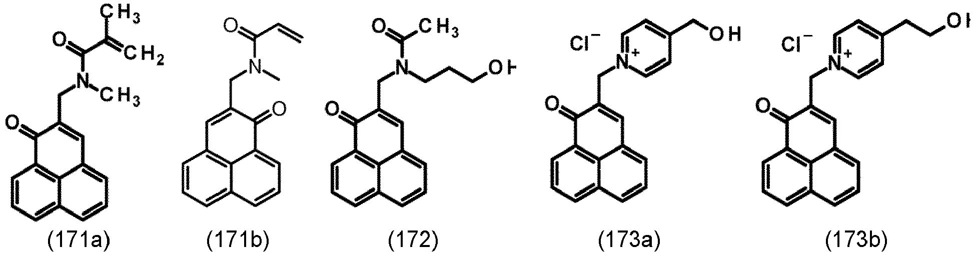

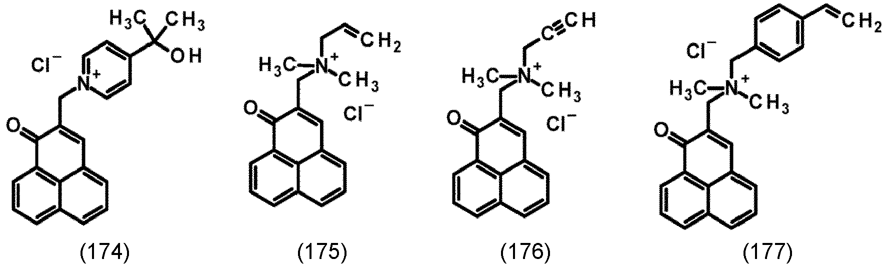

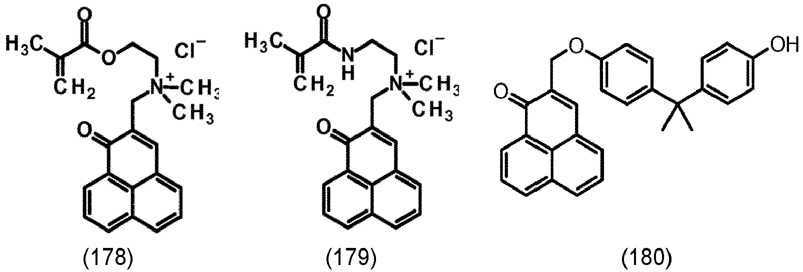

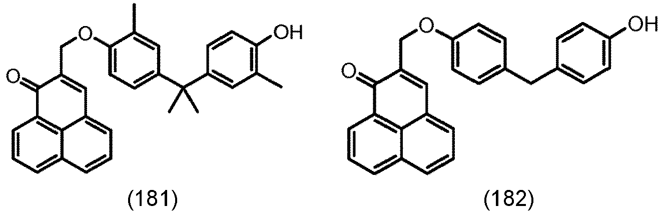

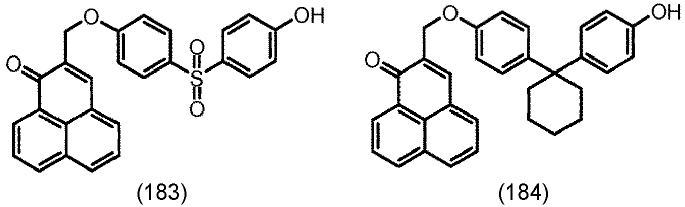

Vorzugsweise ist das wenigstens eine Curcumin-Derivat ein erfindungsgemäß zu verwendetes Curcumin-Derivat der allgemeinen Formel (2a) oder Formel (2b): 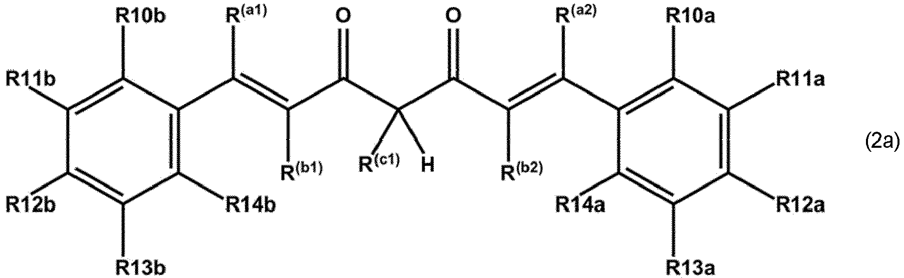 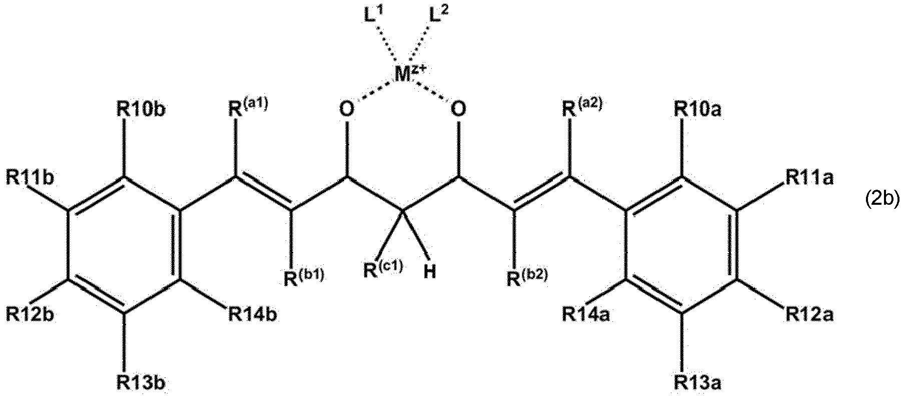
wobei M^{z+} das Kation eines Metalls oder Halbmetalls M bedeutet, das vorzugsweise aus der Gruppe, die aus Bor, Zink, Aluminium und Eisen besteht, ausgewählt ist, wobei z die formale Oxidationszahl des Metalls oder Halbmetalls M ist und wobei z eine ganze Zahl von 1 bis 7, vorzugsweise von 2 bis 3, ist, und wobei L¹ und L² jeweils unabhängig voneinander Wasser, Fluorid, Chlorid, Bromid, lodid, Phosphat, Hydrogenphosphat, Dihydrogenphosphat, Sulfat, Hydrogensulfat, Tosylat, Mesylat oder wenigstens ein Carboxylat-Ion einer Carbonsäure mit 1 bis 15 Kohlenstoffatomen und/oder Mischungen davon bedeuten,
wobei die Reste R^{(a1)}, R^{(a2)}, R^{(b1)} und R^{(b2)}, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, wobei weiter bevorzugt die Reste R^{(a1)} und R^{(a2)} sowie R^{(b1)} und R^{(b2)}, jeweils gleich sind und unabhängig voneinander Wasserstoff, Methyl oder einen unsubstituierten Phenyl-Rest bedeuten,
wobei der Rest R^{(c1)} jeweils unabhängig voneinander Wasserstoff, Halogen, Alkyl, das gradkettig oder verzweigt sein kann, mit 1 bis 4 C-Atomen, vorzugsweise Methyl, Ethyl, n-Propyl oder iso-Propyl, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
und wobei und wobei einer der Reste R^{(b1)} oder R^{(b2)} und der Rest R^{(c1)} jeweils verbrückt sein können und einen Cycloalkylrest mit 4 bis 8 C-Atomen und/oder einen Cycloalkenylrest mit 4 bis 8 C-Atomen bilden können, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können,
und wobei die Reste R10a bis R14b, die jeweils unabhängig voneinander gleich oder voneinander verschieden sein können, jeweils Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, oder einen organischen Rest W1b bedeuten, und
wobei der organische Rest W1b, die jeweils unabhängig voneinander gleich oder voneinander verschieden sein können,:
   b1) ein ungeladener organischer Rest, oder
   b2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder,
   b3) ein organischer Rest mit wenigstens einer Säuregruppe, vorzugsweise 1 oder 2 Säuregruppen, oder
   b4) ein amphoterer organischer Rest, der ein permanent positiv geladenes Stickstoffatom oder ein protonierbares Stickstoffatom, das jeweils nicht direkt am aromatischen Ring angeordnet ist, und eine Säuregruppe umfasst,
vorzugsweise b2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder b3) ein organischer Rest mit wenigstens einer Säuregruppe, vorzugsweise 1 oder 2 Säuregruppen,
bedeutet, mit der Maßgabe, dass wenigstens einer der Reste R10a bis R14a und/oder wenigstens einer der Reste R10b bis R14b, vorzugsweise die Reste R10a und R10b oder die Reste R11a und R11b oder die Reste R12a und R12b oder die Reste R13a und R13b oder die Reste R14a und R14b, ein organischer Rest W1b bedeutet, und wobei optional wenigstens einer der Reste R10a bis R14a und wenigstens einer der Reste R10b bis R14b, vorzugsweise mindestens 2 oder 4 der Reste R10a und R10b oder R11a und R11b oder R12a und R12b oder R13a und R13b oder R14a und R14b, die nicht ein Rest W1b bedeuten, weiterhin Chlor, Brom oder lod bedeutet,
wobei der Rest W1b in Variante b1) ein Rest mit der allgemeinen Formel (10a) bis (16b) bedeutet:

   * - (C(D)(E))ₕ - T (10a)

   * - Q - (C(D)(E))ₕ - T (10b)

   * - [(C(D)(E))_{b} - A]ₐ - (C(D)(E))_{g} - T (11a)

   * - Q - [(C(D)(E))_{b} - A]ₐ - (C(D)(E))_{g} - T (11b)

   * - [(C(D)(E))_{b} - A]ₐ - (C(D)(E))_{y} - Ar - (C(D)(E))_{g} - T (12a)

   * - Q - [(C(D)(E))_{b} - A]ₐ - (C(D)(E))_{y} - Aᵣ - (C(D)(E))_{g} - T (12b)

   * - (C(D)(E))ₕ - Ar - (C(D)(E))_{g} - T (13a)

   * - Q - (C(D)(E))ₕ - Ar - (C(D)(E))_{g} - T (13b)

   * - (C(D)(E))_{y} - Ar - B - (C(D)(E))_{g} - T (14a)

   * - Q - (C(D)(E))_{y} - Ar - B - (C(D)(E))_{g} - T (14b)

   * - (C(D)(E))_{y} - Ar - B - [(C(D)(E))_{b} - A]_{c} - (C(D)(E))_{y} - T (15a)

   * - Q - (C(D)(E))_{y} - Aᵣ - B - [(C(D)(E))_{b} - A]_{c} - (C(D)(E))_{y} - T (15b)

   * - [(C(D)(E))_{b} - A]ₐ - (C(D)(E)_{y} - Ar - B - [(C(D)(E))_{b} - A]_{c} - (C(D)(E))_{y} - T (16a)

   * - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel *-OS(=O)₂-*, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet,
und wobei zwei oder mehr der Reste D und/oder E jeweils verbrückt sein können und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkanonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyklischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeuten, und
wobei der Index a jeweils unabhängig voneinander eine ganze Zahl von 1 bis 10, vorzugsweise 1 bis 6, vorzugsweise 2 bis 4, weiter bevorzugt 1 oder 2, bedeuten, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet und
wobei der Rest W1b in Variante b2) ein Rest mit der allgemeinen Formel (21a) bis (24b) bedeutet:

   * - [(C(D)(E))_{b} - A]_{c} - (C(D)(E))_{g} - X (21a)

   * - Q - [(C(D)(E))_{b} - A]_{c} - (C(D)(E))_{g} - X (21b)

   * - [(C(D)(E))_{b} - A]ₐ - (C(D)(E))_{y} - Ar - (C(D)(E))_{f} - X (22a)

   * - Q - [(C(D)(E))_{b} - A]ₐ - (C(D)(E))_{y} - Aᵣ - (C(D)(E))_{f} - X (22b)

   *-(C(D)(E))_{d}-Ar-(C(D)(E))_{f}-X (23a)

   *-Q-(C(D)(E))_{d}-Ar-(C(D)(E))_{f}-X (23b)

   *-(C(D)(E))_{d}-Ar-[A-(C(D)(E))_{b}]ₐ-X (24a)

   *-Q-(C(D)(E))_{d}-Ar-[A-(C(D)(E))_{b}]ₐ-X (24b)
wobei die Reste Q und A jeweils unabhängig voneinander Sauerstoff, , ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxylalkyl, das gradkettig oder verzweigt sein kann, mit 1 bis 5 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert sein können, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeuten, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices d, und f jeweils unabhängig voneinander eine ganze Zahl von 1 bis 10, vorzugsweise 1 bis 8, vorzugsweise von 2 bis 4, bedeuten, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar einen unsubstituierten Phenylen-Rest oder einen substituierten Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert ist, bedeutet,
wobei der Rest X jeweils unabhängig voneinander ein Rest der Formel (15a), (15b), (16), (17a), (17b), (17c), oder (17d), vorzugsweise ein Rest der Formel (15a), (15b), (17a) oder (17b), bedeutet: 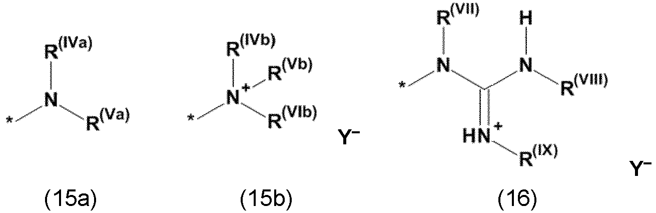 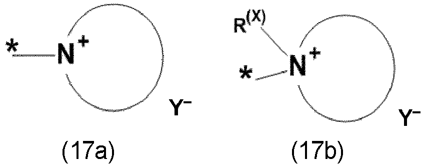 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)} und R^{(X)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (17a): 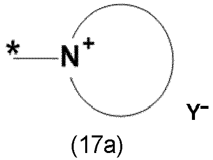 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, bedeutet, wobei der heterocyclische Rest mit 5 bis 10 Ringatomen unsubstituiert oder mit einem oder mehrerer Reste substituiert ist, die aus der Gruppe, die aus Hydroxyl, Chlor, Brom, Fluor, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden, und
wobei der Rest mit der Formel (17b): 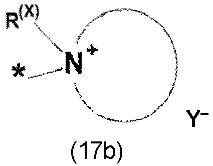 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung mit einem Ringatom, vorzugsweise Kohlenstoffatom, ausbildet, bedeuten, wobei der heterocyclische Rest mit 5 bis 7 Ringatomen, unsubstituiert oder mit einem oder mehrerer Reste substituiert ist, die aus der Gruppe, die aus der Gruppe, die aus Hydroxyl, Chlor, Brom, Fluor, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, und Mischungen davon besteht, ausgewählt ist, und
wobei der Rest W1b in Variante b3) ein Rest mit der allgemeinen Formel (30) bis (24b) bedeutet:

   - Z (30)

   * - [(C(D)(E))_{b} - A]_{c} - (C(D)(E))_{g} - Z (31a)

   * - Q - [(C(D)(E))_{b} - A]_{c} - (C(D)(E))_{g} - Z (31b)

   * - [(C(D)(E))_{b} - A]ₐ - (C(D)(E))_{y} - Ar - (C(D)(E))ₑ - Z (32a)

   * - Q - [(C(D)(E))_{b} - A]ₐ - (C(D)(E))_{y} - Ar - (C(D)(E))ₑ - Z (32b)

   * - (C(D)(E))_{d} - Ar - (C(D)(E))ₑ - Z (33a)

   * - Q - (C(D)(E))_{d} - Ar - (C(D)(E))ₑ - Z (33b)

   *-(C(D)(E))_{d}-Ar-[A-(C(D)(E))_{b}]ₐ-Z (34a)

   *-Q-(C(D)(E))_{d}-Ar-[A-(C(D)(E))_{b}]ₐ-Z (34b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise von 1 bis 6, vorzugsweise 2 bis 4, weiter bevorzugt 1 oder 2, bedeutet, und
wobei der Index b jeweils unabhängig voneinander eine ganze Zahl von 1 bis 4, vorzugsweise von 2 bis 3, weiter bevorzugt 1, bedeutet, und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index d jeweils unabhängig voneinander eine ganze Zahl von 1 bis 10, vorzugsweise 2 bis 4, bedeutet, und
wobei der Index e jeweils unabhängig voneinander eine ganze Zahl von 0 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Rest W1b in Variante b4) ein Rest mit der allgemeinen Formel (41a) oder (41b) bedeutet:

   * - (CH₂)_{g} - Het - (CH₂)ᵢ - Z (41a)

   * - (CH₂)_{g} -Het - [B - (CH₂)ₖ]ₗ - Z (41b)
wobei der Rest B jeweils unabhängig voneinander Sauerstoff, ein Rest der Formel * - C(=O) - *, ein Rest der Formel * - C(=O)O - *, ein Rest der Formel * - OC(=O) - *, oder ein Rest der Formel * - C(=O)N(R⁽⁶⁾) - * bedeutet, wobei der Rest R⁽⁶⁾ jeweils unabhängig voneinander Wasserstoff oder Alkyl, das gradkettig oder verzweigt sein kann, mit 1 bis 4 Kohlenstoffatomen bedeutet,
wobei der Rest Het jeweils einen heterocyclischen Rest mit 5 bis 10 Ringatomen, die mindestens 1 Kohlenstoffatom und mindestens 1 Stickstoffatom sowie optional 1 oder 2 Sauerstoffatome umfassen, bedeutet, der vorzugsweise ein Rest mit der Formel (17a) oder ein Rest mit der Formel (17c) ist: 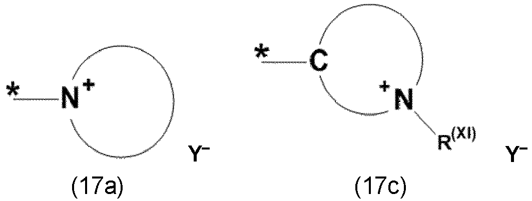
wobei der heterocyclisch Rest 5 bis 10 Ringatome aufweist, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, wobei der Rest R^{(XI)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist, und
wobei der Index g jeweils unabhängig voneinander eine ganze Zahl von 1 bis 35, vorzugsweise von 1 bis 30, bedeutet, und
wobei die Indices i, und k jeweils unabhängig voneinander eine ganze Zahl von 1 bis 4, bedeuten, und
wobei die Index I jeweils unabhängig voneinander eine ganze Zahl von 0 bis 4, vorzugsweise 1 bis 3, bedeutet, und
wobei der Rest Z jeweils unabhängig voneinander ein Säure-Rest ist, der aus der Gruppe, die aus Carboxygruppe und Sulfonsäuregruppe besteht, ausgewählt wird.

Vorzugsweise bedeuten die Reste R10a und R10b oder die Reste R11a und R11b oder die Reste R12a und R12b oder die Reste R13a und R13b oder die Reste R14a und R14b, jeweils einen organischen Rest W1b, wobei die Reste R10a und R10b oder die Reste R11a und R11b oder die Reste R12a und R12b oder die Reste R13a und R13b oder die Reste R14a und R14b, die nicht ein organischer Rest W1b bedeuten, jeweils unabhängig voneinander Halogen, das ausgewählt ist aus Chlor, Brom und lod, bevorzugt Brom, Wasserstoff, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, bedeuten, wobei mindestens 2 und höchstens 4 der Reste R10a und R10b oder R11a und R11b oder R12a und R12b oder R13a und R13b oder R14a und R14b, die nicht eine organischen Rest W1b bedeuten, Chlor, Brom oder lod, bevorzugt Brom, bedeuten.

Weiter bevorzugt bedeutet wenigstens einer der Reste R10a bis R14a und/oder wenigstens einer der Reste R10b bis R14b, vorzugsweise die Reste R10a und R10b oder die Reste R11a und R11b oder die Reste R12a und R12b oder die Reste R13a und R13b oder die Reste R14a und R14b, jeweils einen organischen Rest W1b, wobei der organische Rest W1b ein Rest mit der Formel (11a), (11b), (21a), (21b), (22a), (22b), (31a), (31b), (32a), oder (32b) ist und wobei der Index a jeweils unabhängig voneinander eine ganze Zahl von 0 bis 2, vorzugsweise 1 oder 2, bedeutet und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet und wobei der Index b jeweils unabhängig voneinander eine ganze Zahl von 1 bis 3, vorzugsweise 2 bis 3, bedeutet und wobei der Index y jeweils unabhängig voneinander eine ganze Zahl von 0 bis 2, vorzugsweise 0 bis 1, bedeutet und wobei der Index c jeweils unabhängig voneinander eine ganze Zahl von 0 bis 2, vorzugsweise 1 oder 2, bedeutet, wobei optional wenigstens einer der Reste R10a bis R14a und wenigstens einer der Reste R10b bis R14b, vorzugsweise mindestens 2 oder höchstens 4 der Reste R10a und R10b oder R11a und R11b oder R12a und R12b oder R13a und R13b oder R14a und R14b, die nicht ein Rest W1b bedeuten, weiterhin Chlor, Brom oder lod, vorzugsweise Brom, bedeuten.

Weiter bevorzugt ist das wenigstens eine Curcumin-Derivat ein erfindungsgemäß zu verwendendes Curcumin-Derivat der allgemeinen Formel (2a) oder Formel (2b), wobei wenigstens einer der Reste R10a bis R14a und/oder wenigstens einer der Reste R10b bis R14b, vorzugsweise die Reste R10a und R10b oder die Reste R11a und R11b oder die Reste R12a und R12b oder die Reste R13a und R13b oder die Reste R14a und R14b, ein organischer Rest W1b bedeutet, der ausgewählt ist aus der Gruppe, die aus den Resten mit der Formel (400) bis (585), vorzugsweise den Resten mit der Formel (403) bis (429), (432), (434), (436), (437), (440) bis(443), (448), (450), (451), (454) bis (456), (460), (461), (464) bis (467), (472), (473), (476) bis (478), (480) bis (482), (486) bis (488), (492) bis (494), (498) bis (500), (506), (508), (510), (514) bis (516), (519), (523), (528), (529), (531), (533), (537), (539), (541), (543), und (544) bis (552), besteht.

Weiter bevorzugt wird das wenigstens eine erfindungsgemäß zu verwendende Curcumin-Derivat der allgemeinen Formel (2a) oder Formel (2b) ausgewählt aus der Gruppe, die aus den Verbindungen mit der Formel (200) bis (296) und Mischungen davon, vorzugsweise Verbindungen mit der Formel (200) bis (218) und Mischungen davon, besteht:

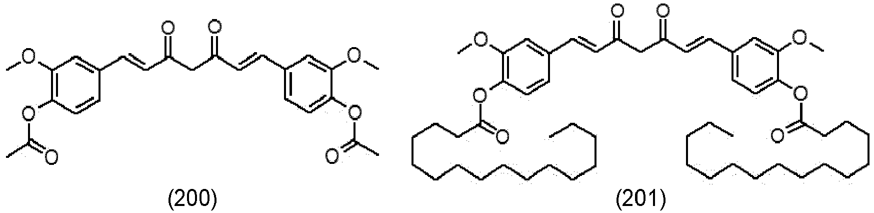

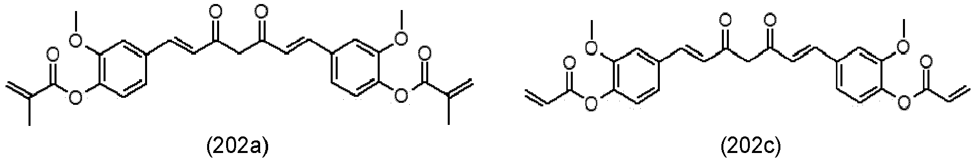

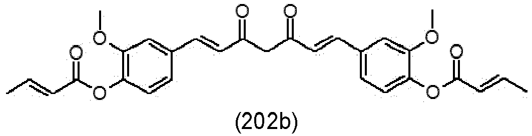

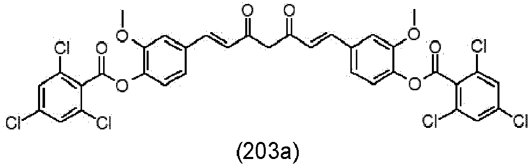

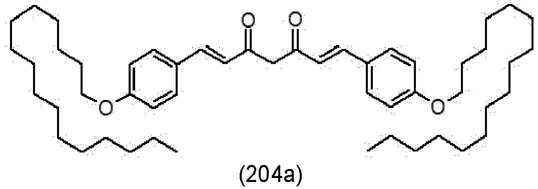

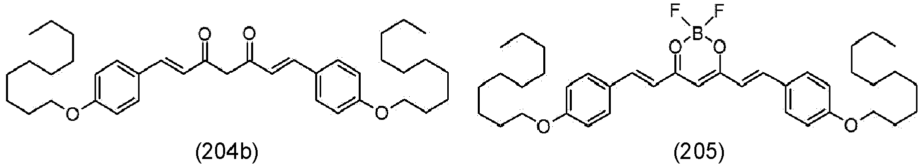

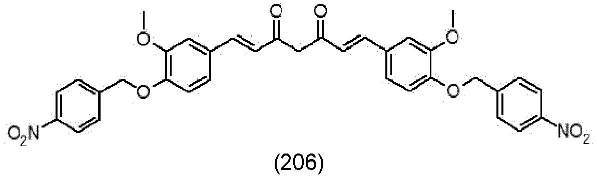

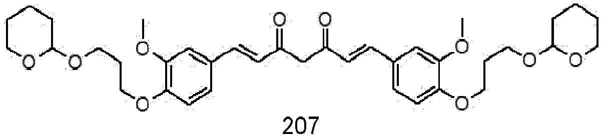

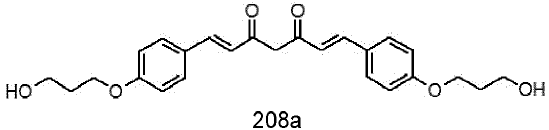

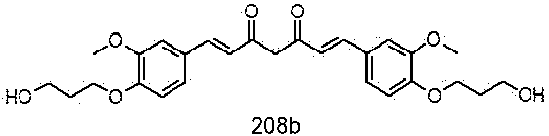

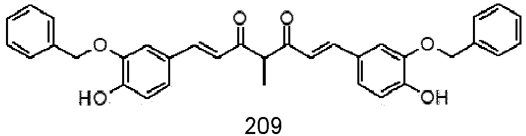

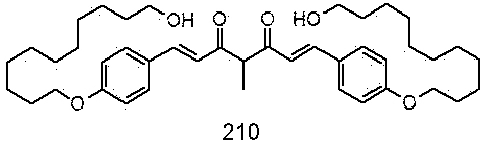

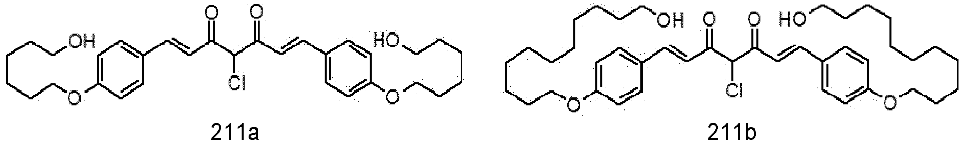

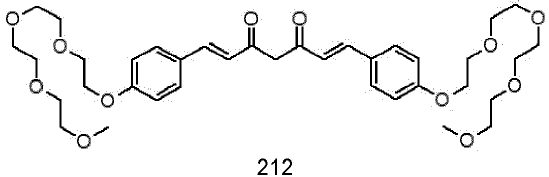

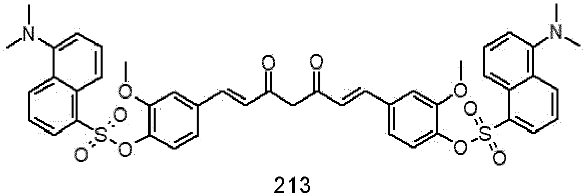

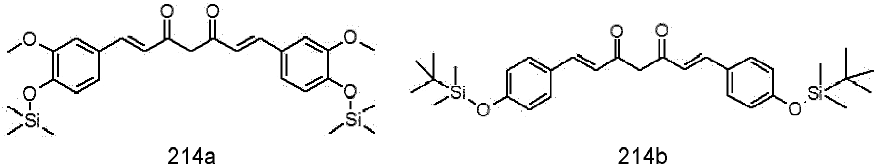

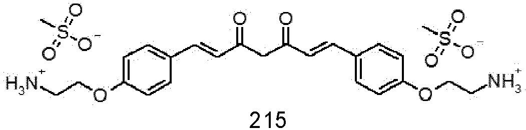

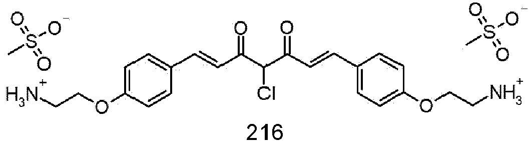

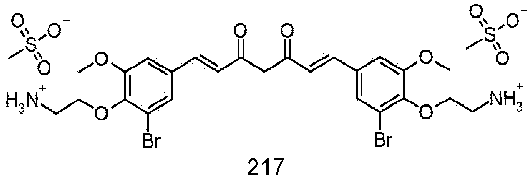

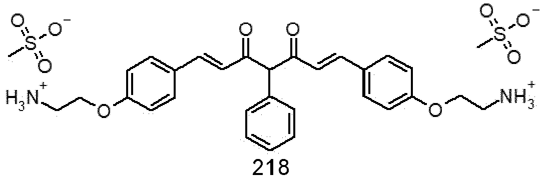

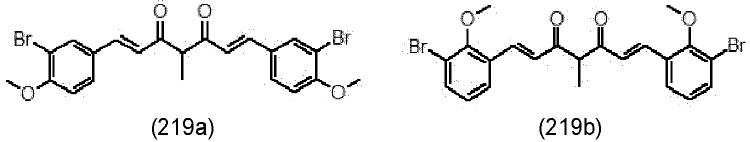

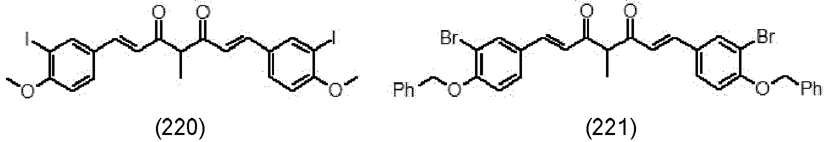

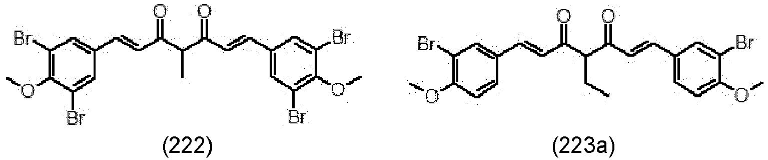

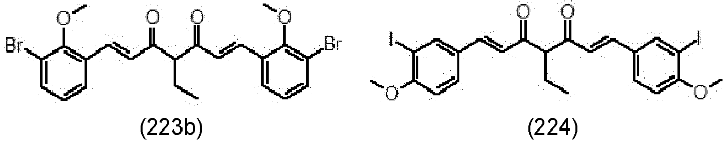

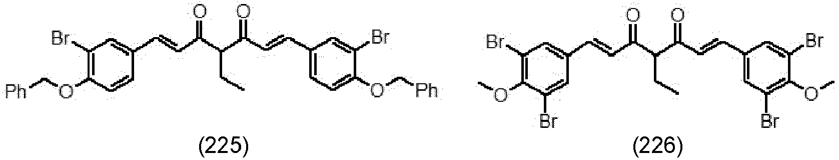

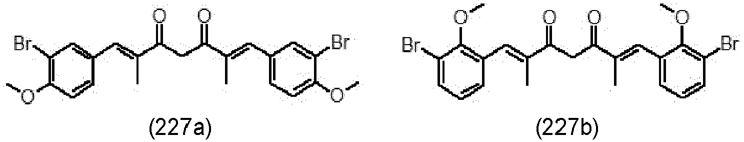

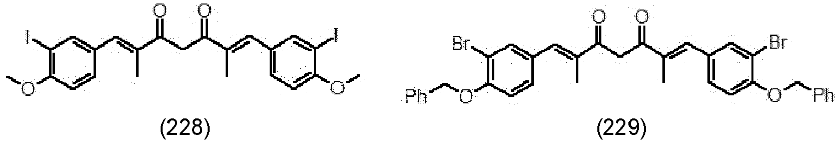

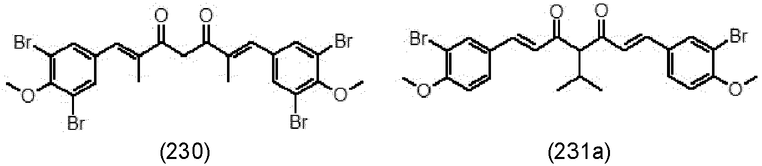

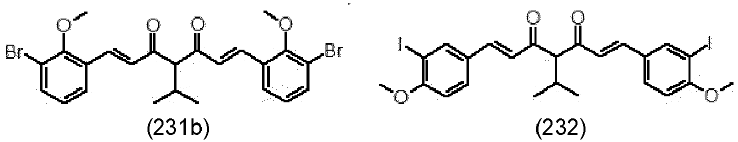

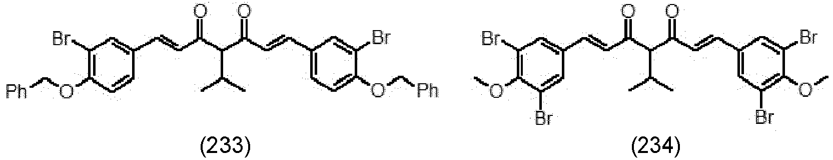

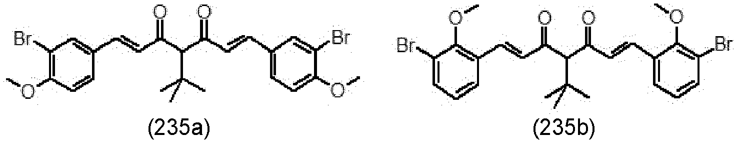

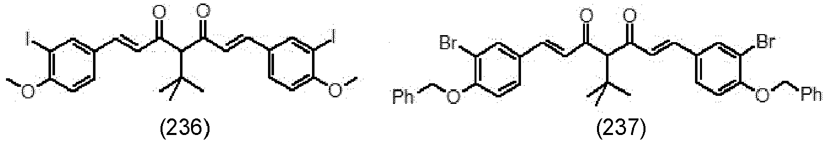

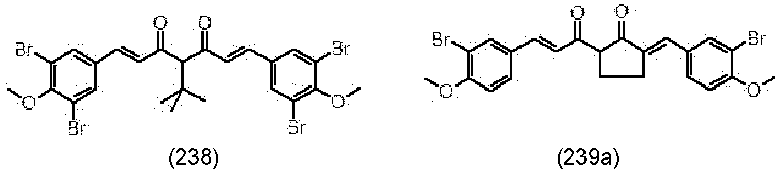

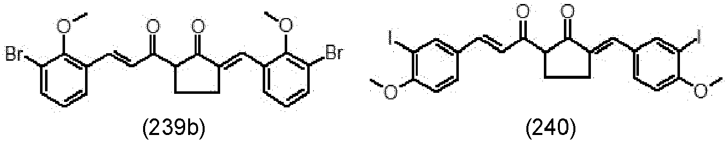

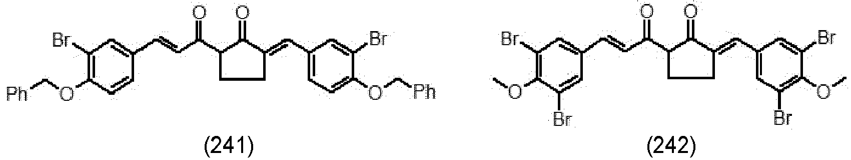

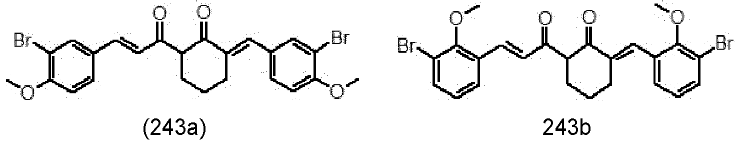

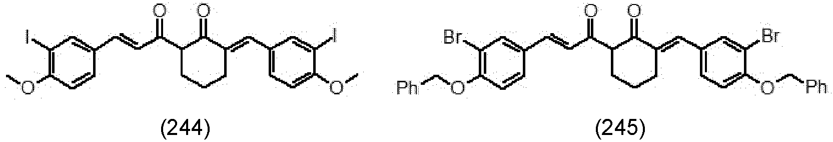

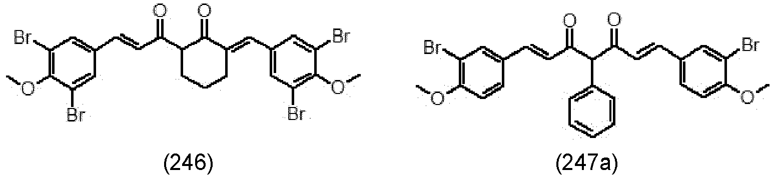

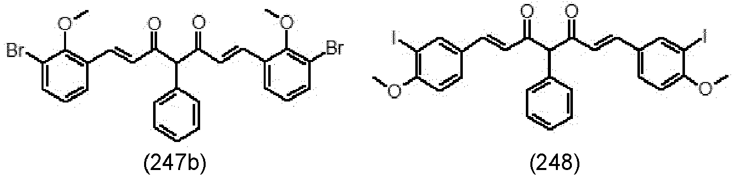

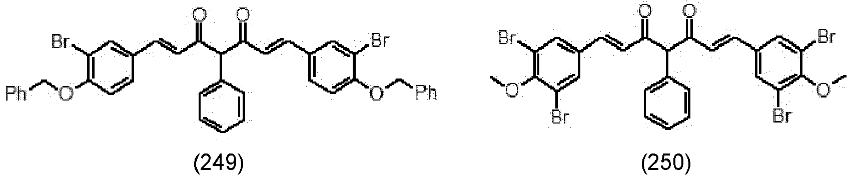

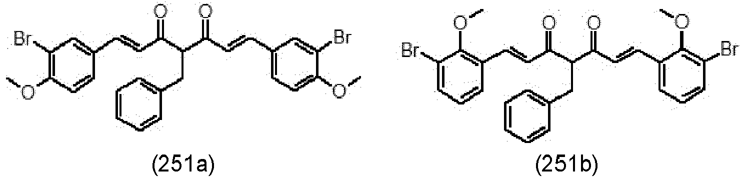

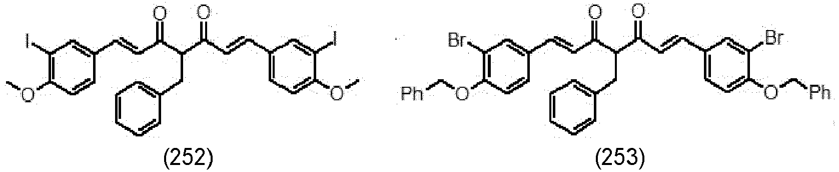

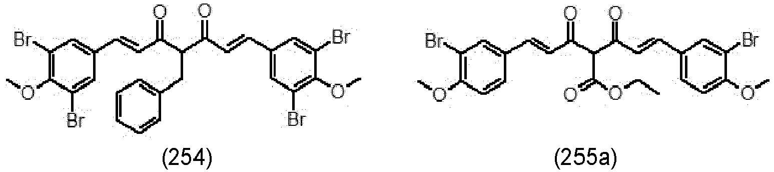

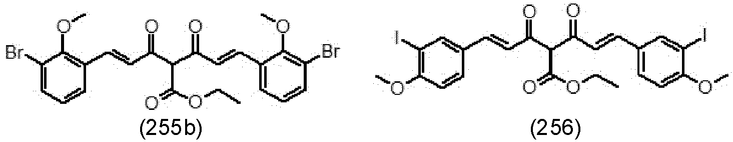

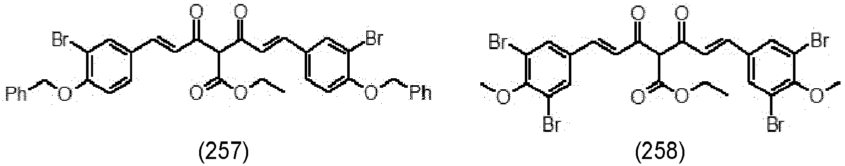

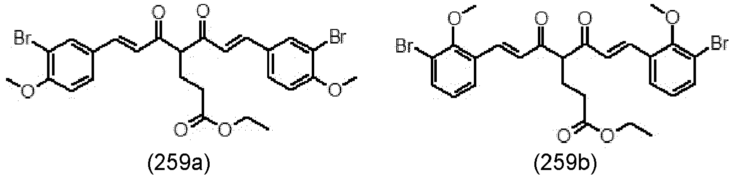

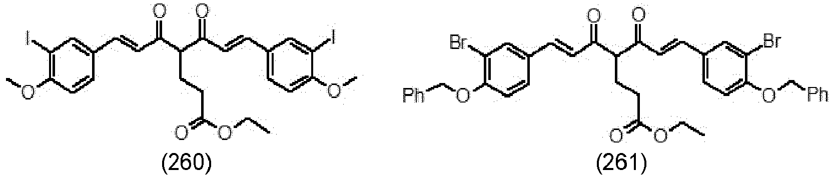

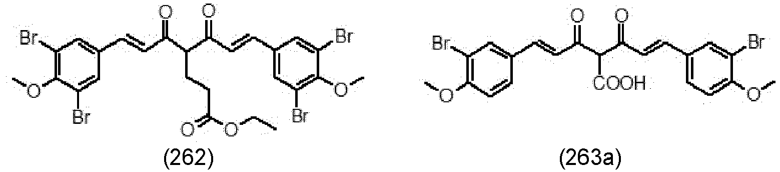

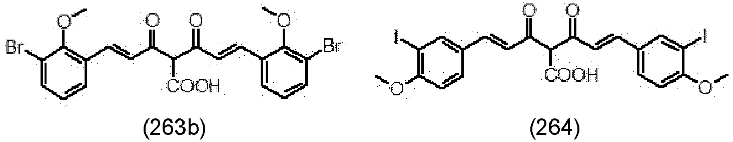

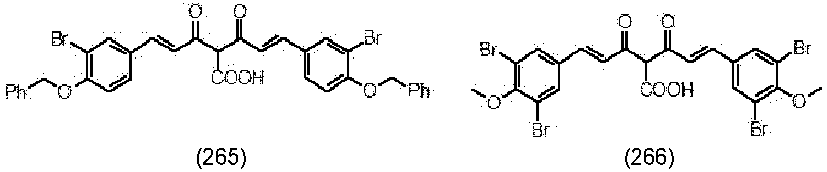

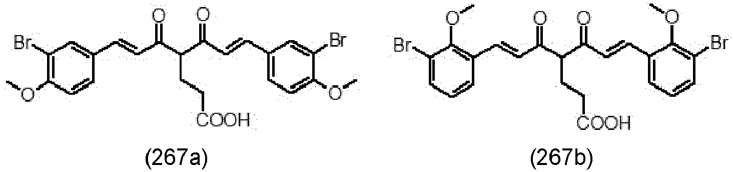

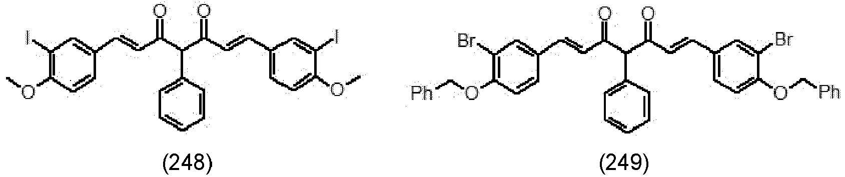

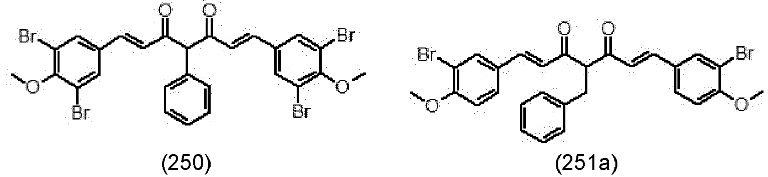

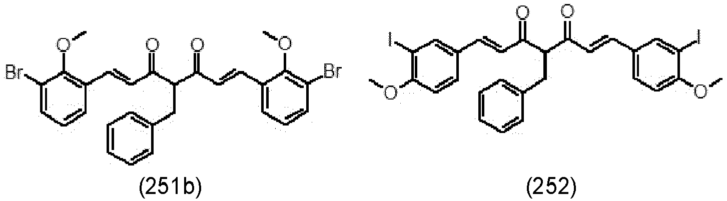

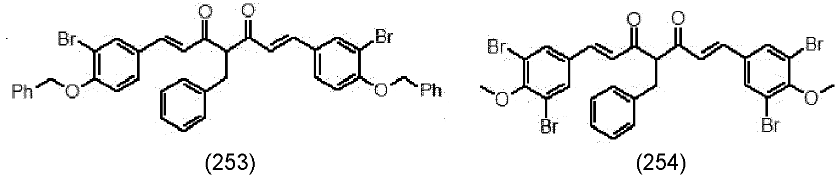

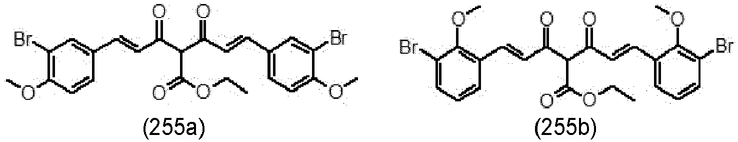

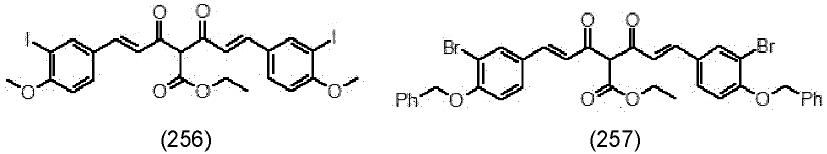

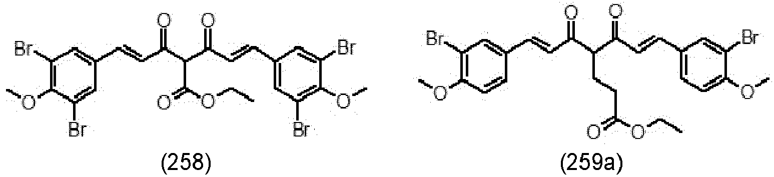

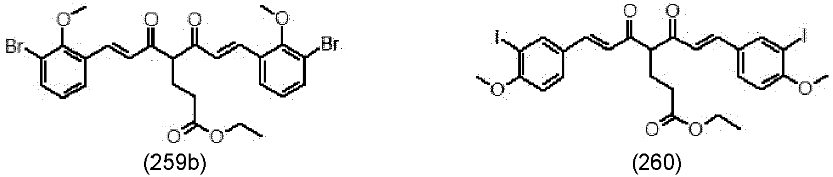

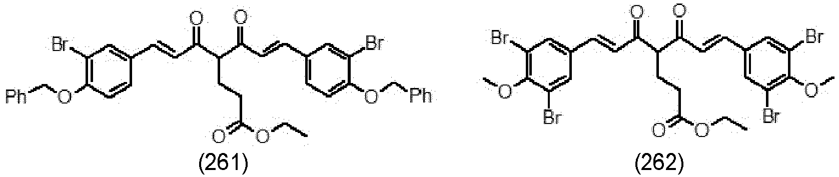

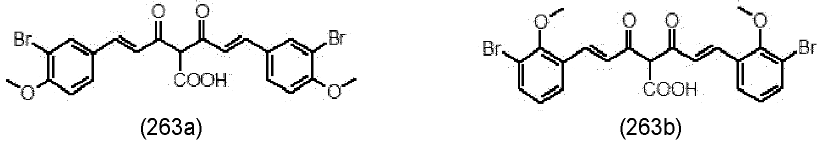

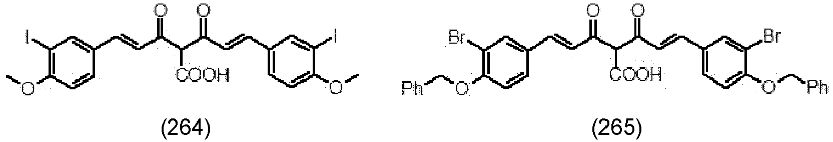

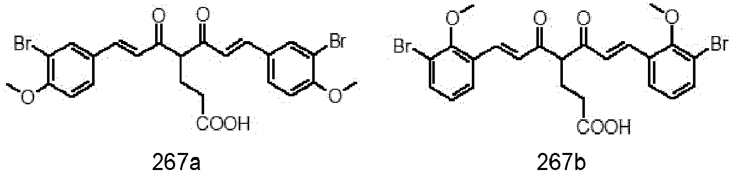

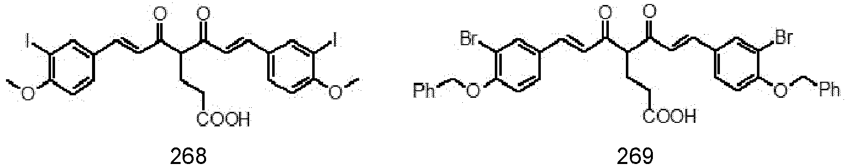

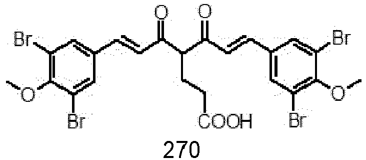

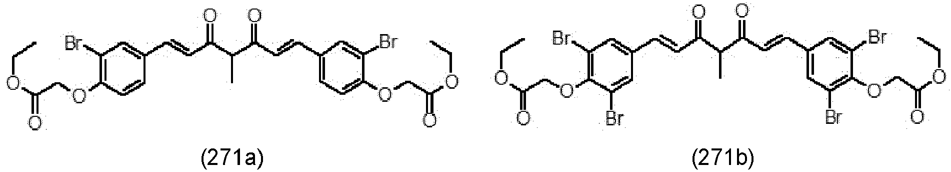

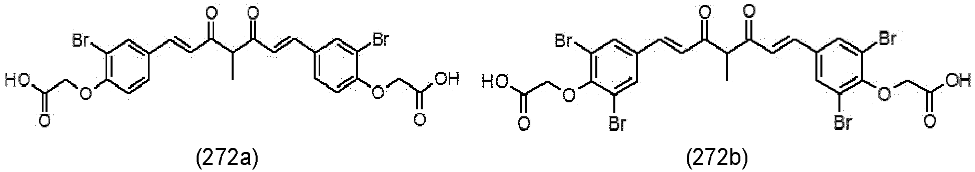

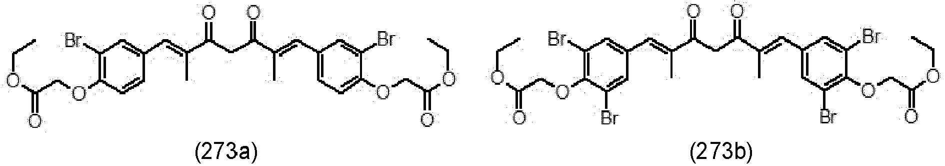

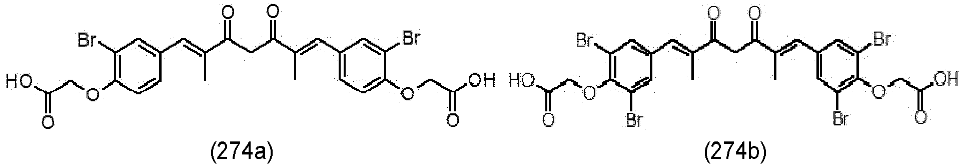

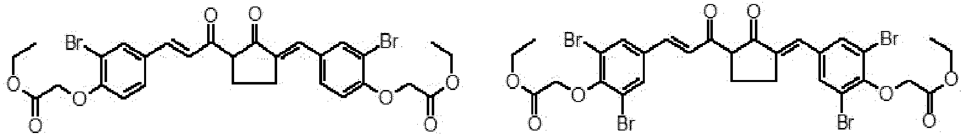

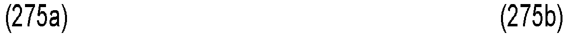

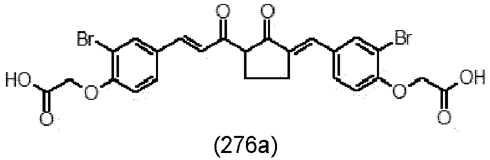

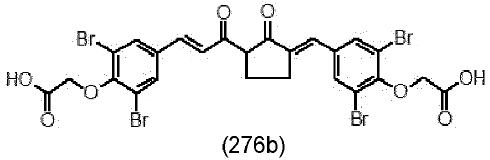

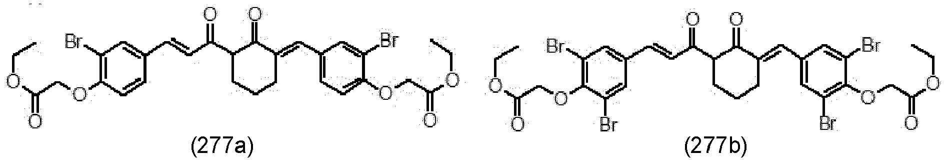

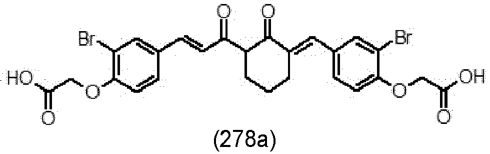

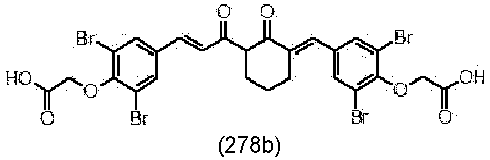

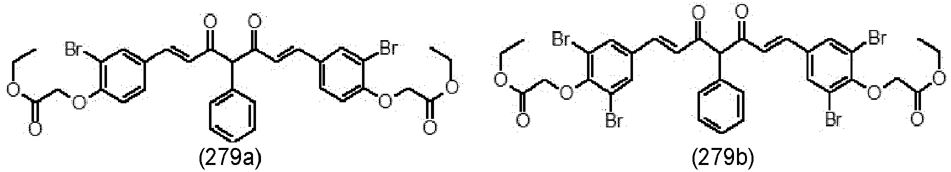

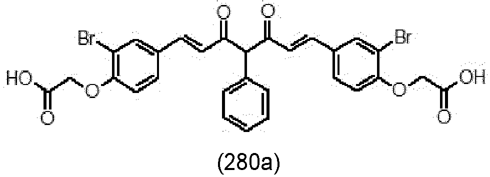

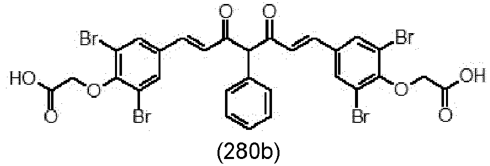

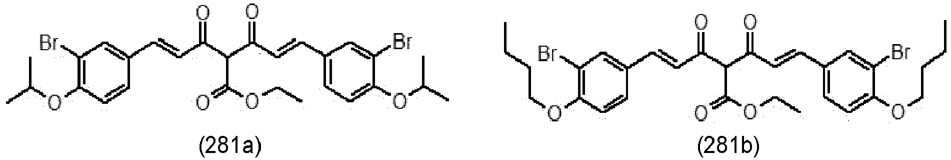

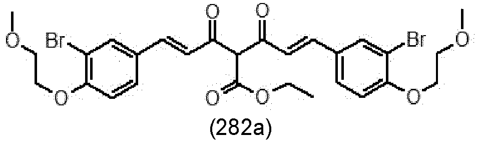

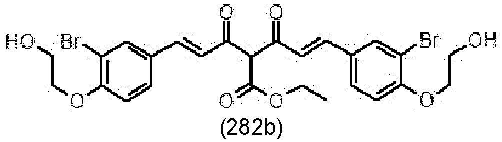

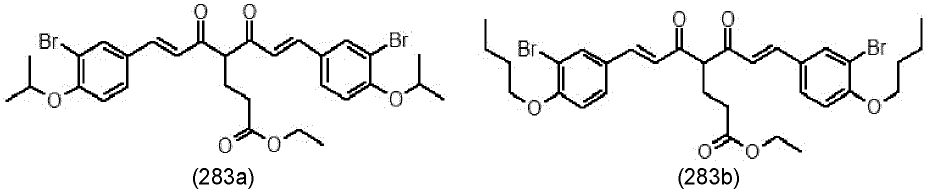

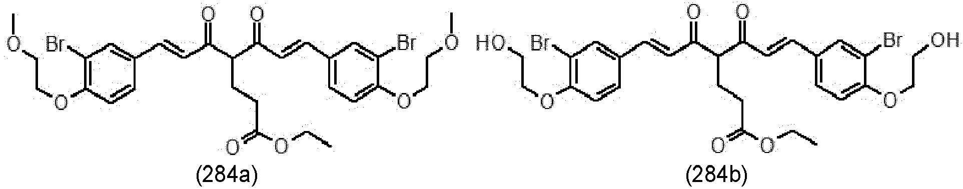

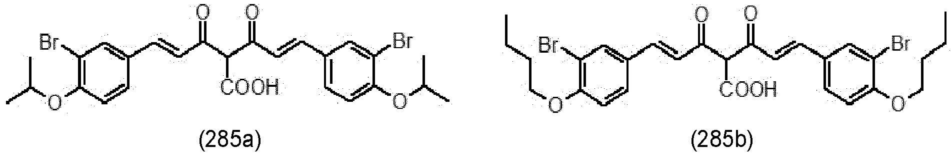

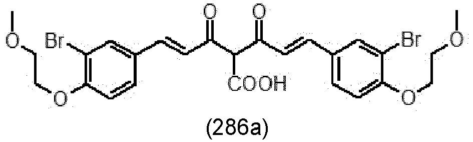

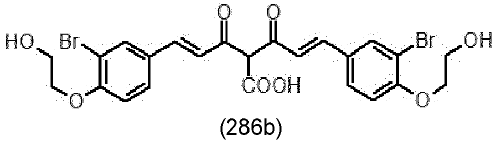

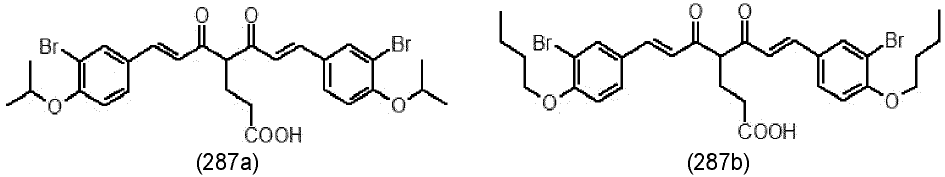

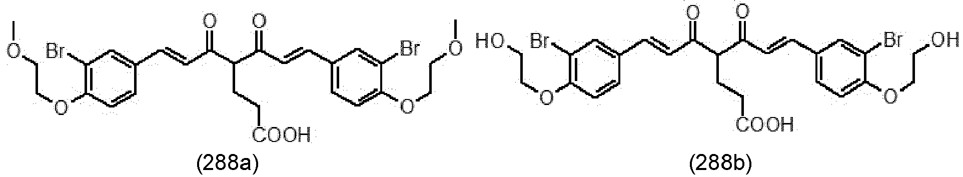

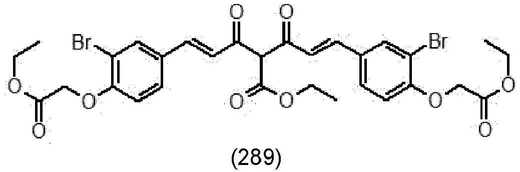

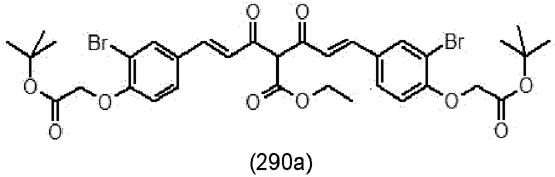

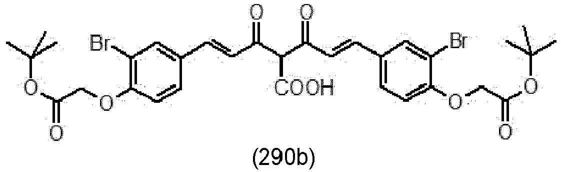

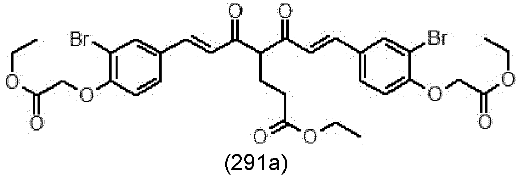

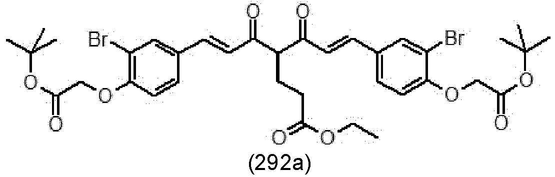

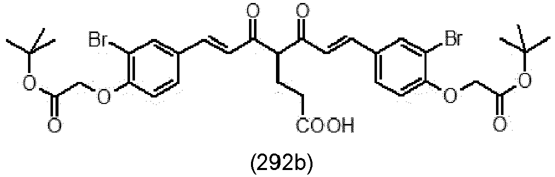

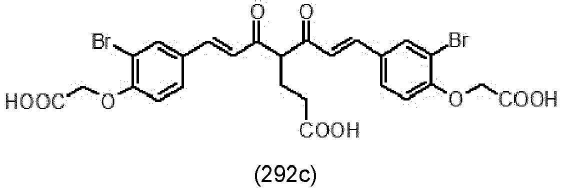

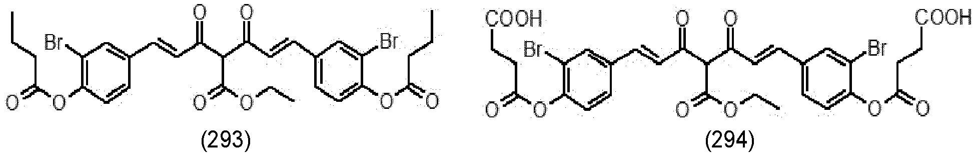

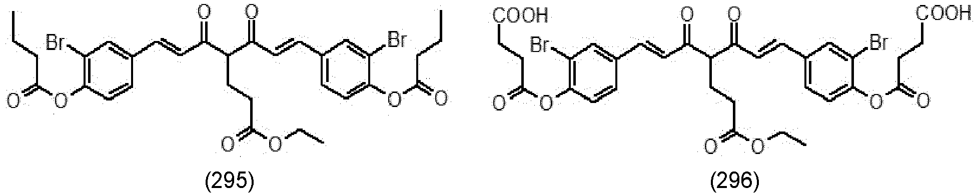

Vorzugsweise ist das wenigstens eine Porphycen-Derivat ein erfindungsgemäß zu verwendendes Porphycen-Derivat der allgemeinen Formel (3): 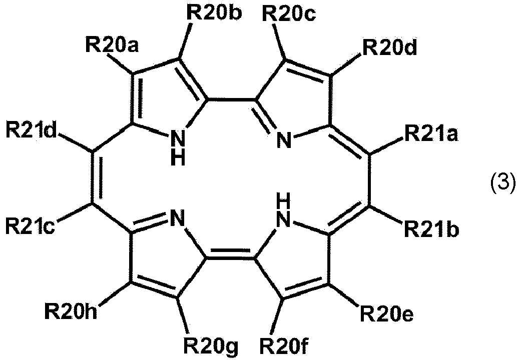
wobei die Reste R20a, R20b, R20c, R20d, R20e, R20f, R20g, und R20h, die jeweils unabhängig voneinander gleich oder voneinander verschieden sein können, jeweils Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel *-[(C(D)(E))ₘ-O)ₙ-W oder ein Rest der allgemeinen Formel *-O[(C(D)(E))ₘ-O)ₙ-W bedeuten,
wobei der Index m jeweils unabhängig voneinander eine ganze Zahl von 1 bis 8, vorzugsweise von 2 bis 4, bedeutet, und wobei der Index n jeweils unabhängig voneinander eine ganze Zahl von 1 bis 6 bedeutet,
wobei der Rest W jeweils unabhängig voneinander Alkyl, das gradkettig oder verzweigt sein kann, mit 1 bis 6 Kohlenstoffatomen, Phenyl oder Benzyl bedeutet, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können,
wobei die Reste R21a, R21b, R21c, und R21d, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, oder einen organischen Rest W1c, der:
   c1) ein ungeladener organischer Rest, oder
   c2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder,
   c3) ein organischer Rest mit wenigstens einer Säuregruppe, vorzugsweise 1 oder 2 Säuregruppen, oder
   c4) ein amphoterer organischer Rest, der ein permanent positiv geladenes Stickstoffatom oder ein protonierbares Stickstoffatom, das jeweils nicht direkt am aromatischen Ring angeordnet ist, und eine Säuregruppe umfasst,
vorzugsweise c2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder c3) ein organischer Rest mit wenigstens einer Säuregruppe, vorzugsweise 1 oder 2 Säuregruppen,
bedeutet, mit der Maßgabe, dass wenigstens einer der Reste R21a, R21b, R21c, und R21d ein organischer Rest W1c bedeutet,
wobei der Rest W1c in Variante c1) ein Rest mit der allgemeinen Formel (10a) bis (16b) bedeutet:

   * - (C(D)(E))ₕ - T (10a)

   * - Q - (C(D)(E))ₕ - T (10b)

   * - [(C(D)(E))_{b} - A]ₐ - (C(D)(E))_{g} - T (11a)

   * - Q - [(C(D)(E))_{b} - A]ₐ - (C(D)(E))_{g} - T (11b)

   * - [(C(D)(E))_{b} - A]ₐ - (C(D)(E))_{y} - Ar - (C(D)(E))_{g} - T (12a)

   * - Q - [(C(D)(E))_{b} - A]ₐ - (C(D)(E))_{y} - Aᵣ - (C(D)(E))_{g} - T (12b)

   * - (C(D)(E))ₕ - Ar - (C(D)(E))_{g} - T (13a)

   * - Q - (C(D)(E))ₕ - Ar - (C(D)(E))_{g} - T (13b)

   * - (C(D)(E))_{y} - Ar - B - (C(D)(E))_{g} - T (14a)

   * - Q - (C(D)(E))_{y} - Ar - B - (C(D)(E))_{g} - T (14b)

   * - (C(D)(E))_{y} - Ar - B - [(C(D)(E))_{b} - A]_{c} - (C(D)(E))_{y} - T (15a)

   * - Q - (C(D)(E))_{y} - Aᵣ - B - [(C(D)(E))_{b} - A]_{c} - (C(D)(E))_{y} - T (15b)

   * - [(C(D)(E))_{b} - A]ₐ - (C(D)(E)_{y} - Ar - B - [(C(D)(E))_{b} - A]_{c} - (C(D)(E))_{y} - T (16a)

   * - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet,
und wobei zwei oder mehr der Reste D und/oder E jeweils verbrückt sein können und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkanonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet, und
wobei der Rest W1c in Variante c2) ein Rest mit der allgemeinen Formel (21a) bis (24b) bedeutet:

   * - [(C(D)(E))_{b} - A]_{c} - (C(D)(E))_{g} - X (21a)

   * - Q - [(C(D)(E))_{b} - A]_{c} - (C(D)(E))_{g} -X (21b)

   * - [(C(D)(E))_{b} - A]ₐ - (C(D)(E))_{y} - Ar - (C(D)(E))_{f} - X (22a)

   * - Q - [(C(D)(E))_{b} - A]ₐ - (C(D)(E))_{y} - Aᵣ - (C(D)(E))_{f} - X (22b)

   *-(C(D)(E))_{d}-Ar-(C(D)(E))_{f}-X (23a)

   *-Q-(C(D)(E))_{d}-Ar-(C(D)(E))_{f}-X (23b)

   *-(C(D)(E))_{d}-Ar-[A-(C(D)(E))_{b}]ₐ-X (24a)

   * - Q - (C(D)(E))_{d} - Ar - [A - (C(D)(E))_{b}]ₐ - X (24b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxylalkyl, das gradkettig oder verzweigt sein kann, mit 1 bis 5 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon besteht, ausgewählt werden, substituiert sein können, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices d, und f jeweils unabhängig voneinander eine ganze Zahl von 1 bis 10, vorzugsweise 1 bis 8, vorzugsweise von 2 bis 4, bedeuten, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar einen unsubstituierten Phenylen-Rest oder einen substituierten Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert ist, bedeutet,
wobei der Rest X jeweils unabhängig voneinander ein Rest der Formel (15a), (15b), (16), (17a), (17b), (17c), oder (17d), vorzugsweise ein Rest der Formel (15a), (15b), (17a) oder (17b), bedeutet: 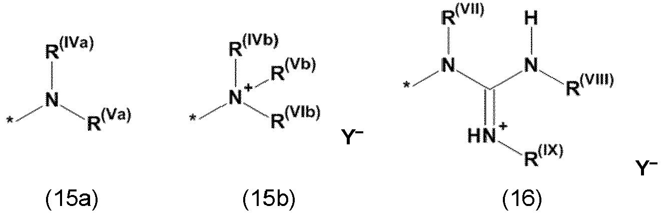 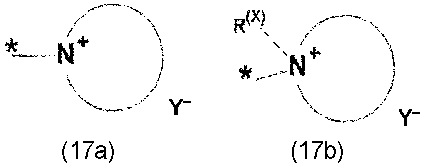 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)} und R^{(X)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (17a): 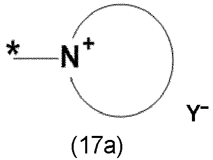 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, bedeutet, wobei der heterocyclische Rest mit 5 bis 10 Ringatomen unsubstituiert oder mit einem oder mehrerer Reste substituiert ist, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden, und
wobei der Rest mit der Formel (17b): 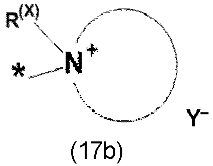 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung mit einem Ringatom, vorzugsweise Kohlenstoffatom, ausbildet, bedeuten, wobei der heterocyclische Rest mit 5 bis 7 Ringatomen, unsubstituiert oder mit einem oder mehrerer Reste substituiert ist, die aus der Gruppe, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, Tosylat und Mischungen davon besteht, ausgewählt ist, und
wobei der Rest W1c in Variante c3) ein Rest mit der allgemeinen Formel (20) bis (24b) bedeutet:

   - Z (30)

   * - [(C(D)(E))_{b} - A]_{c} - (C(D)(E))_{g} - Z (31a)

   * - Q - [(C(D)(E))_{b} - A]_{c} - (C(D)(E))_{g} - Z (31b)

   * - [(C(D)(E))_{b} - A]_{c} - (C(D)(E))_{y} - Ar - (C(D)(E))ₑ - Z (32a)

   * - Q - [(C(D)(E))_{b} - A]_{c} - (C(D)(E))_{y} - Ar - (C(D)(E))ₑ - Z (32b)

   * - (C(D)(E))_{d} - Ar - (C(D)(E))ₑ - Z (33a)

   * - Q - (C(D)(E))_{d} - Ar - (C(D)(E))ₑ - Z (33b)

   * - (C(D)(E))_{d} - Ar - [A - (C(D)(E))_{b}]ₐ - Z (34a)

   * - Q - (C(D)(E))_{d} - Ar - [A - (C(D)(E))_{b}]ₐ - Z (34b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index d jeweils unabhängig voneinander eine ganze Zahl von 1 bis 10, vorzugsweise von 1 bis 4 bedeutet, und
wobei der Index e jeweils unabhängig voneinander eine ganze Zahl von 0 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Rest W1c in Variante c4) ein Rest mit der allgemeinen Formel (41a) oder (41b) bedeutet:

   * - (CH₂)_{g} - Het - (CH₂)ᵢ - Z (41a)

   * - (CH₂)_{g} -Het - [B - (CH₂)ₖ]ₗ - Z (41b)
wobei der Rest B jeweils unabhängig voneinander Sauerstoff, ein Rest der Formel * - C(=O) - *, ein Rest der Formel * - C(=O)O - *, ein Rest der Formel * - OC(=O) - *, oder ein Rest der Formel * - C(=O)N(R⁽⁶⁾) - * bedeutet, wobei der Rest R⁽⁶⁾ jeweils unabhängig voneinander Wasserstoff oder Alkyl, das gradkettig oder verzweigt sein kann, mit 1 bis 4 Kohlenstoffatomen bedeutet,
wobei der Rest Het jeweils einen heterocyclischen Rest mit 5 bis 10 Ringatomen, die mindestens 1 Kohlenstoffatom und mindestens 1 Stickstoffatom sowie optional 1 oder 2 Sauerstoffatome umfassen, bedeutet, der vorzugsweise ein Rest mit der Formel (17a) oder ein Rest mit der Formel (17c) ist: 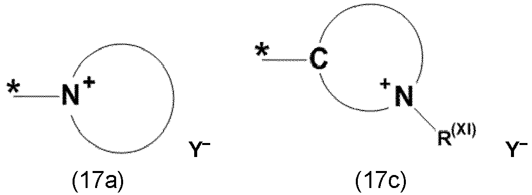
wobei der heterocyclisch Rest 5 bis 10 Ringatome aufweist, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, wobei der Rest R^{(XI)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und wobei die Indices i, und k jeweils unabhängig voneinander eine ganze Zahl von 1 bis 4, vorzugsweise 2 bis 3, bedeutet, und
wobei die Index I jeweils unabhängig voneinander eine ganze Zahl von 0 bis 4, vorzugsweise 1 bis 3, bedeuten, und
wobei der Rest Z jeweils unabhängig voneinander ein Säure-Rest ist, der aus der Gruppe, die aus Carboxygruppe und Sulfonsäuregruppe besteht, ausgewählt wird.

Vorzugsweise sind die Reste R20a, R20d, R20h, und R20e jeweils gleich und die Reste R20b, R20c, R20g, und R20f jeweils gleich, wobei die Reste R20a, R20d, R20h, und R20e und die Reste R20b, R20c, R20g, und R20f jeweils unabhängig voneinander Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - [(C(D)(E))ₘ - O)ₙ - W oder ein Rest der allgemeinen Formel * - O [(C(D)(E))ₘ - O)ₙ - W bedeuten, wobei der Index m jeweils unabhängig voneinander eine ganze Zahl von 1 bis 8, vorzugsweise von 2 bis 4, bedeutet, und wobei der Index n jeweils unabhängig voneinander eine ganze Zahl von 1 bis 6 bedeutet, und wobei der Rest W jeweils unabhängig voneinander Alkyl, das gradkettig oder verzweigt sein kann, mit 1 bis 6 Kohlenstoffatomen, Phenyl oder Benzyl bedeutet, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, bedeutet.

Weiter bevorzugt ist der wenigstens eine organische Rest W1c ein Rest mit der Formel (11a), (11b), (12a), (12b), (21a), (21b), (22a), (22b), (31a), oder (31b), wobei der Index a jeweils unabhängig voneinander eine ganze Zahl von 0 bis 2, vorzugsweise 1 oder 2, bedeutet und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet und wobei der Index b jeweils unabhängig voneinander eine ganze Zahl von 1 bis 3, vorzugsweise 2 bis 3, bedeutet und wobei der Index y jeweils unabhängig voneinander eine ganze Zahl von 0 bis 2, vorzugsweise 0 bis 1, bedeutet und wobei der Index c jeweils unabhängig voneinander eine ganze Zahl von 0 bis 2, vorzugsweise 1 oder 2, bedeutet.

Weiter bevorzugt sind die Reste R20a, R20d, R20h, und R20e jeweils gleich und die Reste R20b, R20c, R20g, und R20f jeweils gleich, wobei die Reste R20a, R20d, R20h, und R20e und die Reste R20b, R20c, R20g, und R20f jeweils unabhängig voneinander Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - [(C(D)(E))ₘ - O)ₙ - W oder ein Rest der allgemeinen Formel * - O [(C(D)(E))ₘ - O)ₙ - W bedeuten, wobei der Index m jeweils unabhängig voneinander eine ganze Zahl von 1 bis 8, vorzugsweise von 2 bis 4, bedeutet, und wobei der Index n jeweils unabhängig voneinander eine ganze Zahl von 1 bis 6 bedeutet, und wobei der Rest W jeweils unabhängig voneinander Alkyl, das gradkettig oder verzweigt sein kann, mit 1 bis 6 Kohlenstoffatomen, Phenyl oder Benzyl bedeutet, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, bedeutet und wobei die Reste R21a, R21b, R21c, und R21d, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, oder einen organischen Rest W1c bedeuten, mit der Maßgabe, dass wenigstens einer, vorzugsweise einer, der Reste R21a, R21b, R21c, und R21d ein organischer Rest W1c, wobei der Rest W1c ein Rest mit der Formel (11a), (11b), (12a), (12b), (21a), (21b), (22a), (22b), (31a), oder (31b), wobei der Index a jeweils unabhängig voneinander eine ganze Zahl von 0 bis 2, vorzugsweise 1 oder 2, bedeutet und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet und wobei der Index b jeweils unabhängig voneinander eine ganze Zahl von 1 bis 3, vorzugsweise 2 bis 3, bedeutet und wobei der Index y jeweils unabhängig voneinander eine ganze Zahl von 0 bis 2, vorzugsweise 0 bis 1, bedeutet und wobei der Index c jeweils unabhängig voneinander eine ganze Zahl von 0 bis 2, vorzugsweise 1 oder 2, bedeutet.

Weiter bevorzugt ist der wenigstens eine organische Rest W1c, ein Rest der ausgewählt ist aus der Gruppe, die aus den Resten mit der Formel (400) bis (585), vorzugsweise den Resten mit der Formel (403) bis (429), (432), (434), (436), (437), (440) bis(443), (448), (450), (451), (454) bis (456), (460), (461), (464) bis (467), (472), (473), (476) bis (478), (480) bis (482), (486) bis (488), (492) bis (494), (498) bis (500), (506), (508), (510), (514) bis (516), (519), (523), (528), (529), (531), (533), (537), (539), (541), (543), und (544) bis (552), besteht

Weiter bevorzugt wird das wenigstens eine erfindungsgemäß zu verwendende Porphycen-Derivat der allgemeinen Formel (3) ausgewählt aus der Gruppe, die aus den Verbindungen mit der Formel (300) bis (330) und Mischungen davon, besteht:

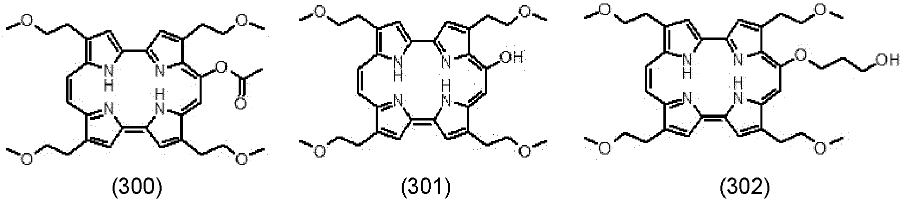

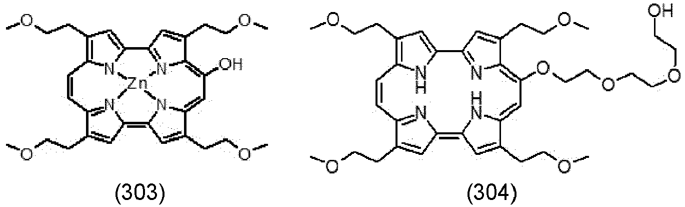

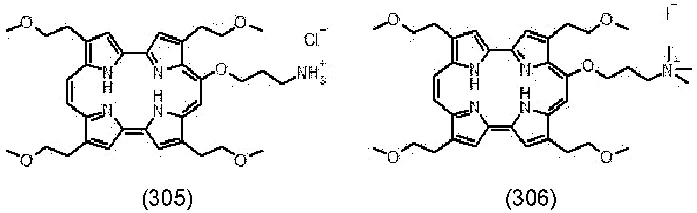

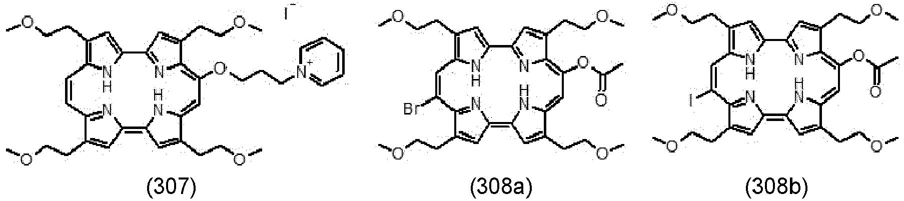

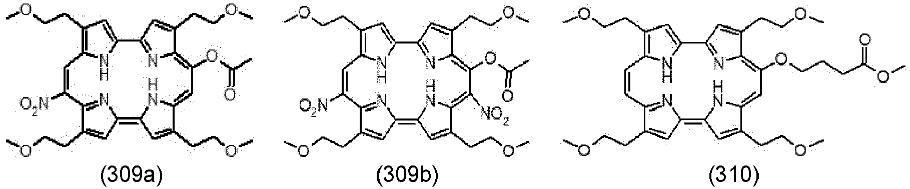

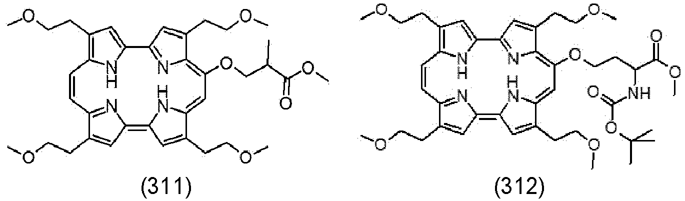

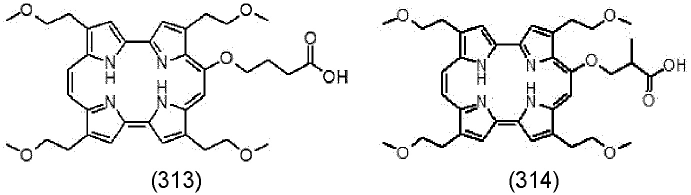

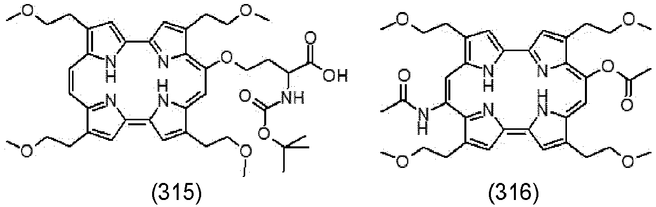

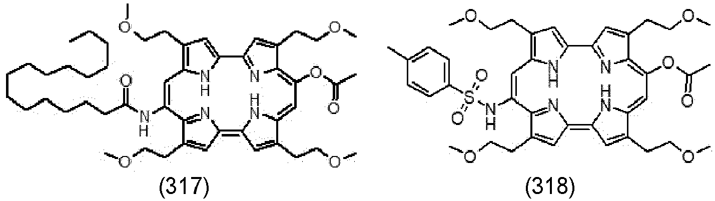

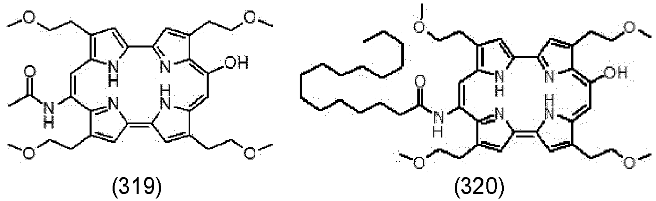

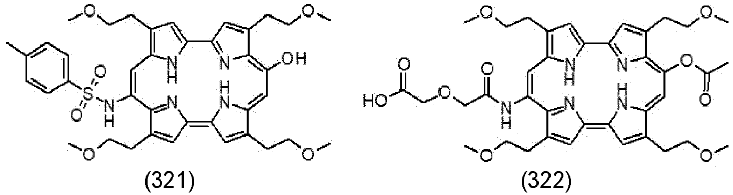

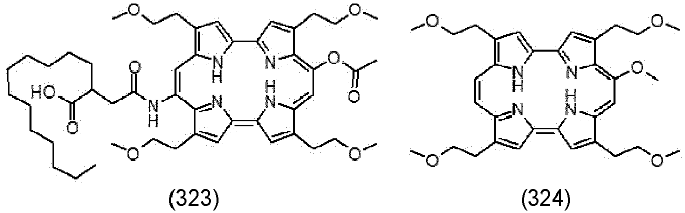

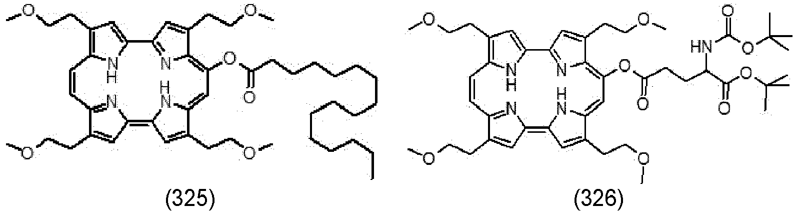

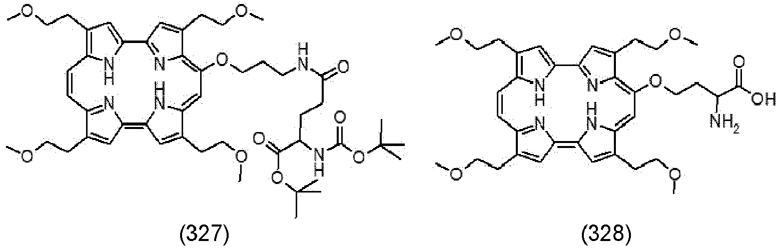

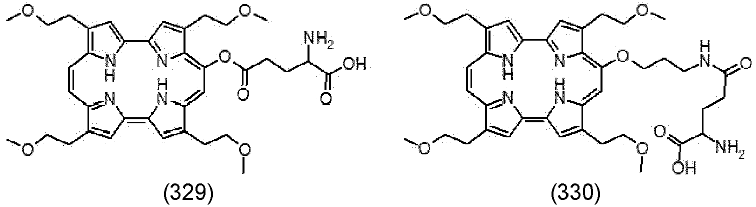

Bei einer bevorzugten Ausführungsform des vorgenannten Phenalen-1-on-Derivats der allgemeinen Formel (1) oder des Curcumin-Derivats der allgemeinen Formel (2a) oder Formel (2b) oder des Porphycen-Derivats der allgemeinen Formel (3) ist der Rest der Formel (17a) ein Rest der Formeln (42a): 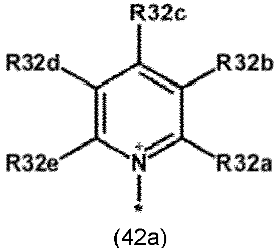 wobei die Reste R32a bis R32e jeweils unabhängig voneinander, Wasserstoff, Hydroxyl, Chlor, Brom, Fluor, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt.

Bei einer bevorzugten Ausführungsform des vorgenannten Phenalen-1-on-Derivats der allgemeinen Formel (1) oder des Curcumin-Derivats der allgemeinen Formel (2a) oder Formel (2b) oder des Porphycen-Derivats der allgemeinen Formel (3) ist der Rest Het ein Rest der allgemeinen Formel (26a) bis (31): 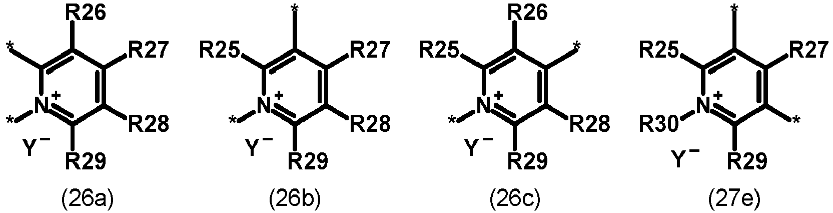 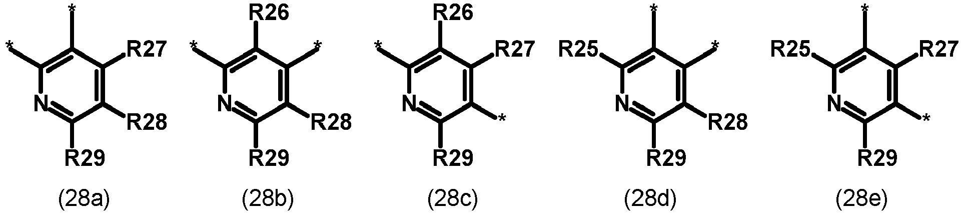 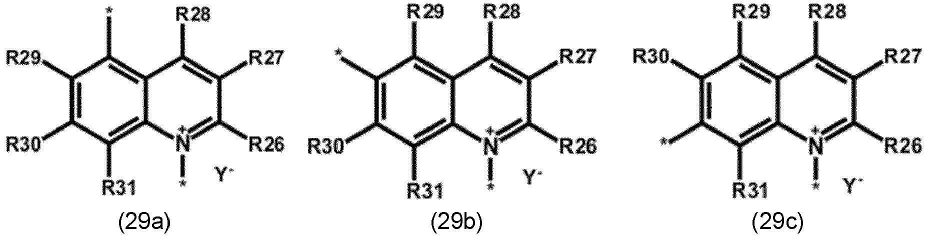 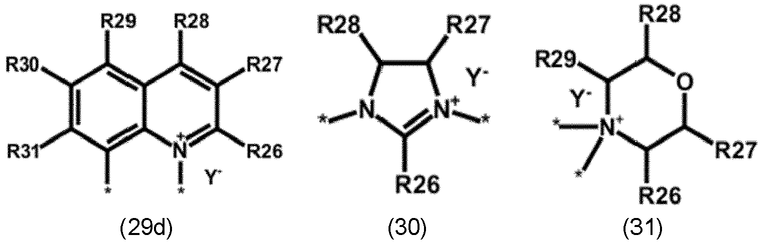
wobei die Reste R26, R27, R28, R29, R30, und R31 jeweils unabhängig voneinander jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist.

Bei einer bevorzugten Ausführungsform des vorgenanntenPhenalen-1-on-Derivats der allgemeinen Formel (1) oder des Curcumin-Derivats der allgemeinen Formel (2a) oder Formel (2b) oder des Porphycen-Derivats der allgemeinen Formel (3) ist der Rest (17b) ein Rest der Formel (32) 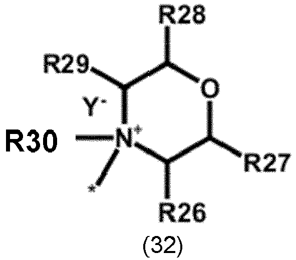 wobei die Reste R26, R27, R28, R29 und R30 jeweils unabhängig voneinander jeweils unabhängig voneinander Wasserstoff, Hydroxyl, Chlor, Brom, Fluor, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, und Mischungen davon besteht, ausgewählt ist.

Bei einer besonders bevorzugten Ausführungsform des erfindungsgemäßen Verfahrens sowie der erfindungsgemäßen Photosensibilisator-Zusammensetzung wird der wenigstens eine Photosensibilisator ausgewählt aus den Phenalen-1-on-Derivaten mit der Formel (101a) bis (184), vorzugsweise mit der Formel (101a) bis (164b), Curcumin-Derivaten mit der Formel (200) bis (296), vorzugsweise mit der Formel (200) bis (218), Porphycen-Derivaten mit der Formel (300) bis (330) und Mischungen davon, vorzugsweise Phenalen-1-on-Derivaten mit der Formel (101a) bis (184), vorzugsweise mit der Formel (101a) bis (164b), Curcumin-Derivaten mit der Formel (200) bis (296), vorzugsweise mit der Formel (200) bis (218) und Mischungen davon, weiter bevorzugt Phenalen-1-on-Derivaten mit der Formel (101a) bis (184), vorzugsweise mit der Formel (101a) bis (164b) und Mischungen davon.

Vorzugsweise umfasst eine Photosensibilisator-haltige Zusammensetzung ferner wenigstens einen Hilfsstoff, vorzugsweise wenigstens ein Komplexbildner, der vorzugsweise ausgewählt ist aus der Gruppe, die aus Ethylendiamintetraessigsäure (EDTA), Diethylentriaminpentaessigsäure (DTPA), Diethylentriaminpentakis(methylenphosphonsäure) (DTPMP), 1-Hydroxyethan-(1,1-diphosphonsäure (HEDP), Aminotrimethylenphosphonsäure (ATMP), Ethylendiamintetra(methylenposphonsäure) (EDTMP), 2-Phosphonbutan-1,2,4-tricarbonsäure (PBTC), Nitriloessigsäure (NTA), jeweils Alkalisalze davon, vorzugsweise Natriumsalze davon, und Mischungen davon besteht, wenigstens ein Konservierungsmittel, vorzugsweise Phenoxyethanol, Phenoxypropanol, Benzoisothiazolinone wie beispielsweise Methylisothiazolinon (MIT), Chlormethylisothiazolinon (CMIT), Benzisothiazolinon (BIT), Dichloroctylisothiazolinon (DCOIT), Benzoesäure/Natriumbenzoat und Zink Pyrrithion oder Mischungen davon und/oder wenigstens ein Pufferungsmittel, vorzugsweise Carbonsäuren und/oder deren Salze, wie beispielsweise Natriumacetat / Essigsäure, Natriumpropionat/Propionsäure, Gluconsäure, Kaliumacetat, Natriumhydrogensulfat, Kaliumhydrogensulfat und Mischungen davon.

Ein erfindungsgemäßes Phenalen-1-on Derivat weist die allgemeine Formel (1) auf: 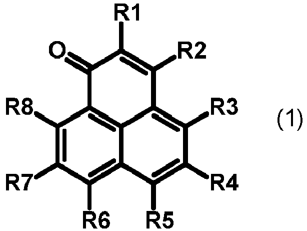
wobei die Reste R1 bis R8, die jeweils unabhängig voneinander gleich oder voneinander verschieden sein können, jeweils Wasserstoff, Chlor, Brom, lod, Alkyl mit 1 bis 4 C-Atomen, vorzugsweise Methyl, Ethyl, n-Propyl oder iso-Propyl, *-O-Alkyl mit 1 bis 4 C-Atomen, vorzugsweise Methoxy oder Ethoxy, oder einen organischen Rest W1a, der ein ungeladener organischer Rest ist, bedeuten,
mit der Maßgabe, dass wenigstens einer der Reste R1 bis R8, vorzugsweise wenigstens einer der Reste R1 bis R6, vorzugsweise wenigstens einer der Reste R1, R2, R4, R6 oder R8, weiter bevorzugt wenigstens einer der Reste R1, R2, R4 oder R6, weiter bevorzugt wenigstens einer der Reste R1 oder R2, weiter bevorzugt der Rest R1, ein organischer Rest W1a bedeutet,
wobei der Rest W1a ein Rest mit der allgemeinen Formel (10a) bis (12b), (13b), (14b), oder (15b) bis (16b) vorzugsweise ein Rest mit der allgemeinen Formel (10b) bis (12b), (13b), (14b), oder (15b) bis (16b), weiter bevorzugt ein Rest mit der allgemeinen Formel (10b), (11a), (12a), (14b) oder (16a), bedeutet:

   * -(C(D)(E))ₕ- T (10a)

   * - Q - (C(D)(E))ₕ- T (10b)

   * - [(C(D)(E))_{b}- A]ₐ-(C(D)(E))_{g}- T (11a)

   * -Q-[(C(D)(E))_{b}-A]a-(C(D)(E))_{g}-T (11b)

   * - [(C(D)(E))_{b}- A]ₐ- (C(D)(E))_{y}-Ar - (C(D)(E))_{g}- T (12a)

   * - Q - [(C(D)(E))_{b}- A]ₐ- (C(D)(E))_{y}-Aᵣ- (C(D)(E))_{g}- T (12b)

   * - Q - (C(D)(E))ₕ- Ar - (C(D)(E))_{g}- T (13b)

   * - Q - (C(D)(E))_{y} - Ar - B - (C(D)(E))_{g}- T (14b)

   * - Q - (C(D)(E))_{y} - Aᵣ- B - [(C(D)(E))_{b}- A]_{c}- (C(D)(E))_{y} - T (15b)

   * - [(C(D)(E))_{b}- A]ₐ- (C(D)(E)_{y}- Ar - B - [(C(D)(E))_{b}- A]_{c}- (C(D)(E))_{y} - T (16a)

   * - Q - [(C(D)(E))_{b}- A]ₐ - (C(D)(E)_{y} - Ar - B - [(C(D)(E))_{b}- A]_{c} - (C(D)(E))_{y} - T (16b)
wobei der Rest Q jeweils unabhängig voneinander Sauerstoff, ein Rest der Formel * - C(=O)O - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR(²)(=O)C - *, oder ein Rest der Formel * - C(=O)NR(²) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R(²))C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
wobei zwei oder mehr der Reste D und/oder E jeweils verbrückt sein können und jeweils einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei wenigstens einer der Reste D und/oder E mit wenigstens einem Ringatom des Rests Ar verbrückt sein kann und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei C-Atome der Cycloalkylreste, Cycloalkenylreste, Cycloalkanonreste, und Cycloalkanonreste jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 1 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei in Rest (11a) Index g eine ganze Zahl von 2 bis 35 bedeutet, wenn Rest A ein Rest der Formel * - OC(=O) - ** und Rest T ein Rest der allgemeinen Formel * - C(D)(E)H bedeutet, und
wobei in Rest (15b) Index c eine ganze Zahl von 1 bis 35 bedeutet, wenn Rest B Sauerstoff und Rest T ein Rest der allgemeinen Formel * - C(D)(E)H bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet.

Weiter bevorzugt ist das wenigstens eine Phenalen-1-on-Derivat ein erfindungsgemäßes Phenalen-1-on-Derivat der allgemeinen Formel (1), wobei einer der Reste R1 bis R8, vorzugsweise einer der Reste R1 bis R6, vorzugsweise einer der Reste R1, R2, R4, R6 oder R8, weiter bevorzugt einer der Reste R1, R2, R4 oder R6, weiter bevorzugt einer der Reste R1 oder R2, weiter bevorzugt der Rest R1, ein organischer Rest W1a bedeutet, wobei der organische Rest W1a ein Rest mit der Formel (11a), (11b), (12a), oder (12b), bedeutet, und wobei der Index a jeweils unabhängig voneinander eine ganze Zahl von 0 bis 2, vorzugsweise 1 oder 2, bedeutet, und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und wobei der Index b jeweils unabhängig voneinander eine ganze Zahl von 1 bis 3, vorzugsweise 2 bis 3, bedeutet, und wobei der Index y jeweils unabhängig voneinander eine ganze Zahl von 0 bis 2, und wobei der Index c jeweils unabhängig voneinander eine ganze Zahl von 0 bis 2, vorzugsweise 1 oder 2, bedeutet.

Bei einer weiter bevorzugten Ausführungsform ist das wenigstens eine Phenalen-1-on-Derivat ein erfindungsgemäßes Phenalen-1-on-Derivat der allgemeinen Formel (1), wobei einer der Reste R1 bis R8, vorzugsweise einer der Reste R1 bis R6, vorzugsweise einer der Reste R1, R2, R4, R6 oder R8, weiter bevorzugt einer der Reste R1, R2, R4 oder R6, weiter bevorzugt einer der Reste R1 oder R2, weiter bevorzugt der Rest R1, ein organischer Rest W1a bedeutet, der ausgewählt ist aus der Gruppe, die aus den Resten mit der Formel (400) bis (405), (412), (414), (416), (418), (420), (422) bis (424), (440) bis (502), (504) bis (508), (512), (515), (517), (518), (521), (522), (525), (526), (529) bis (531), und (534) bis (539), vorzugsweise Resten mit der Formel (400) bis (405), (412), (414), (416), (418), (420), (422) bis (424), (440) bis (449), (451), (453) bis (502), (504) bis (508), (512), (515), (517), (518), (521), (522), (525), (526), (529) bis (531), und (534) bis (539), besteht:

Weiter bevorzugt wird das wenigstens eine erfindungsgemäße Phenalen-1-on-Derivat der allgemeinen Formel (1) ausgewählt aus der Gruppe, die aus den Verbindungen mit der Formel (101j), (101k), (102c) bis (102h), (103b) bis (103g), (104b), (104c), (107b) bis (107d), (114e), (114f), (115c) bis (115e), (116b) bis (116f), (117b), (118b), (119) bis (122), (124a) bis (133), (135), (137a), (138a), (143a), bis (144a) und Mischungen davon, vorzugsweise Verbindungen mit der Formel (101j), (101k), (102c) bis (102h), (103b) bis (103g), (104b), (104c), (107b) bis (107d), (114e), (114f), (115c) bis (115e), (116b) bis (116f), (117b), (118b), (119), (120b) bis (122), (124a) bis (133), (135), (137a), (138a), (143a), bis (144a) und Mischungen davon, besteht.

Ein erfindungsgemäßes Curcumin-Derivat weist die allgemeine Formel (2a) auf: 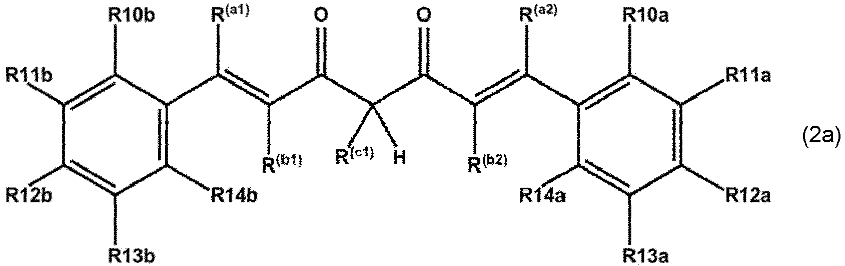
wobei die Reste R^{(a1)}, R^{(a2)}, R^{(b1)} und R^{(b2)}, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, wobei weiter bevorzugt die Reste R^{(a1)} und R^{(a2)} sowie R^{(b1)} und R^{(b2)}, jeweils gleich sind und unabhängig voneinander Wasserstoff, Methyl oder einen unsubstituierten Phenyl-Rest bedeuten,
wobei der Rest R^{(c1)} jeweils unabhängig voneinander Wasserstoff, Chlor, Brom, lod, Alkyl, das gradkettig oder verzweigt sein kann, mit 1 bis 4 C-Atomen, vorzugsweise Methyl, Ethyl, n-Propyl oder iso-Propyl, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
und wobei die Reste R10a bis R14b, die jeweils unabhängig voneinander gleich oder voneinander verschieden sein können, jeweils Wasserstoff, Halogen, vorzugsweise Chlor, Brom oder lod, Alkyl, das gradkettig oder verzweigt sein kann, mit 1 bis 8 C-Atomen, vorzugsweise Methyl, Ethyl, n-Propyl oder iso-Propyl, *-O-Alkyl, das gradkettig oder verzweigt sein kann, mit 1 bis 8 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
wobei in Variante b1) wenigstens einer der Reste R10a bis R14a und wenigstens einer der Reste R10b bis R14b ein organischer Rest W1b bedeutet und wobei optional wenigstens einer der Reste R10a bis R14a und wenigstens einer der Reste R10b bis R14b, vorzugsweise mindestens 2, weiter bevorzugt 2 oder 4, der Reste R10a und R10b oder R11a und R11b oder R12a und R12b oder R13a und R13b oder R14a und R14b, die nicht ein Rest W1b bedeuten, weiterhin Chlor, Brom oder lod, vorzugsweise Brom, bedeutet,
wobei der Rest W1b in Variante b1) ein Rest mit der allgemeinen Formel (10a) bis (11b) bedeutet:

   * - (C(D)(E))ₕ- T (10a)

   * - Q - (C(D)(E))ₕ- T (10b)

   * - [(C(D)(E))_{b}- A]ₐ - (C(D)(E))_{g}- T (11a)

   * - Q - [(C(D)(E))_{b}- A]ₐ- (C(D)(E))_{g}- T (11b)
wobei der Rest Q jeweils unabhängig voneinander Sauerstoff, ein Rest der Formel * - C(=O)O - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet, und
wobei der Rest T jeweils unabhängig voneinander ein Rest der allgemeinen Formel * - (C(D)(E)H, oder * - Ph, vorzugsweise ein Rest der allgemeinen Formel * - (C(D)(E)H, bedeutet
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, oder ein Rest der allgemeinen Formel * - C(=O)O - R⁽⁴⁾ bedeuten,
wobei der Rest R⁽¹⁾, jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeutet, und
wobei der Reste R⁽⁴⁾ jeweils unabhängig voneinander Alkyl, das gradkettig oder verzweigt sein kann, mit 1 bis 4 Kohlenstoffatomen bedeutet,
wobei der Index h jeweils unabhängig voneinander eine ganze Zahl von 1 bis 30, vorzugsweise von 4 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt12, bedeutet, und
wobei der Index a jeweils unabhängig voneinander eine ganze Zahl von 1 bis 10, vorzugsweise 1 bis 6, vorzugsweise 2 bis 4, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet und
wobei in Variante b3) wenigstens einer der Reste R10a bis R14a und wenigstens einer der Reste R10b bis R14b ein organischer Rest W1b bedeutet und wobei optional wenigstens einer der Reste einer der Reste R10a bis R14a und wenigstens einer der Reste R10b bis R14b, vorzugsweise mindestens 2, weiter bevorzugt 2 oder 4, der Reste R10a und R10b oder R11a und R11b oder R12a und R12b oder R13a und R13b oder R14a und R14b, die nicht ein Rest W1b bedeuten, weiterhin Chlor, Brom oder lod, vorzugsweise Brom, bedeutet,
wobei der Rest W1b in Variante b3)_ein Rest mit der allgemeinen Formel (30) bis (31b) bedeutet:

   - Z (30)

   * - [(C(D)(E))_{b}- A]_{c} - (C(D)(E))_{g}- Z (31a)

   * - Q- [(C(D)(E))_{b}- A]_{c} - (C(D)(E))_{g}- Z (31b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, oder ein Rest der allgemeinen Formel * - C(=O)O - R⁽⁴⁾ bedeuten, und
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Index c jeweils unabhängig voneinander eine ganze Zahl von 0 bis 10, vorzugsweise von 0 bis 6, vorzugsweise 0 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet.

Vorzugsweise bedeuten die Reste R10a und R10b oder die Reste R11a und R11b oder die Reste R12a und R12b oder die Reste R13a und R13b oder die Reste R14a und R14b, jeweils einen organischen Rest W1b, wobei die Reste R10a und R10b oder die Reste R11a und R11b oder die Reste R12a und R12b oder die Reste R13a und R13b oder die Reste R14a und R14b, die nicht ein organischer Rest W1b bedeuten, jeweils unabhängig voneinander Halogen, das ausgewählt ist aus Chlor, Brom und lod, bevorzugt Brom, Wasserstoff, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, bedeuten, wobei mindestens 2, weiter bevorzugt 2 oder 4, der Reste R10a und R10b oder R11a und R11b oder R12a und R12b oder R13a und R13b oder R14a und R14b, die nicht ein Rest W1b bedeuten, Chlor, Brom oder lod, bevorzugt Brom, bedeuten.

Weiter bevorzugt bedeutet wenigstens einer der Reste R10a bis R14a und/oder wenigstens einer der Reste R10b bis R14b, vorzugsweise die Reste R10a und R10b oder die Reste R11a und R11b oder die Reste R12a und R12b oder die Reste R13a und R13b oder die Reste R14a und R14b, jeweils einen organischen Rest W1b, wobei der organische Rest W1b ein Rest mit der Formel (11a), (11b), (31a) oder (31b) ist und wobei der Index a jeweils unabhängig voneinander eine ganze Zahl von 0 bis 2, vorzugsweise 1 oder 2, bedeutet und wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet und wobei der Index b jeweils unabhängig voneinander eine ganze Zahl von 1 bis 3, vorzugsweise 2 bis 3, und wobei der Index c jeweils unabhängig voneinander eine ganze Zahl von 0 bis 2, vorzugsweise 1 oder 2, bedeutet.

Weiter bevorzugt wird der organische Rest W1b ausgewählt aus der Gruppe, die aus den Resten mit der Formel (400) bis (585), vorzugsweise den Resten mit der Formel (403) bis (429), (432), (434), (436), (437), (440) bis (443), (448), (450), (451), (454) bis (456), (460), (461), (464) bis (467), (472), (473), (476) bis (478), (480) bis (482), (486) bis (488), (492) bis (494), (498) bis (500), (506), (508), (510), (514) bis (516), (519), (523), (528), (529), (531), (533), (537), (539), (541), (543), und (544) bis (552), besteht.

Weiter bevorzugt wird das wenigstens eine erfindungsgemäße Curcumin-Derivat der allgemeinen Formel (2a) ausgewählt aus der Gruppe, die aus den Verbindungen mit der Formel (200) bis (296) und Mischungen davon, vorzugsweise Verbindungen mit der Formel (200) bis (218), (271a bis 280b) und Mischungen davon, vorzugsweise Verbindungen mit der Formel (200) bis (218) und Mischungen davon, besteht.

Bei einer weiter bevorzugten Ausführungsform des erfindungsgemäßen Verfahrens oder der erfindungsgemäßen Photosensibilisator-haltige Zusammensetzung wird der wenigstens einen Photosensibilisator aus der Gruppe ausgewählt, die aus wenigstens einem erfindungsgemäßen Phenalen-1-on-Derivat gemäß Anspruch 12, wenigstens einem erfindungsgemäßen Curcumin-Derivat gemäß Anspruch 13, und Mischungen davon besteht.

Weiter bevorzugt wird im erfindungsgemäßen Verfahren in Schritt (a) eine erfindungsgemäße Photosensibilisator-haltige Zusammensetzung verwendet, wobei vorzugsweise vor Verwendung in Schritt (a) der Wassergehalt der Zusammensetzung auf einen Anteil von mindestens 50 Gew.-%, bezogen auf das Gesamtgewicht der Photosensibilisator-haltigen Zusammensetzung, eingestellt wird.

Eine erfindungsgemäße Mehrkomponentenzusammensetzung zur gemeinsamen Verwendung in einem erfindungsgemäßen Verfahren umfasst wenigstens eine Komponente A) und eine Komponente B) wobei:
A) eine erste Zusammensetzung, umfassend Wasser, und wenigstens ein Co-Solvens, das aus der Gruppe, die aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon besteht, ausgewählt ist, sowie optional wenigstens einen Alkohol, der vorzugsweise 2 bis 10 C-Atome und 1 bis 4 OH-Gruppen aufweist oder daraus besteht, und/oder wenigstens einen Hilfsstoff, und
B) eine zweite Zusammensetzung, umfassend wenigstens einen Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, besteht, wenigstens ein Co-Solvens, das aus der Gruppe, die aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon besteht, ausgewählt ist, sowie optional Wasser und/oder wenigstens einen Hilfsstoff.

Die erste Zusammensetzung der Komponente A) weist vorzugsweise die flüchtigeren Komponenten der verwendeten Photosensibilisator-haltige Zusammensetzung auf. Daher kann es während der Durchführung des erfinderischen Verfahrens hilfreich sein, wenn die erste Zusammensetzung der Komponente A) regelmäßig ersetzt bzw. hinzugegeben wird, um ggf. verflüchtigte Bestandteile zu ersetzen. Dadurch kann weiterhin die Lebensdauer der verwendeten Photosensibilisator-haltigen Zusammensetzung gewährleistet werden.

Die zweite Zusammensetzung der Komponente B) umfasst insbesondere den verwendeten wenigstens einen Photosensibilisator. Daher kann es zu Regenerierung der verwendeten Photosensibilisator-haltige Zusammensetzung, beispielsweise nach Abschluss eines Durchlaufs, hilfreich sein, wenn die zweite Zusammensetzung der Komponente B) separat hinzugegeben wird, um beispielsweise im Verlaufe des Verfahrens in behandelte Oberflächenteilbereiche eingebrachte, also aus der verwendeten Photosensibilisator-haltigen Zusammensetzung entfernten Photosensibilisator zu ersetzen.

Weiter bevorzugt wird im erfindungsgemäßen Verfahren eine erfindungsgemäße Mehrkomponentenzusammensetzung verwendet, wobei vor und/oder während Schritt (a) die erste Zusammensetzung und die zweite Zusammensetzung der Mehrkomponentenzusammensetzung in einem Verhältnis von 10:1 bis 1:10 unter Erhalt einer als Photosensibilisator-haltigen Zusammensetzung gemischt werden, wobei optional die erhaltene Photosensibilisator-haltige Zusammensetzung nach dem Mischen mit Wasser verdünnt wird und/oder die erste Zusammensetzung und/oder die zweite Zusammensetzung der Mehrkomponentenzusammensetzung vor dem Mischen mit Wasser verdünnt wird.

Ein erfindungsgemäßer Gegenstand, der vorzugsweise unter Verwendung des erfindungsgemäßen Verfahrens hergestellt wurde, umfasst wenigstens einen photokatalytisch aktiven, vorzugsweise photodynamisch aktiven, Teilbereich wenigstens einer Oberfläche, wobei der wenigstens eine Teilbereich wenigstens einer Oberfläche wenigstens ein thermoplastisches Polymer, wenigstens ein elastomeres Polymer, Copolymere davon und Mischungen davon umfasst und in dem wenigstens ein Photosensibilisator, der aus der Gruppe ausgewählt ist, die aus Phenalen-1-on-Derivaten, Curcumin-Derivaten, Porphycen-Derivaten und Mischungen davon besteht, eingebracht ist.

Vorzugsweise liegt der Gegenstand in Form eines selbsttragenden Films, eines Verbunds aus Fasern oder eines Formgegenstandes vor.

Weiter bevorzugt weist ein erfindungsgemäßer Gegenstand wenigstens ein Photosensibilisator auf, der ausgewählt ist aus Phenalen-1-on-Derivaten mit der Formel (101a) bis (184), vorzugsweise mit der Formel (101a) bis (164b), Curcumin-Derivaten mit der Formel (200) bis (296), vorzugsweise mit der Formel (200) bis (218), Porphycen-Derivaten mit der Formel (300) bis (330) und Mischungen davon, vorzugsweise Phenalen-1-on-Derivaten mit der Formel (101a) bis (184), vorzugsweise mit der Formel (101a) bis (164b), Curcumin-Derivaten mit der Formel (200) bis (296), vorzugsweise mit der Formel (200) bis (218) und Mischungen davon, weiter bevorzugt Phenalen-1-on-Derivaten mit der Formel (101a) bis (184), vorzugsweise mit der Formel (101a) bis (164b) und Mischungen davon, wobei der wenigstens ein Photosensibilisator in wenigstens einem Teilbereich einer Oberfläche des Gegenstandes eingebracht ist.

Die Erfindung wird nachfolgend durch Beispiele erläutert, ohne hierauf beschränkt zu sein.

Alle verwendeten Chemikalien wurden von gängigen Anbietern käuflich erworben (Thermo Fisher Scientific, Polysciences Europe GmbH, Sigma Aldrich, TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben.

Dünnschichtchromatographische Analysen (TLC) wurden an Kieselgel 60 mit Fluoreszenz-Indikator F-254 mit 0,20 mm Schichtdicke der Firma Merck (Darmstadt, DE) und Detektion über UV-Licht bei 254 nm / 366 nm oder durch Anfärbung mit Ninhydrin in Ethanol durchgeführt. Die Säulenchromatographie wurde an Kieselgel 60 (0,03-0,2 mm) von Carl Roth durchgeführt.

Schmelzpunkte wurden auf einem Schmelzpunktgerät (MPM-Serie) der Firma Schorpp Gerätetechnik (Überlingen, DE) gemessen und wurden unkorrigiert verwendet.

NMR Spektren wurden an Bruker Avance 300 (¹H 300,13 MHz, ¹³C 75,47 MHz, T = 300 K), Bruker Avance III (¹H 400,13 MHz, ¹³C 100,61 MHz, T = 300 K), Bruker Avance 600 (¹H 600,13 MHz, ¹³C 150,92 MHz, T = 300 K) und Bruker Avance III 600 Kryo (¹H 600,25 MHz, ¹³C 150,95 MHz, T = 300 K) Instrumenten (Bruker Corporation, Billerica, US) gemessen. Die chemischen Verschiebungen sind angegeben als δ[ppm] relativ zum externen Standard ("solvent residual peak"). Die Spektren wurden nach erster Ordnung analysiert, die Kopplungskonstanten J sind angegeben in Hertz [Hz]. Charakterisierung der Signale: s = singlet, d = doublet, t = triplet, q = quartet, m = multiplet, bs = broad singlet, psq = pseudo quintet, dd = double doublet, dt = doublet of triplets, ddd = double double doublet. Die Integration wurde als relative Zahl der Atome bestimmt. Zuordnung der Signale in ¹³C-Spektren erfolgte über 2D-Spektroskopie (COSY, HSQC und HMBC) oder DEPT Verfahren (pulse angle: 135 °) und wird angegeben als (+) für CH₃ oder CH, (-) für CH₂ und (C_{q}) für quaternäre C_{q}. Fehler der angegebenen Werte: Chemische Verschiebung 0.01 ppm (¹H NMR) und 0.1 ppm (¹³C NMR), Kopplungskonstante J 0.1 Hz. Die für die Messungen verwendeten Lösungsmittel sind für jedes Spektrum angegeben.

IR-Spektren wurden mit einem Spektrometer Cary 630 der Firma Agilent aufgenommen. Absorptionsspektren wurden auf einem UV/Vis Spektrometer Cary 60 der Firma Agilent (Santa Clara, CA, USA) aufgenommen. Alle Messungen wurden in 1-cm-UV-Küvetten (Rotilabo) und Lösungsmitteln in UV-Qualität der Firma Merck (Darmstadt, DE) bei Raumtemperatur durchgeführt. Die Intensität der Peaks in rel. [a.u.] (relative "arbitrary units") angegeben. Für exemplarische Verbindungen wurde der Extinktionskoeffizient (ε / [M⁻¹cm⁻¹]) bestimmt. Fehler der angegebenen Werte: Wellenlänge Absorptionsmaximum λ +/-3nm, molarer Extinktionskoeffizient ε +/- 5%. Massenspektren wurden auf einem Agilent Technologies 6540 UHD Accurate - Mass Q-TOF LC/MS (LC-MS, GC-CIMS) und einem Jeol AccuTOF GCX GC-EIMS, FD Spektrometer gemessen (JEOL GmbH, Freising, DE). Xenon diente als lonisationsgas für FAB. Die Lösungsmittel für Absorptions- und Emissionsmessungen wurden in spezieller spektroskopischer Reinheit von Acros oder Baker bzw. Uvasol von Merck gekauft. Milliporewasser (18 MΩ, Milli QPlus) wurde für alle Messungen verwendet.

Nachfolgend verwendete Abkürzungen: DCM = Dichlormethan; DMF = N,N-Dimethylformamid; DIPEA = Diisopropylethylamin; DEAD = Azodicarbonsäurediethylester; EDC = 1-Ethyl-3-(3-dimethylaminopropyl)carbodiimid; NBS = N-Bromosuccinimid; RT = Raumtemperatur; TBAH = Tetrabutylammoniumhydrogensulfat; THF = Tetrahydrofuran;
d.Th = der Theorie

Bei den verwendeten chromatographischen Verfahren, beispielsweise Flashchromatographie oder Säulenchromatographie, bedeutet → jeweils ein Wechsel des Laufmittels in der angegeben Reihenfolge.

Folgende Phenalen-1-on Derivate sind kommerziell verfügbar und wurden unverändert eingesetzt:

**Tabelle 1. kommerziell verfügbare Phenalen-1-on Derivate**

| | | | | |
|---|---|---|---|---|
| 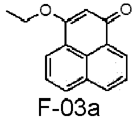 | CAS 76966-10-4 | | 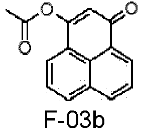 | CAS 18931-04-9 |
| | Oranges Pulver | | | Oranges Pulver |
| | MW: 224,25 g/mol | | | MW: 238,24 g/mol |
| | MF: C₁₅H₁₂O₂ | | | MF: C₁₅H₁₀O |
| 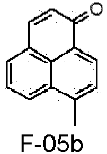 | CAS 10505-82-5 | | 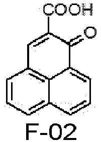 | CAS 7090-52-0 |
| | Oranges Pulver | | | Gelbes Pulver |
| | MW: 194,23 g/mol | | | MW: 224,22 g/mol |
| | MF: C₁₄H₁₀O | | | MF: C₁₄H₈O₃ |
| 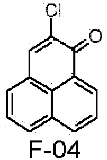 | CAS 59242-56-7 | | 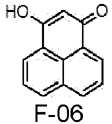 | CAS 5472-84-4 |
| | Gelbes Pulver | | | Gelbes Pulver |
| | MW: 214,65 g/mol | | | MW: 196,2 g/mol |
| | MF: C₁₃H₇ClO | | | MF: C₁₃H₈O |

Die Verbindungen F-02, F-03a, F03b, F-04 und F-05b wurde von der Chemieliva Pharmaceutical Co., Ltd (Chongqing, CN) bezogen. Die Verbindung F-06 wurde von der Alfa Chemistry (Ronkonkoma, NY, US) bezogen.

Folgende Phenalen-1-on Derivate wurden nach literaturbekannten Vorschriften hergestellt:

| | | |
|---|---|---|
| 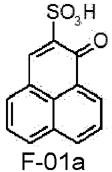 | Oranges Pulver | Hergestellt gemäß Nonell, S. et al.: Afinidad 1993, 50(48), Seite 445 - 450. |
| | MW: 260,28 g/mol | |
| | MF: C₁₃H₈O₃S | |
| 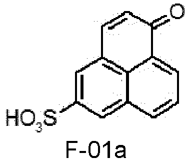 | Rötliches Pulver | Hergestellt gemäß Benniston, A.C und.Bunn, A.: J Chem. Res. 2010, 34(11), Seiten 603 - 605. |
| | MW: 260,28 g/mol | |
| | MF: C₁₃H₈O₃S | |
| 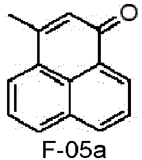 | Gelbes Pulver | Hergestellt gemäß Tavares, H.P. und Borger, J.P.: Canadian Journal of Chemistry 1966, 44(11), Seiten 1323 -1324. |
| | MW: 194,23 g/mol | |
| | MF: C₁₄H₁₀O | |

Folgende Curcumin Derivate wurden nach literaturbekannten Vorschriften hergestellt:

| | | |
|---|---|---|
| 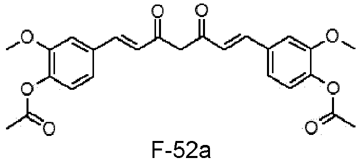 | | Hergestellt gemäß Reddy, A.S. et |
| | Gelbes Pulver | al.: Eur. J. Pharm. Biopharm. 2019, |
| | MW: 452,47 g/mol | 142, Seiten 518 - 530. |
| | MF: C₂₅H₂₄O₈ | |
| 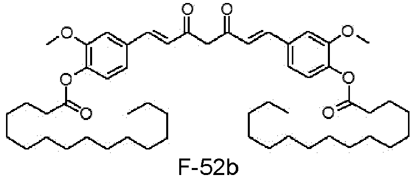 | | Hergestellt gemäß |
| | Oranger Feststoff | DE10245988B4 |
| | MW: 845,22 g/mol | |
| | MF: C₅₃H₈₀O₈ | |
| 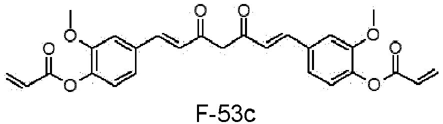 | | Hergestellt gemäß |
| | Oranger Feststoff | Patil, V.S. et al.: J. Nat. |
| | MW: 476,15 g/mol | Prod. 2017, 80(7), |
| | MF: C₂₇H₂₄O₈ | Seiten 1964 - 1971. |

Weiterhin wurde 9-Acetoxy-2,7,12,17-tetrakis(methoxyethyl)porphycen (ATMP) gemäß dem in der US 5,179,120 A beschriebenen Verfahren hergestellt.

| | |
|---|---|
| 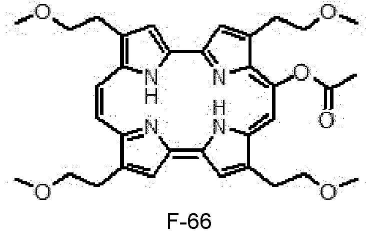 | CAS 147008-54-6 |
| | Dunkelblaues Pulver |
| | MW: 600,72 g/mol |
| | MF: C₃₄H₄₀N₄O₆ |

### Beispiel 1: Herstellung der verwendeten Photosensibilisatoren

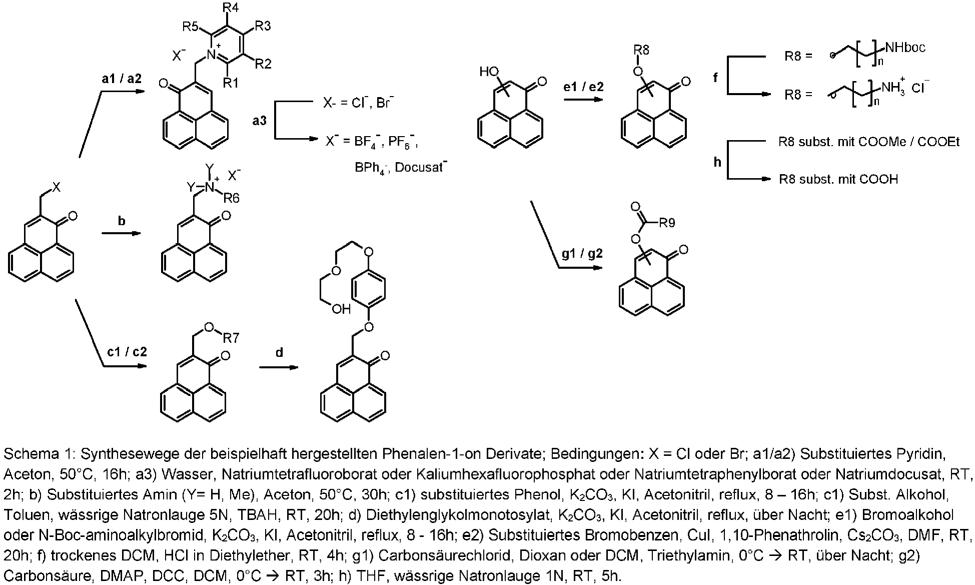

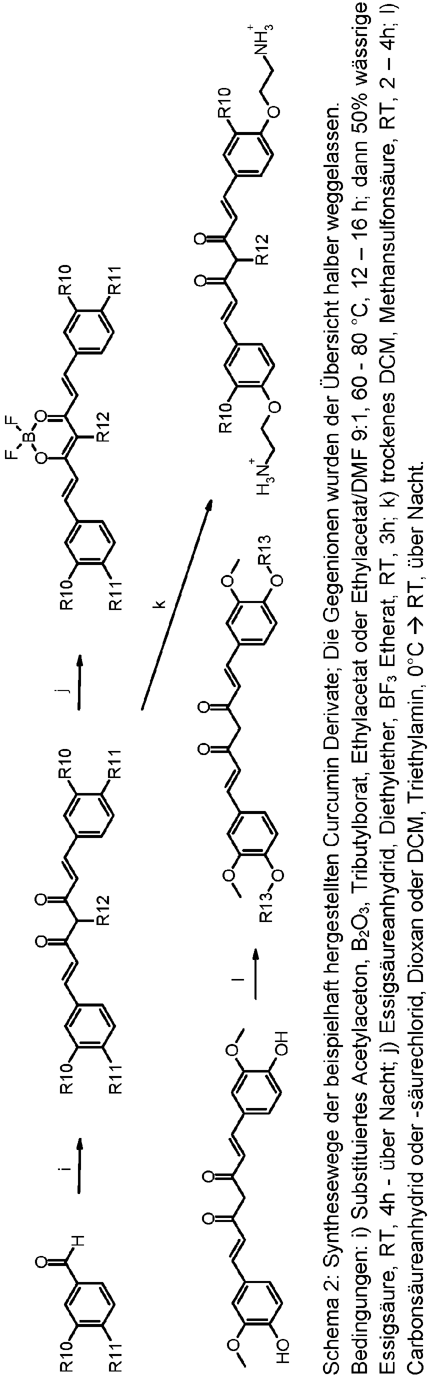

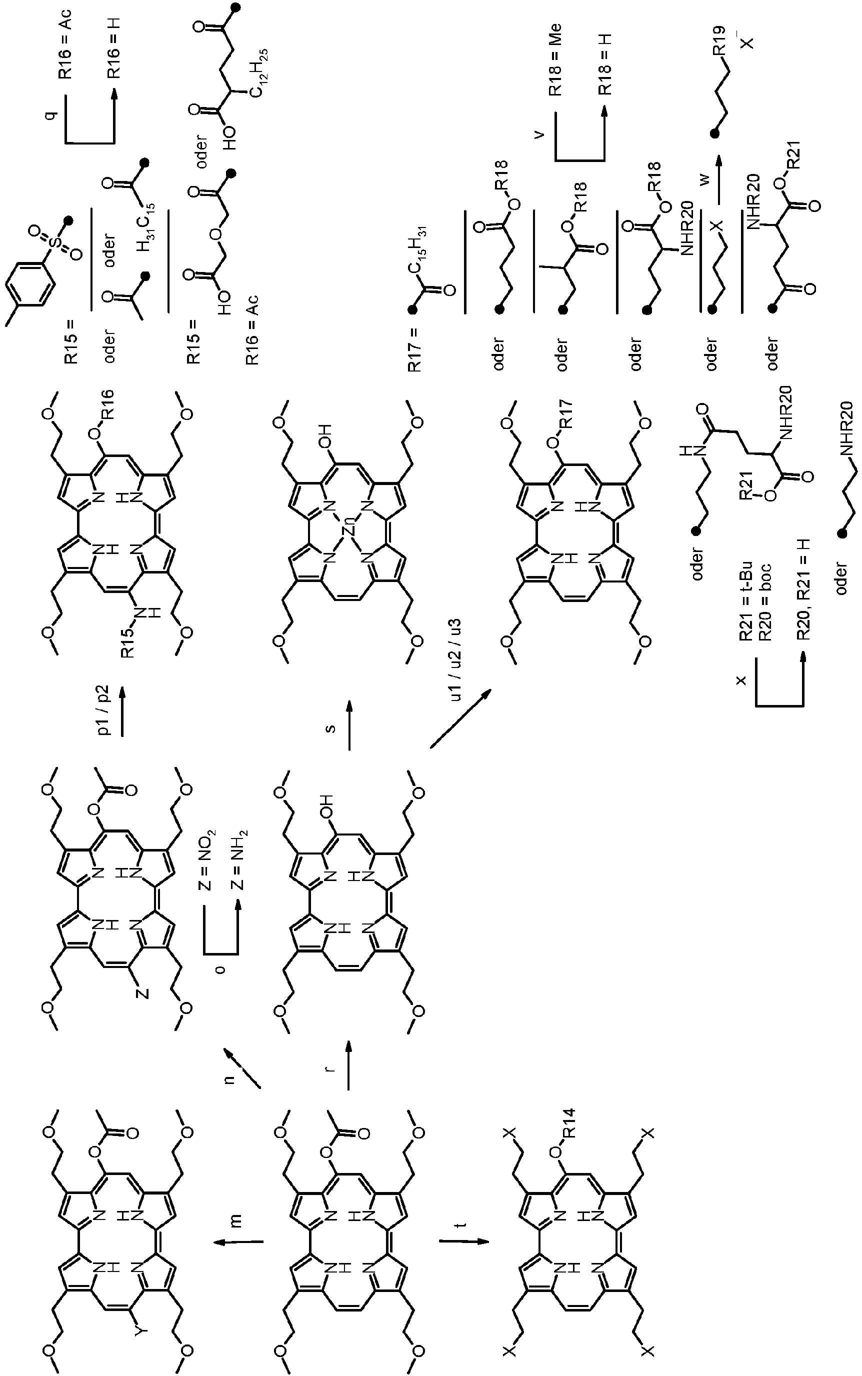

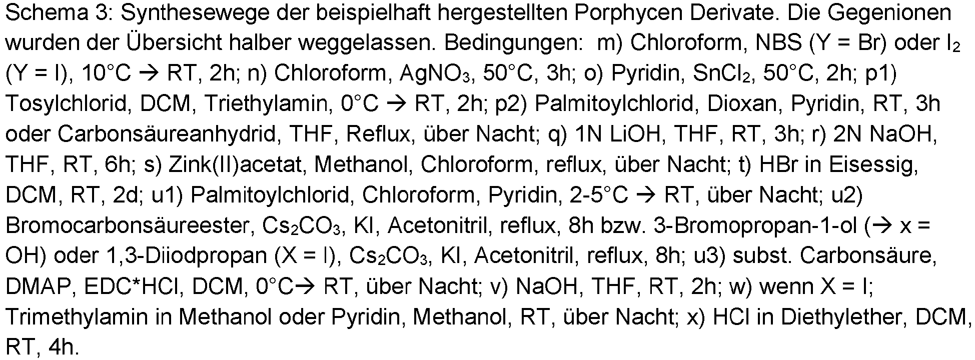

### Synthesevorschriften

### 1.1 Allgemeine Vorschrift a1)

2-Bromomethyl-1H-phenalen-1-on (135 mg, 0,5 mmol) wurde zusammen mit dem Pyridinbaustein (0,75 mmol) in Aceton (3 mL) vorgelegt. Die Lösung wurde 36h bei 50°C gerührt. Es entsteht eine gelbliche Suspension die nach Abkühlen auf Raumtemperatur auf zwei Bluecaps aufgeteilt wurde (nachwaschen / Transfer mit wenig Aceton, max. 2 mL). Das Produkt wurde durch Zugabe von Diethylether auf 15 mL pro Cap gefällt, abzentrifugiert (60 mins, 4400 rpm, 0°C) und der klare Überstand verworfen. Das Präzipitat wurde in Diethylether suspendiert, man ließ absetzen und verwarf den Überstand. Der Reinigungsschritt wurde noch einmal wiederholt und das Produkt anschließend bei vermindertem Druck getrocknet. Falls nötig konnte es durch Umkristallisation aus Isopropanol / Methylethylketon weiter gereinigt werden.

### 1.2 Allgemeine Vorschrift a2)

2-Chloromethyl-1H-phenalen-1-on (115 mg, 0,5 mmol) wurde zusammen mit dem Pyridinbaustein (0,75 mmol) in Aceton (3 mL) vorgelegt. Die Lösung wurde 48h bei 50°C gerührt. Die entstandene Suspension wurde auf zwei Bluecaps aufgeteilt (nachwaschen / Transfer mit wenig Aceton, max. 2 mL) und durch Zugabe von Diethylether auf 15 mL pro Cap gefällt. Das Produkt wurde abzentrifugiert (60 mins, 4400 rpm, 0°C) und der Überstand verworfen. Das Präzipitat wurde in Diethylether suspendiert. Man ließ absetzen und verwarf den Überstand. Der Reinigungsschritt wurde noch einmal wiederholt und das Produkt anschließend bei vermindertem Druck getrocknet. Falls nötig konnte es durch Säulenchromatographie mit Chloroform/Methanol 6:1 weiter gereinigt werden.

Die jeweils hergestellten Verbindungen sind nachfolgend in Tabelle 2 zusammengefasst.

**Tabelle 2: Mit allgemeiner Vorschrift a1) und a2) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Pyridin (CAS 110-86-1) 59,1 g/mol 45 mg | 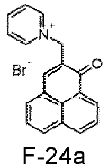 | 148 mg 91 % d.Th Gelbes Pulver | 352,3 g/mol C₁₉H₁₄NOBr | **¹H-NMR** (300 MHz, DMSO-d6): δ [ppm] = 9,27 (d, J = 7,2 Hz, 2H), 8,62 (m, 1H), 8,51 (m, 2H), 8,43 (s, 1H), 8,37 (m, 1H), 8,18 (m, 3H), 7,93 (t, J = 7,6 Hz, 1H), 7,82 (t, J = 8,0 Hz, 1H), 5,83 (s, 2H); - **MS** (ESI-MS, CH₂Cl₂/MeOH + 10 mmol NH₄OAc): e/z (%) = 272,11 (100%, M+); - **UV** (H₂O): λ/ [nm] (ε / [M⁻¹cm⁻¹]) = 255 (20600), 320 (3400), 360 - 420 (9900) |
| II | 2-(4-Pyridyl)-2-propanol (CAS 15031-78-4) 137,18 g/mol 103 mg | 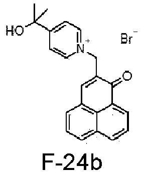 | 191 mg 93 % d.Th. Gelbes Pulver | 410,3 g/mol C₂₂H₂₀NO₂Br | **¹H-NMR** (400 MHz, DMSO-d₆): δ [ppm] = 9,21 (d, J = 8,0 Hz, 2H), 8,46 - 8,51 (m, 3H), 8,34 (d, J = 8,0 Hz, 1H), 8,21 (d, J = 7,2 Hz, 2H), 8,15 (d, J = 7,2 Hz, 1H), 7,90 (t, J = 7,6 Hz, 1H), 7,80 (t, J = 8,0 Hz, 1H), 5,81 (s, 2H), 1,50 (s, 6H); - **MS** (ESI-MS, CH₂Cl₂/MeOH + 10 mmol NH₄OAc): e/z (%) = 330,15 (100%, M+); - **UV** (H₂O): λ/ [nm] (ε / [M⁻¹cm⁻¹]) = 256 (20800), 320 (3300), 360 - 420 (9800) |
| III | 2-(4-Pyridyl)-2-propanol (CAS 15031-78-4) 137,18 g/mol 103 mg | 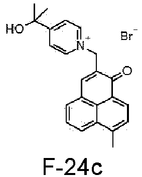 | 174 mg 82 % d.Th. Schmutzig gelbes Pulver | 424,33 g/mol C₂₃H₂₂NO₂Br | **¹H-NMR** (300 MHz, CD₃OD): δ [ppm] = 9,15 (d, J = 8,0 Hz, 2H), 8,45 - 8,52 (m, 2H), 8,38 (s, 1H), 8,17 (d, J = 8,0 Hz, 2H), 8,11 (d, J = 8,0 Hz, 1H), 7,80 (t, J = 8,0 Hz, 1H), 7,73 (d, J = 8,0 Hz, 1H), 5,75 (s, 2H), 2,90 (s, 3H), 1,58 (s, 6H); - **MS** (ESI-MS, 95% H₂O + 0,1% HCOOH / 5% MeCN + 0,1% HCOOH): e/z (%) = 344,16 (100%, M⁺); - **UV** (H₂O): λ/ [nm] (rel. [a.u.]) = 256 (1), 360 - 430 (0,52); |
| IV | Diphenyl(4-pyridyl)methanol (CAS 1620-30-0) 261,32 g/mol 196 mg | 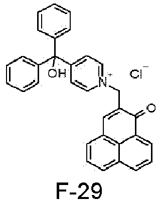 | 171 mg 70 % d.Th. Blassgelbes Pulver | 490 g/mol C₃₂H₂₄NO₂Cl | **¹H-NMR** (400 MHz, DMSO-d₆): δ [ppm] = 9,18 (d, J = 8,0 Hz, 2H), 8,44 - 8,56 (m, 3H), 8,36 (d, J = 8,6 Hz, 1H), 8,16 (d, J = 8,0 Hz, 1H), 8,02 (d, J = 8,0 Hz, 2H), 7,92 (t, J = 8,0 Hz, 1H), 7,81 (t, J = 8,0 Hz, 1H), 7,31 - 7,39 (m, 6H), 7,22 - 7,28 (m, 4H) 5,78 (s, 2H); - MS (ESI-MS, 95% H₂O + 0,1% HCOOH / 5% MeCN + 0,1% HCOOH): e/z (%) = 454,18 (100%, M⁺); - **UV** (H₂O): λ/ [nm] (rel. [a.u.]) = 254 (1), 360 - 425 (0,52); |
| VI | 4-tert-Butylpyridin (CAS: 3978-81-2) 135,21 g/mol 101 mg | 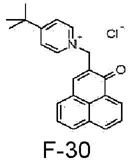 | 142 mg 78 % d.Th. Gelbes Pulver | 363,89 g/mol C₂₃H₂₂NOCl | **¹H-NMR** (400 MHz, DMSO-d₆): δ [ppm] = 9,17 (d, J = 7,2 Hz, 2H), 8,52 (t, J = 8,4 Hz, 2H), 8,46 (s, 1H), 8,37 (d, J = 8,4 Hz, 1H), 8,17 (m, 3H), 7,93 (t, J = 8,0 Hz, 1H), 7,82 (t, J = 7,2 Hz, 1H), 5,79 (s, 2H), 1,36 (s, 9H); - MS (ESI-MS, 95% H₂O + 0,1% HCOOH / 5% MeCN + 0,1% HCOOH): e/z (%) = 328,17 (100%, M⁺); |
| VII | 4-(1-Ethylpropyl)pyridin (CAS: 35182-51-5) 149,24 g/mol 112 mg | 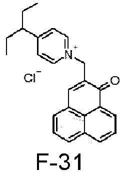 | 134 mg 71 % d.Th. Gelbes Pulver | 377,91 g/mol C₂₄H₂₄NOCl | **¹H-NMR** (400 MHz, DMSO-d₆): δ [ppm] = 9,16 (m, 2H), 8,49 (m, 3H), 8,34 (d, J = 8,4 Hz, 1H), 8,15 (d, J = 7,6 Hz, 1H), 8,03 (d, J = 6,8 Hz, 2H), 7,91 (t, J = 7,2 Hz, 1H), 7,81 (t, J = 8,4 Hz, 1H), 5,76 (s, 2H), 2,88 (sept., J = 4,2 Hz, 1H), 1,66 (m, 2H), 1,59 (m, 2H), 1,20 (m, 4H), 1,11 (m, 2H), 0,96 (m, 2H), 0,77 (t, J = 7,2 Hz, 6H); - MS (ESI-MS, 95% H₂O + 0,1% HCOOH / 5% MeCN + 0,1% HCOOH): e/z (%) = 342,19 (100%, M⁺); |
| VIII | 4-Nonan-5-ylpyridin (CAS: 2961-47-9) 205,34 g/mol 154 mg | 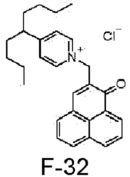 | 145 mg 67 % d.Th. Dunkelgelber Feststoff | 434,02 g/mol C₂₈H₃₂NOCl | **¹H-NMR** (400 MHz, DMSO-d₆): δ [ppm] = 9,16 (m, 2H), 8,49 (m, 3H), 8,34 (d, J = 8,4 Hz, 1H), 8,15 (d, J = 7,6 Hz, 1H), 8,03 (d, J = 6,8 Hz, 2H), 7,91 (t, J = 7,2 Hz, 1H), 7,81 (t, J = 8,4 Hz, 1H), 5,76 (s, 2H), 2,88 (sept., J = 4,2 Hz, 1H), 1,66 (m, 2H), 1,59 (m, 2H), 1,20 (m, 4H), 1,11 (m, 2H), 0,96 (m, 2H), 0,77 (t, J = 7,2 Hz, 6H); - **MS** (ESI-MS, 95% H₂O + 0,1% HCOOH / 5% MeCN + 0,1% HCOOH): e/z (%) = 398,25 (100%, M⁺); - **UV** (H₂O): λ/ [nm] (rel. [a.u.]) = 227 (1), 256 (0,27), 355 - 425 (0,13); |
| IX | 4-Phenylpyridin (CAS: 939-23-1) 155,20 g/m 116 mg | 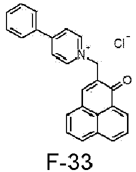 | 123 mg 64 % d.Th. Gelbes Pulver | 383,88 g/mol C₂₅H₁₈NOCl | **¹H-NMR** (400 MHz, DMSO-d₆): δ [ppm] = 9,30 (d, J = 6,5 Hz, 2H), 8,53 (m, 4H), 8,44 (s, 1H), 8,37 (d, J = 8,0 Hz, 1H), 8,17 (d, J = 6,8 Hz, 1H), 8,08 (m, 2H), 7,94 (t, J = 7,6 Hz, 1H), 7,82 (t, J = 7,6 Hz, 1H), 7,65 (m, 3H), 5,84 (s, 2H); - **MS** (ESI-MS, 95% H₂O + 0,1% HCOOH / 5% MeCN + 0,1% HCOOH): e/z (%) = 348,14 (100%, M⁺); |
| X | 4-Benzylpyridin (CAS: 2116-65-6) 169,23 g/mol 127 mg | 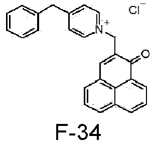 | 143 mg 72 % d.Th. Gelbes Pulver | 397,91 g/mol C₂₆H₂₀NOCl | **¹H-NMR** (400 MHz, DMSO-d₆): δ [ppm] = 9,16 (d, J = 6,8 Hz, 2H), 8,49 (m, 3H), 8,35 (d, J = 8,0 Hz, 1H), 8,15 (d, J = 6,8 Hz, 1H), 8,02 (d, J = 6,4 Hz, 1H), 7,92 (t, J = 7,6 Hz, 1H), 7,81 (t, J = 7,6 Hz, 1H), 7,35 (m, 4H), 7,27 (Sext., J = 4,4 Hz, 1H), 5,76 (s, 2H), 4,29 (s, 2H); - **MS** (ESI-MS, 95% H₂O + 0,1% HCOOH / 5% MeCN + 0,1% HCOOH): e/z (%) = 362,15 (100%, M⁺); |
| XI | 4-(3-Phenylpropyl) pyridin (CAS: 2057-49-0) 197,28 g/mol 148 mg | 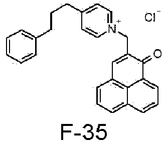 | 141 mg 66 % d.Th. Gelber Feststoff | 425,96 g/mol C₂₈H₂₄NOCl | **¹H-NMR** (400 MHz, DMSO-d₆): δ [ppm] = 9,16 (d, J = 7,2 Hz, 2H), 8,51 (m, 2H), 8,45 (s, 1H), 8,35 (d, J = 8,0 Hz, 1H), 8,15 (d, J = 6,4 Hz, 1H), 8,03 (t, J = 6,8 Hz, 1H), 7,92 (t, J = 7,6 Hz, 1H), 7,81 (t, J = 7,6 Hz, 1H), 7,21 (m, 5H), 5,78 (s, 2H), 2,91 (t, J = 7,6 Hz, 2H), 2,64 (t, J = 8,0 Hz, 2H), 1,98 (quint., J = 7,6 Hz, 2H); - **MS** (ESI-MS, 95% H₂O + 0,1% HCOOH / 5% MeCN + 0,1% HCOOH): e/z (%) = 390,19 (100%, M⁺); |
| XII | 2,5-Diphenylpyridin (CAS: 15827-72-2) 231,30 g/mol 173 mg | 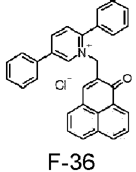 | 67 mg 29 % d.Th. Gelbes Pulver | 459,98 g/mol C₃₁H₂₂NOCl | **MS** (ESI-MS, 95% H₂O + 0,1% HCOOH / 5% MeCN + 0,1% HCOOH): e/z (%) = 424,17 (100%, M⁺); |
| XIII | 4-Phenoxypyridin (CAS: 4783-86-2) 171,20 g/mol 128 mg | 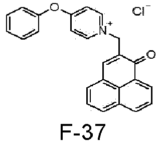 | 126 mg 63 % d.Th. Gelbes Pulver | 399,88 g/mol C₂₅H₁₈NO₂Cl | **¹H-NMR** (400 MHz, CD₃OD): δ [ppm] = 9,00 (d, J = 7,6 Hz, 2H), 8,43 (d, J = 7,2 Hz, 1H), 8,35 (s, 1H), 8,32 (d, J = 8,0 Hz, 1H), 8,19 (d, J = 8,0 Hz, 1H), 8,05 (d, J = 7,2 Hz, 1H), 7,77 (t, J = 7,6 Hz, 1H), 7,70 (t, J = 8,4 Hz, 1H), 7,54 (t, J = 7,6 Hz, 2H), 7,43 (m, 3H), 7,24 (d, J = 8,4 Hz, 2H), 5,63 (s, 2H); - **MS** (ESI-MS, 95% H₂O + 0,1% HCOOH / 5% MeCN + 0,1% HCOOH): e/z (%) = 364,13 (100%, M⁺); - **UV** (H₂O): λ/ [nm] (rel. [a.u.]) = 253 (1), 355 - 430 (0,46); |
| XIV | 4-Cyanopyridin (CAS: 100-48-1) 104,11 g/mol 78 mg | 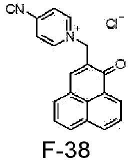 | 118 mg 71 % d.Th. Dunkelgelbes Pulver | 332,79 g/mol C₂₀H₁₃N₂OCl | **¹H-NMR** (400 MHz, DMSO-d₆): δ [ppm] = 9,59 (d, J = 6,8 Hz, 2H), 8,72 (d, J = 7,2 Hz, 2H), 8,54 (d, J = 8,4 Hz, 1H), 8,50 (d, J = 7,6 Hz, 1H), 8,47 (s, 1H), 8,37 (d, J = 8,0 Hz, 1H), 8,16 (d, J = 7,2 Hz, 1H), 7,94 (t, J = 7,6 Hz, 1H), 7,83 (t, J = 7,6 Hz, 1H), 5,92 (s, 2H); - **MS** (ESI-MS, 95% H₂O + 0,1% HCOOH / 5% MeCN + 0,1% HCOOH): e/z (%) = 297,10 (100%, M⁺); |
| XV | 4-Acetylpyridin (CAS: 1122-54-9) 121,14 g/mol 91 mg | 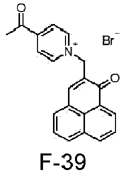 | 156 mg 79 % d.Th. Gelbes Pulver | 394,27 g/mol C₂₁H₁₆NO₂Br | **¹H-NMR** (400 MHz, DMSO-d₆): δ [ppm] = 9,46 (d, J = 6,8 Hz, 2H), 8,50 (m, 4H), 8,45 (s, 1H), 8,38 (d, J = 7,6 Hz, 1H), 8,19 (d, J = 6,4 Hz, 1H), 7,94 (t, J = 7,6 Hz, 1H), 7,84 (t, J = 7,6 Hz, 1H), 5,90 (s, 2H), 2,74 (s, 3H); - **MS** (ESI-MS, 95% H₂O + 0,1% HCOOH / 5% MeCN + 0,1% HCOOH): e/z (%) = 314,12 (100%, M⁺); |
| XVI | 4-Benzoylpyridin (CAS: 14548-46-0) 183,21 g/mol 137 mg | 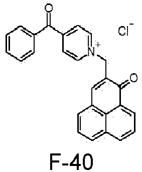 | 128 mg 62 % d.Th. Dunkelgelbes Pulver | 411,89 g/mol C₂₆H₁₈NO₂Cl | **¹H-NMR** (400 MHz, DMSO-d₆): δ [ppm] = 9,48 (d, J = 6,8 Hz, 2H), 8,55 (m, 3H), 8,38 (m, 3H), 8,19 (d, J = 6,8 Hz, 1H), 7,95 (t, J = 8,0 Hz, 1H), 7,82 (m, 4H), 7,62 (t, J = 8,0 Hz, 1H), 5,93 (s, 2H); - **MS** (ESI-MS, 95% H₂O + 0,1% HCOOH / 5% MeCN + 0,1% HCOOH): e/z (%) = 376,13 (100%, M⁺); |
| XVII | Methylisonicotinat (CAS: 2459-09-8) 137,14 g/mol 103 mg | 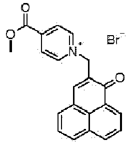 | 96 mg 47 % d.Th. Gelber Feststoff | 410,27 g/mol C₂₁H₁₆NO₃Br | **¹H-NMR** (400 MHz, DMSO-d₆): δ [ppm] = 9,48 (d, J = 6,4 Hz, 2H), 8,50 (m, 5H), 8,36 (d, J = 8,0 Hz, 1H), 8,17 (d, J = 6,8 Hz, 1H), 7,93 (t, J = 7,2 Hz, 1H), 7,82 (t, J = 8,0 Hz, 1H), 5,94 (s, 2H), 3,98 (s, 3H). - **MS** (ESI-MS, 95% H₂O + 0,1% HCOOH / 5% MeCN + 0,1% HCOOH): e/z (%) = 330,11 (100%, M⁺); |
| | | 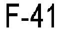 | | | |
| XVIII | Methyl 4-methoxypyridin-2-carboxylat (CAS: 29681-43-4) 167,16 g/mol 125 mg | 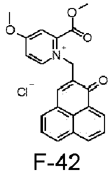 | 46 mg 23 % d.Th. Gelber Feststoff | 395,84 g/mol C₂₂H₁₈NO₄Cl | **MS** (ESI-MS, 95% H₂O + 0,1% HCOOH / 5% MeCN + 0,1% HCOOH): e/z (%) = 360,12 (100%, M⁺); - UV (H₂O): λ/ [nm] (rel. [a.u.]) = 253 (1), 360 - 430 (0,47); |
| XIX | Propyl 3-propoxypicolinat (CAS: 134319-22-5) 223,27 g/mol 167 mg | 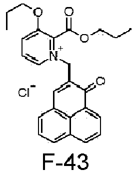 | 77 mg 34 % d.Th. Gelber Feststoff | 451,95 g/mol C₂₆H₂₆NO₄Cl | **¹H-NMR** (400 MHz, DMSO-d₆): δ [ppm] = 8,98 (d, J = 6,0 Hz, 1H), 8,50 (d, J = 7,2 Hz, 1H), 8,47 (d, J = 9,2 Hz, 1H), 8,44 (d, J = 8,0 Hz, 1H), 8,30 (d, J = 8,4 Hz, 1H), 8,16 (m, 3H), 7,88 (t, J = 8,0 Hz, 1H), 7,80 (t, J = 8,0 Hz, 1H), 5,88 (s, 2H), 4,28 (t, J = 6,0 Hz, 2H), 4,19 (t, J = 6,4 Hz, 2H), 1,82 (sext., J = 7,2 Hz, 2H), 1,36 (sext., J = 6,8 Hz, 2H), 1,02 (t, J = 6,7 Hz, 3H), 0,81 (t, J = 6,7 Hz, 3H); - **MS** (ESI-MS, 95% H₂O + 0,1% HCOOH / 5% MeCN + 0,1% HCOOH): e/z (%) = 416,19 (100%, M⁺); |
| XX | Pyridin-3,5-dicarbonsäure diethylester (CAS: 4591-56-4) 223,23 g/mol 167 mg | 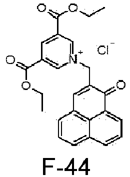 | 93 mg 41 % d.Th. Blassgelbes Pulver | 451,91 g/mol C₂₅H₂₂NO₅Cl | **¹H-NMR** (400 MHz, CD₃OD): δ [ppm] = 9,94 (s, 2H), 9,36 (s, 1H), 8,59 (d, J = 7,2 Hz, 1H), 8,52 (s, 1H), 8,44 (d, J = 7,6 Hz, 1H), 8,30 (d, J = 8,4 Hz, 1H), 8,16 (d, J = 6,8 Hz, 1H), 7,89 (t, J = 7,6 Hz, 1H), 7,79 (t, J = 7,6 Hz, 1H), 5,99 (s, 2H), 4,55 (qart, J = 7,2 Hz, 4H), 1,46 (t, J = 7,2 Hz, 6H); - **MS** (ESI-MS, 95% H₂O + 0,1% HCOOH / 5% MeCN + 0,1% HCOOH): e/z (%) = 416,15 (100%, M⁺); - **UV** (H₂O): λ/ [nm] (rel. [a.u.]) = 254 (1), 350 - 425 (0,55); |
| XXI | 2-(Pyridin-3-carbonylamino) essigsäure (CAS 583-08-4) 180,16 mg 135 mg | 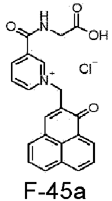 | 106 mg 52 % d.Th. Gelbes Pulver | 408,84 g/mol C₂₂H₁₇N₂O₄Cl | **¹H-NMR** (400 MHz, DMSO-d6): δ [ppm] = 9,63 (s, 1H), 9,58 (m, 1H), 9,40 (d, J = 7,2 Hz, 1H), 8,98 (d, J = 7,9 Hz, 1H), 8,52 (t, J = 7,6 Hz, 2H), 8,43 (s, 1H), 8,37 (d, J = 7,9 Hz, 1H), 8,30 (m, 1H), 8,17 (d, J = 7,6 Hz, 1H), 7,93 (t, 7,6 Hz, 1H), 7,81 (t, 7,6 Hz, 1H), 5,88 (s, 2H), 4,03 (d, J = 4,8 Hz, 2H); - **MS** (ESI-MS, 95% H₂O + 0,1% HCOOH / 5% MeCN + 0,1% HCOOH): e/z (%) = 373,12 (100%, M⁺), 745,23 (2%, (2M⁺-H⁺)⁺); - **UV** (H₂O): λ/ [nm] (rel. [a.u.]) = 256 (1), 320 (0,3), 360 - 420 (0,52); |
| XXII | Pyridin-3-sulfonsäure ⁽¹⁾ (CAS 636-73-7) 159,17 g / mol 120 mg | 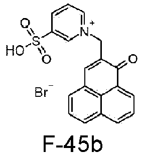 | 162 mg 75 % d.Th. Blassgelbes Pulver | 432,29 g/mol C₁₉H₁₄NO₄SBr | **¹H-NMR** (400 MHz, DMSO-d6): δ [ppm] = 9,41 (s, 1H), 9,18 (d, J = 7,2 Hz, 1H), 8,70 (d, J = 7,9 Hz, 1H), 8,52 (m, 2H), 8,43 (s, 1H), 8,36 (d, J = 7,8 Hz, 1H), 8,19 (d, J = 7,6 Hz, 1H), 8,12 (m, 1H), 7,92 (t, 7,6 Hz, 1H), 7,81 (t, 7,6 Hz, 1H), 5,85 (s, 2H); - **MS** (ESI-MS, 95% H₂O + 0,1% HCOOH / 5% MeCN + 0,1% HCOOH): e/z (%) = 352,06 (100%, M⁺), 703,12 (5%, (2M⁺-H⁺)⁺); - **UV** (H₂O): λ/ [nm] (rel. [a.u.]) = 252 (1), 322 (0,26), 360 - 420 (0,54); |
| XXII | 6-isopropylquinolin (CAS: 135-79-5) 171,24 g/mol 128 mg | 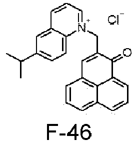 | 162 mg 81 % d.Th. Gelbes Pulver | 399,92 g/mol C₂₆H₂₂NOCl | ¹H-NMR (400 MHz, DMSO-d₆): δ [ppm] = 9,80 (d, J = 5,6 Hz, 1H), 9,32 (d, J = 8,4 Hz, 1H), 8,58 (d, J = 8,4 Hz, 1H), 8,52 (m, 2H), 8,36 (s, 1H), 8,31 (d, J = 7,6 Hz, 1H), 8,26 (m, 1H), 8,16 (m, 2H), 8,04 (d, J = 6,8 Hz, 1H), 7,93 (t, J = 7,6 Hz, 1H), 7,75 (t, J = 8,0 Hz, 1H), 6,30 (s, 2H), 3,19 (sept., J = 6,8 Hz, 1H), 1,30 (d, J = 6,8 Hz, 6H); - MS (ESI-MS, 95% H₂O + 0,1% HCOOH / 5% MeCN + 0,1% HCOOH): e/z (%) = 364,17 (100%, M⁺); - UV (H₂O): λ/ [nm] (rel. [a.u.]) = 246 (1), 322 (0,24), 355 - 425 (0,22); |
| XXIII | 5-Bromoisoquinolin (CAS: 34784-04-8) 208,06 g/mol 156 mg | 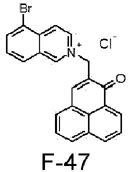 | 162 mg 74 % d.Th. Dunkelgelbes Pulver | 436,74 g/mol C₂₃H₁₅BrNOCl | **¹H-NMR** (400 MHz, DMSO-d₆): δ [ppm] = 10,44 (s, 1H), 9,08 (d, J = 6,4 Hz, 1H), 8,62 (m, 3H), 8,50 (m, 3H), 8,36 (d, J = 8,0 Hz, 1H), 8,17 (d, J = 6,8 Hz, 1H), 7,98 (t, J = 8,4 Hz, 1H), 7,92 (t, J = 8,0 Hz, 1H), 7,82 (t, J = 8,0 Hz, 1H), 6,01 (s, 2H); - **MS** (ESI-MS, 95% H₂O + 0,1% HCOOH / 5% MeCN + 0,1% HCOOH): e/z (%) = 400,03 (100%, M⁺); - **UV** (H₂O): λ/ [nm] (rel. [a.u.]) = 251 (1), 350 - 430 (0,23); |
| XXIV | 3-Palmitoyloxypyridin ⁽²⁾ 333,27 g/mol 250 mg | 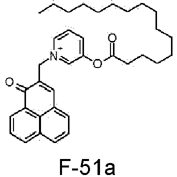 | 143 mg 51 % d.Th. Blassgelber Feststoff | 562,2 g/mol C₃₅H₄₄NO₃Cl | **¹H-NMR** (600 MHz; CDCl3): δ [ppm] = 9,37 (s, 1H), 8,56 (d, J = 7,3 Hz, 1H), 8,38 (m, 1H), 8,23 (m, 2H), 8,18 (d, J = 7,2 Hz, 1H), 8,07 (d, J = 7,8 Hz, 1H), 8,05 (m, 1H), 7,98 (m, 1H), 7,73 (t, J = 7,8 Hz, 1H), 7,66 (t, J = 7,8 Hz, 1H), 6,26 (s, 2H), 2,33 (t, J = 7,8 Hz, 2H), 1,38 (m, 2H), 1,24 (m, 24H), 0,85 (t, J = 7,2 Hz, 3H). - **MS** (ESI-MS, 95% H₂O + 0,1% HCOOH / 5% MeCN + 0,1% HCOOH): e/z (%) = 526,33 (100%, M⁺); - **UV** (H₂O): λ/ [nm] (rel. [a.u.]) = 228 (1), 251 (0,56), 350 - 430 (0,22); |
| XXV | 3-(Palmitoyl-oxymethyl)pyridin (3) 347,28 g/mol 260 mg | 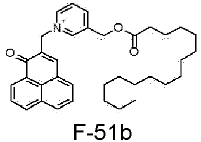 | 135 mg 47 % d.Th. Blassgelber Feststoff | 575,89 g/mol C₃₆H₄₆NO₃Cl | **¹H-NMR** (600 MHz; CDCl3): δ [ppm] = 9,84 (m, 2H), 9,36 (s, 1H), 8,52 (d, J = 7,2 Hz, 1H), 8,33 (d, J = 7,8 Hz, 1H), 8,22 (d, J = 7,2 Hz, 1H), 8,17 (d, J = 7,2 Hz, 1H), 8,08 (d, J = 7,8 Hz, 1H), 7,75 (t, J = 7,8 Hz, 1H), 7,66 (t, J = 7,8 Hz, 1H), 6,23 (s, 2H), 5,34 (s, 2H), 2,40 (t, J = 7,8 Hz, 2H), 1,60 (m, 2H), 1,22 (m, 24H), 0,86 (t, J = 7,2 Hz, 3H). - **MS** (ESI-MS, 95% H₂O + 0,1% HCOOH / 5% MeCN + 0,1% HCOOH): e/z (%) = 540,35 (100%, M⁺); - **UV** (H₂O): λ/ [nm] (rel. [a.u.]) = 253 (1), 350 - 425 (0,46); |

(1) Eingesetzt als Natriumsalz unter Zugabe von 0,3 mL Methanol.
(2) Dieser Ausgangsstoff wurde durch Acylierung von 3-Hydroxypyridin mit Palmitoylchlorid in Dioxan/Pyridin erhalten. Weißer, wachsartiger Feststoff (77% d.Th.); ¹H-NMR (400 MHz, CDC1₃): δ[ppm] = 0,88 (t, 3H), 1,22 - 1,45 (m, 24H), 1,74 (m, 2H), 2,59 (t, 2H), 7,34 (m, 1H), 7,48 (m, 1H), 8,42 (m, 1H), 8,48 (m, 1H);
(3) Dieser Ausgangsstoff wurde durch Acylierung von 3-(Hydroxymethyl)pyridin mit Palmitoylchlorid in Dioxan/Pyridin erhalten. Weißer, wachsartiger Feststoff (72% d.Th.); ¹H-NMR (400 MHz, CDC1₃): δ[ppm] = 0,89 (t, 3H), 1,22 - 1,38 (m, 24H), 1,64 (m, 2H), 2,34 (m, 2H), 5,16 (s, 2H), 7,33 (m, 1H), 7,72 (m, 1H), 8,58 (m, 1H), 8,64 (s, 1H);

### 1.3 Allgemeine Vorschrift a3)

Eine wässrige Lösung von Verbindung F-24b (0,5 mol/L, 6 mL, 3 mmol) wurde vorgelegt. Unter Rühren wurde die Salzlösung langsam zugetropft:
a) Natriumtetrafluoroborat (CAS: 13755-29-8), gesättigte wässrige Lösung, 0,6 mL
b) Kaliumhexafluorophosphat (CAS: 17084-13-8), 0,5 molare wässrige Lösung, 12 mL
c) Natriumtetraphenylborat (CAS: 143-66-8), 0,05 molare wässrige Lösung, 120 mL
d) Natriumdocusat (CAS: 577-11-7), 1 molare Lösung in 30% wässrigem Ethanol, 6 mL

Es wurde 15 min gerührt, danach ließ man den entstandenen Niederschlag absetzen.
I) Der Niederschlag wurde abgesaugt und mit eiskaltem destilliertem Wasser gewaschen. Es wurde an der Luft bis zur Gewichtskonstanz getrocknet.
II) Der Niederschlag wurde abzentrifugiert und mit destilliertem Wasser gewaschen. Das Produkt wurde bei vermindertem Druck getrocknet.

Die jeweils hergestellten Verbindungen sind nachfolgend in Tabelle 3 zusammengefasst.

### 1.4 Allgemeine Vorschrift b)

2-Chloromethyl-1H-phenalen-1-on (115 mg, 0,5 mmol) wurde zusammen mit dem substituierten Dimethylamin (2 mmol) in N,N-Dimethylformamid (0,8 mL) vorgelegt. Die Lösung wurde 48 h bei 50°C im Dunklen gerührt. Der Ansatz wurde auf zwei Bluecaps aufgeteilt (nachwaschen / Transfer mit wenig Dichlormethan, max. 2 mL) und durch Zugabe von Diethylether auf 15 mL pro Cap gefällt. Das Produkt wurde abzentrifugiert (60 mins, 4400 rpm, 0°C) und der Überstand verworfen. Das Präzipitat wurde in Diethylether suspendiert. Man ließ absetzen und verwarf den Überstand. Der Reinigungsschritt wurde zweimal wiederholt und das Produkt anschließend getrocknet. Falls nötig konnte es durch Säulenchromatographie mit Chloroform/Methanol Gemisch weiter gereinigt werden.

Die jeweils hergestellten Verbindungen sind nachfolgend in Tabelle 4 zusammengefasst.

### 1.5 Allgemeine Vorschrift c1)

2-Bromomethyl-1H-phenalen-1-on (272 mg, 1,0 mmol) wurde in DMF (2 mL) vorgelegt. Der Alkohol (2,0 mmol), Kaliumcarbonat (276 mg, 2,0 mmol) und Kaliumiodid (166 mg, 1,0 mmol) wurden zugegeben. Der Ansatz wurde über Nacht bei 60°C unter Stickstoff gerührt. Nach Abkühlen auf Raumtemperatur wurde mit 30 mL Ethylacetat verdünnt und mehrmals mit Wasser und gesättigter Kochsalzlösung (3-4 mal, 20 mL) ausgeschüttelt. Die organische Phase wurde abgetrennt und über Magnesiumsulfat getrocknet. Nach Filtration und Einengen unter vermindertem Druck wurde das Rohprodukt durch Säulenchromatographie mit Ethylacetat/ Petrolether Gemisch gereinigt.

Die jeweils hergestellten Verbindungen sind nachfolgend in Tabelle 5 zusammengefasst.

### 1.6 Allgemeine Vorschrift d)

Verbindung S-12 (151 mg, 0,5 mmol) wurde in Acetonitril (3 mL) vorgelegt. Diethylenglykolmonotosylat (260mg, 1,0 mmol), Kaliumcarbonat (276 mg, 2,0 mmol) und Kaliumiodid (166 mg, 1,0 mmol) wurden zugegeben. Der Ansatz wurde über Nacht bei 60°C unter Stickstoff gerührt. Nach Abkühlen auf Raumtemperatur wurde mit 30 mL Ethylacetat verdünnt und mehrmals mit Wasser und gesättigter Kochsalzlösung (3 mal, 20 mL) ausgeschüttelt. Die organische Phase wurde abgetrennt und über Magnesiumsulfat getrocknet. Nach Filtration und Einengen unter vermindertem Druck wurde das Rohprodukt zunächst durch wiederholte Flashchromatographie mit Ethylacetat / Dichlormethan 1:1, dann mit Ethylacetat/ Petrolether 1:3 → 2:1 gereinigt.

Die jeweils hergestellten Verbindungen sind nachfolgend in Tabelle 6 zusammengefasst.

### 1.7. Allgemeine Vorschrift e1)

Das Hydroxy-phenalen-1-on (98 mg, 0,5 mmol) wurde in DMF (3 mL) vorgelegt. Das entsprechende Bromoalkanol bzw. boc-geschützte Aminoalkylbromid (0,75 mmol), Kaliumcarbonat (276 mg, 2,0 mmol) und Kaliumiodid (83 mg, 0,5 mmol) wurden zugegeben. Der Ansatz wurde über Nacht bei 60°C unter Stickstoff gerührt. Nach Abkühlen auf Raumtemperatur wurde mit 30 mL Ethylacetat verdünnt und mehrmals mit Wasser und gesättigter Kochsalzlösung (3 mal, 20 mL) ausgeschüttelt. Die organische Phase wurde abgetrennt und über Magnesiumsulfat getrocknet. Nach Filtration und Einengen unter vermindertem Druck wurde das Rohprodukt durch Säulenchromatographie mit Ethylacetat/ Petrolether Gemisch gereinigt.

Die jeweils hergestellten Verbindungen sind nachfolgend in Tabelle 7 zusammengefasst.

### 1.8 Vorschrift f) Entschützung der Boc-Gruppe

Das in Tabelle 7 gezeigte boc-geschützte Phenalen-1-on Derivat (0,2 mmol) wurde in trockenem Dichlormethan (3 mL pro 100 mg) vorgelegt. Eine gesättigte Lösung von Chlorwasserstoff in Diethylether (0,5 mL pro mmol Boc-Gruppe) wurde zugetropft. Der Ansatz wurde 3h unter Feuchtigkeitsausschluss gerührt. Das Produkt wurde durch Zugabe von 30 mL getrocknetem Diethylether gefällt. Der Niederschlag wurde abzentrifugiert und mit Diethylether gründlich gewaschen. Das Produkt wurde bei vermindertem Druck getrocknet.

Die jeweils hergestellten Verbindungen sind nachfolgend in Tabelle 8 zusammengefasst.

### 1.9 Allgemeine Vorschrift e2)

In einem getrockneten Rundkolben wurde 3-Hydroxy-phenalen-1-on (196 mg, 1 mmol), das substituierte Bromobenzen (1 mmol) und 1,10-Phenanthrolin (36 mg, 0,2 mmol) in 5 mL trockenem DMF unter Stickstoff vorgelegt. Cäsiumcarbonat (486 mg, 1,5 mmol) und Kupfer(l)iodid (40 mg) wurden zugegeben. Der Ansatz wurde bei Raumtemperatur 20h gerührt. Das Reaktionsgemisch wurde mit Ethylacetat (25 mL) verdünnt, mit gesättigter wässriger Kochsalzlösung (10 mL) und Wasser (10 mL) gewaschen. Die organische Phase wurde über MgSO₄ getrocknet, gefiltert und einrotiert. Das Rohprodukt wurde durch Flash-Chromatographie an Kieselgel mit Ethylacetat / Petrolether Gemischen gereinigt.

Die jeweils hergestellten Verbindungen sind nachfolgend in Tabelle 9 zusammengefasst.

### 1.10 Allgemeine Vorschrift g1)

Das Hydroxy-phenalen-1-on (98 mg, 0,5 mmol) wurde zusammen mit Pyridin (0,55 mL, 0,54 g, 6 mmol) in trockenem Dioxan (3 mL) in einem 5 mL Rundkolben mit Septum unter Stickstoff vorgelegt. Palmitoylchlorid (330 mg, 1,2 mmol) in 1 mL trockenem Dioxan wurde langsam mittels einer Spritze über das Septum unter Kühlung zugetropft. Das Eisbad wurde entfernt und das Reaktionsgemisch über Nacht bei Raumtemperatur gerührt. Es wurde durch Zugabe von 30 mL Petrolether gefällt. Der Niederschlag wurde abfiltriert und in 10 mL Ethylacetat gelöst. Die organische Phase wurde zweimal mit 0,1 N Salzsäure und nachfolgend mit Natriumcarbonatlösung gewaschen, über Natriumsulfat getrocknet und eingeengt. Das erhaltene Rohprodukt wurde durch Säulenchromatographie an Kieselgel mit Petrolether / Ethylacetat Gemischen als Eluent gereinigt.

Die jeweils hergestellten Verbindungen sind nachfolgend in Tabelle 10 zusammengefasst.

### 1.11.1 Allgemeine Vorschrift g2)

3-Hydroxy-phenalen-1-on (196 mg, 1 mmol) wurde zusammen mit der Carbonsäure (1 mmol) und DMAP (122 mg, 1 mmol) in Dichlormethan (4 mL) vorgelegt. Dicyclohexylcarbodiimid (206 mg, 1 mmol) in 1 mL Dichlormethan wurde langsam mittels einer Spritze über das Septum unter Kühlung zugetropft. Das Eisbad wurde entfernt und das Reaktionsgemisch 3h bei Raumtemperatur gerührt. Der Ansatz wurde mit 20 mL Ethylacetat verdünnt und filtriert. Die organische Phase wurde zweimal mit 0,1 N Salzsäure (20 mL) und nachfolgend gesättigter Kochsalzlösung (20 mL) gewaschen, über MgSO₄ getrocknet und einrotiert. Das erhaltene Rohprodukt wurde durch Säulenchromatographie an Kieselgel mit Petrolether / Ethylacetat Gemischen als Eluent gereinigt.

Die jeweils hergestellten Verbindungen sind nachfolgend in Tabelle 11 zusammengefasst.

### 1.11.2 Allgemeine Vorschrift h) Entschützung des Esters

Der in Tabelle 11 gezeigte Ester (0,2 mmol) wurde in THF (2 mL) gelöst. Wässrige Natriumhydroxidlösung (0,4 mL, 1 N, 2 Äquivalente) wurde in einer Portion zugegeben. Nach 5 h Rühren bei RT wurde mit Dichlormethan verdünnt (10 mL). Die Lösung wurde im Scheidetrichter mit gesättigter Kochsalzlösung (2x 10 mL), 1 N HCl (10 mL) und Wasser (10 mL) gewaschen. Die organische Phase wurde getrocknet (MgSO₄), filtriert und unter reduziertem Druck eingeengt.

Die jeweils hergestellten Verbindungen sind nachfolgend in Tabelle 12 zusammengefasst.

### 1.12.1 Allgemeine Vorschrift i)

Das substituierte Acetylaceton (2,0 mmol) wurde zusammen mit Boroxid (139 mg, 2,0 mmol) in 4 mL trockenem Ethylacetat vorgelegt und 30min bei 60°C gerührt. Anschließend wurden das substituierte Aldehyd (4,2 mmol) und Tributylborat (920 mg, 4,0 mmol) nacheinander zugeben und der Ansatz für weitere 30 min gerührt. Im nächsten Schritt wurde 2-Methoxyethylamin (180 mg, 2,4 mmol) gelöst in 2 mL Ethylacetat langsam zugetropft. Der Ansatz wurde 12 bis 16h bei 80°C (DC Kontrolle Reaktionsfortschritt), dann bei Raumtemperatur für 6h gerührt. Es wurden 30 mL 50%ige wässrige Essigsäure zugeben und bis zur vollständigen Hydrolyse des Komplexes gerührt (DC Kontrolle; mind. 4h oder bis über Nacht). Die Lösungsmittel wurden bei vermindertem Druck am Rotationsverdampfer abgezogen.

Aufarbeitung I) Der Rückstand wurde in Ethylacetat (ca. 30 mL) aufgenommen und filtriert. Die organische Lösung wurde mehrmals mit wässriger Kochsalzlösung gewaschen (2-3xje 30 mL), über Magnesiumsulfat getrocknet und einrotiert. Das Rohprodukt wurde durch Säulenchromatographie an Kieselgel mit Petrolether / Aceton Gemischen gereinigt.

Aufarbeitung II) Der Rückstand wurde mit Wasser (2x 10 mL) ausgelaugt und bei vermindertem Druck getrocknet. Anschließend wurde Ethylacetat (ca. 10 mL) suspendiert, über eine Fritte abgesaugt und mit kaltem Ethylacetat und Petrolether gewaschen.

Die jeweils hergestellten Verbindungen sind nachfolgend in Tabelle 13 zusammengefasst.

### 1.12.2 Allgemeine Vorschrift j) Komplexierung mit BF-Gruppe

Verbindung F-55a (0,2 mmol, 118 mg) wurde in einer Mischung aus Essigsäureanhydrid (3 mL) und Diethylether (1,5 mL) vorgelegt. Bortrifluorid Etherat (0,5 mL) wurde unter Feuchtigkeitsausschluss (Septum) bei 0°C unter Rühren langsam zugetropft. Der Ansatz wurde über Nacht in den Tiefkühler gestellt. Das auskristallisierte Produkt wurde abgesaugt, mit eiskaltem Diethylether gewaschen und an der Luft getrocknet

Die jeweils hergestellten Verbindungen sind nachfolgend in Tabelle 14 zusammengefasst.

### 1.12.3 Allgemeine Vorschrift k) Entschützung der Boc-Gruppe

Das entsprechende in Tabelle 13 gezeigte boc-geschützte Curcuminderivat (0,2 mmol) wurde in Dichlormethan/Methanol 8:1 (trockene Lösungsmittel, 3 mL pro 100 mg) in einem Kolben mit Septum vorgelegt. Eine 40%ige Lösung von Methansulfonsäure in DCM (0,5 mL pro mmol Boc-Gruppe) enthaltend 30 mg Triisopropylsilan wurde unter Kühlung über Kanüle/Septum zugetropft. Der Ansatz wurde 2 h unter Feuchtigkeitsausschluss und Lichtschutz gerührt. Das Produkt wurde durch Zugabe von 30 mL trockenem Diethylether gefällt. Der Niederschlag wurde abzentrifugiert und mit trockenem Diethylether gründlich gewaschen. Der Niederschlag wurde in möglichst wenig Methanol gelöst und in den 10fachen Überschuss getrocknetes Ethylacetat eingetropft. Der entstandene Niederschlag wurde abzentrifugiert und zweimal mit wenig trockenem Diethylether gewaschen.

Die jeweils hergestellten Verbindungen sind nachfolgend in Tabelle 15 zusammengefasst.

### 1.13 Allgemeine Vorschrift I)

Curcumin (148 mg, 0,4 mmol) wurde zusammen mit DIPEA (94 mg, 0,8 mmol) in trockenem Tetrahydrofuran (4 mL) in einem 10 mL Rundkolben mit Septum unter Stickstoff vorgelegt. Das Säurechlorid (1 mmol) in 1 mL trockenem Tetrahydrofuran wurde langsam mittels einer Spritze über das Septum bei ca. 0°C zugetropft. Das Eisbad wurde entfernt und das Reaktionsgemisch über Nacht bei Raumtemperatur gerührt. Der Ansatz wurde filtriert. Der Filterkuchen wurde mit EE nachgewaschen und anschließend alle flüchtigen Komponenten bei vermindertem Druck abgezogen. Der Rückstand wurde in Ethylacetat aufgenommen (20 mL) und mit 0,1M K₂CO₃ Lösung, 0,1M wässriger HCl und Wasser gewaschen. Die organische Phase wurde mit MgSO₄ getrocknet, filtriert und eingeengt. Das Rohprodukt wurde durch Säulenchromatographie an Kieselgel mi PE/Aceton 5:1 --> 2:1 gereinigt.

Die jeweils hergestellten Verbindungen sind nachfolgend in Tabelle 16 zusammengefasst.

### 1.14 Allgemeine Vorschrift m)

Eine Lösung von 9-Acetoxy-2,7,12,17-tetra-(beta-methoxyethyl)porphycen (0,06 g, 0,1 mmol) wurden in Dichlormethan (50 mL) mit NBS (35 mg, 0,2 mmol, für Bromierung) oder lod (51 mg, 0,2 mmol, für lodierung) für 3h bei RT gerührt. Es wurde mit verd. Kaliumiodidlösung (0,5%ig, 30 mL), gesättigter Kochsalzlösung (2 × 60 mL) und Wasser (60 mL) gewaschen. Die organische Phase wurde getrocknet (MgSO₄), filtriert und unter reduziertem Druck eingeengt. Das Produkt wurde durch wiederholte Flash-Chromatographie an Kieselgel mit Dichlormethan/Ethylacetat 2:1 als Laufmittel gereinigt

Die jeweils hergestellten Verbindungen sind nachfolgend in Tabelle 17 zusammengefasst.

### 1.15 Allgemeine Vorschrift n)

Eine Lösung von 9-Acetoxy-2,7,12,17-tetra-(beta-methoxyethyl)porphycen (0,12 g, 0,2 mmol) und Silbernitrat (612 mg, 3,6 mmol) in Dichlormethan (40 mL) und Essigsäure (40 mL) wurde bei 50°C gerührt. Bedingung I) für 3 h bzw. Bedingung II) über Nacht

Nach Abkühlen auf Raumtemperatur wurde das Gemisch mit Ethylacetat (40 mL) verdünnt, in einen Scheidetrichter überführt und mit gesättigter Kochsalzlösung (2 × 40 mL) und Wasser (40 mL) gewaschen. Die organische Phase wurde getrocknet (MgSO₄), filtriert und unter reduziertem Druck eingeengt. Das Produkt wurde durch Flash-Chromatographie an Kieselgel mit Dichlormethan/Ethylacetat 1:1 als Laufmittel gereinigt. Der Feststoff wurde in Petrolether suspendiert, abgesaugt und an der Luft bis zur Gewichtskonstanz getrocknet

Die jeweils hergestellten Verbindungen sind nachfolgend in Tabelle 18 zusammengefasst.

### 1.16 Allgemeine Vorschrift o)

Zu einer Lösung von 19-Nitro-9-Acetoxy-2,7,12,17-tetra-(beta-methoxyethyl)porphycen (0,13 g, 0,2 mmol) in Pyridin (6 mL) wurde Zinn(II)chlorid Dihydrat (270 mg, 1,2 mmol) zugegeben und anschließend der Ansatz unter Stickstoff für 1h bei 110°C gerührt. Nach Abkühlen auf Raumtemperatur wurde das Rohprodukt durch Zugabe von 50 mL Petrolether gefällt und der Überstand verworfen. Der Niederschlag wurde mit Dichlormethan (3x 15 mL) ausgelaugt, die vereinigten organischen Extrakte filtriert und in einen Scheidetrichter überführt. Es wurde mit gesättigter Kochsalzlösung (2 × 30 mL) und Wasser (30 mL) gewaschen. Die organische Phase wurde getrocknet (MgSO₄), filtriert und unter reduziertem Druck eingeengt. Der Feststoff wurde in Petrolether suspendiert, abgesaugt und bei vermindertem Druck getrocknet. Das Produkt wurde ohne weitere Reinigung direkt weiter umgesetzt.

Die jeweils hergestellten Verbindungen sind nachfolgend in Tabelle 19 zusammengefasst.

### 1.17 Allgemeine Vorschrift p1)

19-Amino-9-acetoxy-2,7,12,17-tetra-(beta-methoxyethyl)porphycen (62 mg, 0,1 mmol) und Pyridin (0,2 mL, 3 mmol) wurden in trockenem Dichlormethan (2 mL) bei 2-5°C vorgelegt. Das Carbonsäure- oder Sulfonsäurechlorid (0,5 mmol) in trockenem Dichlormethan (0,5 mL) wurde zugetropft und anschließend der Ansatz für 3h bei Raumtemperatur gerührt (DC Kontrolle). Falls nötig wurde 2h bei Raumtemperatur weitergerührt oder 30min refluxiert. Es wurde mit Ethylacetat (20 mL) verdünnt. Die Lösung wurde mit gesättigter Kochsalzlösung (2 × 20 mL) und Wasser (20 mL) gewaschen. Die organische Phase wurde getrocknet (MgSO₄), filtriert und unter reduziertem Druck eingeengt. Der Feststoff wurde durch Säulenchromatographie an Kieselgel mit Dichlormethan / Ethylacetat 2:1 (Verb. S-71 Ac) oder Dichlormethan / Ethylacetat 4:1 → 3:1 (Verb. S-70b Ac) gereinigt und anschließend mit Petrolether gewaschen.

Die jeweils hergestellten Verbindungen sind nachfolgend in Tabelle 20 zusammengefasst.

### 1.18.1 Vorschrift p2)

19-Amino-9-acetoxy-2,7,12,17-tetra-(beta-methoxyethyl)porphycen (31 mg, 0,05 mmol) und Pyridin (0,02 mL, 0,3 mmol) wurden in trockenem Tetrahydrofuran (0,5 mL) bei 2-5°C vorgelegt. Das Carbonsäureanhydrid (0,3 mmol) in trockenem Tetrahydrofuran (0,5 mL) wurde zugetropft und anschließend der Ansatz für 3h bei Raumtemperatur gerührt, dann über Nacht refluxiert. Es wurde mit Dichlormethan (20 mL) verdünnt. Die Lösung wurde mit gesättigter Kochsalzlösung (10 mL), 1N wässriger Salzsäure (10 mL) und Wasser (10 mL) gewaschen. Die organische Phase wurde getrocknet (MgSO₄), filtriert und unter reduziertem Druck eingeengt. Der Feststoff wurde durch Säulenchromatographie an Kieselgel mit Dichlormethan / Ethylacetat 1:1 → 1:2 → Ethylacetat → Ethylacetat / Ethanol 10:1 bzw. Dichlormethan / Ethanol 20:1 → 8:1 gereinigt.

Die jeweils hergestellten Verbindungen sind nachfolgend in Tabelle 21 zusammengefasst.

### 1.18.2 Allgemeine Vorschrift q)

Zur Entschützung der Acetatgruppe wurde das in Tabelle 21 dargestellte Porphycencarbonamid bzw. -sulfonamid (0,1 mmol) in THF (2 mL) vorgelegt. Wässrige Lithiumhydroxidlösung (0,5 mL, 1 N, 5 Äquivalente) wurde in einer Portion zugegeben. Nach 4 h Rühren bei 40°C in einer Stickstoffatmosphäre wurde das Gemisch mit Diethylether/Dichlormethan 1:1 (30 mL) verdünnt, mit gesättigter Kochsalzlösung (2 × 20 mL) und Wasser (20 mL) gewaschen. Die organische Phase wurde getrocknet (MgSO₄), filtriert und unter reduziertem Druck eingeengt. Der Feststoff wurde in Petrolether suspendiert und abgesaugt.

Die jeweils hergestellten Verbindungen sind nachfolgend in Tabelle 22 zusammengefasst.

### 1.19 Allgemeine Vorschrift r)

9-Acetoxy-2,7,12,17-tetra-(beta-methoxyethyl)porphycen (0,1 mmol) wurde in THF (4 mL) gelöst. Wässrige Natriumhydroxidlösung (1 mL, 2 N, 20 Äquivalente) wurde in einer Portion zugegeben. Nach 6 h Rühren bei 40°C in einer Stickstoffatmosphäre wurde das Gemisch mit Diethylether/Dichlormethan 1:1 (20 mL) verdünnt, in einen Scheidetrichter überführt und mit gesättigter Kochsalzlösung (2 × 10 mL) und Wasser (10 mL) gewaschen. Die organische Phase wurde getrocknet (MgSO₄), filtriert und unter reduziertem Druck eingeengt. Der Feststoff wurde in wenig Ethylacetat suspendiert und abgesaugt. Die Kristalle wurden mit eiskaltem Ethylacetat gewaschen und bei reduziertem Druck bis zur Gewichtskonstanz getrocknet. Bei Bedarf konnte das Produkt durch Flash-Chromatographie mit Kieselgel unter Verwendung von Dichlormethan/Ethylacetat 2:1 (Rf ~ 0,4) als Laufmittel gereinigt werden.

Die jeweils hergestellten Verbindungen sind nachfolgend in Tabelle 23 zusammengefasst.

### 1.20 Allgemeine Vorschrift s)

9-Hydroxy-2,7,12,17-tetra-(beta-methoxyethyl)porphycen (Verb. S-58; 0,12 g, 0,2 mmol) in Chloroform (40 mL) wurde mit 5 mL einer gesättigten methanolischen Zink(II)acetatlösung (enthält ca. 75 mg Zinksalz) für 3 Tage bei 60°C unter Stickstoff gerührt. Das Lösungsmittel wurde abgezogen und der Rückstand in Chloroform/Ethanol 50:1 (30 mL) aufgenommen. Das Produkt wurde durch Zugabe von 120 mL Petrolether gefällt. Der Niederschlag wurde abgesaugt, mit Petrolether gründlich gewaschen und an der Luft getrocknet. Bei Bedarf konnte das Produkt durch Flash-Chromatographie an Kieselgel mit Ethylacetat → Ethylacetat/Ethanol 5:1 als Laufmittel gereinigt werden

Die jeweils hergestellten Verbindungen sind nachfolgend in Tabelle 24 zusammengefasst.

### 1.21 Vorschrift t)

9-Acetoxy-2,7,12,17-tetra-(beta-methoxyethyl)porphycen (0,06 g, 0,1 mmol) wurde in trockenem Dichlormethan (5 mL) vorgelegt und im Eisbad gekühlt. Eine Lösung von Bromwasserstoff in Eisessig (33% w/w; 5 mL) wurde unter Feuchtigkeitsausschluss über 15 min zugetropft. Der Ansatz wurde im auftauenden Eisbad für 1h, dann bei Raumtemperatur für 2 Tage gerührt.

Die Lösung wurde mit 30 mL Dichlormethan verdünnt und mit gesättigter Kochsalzlösung (2x 30 mL), Natriumhydrogencarbonatlösung (5%ig, 30mL) und Wasser (30 mL) gewaschen. Die organische Phase wurde getrocknet (MgSO₄), filtriert und unter reduziertem Druck eingeengt. Das Produkt wurde durch Flash-Chromatographie an Kieselgel unter Verwendung von Dichlormethan / Ethylacetat 3:1 als Laufmittel gereinigt und bei reduziertem Druck bis zur Gewichtskonstanz getrocknet. Es wurden ein Hauptprodukt (S-60a) und ein Nebenprodukt (S-60b) isoliert.

Die jeweils hergestellten Verbindungen sind nachfolgend in Tabelle 25 zusammengefasst.

### 1.22 Allgemeine Vorschrift u1)

9-Hydroxy-2,7,12,17-tetra-(beta-methoxyethyl)porphycen (56 mg, 0,1 mmol) wurde zusammen mit Pyridin (0,1 mL, 98 mg, 1,1 mmol) in trockenem Dioxan (5 mL) in einem 10 mL Rundkolben mit Septum unter Stickstoff vorgelegt. Palmitoylchlorid (83 mg, 0,3 mmol) in 0,5 mL trockenem Dioxan wurde langsam mittels einer Spritze über das Septum unter Kühlung zugetropft. Das Eisbad wurde entfernt und das Reaktionsgemisch über Nacht bei Raumtemperatur gerührt. Der Ansatz wurde mit 20 mL Chloroform verdünnt und die organische Lösung zweimal mit 0,1 N Salzsäure (20 mL) und nachfolgend mit Natriumcarbonatlösung (10 mL) gewaschen, über Natriumsulfat getrocknet und eingeengt. Das erhaltene Rohprodukt wurde mit Petrolether ausgewaschen um Palmitinsäurereste zu entfernen. Der Rückstand wurde durch Säulenchromatographie an Kieselgel mit Ethylacetat / Petrolether 1:3 → 2:3 als Eluent gereinigt.

Die jeweils hergestellten Verbindungen sind nachfolgend in Tabelle 26 zusammengefasst.

### 1.23 Allgemeine Vorschrift u2)

9-Hydroxy-2,7,12,17-tetra-(beta-methoxyethyl)porphycen (Verb. F-67a; 0,17 g, 0,3 mmol) wurde in trockenem DMF (4 mL) gelöst. Anschließend wurde das Alkylierungsreagenz (1,0 mmol), Kaliumiodid (83 mg, 0,5 mmol) und Caesiumcarbonat (488 mg, 1,5 mmol) zugegeben. Nach Rühren bei 60°C in einer Stickstoffatmosphäre über Nacht (DC-Kontrolle) wurde das Gemisch mit Ethylacetat (30 mL) verdünnt, in einen Scheidetrichter überführt und mit gesättigter Kochsalzlösung (3 × 20 mL) und Wasser (20 mL) gewaschen. Die organische Schicht wurde getrocknet (MgSO₄), filtriert und unter reduziertem Druck eingeengt. Das Rohmaterial wurde durch Säulenchromatographie mit Silicagel unter Verwendung von Dichlormethan / Ethylacetat Gemisch als Elutionsmittel gereinigt (Bsp. I und II mit 3:1 --> 1:1, die übrigen Bsp. mit 6:1 -7 4:1). Schließlich wurde das Produkt in Petrolether suspendiert, abfiltriert und mit Petrolether gewaschen.

Die jeweils hergestellten Verbindungen sind nachfolgend in Tabelle 27 zusammengefasst.

### 1.24 Allgemeine Vorschrift u3)

9-Hydroxy-2,7,12,17-tetra-(beta-methoxyethyl)porphycen (Verb. F-67a; 0,12 g, 0,2 mmol) wurde in trockenem DCM (4 mL) gelöst im Eisbad unter Feuchtigkeitsauschluss gerührt. Anschließend wurde in dieser Reihenfolge die Carbonsäure (0,5 mmol), DMAP (61 mg, 0,5 mmol) und EDC Hydrochlorid (95 mg, 1 mmol) nacheinander zugegeben. Der Ansatz wurde 1h im auftauenden Eisbad, dann bei RT über Nacht gerührt. Das Gemisch wurde mit Ethylacetat (30 mL) verdünnt, in einen Scheidetrichter überführt und mit gesättigter Kochsalzlösung (2 × 20 mL) und Wasser (20 mL) gewaschen. Die organische Phase wurde getrocknet (MgSO₄), filtriert und unter reduziertem Druck eingeengt. Das Rohmaterial wurde durch Säulenchromatographie mit Silicagel unter Verwendung von Dichlormethan / Ethylacetat 4:1 und Ethylacetat / Petrolether 2:3 → 1:1 als Laufmittel gereinigt.

Die jeweils hergestellten Verbindungen sind nachfolgend in Tabelle 28 zusammengefasst.

### 1.25.1 Allgemeine Vorschrift w)

9-(3-lodoprop-1-oxy)-2,7,12,17-tetra-(beta-methoxyethyl)porphycen (0,15 g, 0,2 mmol) wurden in 1 mL Methanol suspendiert. Das tertiäre Amin wurde als 1 M Lösung in Methanol zugegeben (2 mL) und der Ansatz für 36 bei Raumtemperatur gerührt. Alle flüchtigen Komponenten wurden bei vermindertem Druck abgezogen, der tiefblaue Rückstand in möglichst wenig Methanol gelöst und durch Zugabe von trockenem Diethylether gefällt (ca. 20-facher Überschuss). Der Niederschlag wurde abzentrifugiert und mit Petrolether gründlich gewaschen. Das Produkt wurde bei vermindertem Druck getrocknet.

Die jeweils hergestellten Verbindungen sind nachfolgend in Tabelle 29 zusammengefasst.

### 1.25.2 Allgemeine Vorschrift v) Entschützung des Methylesters

Der in Tabelle 29 dargestellte Methylester (0,1 mmol) wurde in THF (4 mL) gelöst. Wässrige Natriumhydroxidlösung (0,5 mL, 1 N, 5 Äquivalente) wurde in einer Portion zugegeben. Nach 4 h Rühren bei RT wurde mit Ethylacetat verdünnt (20 mL). Die Lösung wurde im Scheidetrichter mit gesättigter Kochsalzlösung (2x 10 mL), 1 N HCl (10 mL) und Wasser (10 mL) gewaschen. Die organische Phase wurde getrocknet (MgSO₄), filtriert und unter reduziertem Druck eingeengt. Falls nötig konnte mit Flashchromatographie an Kieselgel mit Dichlormethan / Ethylacetat 1:1 weiter gereinigt werden.

Die jeweils hergestellten Verbindungen sind nachfolgend in Tabelle 30 zusammengefasst.

### 1.25.3 Allgemeine Vorschrift x) Entschützung der tert.-Butyl bzw. Boc-Gruppe

Das in Tabelle 30 dargestellte geschützte Porphycenderivat (0,1 mmol) wurde in Dichlormethan (5 mL pro 100 mg) vorgelegt. Eine gesättigte Lösung von Chlorwasserstoff in Diethylether (0,5 mL pro mmol tert.-Butyl oder Boc-Gruppe) wurde zugetropft. Der Ansatz wurde 6 h unter Feuchtigkeitsausschluss gerührt. Das Produkt wurde durch Zugabe von 30 mL Petrolether gefällt. Der Niederschlag wurde abzentrifugiert und mit Petrolether gründlich gewaschen. Das Produkt wurde bei vermindertem Druck getrocknet.

Die jeweils hergestellten Verbindungen sind nachfolgend in Tabelle 31 zusammengefasst.

**Tabelle 3. Mit allgemeiner Vorschrift a3) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-24b 410,3 g/mol 1,23 g | 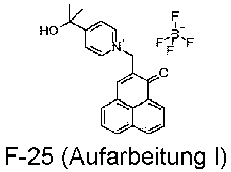 | 0,98 g 78 % d.Th. Gelbes Pulver | 417,21 g/mol C₂₂H₂₀NO₂BF₄ | **¹H-NMR** (400 MHz, DMSO-d₆): δ [ppm] = 9,15 (d, J = 8,0 Hz, 2H), 8,52 (m, 2H), 8,41 (s, 1H), 8,37 (d, J = 7,2 Hz, 2H), 8,16 - 8,21 (m, 3H), 7,94 (t, J = 7,7 Hz, 1H), 7,83 (t, J = 8,0 Hz, 1H), 5,75 (s, 2H), 1,48 (s, 6H); **¹⁹F-NMR** (376 MHz, CDC1₃): δ[ppm] = -147,8; - **MS** (+ESI-MS, CH₂Cl₂/MeOH + 10 mmol NH₄OAc): e/z (%) = 330,15 (100%, M⁺); |
| II | F-24b 410,3 g/mol 1,23 g | 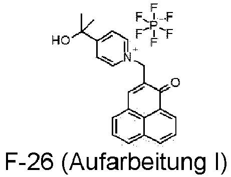 | 1,23 g 86 % d.Th. Gelbes Pulver | 475,37 g/mol C₂₂H₂₀NO₂F₆P | **¹H-NMR** (400 MHz, DMSO-d₆): δ [ppm] = 9,24 (d, J = 8,0 Hz, 2H), 8,52 (m, 2H), 8,41 (s, 1H), 8,36 (d, J = 7,2 Hz, 2H), 8,14 - 8,19 (m, 3H), 7,93 (t, J = 7,6 Hz, 1H), 7,82 (t, J = 8,0 Hz, 1H), 5,75 (s, 2H), 1,48 (s, 6H); **¹⁹F-NMR** (376 MHz, CDC1₃): δ[ppm] = -68,8, - 70,6; - **MS** (+ESI-MS, CH₂Cl₂/MeOH + 10 mmol NH₄OAc): e/z (%) = 330,15 (100%, M⁺) / (-ESI-MS, CH₂Cl₂/MeOH + 10 mmol NH₄OAc): e/z (%) = 144,97 (100%, M⁻); |
| III | F-24b 410,3 g/mol 1,23 g | 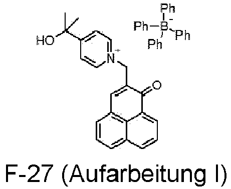 | 1,79 g 92 % d.Th. Gelbes Pulver | 649,65 g/mol C₄₆H₄₀BNO₂ | **¹H-NMR** (400 MHz, CDCla): δ [ppm] = 9,53 (d, J = 8,0 Hz, 2H), 9,19 (s, 1H), 8,58 (m, 1H), 8,24 (d, J = 8,0 Hz, 1H), 8,17 (d, J = 7,3 Hz, 1H), 8,06 - 8,11 (m, 3H), 7,81 (m, 2H), 7,58 - 7,69 (m, 12H), 7,41 - 7,49 (m, 8H), 6,03 (s, 2H), 1,61 (s, 6H); - MS (+ESI-MS, CH₂Cl₂/MeOH + 10 mmol NH₄OAc): e/z (%) = 330,15 (100%, M⁺) / (-ESI-MS, CH₂Cl₂/MeOH + 10 mmol NH₄OAc): e/z (%) = 319,17 (100%, M⁻); |
| IV | F-24b 410,3 g/mol 1,23 g | 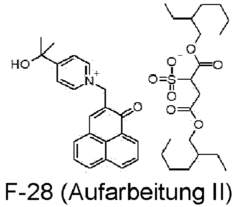 | 1,99 g 88 % d.Th. Dunkelgelbes,zähes Öl, das mit der Zeit zum Glas erstarrt | 751,99 g/mol C₄₂H₅₇NO₉S | **¹H-NMR** (400 MHz, CDCla): δ [ppm] = 9,22 (d, J = 8,0 Hz, 2H), 8,81 (s, 1H), 8,48 (d, J = 8,0 Hz, 2H), 8,18 (m, 2H), 8,01 - 8,08 (m, 3H), 7,71 (t, J = 7,6 Hz, 1H), 7,61 (t, J = 8,0 Hz, 1H), 5,78 (s, 2H), 4,21 (m, 1H), 3,88 - 4,07 (m, 6H), 3,10 - 3,31 (m, 2H), 1,56 (m, 2H), 1,48 (s, 6H) 1,14 - 1,35 (m, 14H), 0,76 - 0,88 (m, 12H); - **MS** (+ESI-MS, CH₂Cl₂/MeOH + 10 mmol NH₄OAc): e/z (%) = 330,15 (100%, M⁺) / (-ESI-MS, CH₂Cl₂/MeOH + 10 mmol NH₄OAc): e/z (%) = 421,23 (100%, M⁻); |

**Tabelle 4. Mit allgemeiner Vorschrift b) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Diphenhydramin (CAS: 58-73-1) 255,36 g/mol 510 mg | 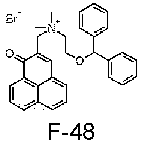 | 153 mg 58 % d.Th. Blassgelber Feststoff | 528,49 g/mol C₃₁H₃₉NO₂Br | **¹H-NMR** (400 MHz, CD₃OD): δ [ppm] = 8,64 (d, J = 7,2 Hz, 1H), 8,41 (m, 2H), 8,29 (d, J = 8,4 Hz, 1H), 8,05 (d, J = 6,8 Hz, 1H), 7,89 (t, J = 8,0 Hz, 1H), 7,75 (t, J = 8,0 Hz, 1H), 7,43 (d, J = 7,2 Hz, 4H), 7,38 (t, J = 8,0 Hz, 4H), 7,25 (tt, J = 7,2 Hz, 2H), 5,61 (s, 1H), 4,67 (s, 2H), 4,02 (m, 2H), 3,79 (s, 2H), 3,22 (s, 6H); - **MS** (ESI-MS, 95% H₂O + 0,1% HCOOH / 5% MeCN + 0,1% HCOOH): e/z (%) = 448,23 (100%, M⁺); |
| II | 4-(Dimethylamino) benzophenon (CAS: 530-44-9) 225,29 g/mol 450 mg | 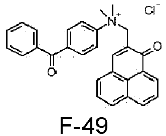 | 84 mg 37 % d.Th. Gelber Feststoff | 453,97 g/mol C₂₉H₂₄NO₂Cl | **¹H-NMR** (400 MHz, CD₃OD): δ [ppm] = 8,65 (d, J = 7,6 Hz, 1H), 8,41 (d, J = 8,0 Hz, 1H), 8,22 (d, J = 8,8 Hz, 1H), 8,14 (s, 1H), 8,01 (d, J = 6,4 Hz, 1H), 7,90 (t, J = 8,4 Hz, 1H), 7,74 (m, 3H), 7,66 (d, J = 7,2 Hz, 2H), 7,58 (d, J = 6,8 Hz, 1H), 7,51 (m, 2H), 6,78 (d, J = 8,8 Hz, 2H), 4,65 (s, 2H), 3,09 (s, 6H); - **MS** (ESI-MS, 95% H₂O + 0,1% HCOOH / 5% MeCN + 0,1% HCOOH): e/z (%) = 418,18 (100%, M⁺); |
| III | Ethyl-4-(dimethyl-amino)benzoat (CAS 10287-53-3) 193,25 g/mol 386 mg | 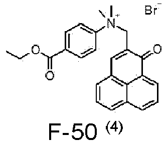 | 47 mg 23 % d.Th. Dunkelgelbes Pulver | 466,37 g/mol C₂₅H₂₄NO₃Br | **¹H-NMR** (600 MHz, DMSO-d6): δ [ppm] = 8,48 (d, J = 7,7 Hz, 1H), 8,42 (d, J = 7,8 Hz, 1H), 8,18 (d, J = 8,0 Hz, 1H), 8,02 (d, J = 7,6 Hz, 1H), 7,93 (s, 1H), 7,87 (t, J = 7,8 Hz, 1H), 7,76 (d, J = 7,6 Hz, 2H), 7,70 (t, J = 7,6 Hz, 1H), 6,79 (m, 2H), 5,03 (s, 2H), 4,21 (q, J = 6,6 Hz, 2H), 2,98 (s, 6H), 1,27 (d, J = 6,4 Hz, 3H); |

### (4) Reaktion durchgeführt unter Zusatz von 0,12 mL Diisopropylethylamin (DIPEA) (2 eq).

**Tabelle 5. Mit allgemeiner Vorschrift c) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Hydrochinon (CAS 123-31-9) 110,11 g/mol 220 mg | 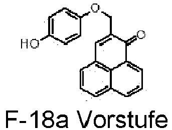 | 178 mg 59 % d.Th. Gelbes Glas | 302,33 g/mol C₂₀H₁₄O₃ | **¹H-NMR** (400 MHz, CDCl₃): δ [ppm] = 8,72 (d, J = 5,6 Hz, 1H), 8,24 (d, J = 7,6 Hz, 1H), 8,05 (d, J = 8,4 Hz, 1H), 7,97 (s, 1H), 7,82 (m, 2H), 7,62 (t, J = 7,6 Hz, 1H), 7,03 (d, J = 8,4 Hz, 2H), 6,87 (d, J = 8,6 Hz, 2H), 5,17 (s, 2H); - **MS** (ESI-MS, 95% H₂O + 0,1% HCOOH / 5% MeCN + 0,1% HCOOH): e/z (%) = 303,10 (100%, MH⁺), 627,18 (54%, (2M+Na⁺)⁺); |
| II | Cholesterol (CAS: 57-88-5) 386,66 g/mol 772 mg | 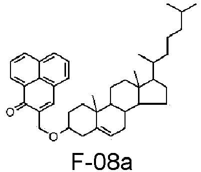 | 255 mg 44 % d.Th. Blassgelber wachsartiger Feststoff | 578,89 g/mol C₄₁H₅₄O₂ | **¹H-NMR** (400 MHz, CDCla): δ [ppm] = 8,65 (dd, J = 7,6 Hz, 1H), 8,21 (d, J = 8,0 Hz, 1H), 8,00 (d, J = 8,0 Hz, 1H), 7,88 (s, 1H), 7,791 (m, 2H), 7,60 (t, J = 7,6 Hz, 1H), 5,39 (m, 1H), 4,66 (s, 2H), 3,40 (m, 1H), 2,54 (m, 2H), 2,36 (m, 2H), 2,14 - 0,77 (m, 36H), 0,69 (s, 3H); - **MS** (ESI-MS, CH₂Cl₂/MeOH + 10 mmol NH₄OAc): e/z (%) = 579,42 (19%, MH⁺), 601,40 (100%, MNa⁺); - **UV** (H₂O): λ/ [nm] (rel. [a.u.]) = 252 (1), 350 - 420 (0,46); |
| III | Estradiol (CAS: 50-28-2) 272,39 g/mol 545 mg | 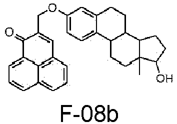 | 181 mg 39 % d.Th. Blassgelber Feststoff | 464,61 g/mol C₃₂H₃₂O₃ | **MS** (ESI-MS, CH₂Cl₂/MeOH + 10 mmol NH₄OAc): e/z (%) = 465,24 (16%, MH⁺), 487,22 (100%, MNa⁺); |
| IV | Estron (CAS: 53-16-7) 270,37 g/mol 541 mg | 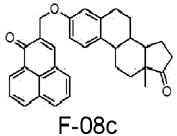 | 213 mg 46 % d.Th. Blassgelber Feststoff | 462,59 g/mol C₃₂H₃₀O₃ | **MS** (ESI-MS, CH₂Cl₂/MeOH + 10 mmol NH₄OAc): e/z (%) = 463,23 (19%, MH⁺), 485,21 (100%, MNa⁺); |
| V | 4-Chloro-3,5-dimethylphenol (CAS: 88-04-0) 156,61 g/mol 312 mg | 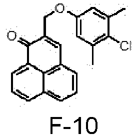 | 206 mg 59 % d.Th. Gelbes Pulver | 348,83 g/mol C₂₂H₁₇ClO₂ | **¹H-NMR** (400 MHz, CDCla): δ [ppm] = 8,70 (d, J = 7,2 Hz, 1H), 8,25 (d, J = 8,0 Hz, 1H), 8,05 (d, J = 8,4 Hz, 1H), 7,93 (t, J = 1,2 Hz, 1H), 7,82 (m, 2H), 7,62 (t, J = 8,0 Hz, 1H), 6,82 (s, 2H), 5,13 (s, 2H), 2,36 (s, 6H); - **MS** (ESI-MS, CH₂Cl₂/MeOH + 10 mmol NH₄OAc): e/z (%) = 349,10 (21%, MH⁺), 719,17 (100%, (2M+Na⁺)⁺); - **UV** (H₂O): λ/ [nm] (rel. [a.u.]) = 255 (1), 340 - 420 (0,51); |
| VI | 4-(1,1,3,3-Tetramethyl butyl)phenol (CAS: 140-66-9) 206,33 g/mol 412 mg | 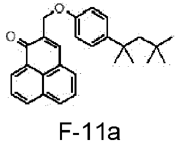 | 255 mg 64 % d.Th. Gelbes Pulver | 398,55 g/mol C₂₈H₃₀O₂ | **¹H-NMR** (400 MHz, CDCla): δ [ppm] = 8,69 (d, J = 7,2 Hz, 1H), 8,88 (d, J = 8,0 Hz, 1H), 8,03 (d, J = 8,4 Hz, 1H), 7,96 (t, 2,0 Hz, 1H), 7,81 (m, 2H), 7,61 (t, J = 7,6 Hz, 1H), 7,31 (d, J = 9,2 Hz, 2H), 7,00 (d, J = 8,4 Hz, 2H), 5,17 (s, 2H), 1,72 (s, 2H), 1,36 (s, 6H), 0,72 (s, 9H); - **MS** (ESI-MS, CH₂Cl₂/MeOH + 10 mmol NH₄OAc): e/z (%) = 399,23 (100%, MH⁺), 421,21 (11%, MNa⁺), 819,44 (79%, (2M+Na⁺)⁺); |
| VII | 4-Hexyloxyphenol (CAS: 18979-55-0) 194,27 g/mol 388 mg | 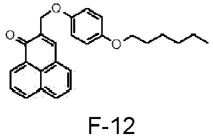 | 236 mg 61 % d.Th. Gelber Feststoff | 386,50 g/mol C₂₆H₂₆O₃ | **¹H-NMR** (400 MHz, CDCla): δ [ppm] = 8,70 (d, J = 5,2 Hz, 1H), 8,23 (d, J = 7,6 Hz, 1H), 8,03 (d, J = 8,4 Hz, 1H), 7,96 (s, 1H), 7,81 (m, 2H), 7,61 (t, J = 7,6 Hz, 1H), 7,00 (d, J = 8,4 Hz, 2H), 6,86 (d, J = 8,8 Hz, 2H), 5,14 (s, 2H), 3,91 (m, 2H), 1,75 (m, 2H), 1,45 (m, 2H), 1,34 (m, 4H), 0,91 (t, J = 6,8 Hz, 3H); - **MS** (ESI-MS, CH₂Cl₂/MeOH + 10 mmol NH₄OAc): e/z (%) = 387,20 (48%, MH⁺), 795,37 (100%, (2M+Na⁺)⁺); |
| VIII | 3-n-Pentadecylphenol (CAS: 501-24-6) 304,52 g/mol 609 mg | 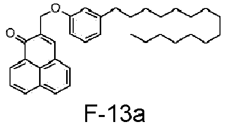 | 283 mg 57 % d.Th. Gelber Feststoff | 496,74 g/mol C₃₅H₄₄O₂ | **¹H-NMR** (400 MHz, CDCla): δ [ppm] = 8,70 (d, J = 7,2 Hz, 1H), 8,24 (d, J = 8,4 Hz, 1H), 8,03 (d, J = 8,4 Hz, 1H), 7,97 (s, 1H), 7,81 (m, 2H), 7,61 (t, J = 8,0 Hz, 1H), 7,22 (t, J = 7,2 Hz, 1H), 6,91 (m, 2H), 6,82 (d, J = 7,6 Hz, 1H), 5,18 (s, 2H), 2,60 (t, J = 7,6 Hz, 2H), 1,62 (quint., J = 6,8 Hz, 2H), 1,25 (m, 24H), 0,88 (t, J = 7,2 Hz, 3H); - **MS** (ESI-MS, CH₂Cl₂/MeOH + 10 mmol NH₄OAc): e/z (%) = 497,36 (100%, MH⁺); - **UV** (H₂O): λ/ [nm] (rel. [a.u.]) = 253 (1), 350 - 420 (0,50); |
| IX | 4-Cumylphenol (CAS: 599-64-4) 212,29 g/mol 424 mg | 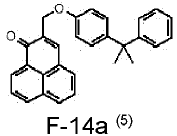 | 279 mg 69 % d.Th. Gelbes Glas | 404,51 g/mol C₂₉H₂₄O₂ | **¹H-NMR** (400 MHz, CDCl₃): δ [ppm] = 8,69 (d, J = 7,6 Hz, 1H), 8,23 (d, J = 8,0 Hz, 1H), 8,03 (d, J = 7,2 Hz, 1H), 7,96 (t, J = 1,6 Hz, 1H), 7,80 (t, J = 8,0 Hz, 2H), 7,61 (t, J = 7,6 Hz, 1H), 7,26 (m, 4H), 7,19 (m, 3H), 6,99 (d, J = 10,4 Hz, 2H), 5,16 (s, 2H), 1,68 (s, 6H); - **MS** (ESI-MS, CH₂Cl₂/MeOH + 10 mmol NH₄OAc): e/z (%) = 405,19 (36%, MH⁺), 427,16 (9%, MNa⁺), 831,35 (100%, (2M+Na⁺)⁺); |
| X | 4-Tritylphenol (CAS: 978-86-9) 336,43 g/mol 673 mg | 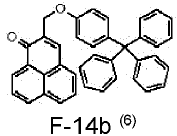 | 333 mg 63 % d.Th. Gelber Feststoff | 528,66 g/mol C₈₉H₂₈O₂ | **¹H-NMR** (400 MHz, CDCla): δ [ppm] = 8,68 (d, J = 7,6 Hz, 1H), 8,24 (d, J = 8,0 Hz, 1H), 8,04 (d, J = 7,2 Hz, 1H), 7,95 (t, J = 1,6 Hz, 1H), 7,81 (t, J = 8,0 Hz, 2H), 7,62 (t, J = 7,6 Hz, 1H), 7,23 - 7,36 (m, 8H), 7,15 - 7,21 (m, 9H), 6,98 (d, J = 10,2 Hz, 2H), 5,18 (s, 2H); - **MS** (ESI-MS, CH₂Cl₂/MeOH + 10 mmol NH₄OAc): e/z (%) = 529,22 (100%, MH⁺), 551,20 (5%, MNa⁺), 1079,41 (13%, (2M+Na⁺)⁺); - **UV** (H₂O): λ/ [nm] (rel. [a.u.]) = 226 (1), 350 - 420 (0,18); |
| XI | 4-Hydroxybenzophenon (CAS: 1137-42-4) 198,22 g/mol 396 mg | 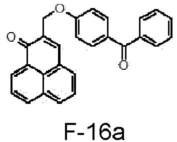 | 257 mg 66 % d.Th. Gelber Feststoff | 390,44 g/mol C₂₇H₁₈O₃ | **¹H-NMR** (400 MHz, CDCla): δ [ppm] = 8,70 (d, J = 7,6 Hz, 1H), 8,25 (d, J = 7,6 Hz, 1H), 8,05 (d, J = 8,4 Hz, 1H), 7,95 (s, 1H), 7,84 (m, 4H), 7,76 (m, 2H), 7,63 (t, J = 8,0 Hz, 1H), 7,57 (t, J = 7,2 Hz, 1H), 7,47 (t, J = 7,2 Hz, 2H), 7,14 (d, J = 9,2 Hz, 2H), 5,26 (s, 2H); - **MS** (ESI-MS, CH₂Cl₂/MeOH + 10 mmol NH₄OAc): e/z (%) = 391,13 (100%, MH⁺), 413,11 (4%, MNa⁺), 803,24 (66%, (2M+Na⁺)⁺); - **UV** (H₂O): λ/ [nm] (rel. [a.u.]) = 228 (1), 292 (0,26), 350 - 420 (0,16); |
| XII | (4-Fluorophenyl)-(4-hydroxyphenyl)-methanon (CAS: 25913-05-7) 216,21 g/mol 432 mg | 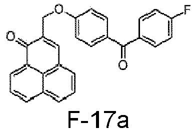 | 167 mg 41 % d.Th. Gelbes Pulver | 408,43 g/mol C₂₇H₁₇F0₃ | **¹H-NMR** (400 MHz, CDCla): δ [ppm] = 8,71 (d, J = 7,6 Hz, 1H), 8,26 (d, J = 8,0 Hz, 1H), 8,06 (d, J = 8,0 Hz, 1H), 7,94 (s, 1H), 7,83 (m, 6H), 7,63 (t, J = 8,0 Hz, 1H), 7,16 (t, J = 8,0 Hz, 4H), 5,27 (s, 2H); - **MS** (ESI-MS, CH₂Cl₂/MeOH + 10 mmol NH₄OAc): e/z (%) = 409,12 (100%, MH⁺), 431,10 (17%, MNa⁺), 839,22 (39%, (2M+Na⁺)⁺); |
| XIII | 2-(2-(2-(2-methoxyethoxy)-ethoxy)ethoxy)ethyl-4-hydroxybenzoat ⁽⁷⁾ 328,15 g/mol 656 mq | 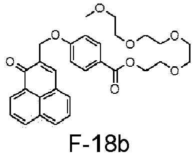 | 146 mg 28 % d.Th. Gelbes Glas | 520,58 g/mol C₃₀H₃₂O₈ | **MS** (ESI-MS, CH₂Cl₂/MeOH + 10 mmol NH₄OAc): e/z (%) = 521,22 (100%, MH⁺), 543,20 (51%, MNa⁺), 1063,41 (4%, (2M+Na⁺)⁺); |
| XIV | Methyl-2-(4-hydroxy-phenoxy)propanoat (CAS: 96562-58-2) 196,20 g/mol 392 mg | 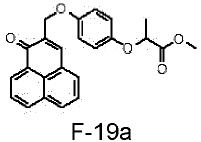 | 237 mg 61 % d.Th. Gelber Feststoff | 388,42 g/mol C₂₄H₂₀O₅ | **¹H-NMR** (400 MHz, CDCl₃): δ [ppm] = 8,69 (d, J = 7,2 Hz, 1H), 8,23 (d, J = 8,0 Hz, 1H), 8,03 (d, J = 8,4 Hz, 1H), 7,93 (s, 1H), 7,81 (m, 2H), 7,61 (t, J = 7,6 Hz, 1H), 6,99 (d, J = 8,8 Hz, 2H), 6,86 (d, 8,8 Hz, 2H), 5,12 (s, 2H), 4,69 (q, J = 7,2 Hz, 1H), 3,76 (s, 3H), 1,60 (d, J = 7,2 Hz, 3H); - **MS** (ESI-MS, CH₂Cl₂/MeOH + 10 mmol NH₄OAc): e/z (%) = 389,14 (100%, MH⁺), 411,12 (76%, MNa⁺), 799,25 (18%, (2M+Na⁺)⁺); - **UV** (H₂O): λ/ [nm] (rel. [a.u.]) = 255 (1), 320 (0,24), 345 - 425 (0,43); |
| XV | 4-Hydroxybenzoesäure 2-ethylhexylester (CAS : 5153-25-3) 250,34 g/mol 500 mg | 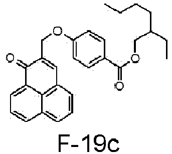 | 252 mg 57 % d.Th. Gelber Feststoff | 442,56 g/mol C₂₉H₃₀O₄ | **MS** (ESI-MS, CH₂Cl₂/MeOH + 10 mmol NH₄OAc): e/z (%) = 443,22 (100%, MH⁺), 465,20 (67%, MNa⁺), 907,42 (7%, (2M+Na⁺)⁺); |
| XVI | 4-Hydroxybenzoesäure 2-hydroxyethylester (CAS: 2496-90-4) 182,18 g/mol 364 mg | 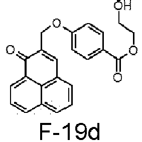 | 209 mg 56 % d.Th. Gelber Feststoff | 374,40 g/mol C₂₃H₁₈O₅ | **MS** (ESI-MS, CH₂Cl₂/MeOH + 10 mmol NH₄OAc): e/z (%) = 375,12 (100%, MH⁺), 397,11 (44%, MNa⁺), 771,22 (13%, (2M+Na⁺)⁺); |
| XVII | N-Boc-tyrosinmethylester (CAS: 76757-90-9) 295,34 g/mol 590 mg | 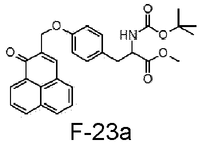 | 249 mg 51 % d.Th. Gelbes Pulver | 487,56 g/mol C₂₉H₂₉NOe | **¹H-NMR** (400 MHz, CDCl₃): δ [ppm] = 8,69 (d, J = 7,8 Hz, 1H), 8,23 (d, J = 8,0 Hz, 1H), 8,05 (d, J = 8,3 Hz, 1H), 7,94 (s, 1H), 7,82 (m, 2H), 6,98 (d, J = 8,6 Hz, 2H), 6,76 (d, J = 8,8 Hz, 2H), 5,56 (bs, 1H), 5,16 (s, 2H), 4,54 (m, 2H), 3,71 (s, 3H), 3,02 (m, 1H), 1,41 (s, 9H). - **MS** (ESI-MS, CH₂Cl₂/MeOH + 10 mmol NH₄OAc): e/z (%) = 431,14 (16%, ((M - C₄H₉)H⁺)⁺), 488,21 (100%, MH⁺), 510,19 (73%, MNa⁺), 997,39 (2%, (2M+Na⁺)⁺); |
| XVIII | N-Benzyloxy-carbonyltyrosin (CAS: 5618-98-4) 315,33 g/mol 630 mg | 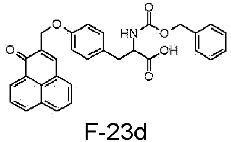 | 234 mg 46 % d.Th. Gelbes Pulver | 507,55 g/mol C₃₁H₂₅NO₆ | **¹H-NMR** (400 MHz, CDCl₃): δ [ppm] = 8,64 (d, J = 7,6 Hz, 1H), 8,21 (d, J = 8,0 Hz, 1H), 8,03 (d, J = 8,3 Hz, 1H), 7,73 - 7,84 (m, 3H), 7,61 (m, 1H), 7,22 - 7,36 (m, 5H), 6,97 (d, J = 8,8 Hz, 2H), 6,64 (d, 8,8 Hz, 2H), 5,36 (m, 1H), 5,23 (s, 2H), 5,09 (m, 2H), 4,73 (m, 1H), 3,08 (m, 2H). - **MS** (ESI-MS, CH₂Cl₂/MeOH + 10 mmol NH₄OAc): e/z (%) = 508,18 (100%, MH⁺), 530,16 (53%, MNa⁺), 1037,33 (6%, (2M+Na⁺)⁺); |

**Tabelle 6. Mit allgemeiner Vorschrift d) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Diethylenglykolmonotosylat 260,2 g/mol 260 mg | 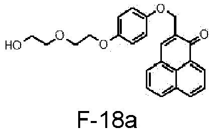 | 76 mg 39 % d.Th. Bräunliches zähes Öl | 390,44 g/mol C₂₄H₂₂O₅ | **MS** (ESI-MS, 95% H₂O + 0,1% HCOOH / 5% MeCN + 0,1% HCOOH): e/z (%) = 391,15 (100%, MH⁺), 413,13 (82%, MNa⁺); - **UV** (H₂O): λ/ [nm] (rel. [a.u.]) = 254 (1), 360 - 420 (0,5); |

**Tabelle 7. Mit allgemeiner Vorschrift e1) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | 3-(Boc-amino)-propylbromid 175,2 g/mol 131 mg | 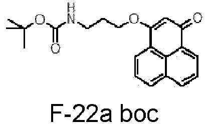 | 76 mg 43 % d.Th. Oranges Glas | 353,42 g/mol C₂₁H₂₃NO₄ | Siehe WO2018167264A1 |
| II | 2-(Boc-amino)-ethylbromid (CAS 39684-80-5) 161,1 g/mol 120 mg | 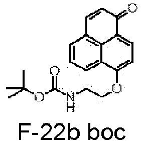 | 78 mg 46 % d.Th. Oranges Glas | 339,39 g/mol C₂₀H₂₁ NO₄ | **¹H-NMR** (400 MHz, CDCl₃): δ [ppm] = 8,55 (d, J = 8,8 Hz, 1H), 8,38 (d, J = 8,4 Hz, 1H), 7,71 (d, 6,4 Hz, 1H), 7,66 (d, 10,0 Hz, 1H), 7,51 (t, 7,6 Hz, 1H), 7,03 (d, 9,2 Hz, 1H), 6,66 (d, J = 10,0 Hz, 1H), 5,00 (s, 1H), 4,28 (t, 2H), 3,66 (t, 2H), 1,42 (s, 9H); - **MS** (ESI-MS, CH₂Cl₂/MeOH + 10 mmol NH₄OAc): e/z (%) = 341,15 (100%, MH+); - **UV** (H₂O): λ/ [nm] (ε / [M⁻¹cm⁻¹]) = 260 (20100), 340 (3600), 370 - 450 (10300) |
| III | 3-(Boc-amino)-propylbromid (CAS 83948-53-2) 175,2 g/mol 131 mg | 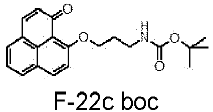 | 90 mg 51 % d.Th. Dunkelgelbes Pulver | 353,42 g/mol C₂₁H₂₃NO₄ | **¹H-NMR** (400 MHz, CDCl₃): δ [ppm] = 8,22 (d, J = 8,0 Hz, 1H), 8,01 (d, J = 7,9 Hz, 1H), 7,85 (d, 7,9 Hz, 1H), 7,75 (d, 8,0 Hz, 1H), 7,56 (t, 8,0 Hz, 1H), 7,49 (d, 8,0 Hz, 1H), 6,79 (d, J = 8,0 Hz, 1H), 4,46 (t, J = 7,2 Hz, 2H), 3,53 (m, 2H), 2,21 (m, 2H), 1,48 (s, 9H); - **MS** (ESI-MS, 95% H₂O + 0,1% HCOOH / 5% MeCN + 0,1% HCOOH): e/z (%) = 354,17 (100%, MH⁺), 376,15 (43%, MNa⁺), 729,31 (47%, (2M+Na⁺)⁺); - **UV** (H₂O): λ/ [nm] (rel. [a.u.]) = 260 (0,96), 355 (1), 370 - 440 (0,5); |

**Tabelle 8. Mit allgemeiner Vorschrift f) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | S-08 Boc 353,2 g/mol 70 mg | 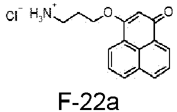 | 51 mg 90 % d.Th. Oranges, hygroskopisches Pulver | 289,76 g/mol C₁₆H₁₆NO₂Cl | **MS** (ESI-MS, 95% H₂O + 0,1% HCOOH / 5% MeCN + 0,1% HCOOH): e/z (%) = 254,12 (100%, M⁺), 529,21 (1%, (2M+Na⁺)⁺); - **UV** (H₂O): λ/ [nm] (rel. [a.u.]) = 250 (1), 315 - 410 (0,57); |
| II | S-09 Boc 339,4 g/mol 68 mg | 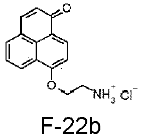 | 49 mg 93 % d.Th. Oranges, hygroskopisches Pulver | 275,73 g/mol C₁₅H₁₄NO₂Cl | **MS** (ESI-MS, CH₂Cl₂/MeOH + 10 mmol NH₄OAc): e/z (%) = 240,10 (100%, M⁺); - **UV** (H₂O): λ/ [nm] (ε / [M⁻¹cm⁻¹]) = 260 (20300), 330 (3400), 365 - 455 (10400) |
| III | S-10 Boc 353,2 g/mol 70 mg | 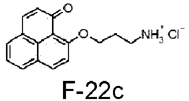 | 53 mg 94 % d.Th. Dunkelgelbes, hygroskopisches Pulver | 289,76 g/mol C₁₆H₁₆NO₂Cl | **MS** (ESI-MS, 95% H₂O + 0,1% HCOOH / 5% MeCN + 0,1% HCOOH): e/z (%) = 254,12 (100%, M⁺), 507,23 (7%, (2M+H⁺)⁺); - **UV** (H₂O): λ/ [nm] (rel. [a.u.]) = 265 (0,88), 355 (1), 370 - 440 (0,46); |

**Tabelle 9. Mit allgemeiner Vorschrift e2) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | 4-Brom-2-methylbenzonitril (CAS 67832-11-5) 196,04 g/mol 196 mq | 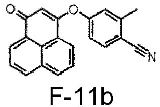 | 124 mg 41 % d.Th. Hellbrauner Feststoff | 302,33 g/mol C₂₀H₁₄O₃ | **MS** (ESI-MS, CH₂Cl₂/MeOH + 10 mmol NH₄OAc): e/z (%) = 303,10 (100%, MH⁺), 325,08 (41%, MNa⁺), 627,18 (9%, (2M+Na⁺)⁺); |
| II | 4-Bromobenzophenon (CAS 90-90-4) 261,12 g/mol 261 mq | 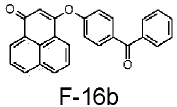 | 181 mg 48 % d.Th. Oranger Feststoff | 376,42 g/mol C₂₆H₁₆O₃ | **MS** (ESI-MS, CH₂Cl₂/MeOH + 10 mmol NH₄OAc): e/z (%) = 377,12 (100%, MH⁺), 399,10 (57%, MNa⁺), 775,21 (14%, (2M+Na⁺)⁺); |
| III | Ethyl 4-bromobenzoat (CAS 5798-75-4) 229,07 g/mol 229 mg | 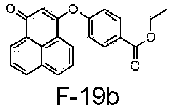 | 182 mg 53 % d.Th. Dunkelgelber Feststoff | 344,37 g/mol C₂₂H_{16O4} | **MS** (ESI-MS, CH₂Cl₂/MeOH + 10 mmol NH₄OAc): e/z (%) = 345,11 (100%, MH⁺), 367,10 (55%, MNa⁺), 711,20 (19%, (2M+Na⁺)⁺); |
| IV | 4-Bromobenzyl alcohol (CAS 873-75-6) 187,04 g/mol 187 mq | 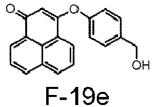 | 143 mg 46 % d.Th. Dunkelgelber Feststoff | 311,34 g/mol C₂₁H₁₃NO₂ | **MS** (ESI-MS, CH₂Cl₂/MeOH + 10 mmol NH₄OAc): e/z (%) = 312,10 (100%, MH⁺), 334,08 (48%, MNa⁺), 645,18 (11%, (2M+Na⁺)⁺); |
| V | Boc-4-bromo-DL-phenylalanin methyl ester (CAS 132067-41-5) 358,32 g/mol 358 mq | 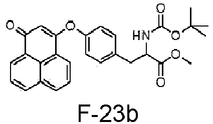 | 175 mg 37 % d.Th. Oranger Feststoff | 473,53 g/mol C₂₈H₂₇NO₆ | **MS** (ESI-MS, CH₂Cl₂/MeOH + 10 mmol NH₄OAc): e/z (%) = 417,12 (12%, ((M - C₄H₉)H⁺)⁺), 474,19 (100%, MH⁺), 496,17 (76%, MNa⁺), 799,25 (18%, (2M+Na⁺)⁺); |

**Tabelle 10. Mit allgemeiner Vorschrift g1) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | 3-Hydroxyphenalen-1-on (CAS 5472-84-4) 98 mg | 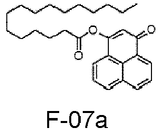 | 67 mg 31 % d.Th. Dunkelgelber Feststoff | 434,62 g/mol C₂₉H₃₈O₃ | **¹H-NMR** (400 MHz; CDCl₃): δ [ppm] = 8,67 (dd, J = 7,6 Hz, 1H), 8,25 (dd, J = 8,4 Hz, 1H), 8,12 (d, J = 8,4 Hz, 1H), 7,96 (dd, J = 7,2 Hz, 1H), 7,82 (t, J = 7,6 Hz, 1H), 7,66 (t, J = 7,6 Hz, 1H), 6,67 (s, 1H), 2,77 (t, J = 8,0 Hz, 2H), 1,89 (quin, J = 7,6 Hz, 2H), 1,50 (m, 2H), 1,31 (m, 22H), 0,92 (t, J = 6,8 Hz, 3H); - **MS** (ESI-MS, CH₂Cl₂/MeOH + 10 mmol NH₄OAc): e/z (%) = 435,28 (100%, MH⁺), 457,27 (62%, MNa⁺), 891,55 (56%, (2M+Na⁺)⁺); - **UV** (H₂O): λ/ [nm] (rel. [a.u.]) = 245 (1), 355 - 420 (0,42); |
| II | 7-Hydroxyphenalen-1-on (CAS 21913-87-1) 98 mg | 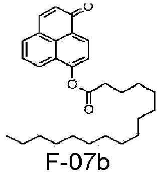 | 85 mg 39 % d.Th. Oranger Feststoff | 434,62 g/mol C₂₉H₃₈O₃ | **MS** (ESI-MS, CH₂Cl₂/MeOH + 10 mmol NH₄OAc): e/z (%) = 435,28 (100%, MH⁺), 457,27 (34%, MNa⁺), 891,55 (46%, (2M+Na⁺)⁺); - **UV** (H₂O): λ/ [nm] (rel. [a.u.]) = 250 (1), 345 - 410 (0,47); |
| III | 9-Hydroxyphenalen-1-on (CAS 7465-58-9) 98 mg | 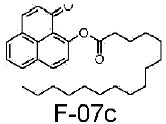 | 48 mg 22 % d.Th. Dunkelgelber Feststoff | 434,62 g/mol C₂₉H₃₃O₃ | **¹H-NMR** (600 MHz, CDCl₃): δ [ppm] = 8,10 (d, J = 9,0 Hz, 2H), 8,02 (d, J = 7,2 Hz, 2H), 7,60 (t, 7,2 Hz, 1H), 7,18 (d, J = 9,6 Hz, 2H), 2,35 (t, 7,2 Hz, 2H), 1,64 (quint., J = 7,8 Hz, 2H), 1,26 (m, 24H), 0,89 (t, J = 7,2 Hz, 3H). - **MS** (ESI-MS, CH₂Cl₂/MeOH + 10 mmol NH₄OAc): e/z (%) = 435,28 (37%, MH⁺), 457,27 (100%, MNa⁺); - **UV** (H₂O): λ/ [nm] (rel. [a.u.]) = 255 (1), 352 (0,61), 355 - 415 (0,44); |

**Tabelle 11. Mit allgemeiner Vorschrift g2) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | 3-Phenoxybenzoesäure (CAS: 3739-38-6) 214,22 g/mol 214 mg | 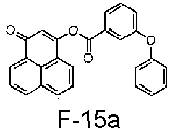 | 255 mg 65 % d.Th. Oranger Feststoff | 392,42 g/mol C₂₆H₁₆O₄ | **¹H-NMR** (400 MHz, CDCla): δ [ppm] = 8,61 (d, J = 7,6 Hz, 1H), 8,19 (d, J = 8,0 Hz, 1H), 8,06 (d, J = 8,4 Hz, 1H), 8,02 (d, J = 8,0 Hz, 1H), 7,91 (m, 2H), 7,76 (t, J = 8,0 Hz, 1H), 7,55 (m, 2H), 7,40 (t, J = 8,4 Hz, 2H), 7,34 (dd, J = 8,0 Hz, 1H), 7,18 (t, J = 7,6 Hz, 1H), 6,74 (s, 1H). - **MS** (ESI-MS, CH₂Cl₂/MeOH + 10 mmol NH₄OAc): e/z (%) = 393,11 (100%, MH⁺), 415,10 (31%, MNa⁺), 807,20 (12%, (2M+Na⁺)⁺); - **UV** (H₂O): λ/ [nm] (rel. [a.u.]) = 249 (1), 321 (0,18), 360 - 420 (0,31); |
| II | 4-Octylbenzoesäure (CAS: 3575-31-3) 234,34 g/mol 234 mg | 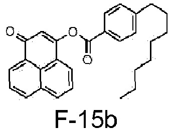 | 256 mg 62 % d.Th. Oranger Feststoff | 412,53 g/mol C₂₈H₂₈O₃ | **¹H-NMR** (400 MHz, CDCl₃): δ [ppm] = 8,61 (d, J = 7,6 Hz, 1H), 8,18 (m, 3H), 8,04 (d, J = 8,0 Hz, 1H), 7,96 (d, J = 7,2 Hz, 1H), 7,75 (m, J = 8,0 Hz, 1H), 7,56 (t, J = 8,0 Hz, 1H), 7,38 (d, J = 8,4 Hz, 2H), 6,73 (s, 1H), 2,73 (t, J = 8,0 Hz, 2H), 1,68 (quint., J = 8,0 Hz, 2H), 1,32 (m, 10H), 0,89 (t, J = 7,2 Hz, 3H). - **MS** (ESI-MS, CH₂Cl₂/MeOH + 10 mmol NH₄OAc): e/z (%) = 413,21 (100%, MH⁺), 435,19 (2%, MNa⁺), 847,40 (44%, (2M+Na⁺)⁺); - **UV** (H₂O): λ/ [nm] (rel. [a.u.]) = 251 (1), 322 (0,16), 360 - 420 (0,23); |
| III | 4-Decyloxybenzoesäure (CAS: 5519-23-3) 278,39 g/mol 278 mg | 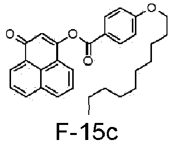 | 311 mg 68 % d.Th. Gelboranger Feststoff | 456,59 g/mol C₃₃H₃₂O₄ | **MS** (ESI-MS, CH₂Cl₂/MeOH + 10 mmol NH₄OAc): e/z (%) = 457,23 (100%, MH⁺), 479,22 (61%, MNa⁺), 935,45 (9%, (2M+Na⁺)⁺); |
| IV | 4-Benzoylbenzoesäure (CAS: 611-95-0) 226,23 g/mol 226 mq | 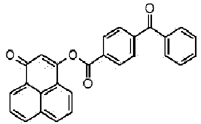 | 230 mg 57 % d.Th. Oranger Feststoff | 404,43 g/mol C₂₇H₁₆O₄ | **MS** (ESI-MS, CH₂Cl₂/MeOH + 10 mmol NH₄OAc): e/z (%) = 405,11 (100%, MH⁺), 427,10 (73%, MNa⁺), 831,20 (16%, (2M+Na⁺)⁺); |
| | | 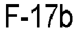 | | | |
| V | Boc-O-benzyl-D-tyrosin (CAS: 63769-58-4) 371,43 g/mol 371 mg | 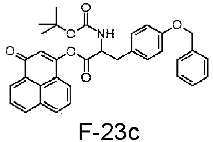 | 297 mg 54 % d.Th. Gelboranger Feststoff | 549,63 g/mol C₃₄H₃₁NOe | **MS** (ESI-MS, CH₂Cl₂/MeOH + 10 mmol NH₄OAc): e/z (%) = 493,15 (15%, ((M - C₄H₉)H⁺)⁺), 550,22 (100%, MH⁺), 572,20 (37%, MNa⁺), 1121,42 (7%, (2M+Na⁺)⁺); |
| VI | Diglykolsäureanhydrid (CAS 4480-83-5) 116,07 g/mol 116 mg | 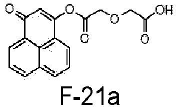 | 122 mg 39 % d.Th. Dunkelgelber Feststoff | 312,28 g/mol C₁₇H₁₂O₆ | **MS** (ESI-MS, CH₂Cl₂/MeOH + 10 mmol NH₄OAc): e/z (%) = 313,07 (100%, MH⁺), 335,05 (62%, MNa⁺), 647,12 (11%, (2M+Na⁺)⁺); |
| VII | 3-Dodecyldihydro-furan-2,5-dion (CAS 2561-85-5) 268,4 g/mol 268 mg | 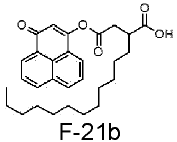 | 172 mg 37 % d.Th. Bräunlicher Feststoff | 464,61 g/mol C₂₉H₃₆O₅ | **MS** (ESI-MS, CH₂Cl₂/MeOH + 10 mmol NH₄OAc): e/z (%) = 465,26 (100%, MH⁺), 487,25 (76%, MNa⁺), 951,50 (18%, (2M+Na⁺)⁺); |

**Tabelle 12. Mit allgemeiner Vorschrift h) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-19a 388,42 g/mol 78 mg | 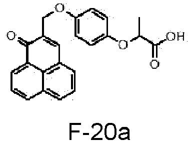 | 68 mg 90 % d.Th. Gelbes Pulver | 374,40 g/mol C₂₃H₁₈O₅ | **¹H-NMR** (400 MHz, CDCl₃): δ [ppm] = 8,68 (d, J = 7,9 Hz, 1H), 8,23 (d, J = 8,0 Hz, 1H), 8,04 (d, J = 8,0 Hz, 1H), 7,94 (s, 1H), 7,81 (m, 2H), 7,62 (t, J = 7,9 Hz, 1H), 7,01 (d, J = 8,6 Hz, 2H), 6,88 (d, J = 8,6 Hz, 2H), 6,78 (s, 1H), 5,12 (s, 2H), 1,63 (d, J = 7,8 Hz, 3H); - **MS** (ESI-MS, 95% H₂O + 0,1% HCOOH / 5% MeCN + 0,1% HCOOH): e/z (%) = 375,12 (100%, MH⁺), 397,10 (100%, MNa⁺), 771,22 (15%, (2M+Na⁺)⁺); - **UV** (H₂O): λ/ [nm] (rel. [a.u.]) = 252 (1), 360 - 420 (0,47); |
| II | F-19b 344,37 g/mol 69 mg | 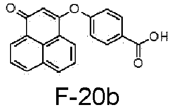 | 56 mg 89 % d.Th. Hellbraunes Pulver | 316,32 g/mol C₂₀H₁₂O₄ | **MS** (ESI-MS, CH₂Cl₂/MeOH + 10 mmol NH₄OAc): e/z (%) = 317,08 (100%, MH⁺), 339,06 (81%, MNa⁺), 655,14 (22%, (2M+Na⁺)⁺); |
| III | F-19c 442,56 g/mol 88 mg | 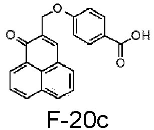 | 55 mg 83 % d.Th. Schmutzig gelbes Pulver | 330,34 g/mol C₂₁H₁₄O₄ | **MS** (ESI-MS, 95% H₂O + 0,1% HCOOH / 5% MeCN + 0,1% HCOOH): e/z (%) = 331,07 (100%, MH⁺), 353,05 (81%, MNa⁺); |
| IV | F-23b 473,53 g/mol 94 mg | 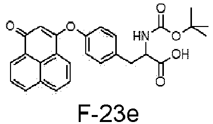 | 66 mg 72 % d.Th. Oranges Pulver | 459,50 g/mol C₂₇H₂₅NO₆ | **MS** (ESI-MS, CH₂Cl₂/MeOH + 10 mmol NH₄OAc): e/z (%)= 403,11 (7%, ((M - C₄H₉)H⁺)⁺), 460,18 (100%, MH⁺), 482,16 (79%, MNa⁺), 941,33 (26%, (2M+Na⁺)⁺); |

**Tabelle 13. Mit allgemeiner Vorschrift i) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | 4-(2-N-boc-aminoethoxy) benzaldehyd (CAS 198990-09-9) 265,31 g/mol 1,11 g | 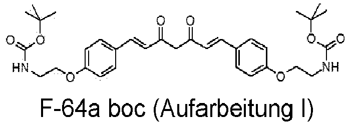 | 0,90 g 76 % d.Th. Gelbes Pulver | 594,67 g/mol C₃₃H₄₂N₂O₈ | Siehe WO2017032892A1 |
| II | 4-(2-N-boc-aminoethoxy) benzaldehyd (CAS 198990-09-9) 265,31 g/mol 1,11 g | 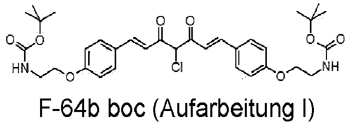 | 0,79 g 63 % d.Th. Oranges Glas | 629,16 g/mol C₃₃H₄₁ClN₂O₈ | **¹H-NMR** (400 MHz, CDCl₃): δ[ppm] = 7,61 (d, J = 15,6 Hz, 2H), 7,49 (d, J = 8,2 Hz, 4H), 6,90 (d, J = 8,2 Hz, 4H), 6,49 (d, J = 15,6 Hz, 2H), 5,78 (s, 1H), 5,01 (bs, 2H), 4,06 (m, 4H), 3,54 (m, 4H), 1,45 (s, 18H); - **MS** (ESI-MS, 95% H₂O + 0,1% HCOOH / 5% MeCN + 0,1% HCOOH): e/z (%) = 629,26 (100%, MH⁺), 651,25 (72%, MNa⁺); - **UV** (H₂O): λ/ [nm] (rel. [a.u.]) = 255 (0,26), 445 (1); |
| III | 4-(2-N-boc-aminoethoxy) benzaldehyd (CAS 198990-09-9) 265,31 g/mol 1,11 g | 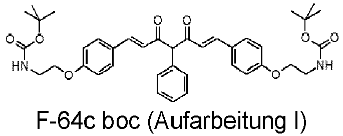 | 0,87 g 65 % d.Th. Oranges Glas | 670,81 g/mol C₃₉H₄₆N₂O₈ | **¹H-NMR** (400 MHz, CDCl₃): δ[ppm] = 7,61 (d, *J* = 15,6 Hz, 2H), 7,42 (m, 2H), 7,21 - 7,26 (m, 5H), 6,76 (d, *J* = 8,1 Hz, 4H), 6,32 (d, *J* = 15,6 Hz, 2H), 4,91 (bs, 2H), 3,97 (m, 4H), 3,48 (m, 4H), 1,41 (s, 18H); - **MS** (ESI-MS, 95% H₂O + 0,1% HCOOH / 5% MeCN + 0,1% HCOOH): e/z (%) = 671,33 (100%, MH⁺), 693,31 (6%, MNa⁺); - **UV** (H₂O): λ/ [nm] (rel. [a.u.]) = 430 (1); |
| IV | 3-Bromo-4-(2-N-boc-aminoethoxy) benzaldehyd 244,2 g/mol 1,45 g | 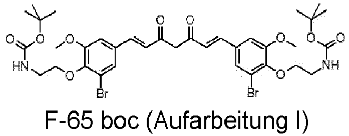 | 0,96 g 59 % d.Th. Oranges Glas | 812,56 g/mol C₃₅H₄₄Br₂N₂O₁₀ | **¹H-NMR** (600 MHz, CDCl₃): δ[ppm] = 7,53 (d, *J* = 15,7 Hz, 2H), 7,38 (d, *J* = 1,7 Hz, 2H), 7,01 (d, *J* = 1,7 Hz, 2H), 6,53 (d, *J =* 15,7 Hz, 2H), 5,84 (s, 1H), 5,50 (bs, 2H), 4,15 (t, J = 4,6 Hz, 4H), 3,93 (s, 6H), 3,51 (m, 4H), 1,48 (s, 18H); - **MS** (ESI-MS, 95% H₂O + 0,1% HCOOH / 5% MeCN + 0,1% HCOOH): e/z (%) = 813,14 (100%, MH⁺), 835,12 (27%, MNa⁺); - **UV** (H₂O): λ/ [nm] (rel. [a.u.]) = 408 (1); |
| V | 4-(3-Hydroxy-propoxy) benzaldehyd (CAS 99186-35-3) 180,2 g/mol 0,76 g | 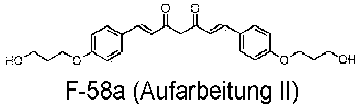 | 0,48 g 57 % d.Th. Oranges Pulver | 424,50 g/mol C₂₅H₂₈O₆ | **¹H-NMR** (400 MHz, CDCl₃): δ[ppm] = 7,62 (d, *J* = 15,6 Hz, 2H), 7,51 (d, J = 8,1 Hz, 4H), 6,92 (d, *J* = 8,1 Hz, 4H), 6,49 (d, *J =* 15,6 Hz, 2H), 4,18 (t, J = 6,8 Hz, 4H), 3,87 (m, 4H), 2,08 (m, 4H), 2,06 (bs, 2H); - **MS** (ESI-MS, 95% H₂O + 0,1% HCOOH / 5% MeCN + 0,1% HCOOH): e/z (%) = 425,12 (100%, MH⁺), 447,11 (9%, MNa⁺); - **UV** (H₂O): λ/ [nm] (rel. [a.u.]) = 420 (1); |
| VI | 3-Methoxy-4-(3-hydroxypropoxy) benzaldehyd (CAS 107115-25-3) 210,2 g/mol 0,88 g | 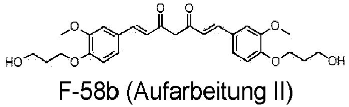 | 0,48 g 49 % d.Th. Rotoranges Pulver | 484,55 g/mol C₂₇H₃₂O₈ | **¹H-NMR** (400 MHz, CDCl₃): δ[ppm] = 7,56 (d, *J* = 15,6 Hz, 2H), 7,08 (m, 2H), 7,03 (m, 2H), 6,85 (d, *J* = 8,2 Hz, 2H), 6,46 (d, *J =* 15,6 Hz, 2H), 4,11 (m, 4H), 3,68 (m, 4H), 2,11 (bs, 2H), 2,06 (m, 4H); - **MS** (ESI-MS, 95% H₂O + 0,1% HCOOH / 5% MeCN + 0,1% HCOOH): e/z (%) = 485,22 (100%, MH⁺), 507,21 (11%, MNa⁺); - UV (H₂O): λ/ [nm] (rel. [a.u.]) = 425 (1); |
| VII | 3-Methoxy-4-(3-(2-(Tetrahydro-2H-pyran-2-yl)oxy)propoxy) benzaldehyd ⁽⁸⁾ 294,3 g/mol 1,23 g | 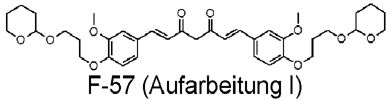 | 0,74 g 56 % d.Th. Oranger, zäher Feststoff | 652,79 g/mol C₃₇H₄₈O₁₀ | **¹H-NMR** (400 MHz, CDCl₃): δ[ppm] = 7,61 (d, J = 15,6 Hz, 2H), 7,06 - 7,17 (m, 4H), 6,91 (m, 2H), 6,48 (d, *J* = 15,6 Hz, 2H), 5,83 (s, 1H), 4,59 (m, app. s, 2H), 3,92 (s, 6H), 3,79 - 3,98 (m, 4H), 3,46 - 3,64 (m, 4H), 2,08 - 2,21 (m, 4H), 1,63 - 1,88 (m, 4H), 1,46 - 1,61 (m, 8H); - **MS** (ESI-MS, 95% H₂O + 0,1% HCOOH / 5% MeCN + 0,1% HCOOH): e/z (%) = 653,33 (100%, MH⁺), 675,12 (19%, MNa⁺), 1306,16 (8%, (2M+H⁺)⁺), 1327,64 (6%, 2M+Na⁺)⁺); |
| VIII | 4-(11 -Hydroxy undecyloxy) benzaldehyd (CAS 124389-14-6) 292,4 g/mol 1,23 g | 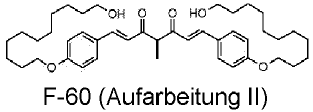 | 0,64 g 48 % d.Th. Oranger, zäher Feststoff | 662,96 g/mol C₄₂H₆₂O₆ | **MSI** (ESI-MS, 95% H₂O + 0,1% HCOOH / 5% MeCN + 0,1% HCOOH): e/z (%) = 663,46 (100%, MH⁺), 685,44 (71%, MNa⁺); - **UV** (H₂O): λ/ [nm] (rel. [a.u.]) = 230 (1), 410 (0,65); |
| IX | 4-(6-Hydroxy hexyloxy) benzaldehyd (CAS 96735-91-0) 222,3 g/mol 0,94 q | 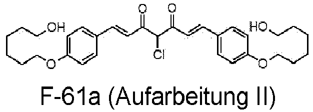 | 0,49 g 45 % d.Th. Oranger, zäher Feststoff | 543,11 g/mol C₃₁H₃₉ClO₆ | **MS** (ESI-MS, 95% H₂O + 0,1% HCOOH / 5% MeCN + 0,1% HCOOH): e/z (%) = 543,25 (100%, MH⁺), 565,23 (71%, MNa⁺); - **UV** (H₂O): λ/ [nm] (rel. [a.u.]) = 233 (1), 428 (0,88); |
| X | 4-(11-Hydroxy undecyloxy) benzaldehyd (CAS 124389-14-6) 292,4 g/mol 1,23 g | 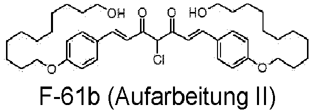 | 0,41 g 30 % d.Th. Oranger, zäher Feststoff | 683,38 g/mol C₄₁H₅₉ClO₆ | **MS** (ESI-MS, 95% H₂O + 0,1% HCOOH / 5% MeCN + 0,1% HCOOH): e/z (%) = 683,41 (100%, MH⁺), 705,40 (92%, MNa⁺); - **UV** (H₂O): λ/ [nm] (rel. [a.u.]) = 230 (1), 425 (0,96); |
| XI | 4-[2-[2-[2-(2-Methoxyethoxy) ethoxy]ethoxy]-ethoxy]-benzaldehyd (CAS 197513-69-2) 312,36 g/mol 1,31 g | 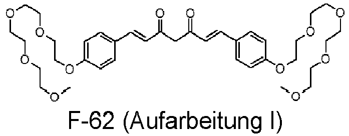 | 0,88 g 64 % d.Th. Oranger, zäher Feststoff | 688,82 g/mol C₃₇H₅₂O₁₂ | **¹H-NMR** (400 MHz, CDCl₃): δ [ppm] = 7,61 (d, J = 16,0 Hz, 2H), 7,49 (d, J = 8,8 Hz, 4H), 6,93 (d, J = 8,4 Hz, 4H), 6,49 (d, J = 16,0 Hz, 2H), 5,78 (s, 1H), 4,17 (t, J = 4,8 Hz, 4H), 3,87 (d, J = 4,8 Hz, 4H), 3,73 (m, 4H), 3,67 (m, 16H), 3,54 (m, 4H), 3,37 (s, 6H); - **MS** (ESI-MS, 95% H₂O + 0,1% HCOOH / 5% MeCN + 0,1% HCOOH): e/z (%) = 683,41 (100%, MH⁺), 705,40 (92%, MNa⁺); - **UV** (H₂O): λ/ [nm] (rel. [a.u.]) = 252 (0,16), 423 (1); |
| XII | 3-Benzyloxy-4-hydroxy benzaldehyd (CAS 50773-56-3) 228,25 g/mol 0,96 g | 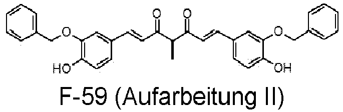 | 0,54 g 50 % d.Th. Rotoranges Pulver | 534,61 g/mol C₃₄H₃₀O₆ | **¹H-NMR** (300 MHz, DMSO-d6): δ[ppm] = 9.65 (bs, 2H), 7,55 (d, *J* = 15,6 Hz, 2H), 7,42 - 7,30 (m, 10H), 7,15 - 7,02 (m, 4H), 6,88 (d, *J* = 8,2 Hz, 2H), 6,49 (d, *J* = 15.6 Hz, 2H), 5,22 (s, 4H), 3,61 (s, 3H); - **MS** (ESI-MS, 95% H₂O + 0,1% HCOOH / 5% MeCN + 0,1% HCOOH): e/z (%) = 535,21 (100%, MH⁺), 557,19 (38%, MNa⁺); - UV (H₂O): λ/ [nm] (rel. [a.u.]) = 265 (0,26), 440 (1); |
| XIII | 3-methoxy-4-[(4-nitrobenzyl)oxy]-benzaldehyd (CAS: 81307-09-7) 287,27 g/mol 1,21 g | 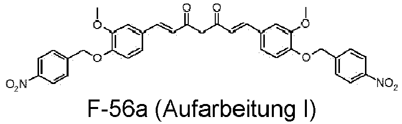 | 0,60 g 47 % d.Th. Rotoranges Pulver | 638,64 g/mol C₃₅H₃₀N₂O₁₀ | **¹H-NMR** (400 MHz, CDCl₃): δ [ppm] = 8,29 (d, J = 8,8 Hz, 4H), 7,66 (m, 6H), 7,16 (s, 2H), 7,13 (d, J = 8,4 Hz, 2H), 6,89 (d, J = 8,4 Hz, 2H), 6,54 (d, J = 15,6 Hz, 2H), 5,86 (s, 1H), 5,32 (s, 4H), 4,00 (s, 6H); - **MS** (ESI-MS, 95% H₂O + 0,1% HCOOH / 5% MeCN + 0,1% HCOOH): e/z (%) = 639,20 (100%, MH⁺), 661,18 (13%, MNa⁺); - **UV** (H₂O): λ/ [nm] (rel. [a.u.]) = 270 (0,54), 422 (1); |
| XIV | 4-Decyloxy benzaldehyd (CAS 24083-16-7) 262,4 g/mol 1,10 g | 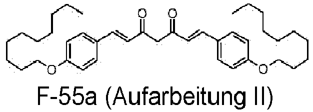 | 0,78 g 66 % d.Th. Oranger, zäher Feststoff | 588,88 g/mol C₃₉H₅₆O₄ | **¹H-NMR** (400 MHz, CDCl₃): δ[ppm] = 7,62 (d, *J* = 15,8 Hz, 2H), 7,50 (d, *J* = 8,2 Hz, 4H), 6,90 (d, *J* = 8,2 Hz, 4H), 6,49 (d, *J =* 15,8 Hz, 2H), 5,78 (s, 1H), 3,89 (t, *J* = 6,8 Hz, 4H), 1,79 (m, 4H), 1,45 (m, 4H), **1,22** - 1,39 (m, 24H), 0,89 (t, *J* = 6,8 Hz, 6H); - MS (ESI-MS, 95% H₂O + 0,1% HCOOH / 5% MeCN + 0,1% HCOOH): e/z (%) = 611,41 (100%, MNa⁺); - UV (H₂O): λ/ [nm] (rel. [a.u.]) = 245 (0,23), 415 (1); |
| XV | 4-(Hexadecyloxy) benzaldehyd 346,55 g/mol 1,46 g | 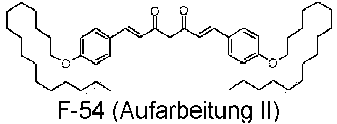 | 0,26 g 17 % d.Th. Gelber Feststoff | 757,20 g/mol C₅₁H₈₀O₄ | **MS** (ESI-MS, 95% H₂O + 0,1% HCOOH / 5% MeCN + 0,1% HCOOH): e/z (%) = 757,61 (3%, MH⁺), 779,59 (100%, MNa⁺); |
| XVI | 4-(tert.-Butyl-dimethylsilyloxy)-benzaldehyd (CAS: 120743-99-9) 236,38 g/mol 0,99 g | 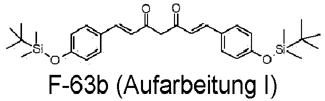 | 0,62 g 58 % d.Th. Gelber Feststoff | 536,87 g/mol C₃₁H₄₄O₄Si₂ | **¹H-NMR** (400 MHz, CDCl₃): δ [ppm] = 7,54 (d, J = 16,0 Hz, 2H), 7,38 (d, J = 8,8 Hz, 4H), 6,78 (d, J = 8,4 Hz, 4H), 6,42 (d, J = 16,4 Hz, 2H), 5,72 (s, 1H), 0,92 (s, 18H), 0,15 (s, 12H); - **MS** (ESI-MS, 95% H₂O + 0,1% HCOOH / 5% MeCN + 0,1% HCOOH): e/z (%) = 536,40 (100%, MH⁺); |

### (8) Dieser Ausgangsstoff wurde durch THP Schützung von 3-Methoxy-4-(3-hydroxypropoxy)benzaldehyd erhalten. Gelblicher Feststoff (81% d.Th.).

**Tabelle 14. Mit allgemeiner Vorschrift j) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | S-52a 588,88 g/mol 118 mg | 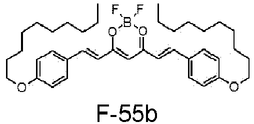 | 100 mg 79 % d.Th. Rotbrauner Feststoff | 636,68 g/mol C₃₉H₅₅BFN₂O₄ | **¹H-NMR** (400 MHz, CDCl₃): δ[ppm] = 8,01 (d, *J =* 15,6 Hz, 2H), 7,57 (d, *J* = 8,1 Hz, 4H), 6,93 (d, *J* = 8,1 Hz, 4H), 6,58 (d, *J* = 15,6 Hz, 2H), 6,00 (s, 1H), 4,01 (t, *J* = 6,8 Hz, 4H), 1,81 (m, 4H), 1,43 (m, 4H), 1,22 - 1,40 (m, 24H), 0,89 (t, *J* = 6,8 Hz, 6H); **¹⁹F-NMR** (376 MHz, CDCl₃): δ[ppm] = -141,1; - **MS** (ESI-MS, 95% H₂O + 0,1% HCOOH / 5% MeCN + 0,1% HCOOH): e/z (%) = 659,41 (100%, MNa⁺); - UV (H₂O): λ/ [nm] (rel. [a.u.]) = 260 (0,14), 490 (1); |

**Tabelle 15. Mit allgemeiner Vorschrift k) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-64a boc 593,71 g/mol 118 mg | 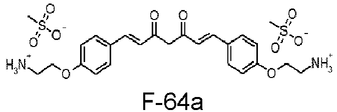 | 99 mg 84 % d.Th. Gelber hygroskopischer Feststoff | 586,69 g/mol C₂₃H₂₈N₂O₄ + 2x CH₃O₃S | **¹H-NMR** (400 MHz, DMSO-d6): δ[ppm] = 8,01 (bs, 6H), 7,71 (d, J = 15,8 Hz, 4H), 7,60 (d, J = 8,4 Hz, 2H), 7,06 (d, J = 8,4 Hz, 2H), 6,81 (d, J = 15,8 Hz, 4H), 6,13 (s, 1H), 4,22 (t, J = 6,4 Hz, 4H), 3,34 (m, 4H), 3,21 (m, 4H), 2,46 (s, 6H); - **MS** (ESI-MS, 95% H₂O + 0,1% HCOOH / 5% MeCN + 0,1% HCOOH): e/z (%) = 198,10 (100%, M²⁺), 395,20 (9%, M⁺); - **UV** (H₂O): λ/ [nm] (rel. [a.u.]) = 422 (1); |
| II | F-64b boc 629,16 g/mol 126 mg | 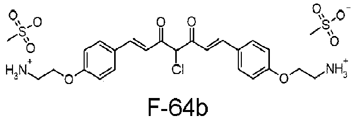 | 101 mg 81 % d.Th. Oranger hygroskopischer Feststoff | 621,14 g/mol C₂₃H₂₇ClN₂O₄ + 2 x CH₃O₃S | **MS** (ESI-MS, 95% H₂O + 0,1% HCOOH / 5% MeCN + 0,1% HCOOH): e/z (%) = 216,09 (100%, M²⁺), 431,17 (5%, M⁺); |
| III | F-64c boc 670,81 g/mol 134 mg | 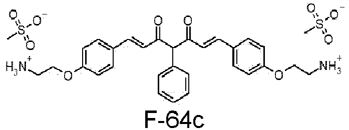 | 105 mg 86 % d.Th. Rotoranger hygroskopischer Feststoff | 662,79 g/mol C₂₉H₃₂N₂O₄ + 2 x CH₃O₃S | **MS** (ESI-MS, 95% H₂O + 0,1% HCOOH / 5% MeCN + 0,1% HCOOH): e/z (%) = 236,12 (100%, M²⁺), 471,22 (4%, M⁺); - **UV** (H₂O): λ/ [nm] (rel. [a.u.]) = 230 (0,82), 427 (1); |
| IV | F-65 boc 812,56 g/mol 162 mg | 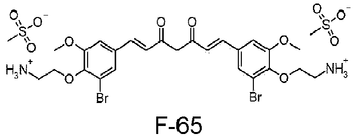 | 126 mg 78 % d.Th. Oranger hygroskopischer Feststoff | 804,53 g/mol C₂₅H₃₀Br₂N₂O₆ + 2 x CH₃O₃S | **¹H-NMR** (600 MHz, DMSO-d6): δ[ppm] = 8,00 (bs, 6H), 7,26-7,65 (m, 6H), 7,03-7,08 (m, 2H), 6,18 (s, 1H), 4,15 (m, 4H), 3,90 (m, 6H), 3,21 (m, 4H), 2,34 (s, 6H); - **MS** (ESI-MS, 95% H₂O + 0,1% HCOOH / 5% MeCN + 0,1% HCOOH): e/z (%) = 307,02 (100%, M²⁺), 613,03 (7%, M⁺); - **UV** (H₂O): λ/ [nm] (rel. [a.u.]) = 232 (0,89), 408 (1); |

**Tabelle 16. Mit allgemeiner Vorschrift I) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Methacryloylchlorid (CAS: 920-46-7) 104,54 g/mol 105 mg | 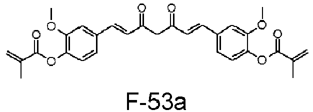 | 97 mg 48 % d.Th. Dunkelgelber Feststoff | 504,54 g/mol C₂₉H₂₈O₈ | **MS** (ESI-MS, 95% H₂O + 0,1% HCOOH / 5% MeCN + 0,1% HCOOH): e/z (%) = 505,19 (100%, MH⁺), 527,17 (15%, MNa⁺), 1009,37 (11%, (2M+H⁺)⁺); |
| II | Crotonylchlorid (CAS: 10487-71-5) 104,54 g/mol 105 mg | 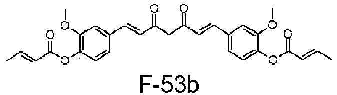 | 85 mg 46 % d.Th. Tiefgelber Feststoff | 504,54 g/mol C₂₉H₂₈O₈ | **¹H-NMR** (400 MHz, CDCl₃): δ [ppm] = 7,62 (d, J = 16,0 Hz, 2H), 7,26-7,06 (m, 8H), 6,57 (d, J = 16,0 Hz, 2H), 6,08 (dd, J = 15,6 Hz, 2H), 5,86 (s, 1H), 3,87 (s, 6H), 1,98 (dd, J = 6,4 Hz, 6H); - **MS** (ESI-MS, 95% H₂O + 0,1% HCOOH / 5% MeCN + 0,1% HCOOH): e/z (%) = 505,19 (100%, MH⁺), 527,17 (7%, MNa⁺), 1009,37 (16%, (2M+H⁺)⁺), 1031,35 (4%, (2M+Na⁺)⁺); |
| III | 2,4,6-Trichlorobenzoyl chlorid (CAS: 4136-95-2) 243,90 g/mol 244 mg | 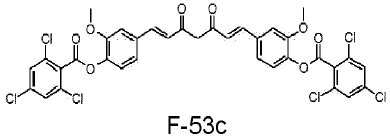 | 194 mg 62 % d.Th. Oranges Pulver | 783,28 g/mol C₃₅H₂₂Cl₆O₈ | **¹H-NMR** (600 MHz, CDCl₃): δ [ppm] = 7,66 (d, J = 16,2 Hz, 2H), 7,45 (s, 4H), 7,29 (s, 2H), 7,27 (d, J = 7,8 Hz, 2H), 7,23 (m, 2H), 6,33 (d, J = 16,2 Hz, 2H), 5,31 (s, 1H), 3,95 (s, 6H). - **MS** (ESI-MS, 95% H₂O + 0,1% HCOOH / 5% MeCN + 0,1% HCOOH): e/z (%) = 782,95 (100%, MH⁺), 804,92 (2%, MNa⁺); - **UV** (H₂O): λ/ [nm] (rel. [a.u.]) = 252 (0,36), 402 (1); |
| IV | Dansylchlorid) (CAS: 605-65-2) 269,75 g/mol 270 mg | 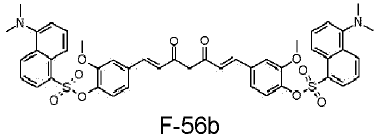 | 190 mg 57 % d.Th. Oranges Glas | 834,97 g/mol C₄₅H₄₂N₂O₁₀S₂ | **¹H-NMR** (400 MHz, CDCl₃): δ [ppm] = 8,62 (d, J = 8,0 Hz, 2H), 8,53 (d, J = 8,4 Hz, 2H), 8,11 (d, J = 7,2 Hz, 2H), 7,64 (t, J = 8,4 Hz, 2H), 7,49 (m, 4H), 7,25 (d, J = 7,6 Hz, 2H), 7,03 (m, 4H), 6,90 (s, 2H), 6,49 (d, J = 16,0 Hz, 2H), 5,79 (s, 1H), 3,35 (s, 6H), 2,91 (s, 12H); - **MS** (ESI-MS, 95% H₂O + 0,1% HCOOH / 5% MeCN + 0,1% HCOOH): e/z (%) = 418,12 (100%, (M+2H⁺)²⁺), 835,24 (24%, MH⁺); - UV (H₂O): λ/ [nm] (rel. [a.u.]) = 258 (0,62), 403 (1); |

**Tabelle 17. Mit allgemeiner Vorschrift m) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-66 600,7 g/mol 60 mg | 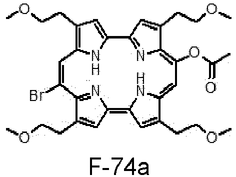 | 36 mg 53 % d.Th. Dunkelblaues Pulver | 679,62 g/mol C₃₄H₃₉BrN₄O₆ | **¹H-NMR** (300 MHz, CDCl₃): δ[ppm] = 10,12 (s, 1H), 9,52 (s, 1H), 9,18 (s, 1H), 9,09 (s, 1H), 9,07 (s, 1H), 8,99 (s, 1H), 4,16 - 4,24 (m, 8H), 4,05 - 4,14 (m, 4H), 3,97 - 4,04 (m, 2H), 3,88 - 3,95 (m, 2H), 3,63 (s, 3H), 3,62 (s, 3H), 3,59 (s, 6H), 2,89 (s, 3H); - **MS** (ESI-MS, 95% H₂O + 0,1% HCOOH / 5% MeCN + 0,1% HCOOH): e/z (%) = 681,21 (100%, MH⁺), 1359,42 (7%, (2M+H⁺)⁺); - **UV** (H₂O): λ/ [nm] (rel. [a.u.]) = 365 (1), 560 (0,16), 610 (0,24), 630 (0,27); |
| II | F-66 600,7 g/mol 60 mg | 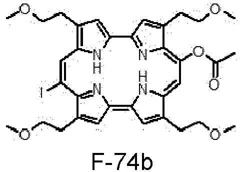 | 30 mg 41 % d.Th. Dunkelblaues Pulver | 726,62 g/mol C₃₄H₃₉IN₄O₆ | **MS** (ESI-MS, 95% H₂O + 0,1% HCOOH / 5% MeCN + 0,1% HCOOH): e/z (%) = 727,20 (100%, MH⁺), 1453,39 (6%, (2M+H⁺)⁺); - **UV** (H₂O): λ/ [nm] (rel. [a.u.]) = 365 (1), 560 (0,29), 610 (0,3), 630 (0,32); |

**Tabelle 18. Mit allgemeiner Vorschrift n) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-66 600,7 g/mol 120 mg | 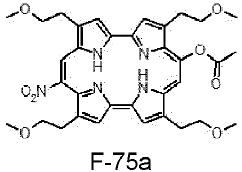 | 86 mg 67 % d.Th. Grünblaue Schuppen (Bedingung I) | 645,72 g/mol C₃₄H₃₉N₅O₈ | **¹H-NMR** (300 MHz, CDCl₃): δ[ppm] = 9,78 (s, 1H), 9,12 (s, 1H), 9,08 (s, 1H), 9,06 (s, 1H), 8,97 (s, 1H), 8,88 (s, 1H), 4,17 - 4,26 (m, 8H), 4,06 - 4,16 (m, 4H), 3,99 - 4,05 (m, 2H), 3,91 - 3,97 (m, 2H), 3,62 (s, 3H), 3,61 (s, 3H), 3,58 (s, 6H), 2,88 (s, 3H); - **MS** (ESI-MS, 95% H₂O + 0,1% HCOOH / 5% MeCN + 0,1% HCOOH): e/z (%) = 646,28 (100%, MH⁺), 1291,57 (48%, (2M+H⁺)⁺); - UV (H₂O): λ/[nm] (rel. [a.u.]) = 372 (1), 562 (0,26), 605 (0,34), 635 (0,36); |
| II | F-66 600,7 g/mol 120 mg | 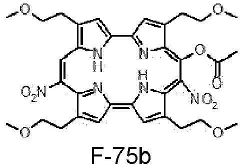 | 70 mg 55 % d.Th. Dunkelgrünes Pulver (Bedingung II) | 690,72 g/mol C₃₄H₃₆N₆O₁₀ | **MS** (ESI-MS, 95% H₂O + 0,1% HCOOH / 5% MeCN + 0,1% HCOOH): e/z (%) = 691,27 (100%, MH⁺), 713,25 (31%, MH⁺), 1381,54 (7%, (2M+H⁺)⁺), 1403,52 (4%, (2M+Na⁺)⁺); - UV (H₂O): λ/ [nm] (rel. [a.u.]) = 376 (1), 564 (0,23), 606 (0,31), 638 (0,34); |

**Tabelle 19. Mit allgemeiner Vorschrift o) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Zinn(II)chlorid Dihydrat (CAS 10025-69-1) 225,64 g/mol 451 mg | 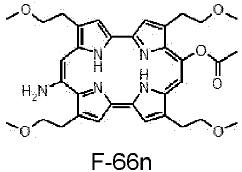 | 96 mg 78 % d.Th. Grünliches Pulver | 615,74 g/mol C₃₄H₄₁N₅O₆ | **¹H-NMR** (300 MHz, CDCl₃): δ[ppm] = 9,12 (s, 1H), 8,82 (s, 1H), 8,77 (s, 1H), 8,44 (m, 2H), 7,81 (s, 1H), 5,33 (bs, 2H), 4,21 - 4,28 (m, 4H), 4,11 - 4,20 (m, 2H), 4,01 - 4,10 (m, 4H), 3,92 - 3,99 (m, 2H), 3,64 (s, 3H), 3,62 (s, 3H), 3,52 - 3,58 (m, 2H), 3,51 (s, 3H), 3,46 (s, 3H), 2,88 (s, 3H); - MS (ESI-MS, 95% H₂O + 0,1% HCOOH / 5% MeCN + 0,1% HCOOH): e/z (%) = 616,31 (100%, MH⁺), 628,29 (2%, MNa⁺); |

**Tabelle 20. Mit allgemeiner Vorschrift p1) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Essigsäureanhydrid (CAS 108-24-7) 102,09 g/mol 51 mg | 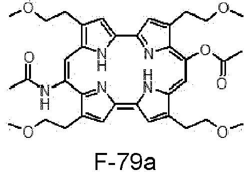 | 56 mg 84 % d.Th. Dunkelblauer Feststoff | 657,77 g/mol C₃₆H₄₃N₅O₇ | **¹H-NMR** (300 MHz, CDCl₃): δ[ppm] = 11,50 (s, 1H), 10,16 (s, 1H), 9,39 (s, 1H), 9,26 (s, 1H), 9,24 (s, 1H), 9,20 (s, 1H), 9,17 (s, 1H), 4,21 -4,39 (m, 12H), 4,11 - 4,19 (m, 4H), 3,62 (s, 3H), 3,60 (s, 3H), 3,58 (s, 3H), 3,50 (s, 3H), 2,89 (s, 3H), 2,67 (s, 3H); - **MS** (ESI-MS, 95% H₂O + 0,1% HCOOH / 5% MeCN + 0,1% HCOOH): e/z (%) = 658,32 (100%, MH⁺), 680,30 (7%, MNa⁺), 1337,62 (11%, (2M+H⁺)⁺), 1359,60 (10%, (2M+Na⁺)⁺); - UV (H₂O): λ/ [nm] (rel. [a.u.]) = 374 (1), 560 (0,18), 629 (0,27); |
| II | Palmitoylchlorid (CAS 112-67-4) 274,87 g/mol 137 mg | 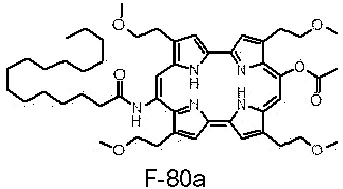 | 42 mg 49 % d.Th. Blauer Feststoff | 854,15 g/mol C₅₀H₇₁N₅O₇ | **MS** (ESI-MS, 95% H₂O + 0,1% HCOOH / 5% MeCN + 0,1% HCOOH): e/z (%) = 854,54 (100%, MH⁺), 876,52 (41%, MNa⁺); - **UV** (H₂O): λ/ [nm] (rel. [a.u.]) = 370 (1), 560 (0,15), 670 (0,2); |
| III | Tosylchlorid (CAS 98-59-9) 190,65 g/mol 95 mg | 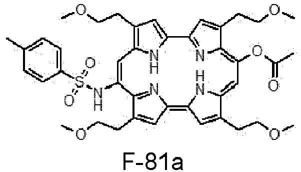 | 38 mg 47 % d.Th. Grünblauer Feststoff | 769,92 g/mol C₄₁H₄₇N₅O₈S | **¹H-NMR** (300 MHz, CDCl₃): δ[ppm] = 11,38 (s, 1H), 9,78 (s, 1H), 9,40 (s, 1H), 9,17 - 9,28 (m, 4H), 7,78 (d, J = 8,2 Hz, 2H), 7,16 (d, J = 8,2 Hz, 2H), 4,08 - 4,36 (m, 14H), 4,01 (m, 2H), 3,63 (s, 3H), 3,61 (s, 3H), 3,59 (s, 3H), 3,53 (s, 3H), 2,89 (s, 3H), 2,63 (s, 3H); - **MS** (ESI-MS, 95% H₂O + 0,1% HCOOH / 5% MeCN + 0,1% HCOOH): e/z (%) = 770,32 (100%, MH⁺), 1539,63 (35%, (2M+H⁺)⁺); - **UV** (H₂O): λ/ [nm] (rel. [a.u.]) = 375 (1), 560 (0,2), 605 (0,25), 630 (0,25); |

**Tabelle 21. Mit allgemeiner Vorschrift p2) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Diglykolsäureanhydrid (CAS 4480-83-5) 116,07 g/mol 34 mg | 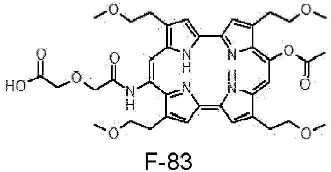 | 20 mg 56 % d.Th. Dunkelblauer Feststoff | 731,81 g/mol C₃₈H₄₅N₅O₁₀ | **¹H-NMR** (300 MHz, MeOD): δ[ppm] = 9,38 (s, 1H), 9,01 (s, 1H), 7,75 - 8,84 (m, 4H), 4,12 - 4,27 (m, 20H), 3,64 (s, 3H), 3,59 (s, 3H), 3,56 (s, 3H), 3,51 (s, 3H), 2,82 (s, 3H); - **MS** (ESI-MS, 95% H₂O + 0,1% HCOOH / 5% MeCN + 0,1% HCOOH): e/z (%) = 732,32 (100%, MH⁺), 1463,64 (19%, (2M+H⁺)⁺); - **UV** (H₂O): λ/ [nm] (rel. [a.u.]) = 370 (1), 560 (0,18), 605 (0,23), 630 (0,22); |
| II | Diglykolsäureanhydrid (CAS 4480-83-5) 116,07 g/mol 34 mg | 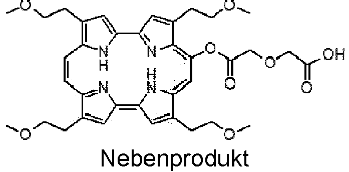 | 3 mg 8 % d.Th. Dunkelblauer Feststoff | 673,74 g/mol C₃₆H₄₁N₄O₉ | **MS** (ESI-MS, 95% H₂O + 0,1% HCOOH / 5% MeCN + 0,1% HCOOH): e/z (%) = 674,30 (100%, MH⁺), 1347,58 (6%, (2M+H⁺)⁺); |
| III | 3-Dodecyldihydrofuran-2,5-dion (CAS 2561-85-5) 268,4 g/mol 81 mg | 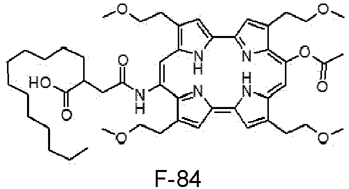 | 19 mg 43 % d.Th. Dunkelblauer Feststoff | 884,14 g/mol C₅₀H₆₉N₅O₉ | **¹H-NMR** (300 MHz, CDCl₃): δ[ppm] = 11,12 (s, 1H), 9,30 (s, 1H), 8,82 (m, 2H), 8,71 (s, 1H), 8,37 (s, 2H), 7,16 (d, J = 8,2 Hz, 2H), 3,96 - 4,38 (m, 16H), 3,82 (m, 1H), 3,71 (s, 3H), 3,68 (s, 3H), 3,52 (s, 3H), 3,41 (s, 3H), 3,38 - 3,46 (m, 2H), 2,92 (s, 3H), 1,61 (m, 2H), 1,21 - 1,36 (m, 20H), 0,88 (m, 3H); - **MS** (ESI-MS, 95% H₂O + 0,1% HCOOH / 5% MeCN + 0,1% HCOOH): e/z (%) = 884,52 (100%, MH⁺); - **UV** (H₂O): λ/ [nm] (rel. [a.u.]) = 374 (1), 562 (0,19), 627 (0,26); |
| IV | 3-Dodecyldihydrofuran-2,5-dion (CAS 2561-85-5) 268,4 g/mol 81 mg | 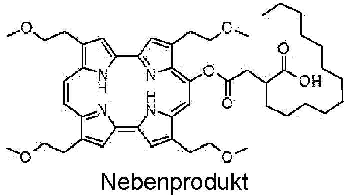 | 2 mg 4 % d.Th. Dunkelblauer Feststoff | 826,07 g/mol C₄₈H₆₅N₄O₈ | **MS** (ESI-MS, 95% H₂O + 0,1% HCOOH / 5% MeCN + 0,1% HCOOH): e/z (%) = 828,45 (100%, MH⁺); - **UV** (H₂O): λ/ [nm] (rel. [a.u.]) = 371 (1), 562 (0,18), 610 (0,21), 628 (0,27); |

**Tabelle 22. Mit allgemeiner Vorschrift q) hergestellte Verbindungen**

| No | Reaktant Molmasse Einwaage | Zielverbindung | Ausbeute | Molekülmasse / Summenformel | Analytik |
|---|---|---|---|---|---|
| I | F-81a 769,92 g/mol 77 mg | 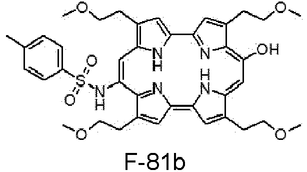 | 55 mg 72 % d.Th. Schwarzblauer Feststoff | 727,88 g/mol C₃₉H₄₅N₅O₇S | **MS** (ESI-MS, 95% H₂O + 0,1% HCOOH / 5% MeCN + 0,1% HCOOH): e/z (%) = 728,31 (100%, MH⁺); |
| II | F-80a 854,15 g/mol 85 mg | 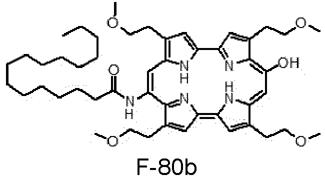 | 53 mg 65 % d.Th. Blauer Feststoff | 812,06 g/mol C₄₈H₆₉N₅O₆ | **MS** (ESI-MS, 95% H₂O + 0,1% HCOOH / 5% MeCN + 0,1% HCOOH): e/z (%) = 812,53 (100%, MH⁺); - **UV** (H₂O): λ/ [nm] (rel. [a.u.]) = 370 (1), 405 (0,66), 570 (0,15), 670 (0,2); |

**Tabelle 23. Mit allgemeiner Vorschrift r) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-66 600,7 g/mol 60 mg | 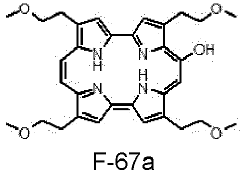 | 52 mg 93 % d.Th. Violett schimmerndes, dunkelblaues feines Pulver | 558,68 g/mol C₃₂H₃₈N₄O₅ | **¹H-NMR** (300 MHz, CDCl₃): δ[ppm] = 11,12 (bs, 1H), 9,49 (m, 1H), 9,28 (m, 2H), 8,92 - 9,07 (m, 3H), 4,08 - 4,36 (m, 16H), 3,61 (s, 6H), 3,59 (s, 3H), 3,57 (s, 3H); - **MS** (ESI-MS, 95% H₂O + 0,1% HCOOH / 5% MeCN + 0,1% HCOOH): e/z (%) = 559,29 (84%, MH⁺), 1117,58 (100%, (2M+H⁺)⁺); - **UV** (n-Octanol): λ/ [nm] (ε / [M⁻¹cm^{- 1}]) = 368 (135400), 388 (83500), 563 (17800), 638 (36600), 677 (23600); |
| II | F-79a 657,8 g/mol 66 mg | 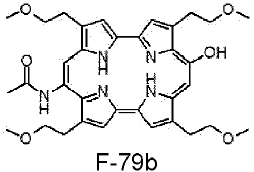 | 51 mg 83 % d.Th. Dunkelblaues feines Pulver | 615,74 g/mol C₃₄H₄₁N₅O₆ | **MS** (ESI-MS, 95% H₂O + 0,1% HCOOH / 5% MeCN + 0,1% HCOOH): e/z (%) = 616,31 (100%, MH⁺), 1231,62 (7%, (2M+H⁺)⁺); - **UV** (H₂O): λ/ [nm] (rel. [a.u.]) = 365 (1), 390 (0,66), 562 (0,21), 633 (0,27); |

**Tabelle 24. Mit allgemeiner Vorschrift s) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Zink(II)acetat (CAS 557-34-6) 183,48 g/mol 75 mg | 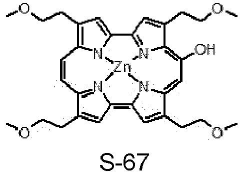 | 24 mg 20 % d.Th. Schmutzig blaues Pulver | 624,05 g/mol C₃₂H₃₈N₄O₅Zn | **MS** (ESI-MS, 95% H₂O + 0,1% HCOOH / 5% MeCN + 0,1% HCOOH): e/z (%) = 311,1 (19%, M²⁺), 622,2 (100%, M⁺); |
| | | | | | |

**Tabelle 25. Mit allgemeiner Vorschrift t) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-66 600,7 g/mol 60 mg | 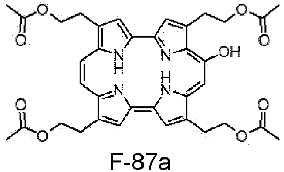 | 21 mg 31 % d.Th. Dunkelblaues feines Pulver | 670,3 g/mol C₃₆H₃₈N₄O₉ | **¹H-NMR** (300 MHz, CDCl₃): δ[ppm] = 11,18 (bs, 1H), 9,58 (m, 1H), 9,37 (m, 1H), 8,92 - 9,18 (m, 4H), 8,48 (s, 1H), 5,03 (m, 2H), 4,56 (m, 4H), 4,42 (m, 4H), 4,22 - 4,38 (m, 6H), 2,23 (s, 3H), 2,21 (s, 6H), 2,14 (s, 3H); - **MS** (ESI-MS, 95% H₂O + 0,1% HCOOH / 5% MeCN + 0,1% HCOOH): e/z (%) = 671,27 (100%, MH⁺), 1341,54 (29%, (2M+H⁺)⁺); - **UV** (H₂O): λ/ [nm] (rel. [a.u.]) = 364 (1), 386 (0,63), 558 (0,21), 636 (0,24); |
| II | F-66 600,7 g/mol 60 mg | 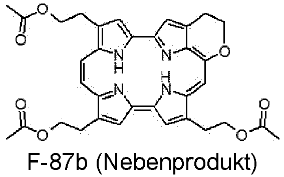 | 8 mg 13 % d.Th. Schwarzblaues feines Pulver | 610,67 g/mol C₃₄H₃₄N₄O₇ | **¹H-NMR** (300 MHz, CDCl₃): δ[ppm] = 9,61 (m, 1H), 9,50 (m, 1H), 9,21 - 9,28 (m, 3H), 9,17 (s, 1H), 8,98 (s, 1H), 5,25 (m, 2H), 5,01 (m, 6H), 4,25 - 4,39 (m, 6H), 4,08 (m, 2H), 2,22 (s, 3H), 2,20 (s, 3H), 2,17 (s, 3H); - **MS** (ESI-MS, 95% H₂O + 0,1% HCOOH / 5% MeCN + 0,1% HCOOH): e/z (%) = 611,25 (100%, MH⁺), 633,23 (13%, MNa⁺), 1221,49 (49%, (2M+H⁺)⁺); - **UV** (H₂O): λ/ [nm] (rel. [a.u.]) = 371 (1), 554 (0,33), 626 (0,17); |

**Tabelle 26. Mit allgemeiner Vorschrift u1) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-67a 558,7 g/mol 56 mg | 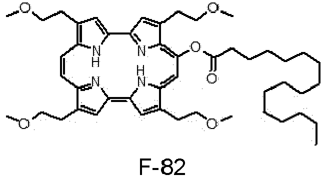 | 61 mg 77 % d.Th. Dunkelblauer Feststoff | 797,10 g/mol C₄₈H₆₈N₄O₆ | **¹H-NMR** (400 MHz, CDCl₃): δ [ppm] = 9,78 (d, J = 10,8 Hz, 1H), 9,70 (d, J = 11,2 Hz, 1H), 9,45 (s, 1H), 9,36 (m, 3H), 4,31 (m, 14H), 4,19 (t, J = 7,6 Hz, 2H), 3,62 (s, 3H), 3,61 (s, 6H), 3,61 (s, 3H), 3,60 (s, 3H), 3,20 (t, J = 8,0 Hz, 2H), 2,14 (quint., J = 7,6 Hz, 2H), 1,72 (quint, |
| | | | | | 8,0 Hz, 2H), 1,64 (m, 2H), 1,55 (m, 18H), 0,88 (m, 3H); - **MS** (ESI-MS, 95% H₂O + 0,1% HCOOH / 5% MeCN + 0,1% HCOOH): e/z (%) = 797,52 (100%, MH⁺), 819,50 (27%, MNa⁺); - **UV** (n-Octanol): λ/ [nm] (ε / [M⁻¹cm⁻¹]) = 372 (132200), 565 (27500), 607 (31700), 634 (30800); |

**Tabelle 27. Mit allgemeiner Vorschrift u2) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Methyliodid (CAS 74-88-4) 141,94 g/mol 141 mg | 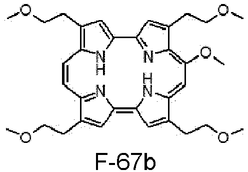 | 122 mg 71 % d.Th. Blauer, amorpher Feststoff | 572,71 g/mol C₃₃H₄₀N₄O₅ | **¹H-NMR** (400 MHz, CDCl₃): δ [ppm] = 9,67 (d, J = 11,2 Hz, 1H), 9,49 (d, J = 10,8 Hz, 1H), 9,34 (s, 1H), 9,27 (m, 3H), 4,78 (s, 3H), 4,30 (m, 16H), 3,63 (s, 3H), 3,61 (s, 3H), 3,60 (s, 6H); - **MS** (ESI-MS, 95% H₂O + 0,1% HCOOH / 5% MeCN + 0,1% HCOOH): e/z (%) = 573,31 (100%, MH⁺), 1145,61 (76%, (2M+H⁺)⁺); |
| II | 3-Brom-1-propanol (CAS 627-18-9) 139,0 g/mol 139 mg | 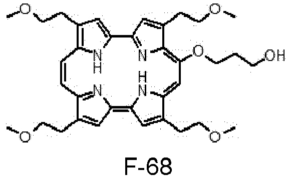 | 152 mg 82 % d.Th. Blauer, amorpher Feststoff | 616,76 g/mol C₃₅H₄₄N₄O₆ | **¹H-NMR** (300 MHz, CDCl₃): δ[ppm] = 9,64 (d, J = 10,2 Hz, 1H), 9,46 (d, J = 10,2 Hz, 1H), 9,39 (s, 1H), 9,33 (s, 4H), 5,16 (m, 2H), 4,18 - 4,34 (m, 18H), 3,71 (m, 2H), 3,61 (s, 3H), 3,59 (s, 6H), 3,58 (s, 3H), 2,62 (m, 2H); - MS (ESI-MS, 95% H₂O + 0,1% HCOOH / 5% MeCN + 0,1% HCOOH): e/z (%) = 617,33 (100%, MH⁺), 1233,66 (61%, (2M+H⁺)⁺); - UV (n-Octanol): λ/ [nm] (ε / [M⁻¹cm⁻¹]) = 366 (131000), 386 (88900), 565 (27300), 634 (42700), 683 (23500); |
| III | Triethylenglykol monotosylat (CAS 77544-68-4) 304,3 g/mol 304 mg | 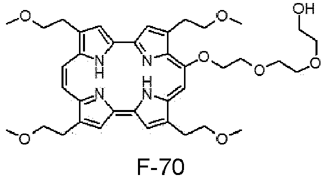 | 147 mg 71 % d.Th. Blaues, violett schimmerndes Glas | 690,84 g/mol C₃₈H₅₀N₄O₈ | **¹H-NMR** (400 MHz, CDCl₃): δ[ppm] = 9,63 (d, J = 10,4 Hz, 1H), 9,45 (d, J = 10,4 Hz, 1H), 9,25 (s, 1H), 9,24 (s, 1H), 9,22 (s, 1H), 9,20 (s, 1H), 9,19 (s, 1H), 5,07 (m, 2H), 4,15 - 4,36 (m, 18H), 3,96 (m, 2H), 3,85 (m, 2H), 3,77 (m, 2H), 3,68 (m, 2H), 3,61 (s, 3H), 3,60 (s, 3H), 3,60 (s, 3H), 3,59 (s, 3H); - **MS** (ESI-MS, 95% H₂O + 0,1% HCOOH / 5% MeCN + 0,1% HCOOH): e/z (%) = 691,37 (100%, MH⁺), 1381,74 (83%, (2M+H⁺)⁺); - **UV** (n-Octanol): λ/ [nm] (ε / [M⁻¹cm⁻¹]) = 366 (134000), 387 (91200), 565 (32600), 632 (56700), 682 (19800); |
| IV | 1,3-Diiodpropan (CAS 627-31-6) 295,8 g/mol 296 mg | 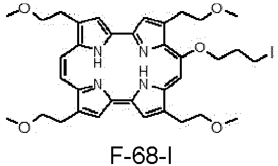 | 155 mg 67 % d.Th. Blauviolettes Glas | 726,65 g/mol C₃₅H₄₃N₄O₅I | **¹H-NMR** (300 MHz, CDCl₃): δ[ppm] = 9,63 (d, J = 10,3 Hz, 1H), 9,47 (d, J = 10,3 Hz, 1H), 9,33 (s, 1H), 9,27 (s, 4H), 5,01 (m, 2H), 4,19 - 4,32 (m, 16H), 3,79 (m, 2H), 3,61 (s, 3H), 3,60 (s, 6H), 3,59 (s, 3H), 2,81 (m, 2H); |
| V | 3-(Boc-amino)-propylbromid (CAS 83948-53-2) 175,2 g/mol 175 mg | 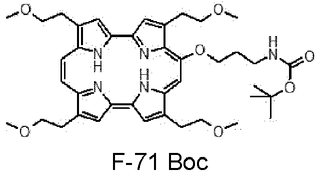 | 165 mg 77 % d.Th. Blaues Glas | 715,89 g/mol O₁₀H₅₃N₅O₇ | **¹H-NMR** (300 MHz, CDCl₃): δ[ppm] = 9,62 (d, J = 10,4 Hz, 1H), 9,42 (d, J = 10,4 Hz, 1H), 9,18 - 9,24 (m, 5H), 5,27 (bs, 1H), 4,93 (m, 2H), 4,16 - 4,33 (m, 16H), 3,71 (m, 2H), 3,60 (s, 3H), 3,58 (s, 6H), 3,57 (s, 3H), 2,56 (m, 2H), 1,49 (s, 1H); - **MS** (ESI-MS, 95% H₂O + 0,1% HCOOH / 5% MeCN + 0,1% HCOOH): e/z (%) = 716,42 (100%, MH⁺), 1431,79 (3%, (2M+H⁺)⁺); - **UV** (H₂O): λ/ [nm] (rel. [a.u.]) = 366 (1), 560 (0,21), 629 (0,24); |
| VI | Methyl 4-Bromobutyrat (CAS 4897-84-1) 181,03 g/mol 181 mg | 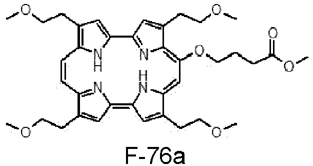 | 132 mg 67 % d.Th. Blauer amorpher Feststoff | 658,80 g/mol C₃₇H₄₆N₄O₇ | **¹H-NMR** (400 MHz, CDCl₃): δ[ppm] = 9,63 (d, J = 10,4 Hz, 1H), 9,45 (d, J = 10,4 Hz, 1H), 9,20 - 9,26 (m, 5H), 4,95 (m, 2H), 4,16 - 4,33 (m, 16H), 3,82 (s, 3H), 3,61 (s, 12H), 2,93 (t, J = 7,1 Hz, 2H), 2,68 (m, 2H); - **MS** (ESI-MS, 95% H₂O + 0,1% HCOOH / 5% MeCN + 0,1% HCOOH): e/z (%) = 659,34 (100%, MH⁺), 1317,68 (18%, (2M+H⁺)⁺); - **UV** (H₂O): λ/ [nm] (rel. [a.u.]) = 368 (1), 560 (0,22), 626 (0,36); |
| VII | Methyl 3-bromo-2-methylpropanoate (CAS 20609-71-6) 181,03 g/mol 181 mg | 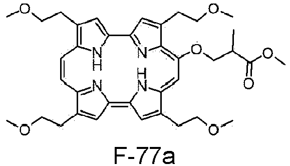 | 61 mg 30 % d.Th. Blauer amorpher Feststoff | 658,80 g/mol C₃₇H₄₆N₄O₇ | **¹H-NMR** (400 MHz, CDCl₃): δ[ppm] = 9,64 (d, J = 10,4 Hz, 1H), 9,46 (d, J = 10,4 Hz, 1H), 9,19 - 9,25 (m, 5H), 4,16 - 4,34 (m, 16H), 3,71 (s, 3H), 3,62 (s, 3H), 3,61 (s, 6H), 3,59 (s, 3H), 3,46 - 3,64 (m, 2H), 2,88 (m, 1H), 1,34 (d, J = 5,4 Hz, 3H); - **MS** (ESI-MS, 95% H₂O + 0,1% HCOOH / 5% MeCN + 0,1% HCOOH): e/z (%) = 659,34 (100%, MH⁺), 1317,68 (10%, (2M+H⁺)⁺); - **UV** (H₂O): λ/ [nm] (rel. [a.u.]) = 367 (1), 558 (0,28), 627 (0,39); |
| VIII | tert-Butyl 1-(methoxycarbonyl)-3-bromopropyl carbamate (CAS 168077-38-1) 296,16 g/mol 296 mg | 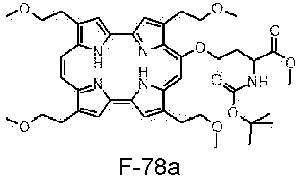 | 77 mg 33 % d.Th. Blauer amorpher Feststoff | 773,93 g/mol C₄₂H₅₅N₅O₉ | **¹H-NMR** (400 MHz, CDCl₃): δ[ppm] = 9,63 (d, J = 10,4 Hz, 1H), 9,46 (d, J = 10,4 Hz, 1H), 9,29 (s, 1H), 9,20 - 9,25 (m, 4H), 5,81 (m, 1H), 4,98 (m, 2H), 4,89 (m, 1H), 4,19 - 4,34 (m, 16H), 3,82 (s, 3H), 3,62 (s, 3H), 3,61 (s, 6H), 3,59 (s, 3H), 2,90 (m, 1H), 2,71 (m, 1H), 1,51 (s, 9H); - **MS** (ESI-MS, 95% H₂O + 0,1% HCOOH / 5% MeCN + 0,1% HCOOH): e/z (%) = 774,41 (100%, MH⁺), 1547,81 (8%, (2M+H⁺)⁺); - **UV** (H₂O): λ/ [nm] (rel. [a.u.]) = 372 (1), 560 (0,21), 626 (0,33); |
| IX | α-N-(tert-butoxy carbonyl)-glutaminsäure-(3-bromopropyl)amid-tert-butylester ⁽⁹⁾ 423 mg 423,35 g/mol | 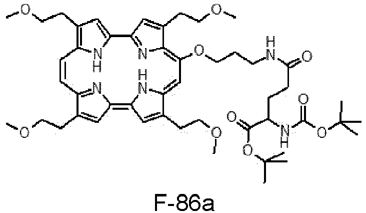 | 127 mg 47 % d.Th. Blauer amorpher Feststoff | 901,12 g/mol C₄₉H₆₈N₆O₁₀ | **¹H-NMR** (600 MHz, CDCl₃): δ[ppm] = 9,65 (d, J = 10,4 Hz, 1H), 9,48 (d, J = 10,4 Hz, 1H), 9,34 (s, 1H), 9,22 - 9,27 (m, 4H), 5,35 (bs, 1H), 5,25 (bs, 1H), 5,02 (m, 2H), 4,18 - 4,36 (m, 16H), 3,86 (m, 2H), 3,61 (s, 3H), 3,59 (s, 3H), 3,59 (s, 3H), 3,58 (s, 3H), 3,38 (m, 2H), 2,61 (m, 2H), 2,37 (m, 1H), 2,27 (m, 2H), 1,46 (s, 9H), 1,25 (s, 9H); - **MS** (ESI-MS, 95% H₂O + 0,1% HCOOH / 5% MeCN + 0,1% HCOOH): e/z (%) = 901,51 (100%, MH⁺); |

### (9) Dieser Ausgangsstoff wurde durch Peptidkupplung mit HOBt/EDC*HCl/DIPEA in DMF aus N-Boc- glutaminsäure-1-tert.-butylester und 3-Bromopropylamin Hydrobromid erhalten. Weisser, wachsartiger Feststoff (74% d.Th.); ¹H-NMR (600 MHz, CDCl₃): δ[ppm] = 1,42 (s, 9H), 1,45 (s, 9H), 1,85 (m, 1H), 2,03 (m, 2H), 2,15 (m, 2H), 2,27 (t, J = 11,6 Hz, 2H), 3,37 (m, 2H), 3,57 (t, J = 11,6 Hz, 2H), 5,27 (bs, 1H), 6,55 (bs, 1H);

**Tabelle 28. Mit allgemeiner Vorschrift u3) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | N-Bocglutaminsäure-1-tert.-butylester (CAS 24277-39-2) 303,35 g/mol 151 mg | 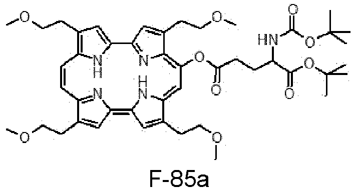 | 124 mg 74 % d.Th. Blauer, amorpher Feststoff | 844,03 g/mol C₄₆H₆₁N₅O₁₀ | **¹H-NMR** (400 MHz, CDCl₃): δ[ppm] = 9,52 - 9,68 (m, 3H), 9,22 - 9,34 (m, 4H), 5,51 (m, 1H), 4,60 (m, 1H), 4,22 - 4,35 (m, 14H), 4,17 (m, 2H), 3,62 (s, 6H), 3,60 (s, 6H), 3,34 (m, 2H), 2,71 (m, 1H), 2,68 (m, 1H), 2,34 (m, 1H), 1,58 (s, 9H), 1,27 (s, 9H); - **MS** (ESI-MS, 95% H₂O + 0,1% HCOOH / 5% MeCN + 0,1% HCOOH): e/z (%) = 844,45 (100%, MH⁺), 1005,45 (12%, (2M+H⁺)⁺); - **UV** (H₂O): λ/ [nm] (rel. [a.u.]) = 365 (1), 555 (0,21), 600 (0,21), 630 (0,24); |

**Tabelle 29. Mit allgemeiner Vorschrift w) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | Trimethylamin (CAS 75-50-3) 79,1 g/mol 158 mg | 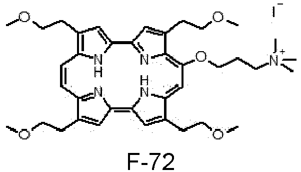 | 132 mg 84 % d.Th. Blaues feines Pulver | 785,77 g/mol C₃₈H₅₂N₅O₅I | **¹H-NMR** (300 MHz, CD₃OD): δ[ppm] = 9,68 (d, J = 10,4 Hz, 1H), 9,49 (d, J = 10,4 Hz, 1H), 9,32 - 9,38 (m, 3H), 9,03 (s, 1H), 8,69 (s, 1H), 4,58 (m, 2H), 4,16 - 4,35 (m, 12H), 3,93 - 4,07 (m, 4H), 3,56 - 3,63 (m, 2H), 3,59 (s, 3H), 3,56 (s, 6H), 3,53 (s, 3H), 3,36 (m, 2H), 3,02 (s, 9H), 1,84 (m, 2H); - **MS** (ESI-MS, 95% H₂O + 0,1% HCOOH / 5% MeCN + 0,1% HCOOH): e/z (%) = 329,70 (83%, (M⁺+H⁺)²⁺), 658,40 (100%, M⁺); - **UV** (n-Octanol): λ/ [nm] (ε / [M⁻¹cm⁻¹]) = 371 (128000), 389 (86800), 564 (35600), 632 (56000), 680 (23600); |
| II | Pyridin (CAS 110-86-1) 59,1 g/mol 118 mg | 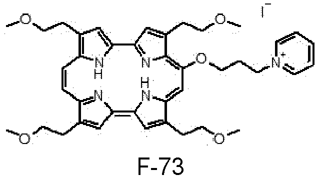 | 148 mg 92 % d.Th. Blaues feines Pulver | 805,76 g/mol C₄₀H₄₈N₅O₅I | **¹H-NMR** (300 MHz, DMSO-d6): δ[ppm] = 9,81 (d, J = 10,4 Hz, 1H), 9,51 - 9,68 (m, 5H), 9,38 (s, 1H), 9,31 (d, J = 6,6 Hz, 2H), 8,22 (m, 3H), 5,21 (m, 2H), 5,12 (m, 2H), 4,15 - 4,28 (m, 16H), 3,59 (s, 3H), 3,58 (s, 6H), 3,56 (s, 3H), 3,01 (m, 2H); - **MS** (ESI-MS, 95% H₂O + 0,1% HCOOH / 5% MeCN + 0,1% HCOOH): e/z (%) = 339,690 (57%, (M⁺+H⁺)²⁺), 678,37 (100%, M⁺); |

**Tabelle 30. Mit allgemeiner Vorschrift v) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-76a 658,8 g/mol 66 mg | 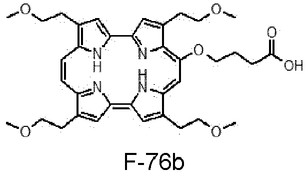 | 55 mg 85 % d.Th. Blaugrünes Pulver | 644,77 g/mol C₃₆H₄₄N₄O₇ | **¹H-NMR** (600 MHz, CDCl₃): δ[ppm] = 9,48 (d, J = 10,4 Hz, 1H), 9,30 (d, J = 10,4 Hz, 1H), 9,13 (s, 1H), 9,10 (s, 1H), 9,01 (s, 1H), 8,94 (s, 1H), 8,93 (s, 1H), 4,73 (t, J = 6,0 Hz, 2H), 4,21 - 4,29 (m, 10H), 4,12 - 4,19 (m, 6H), 3,96 (t, J = 7,2 Hz, 2H), 3,60 (s, 6H), 3,59 (s, 3H), 3,55 (s, 3H), 2,91 (t, J = 7,2 Hz, 2H), 2,55 (m, 2H); - **MS** (ESI-MS, 95% H₂O + 0,1% HCOOH / 5% MeCN + 0,1% HCOOH): e/z (%) = 645,33 (100%, MH⁺), 1289,65 (90%, (2M+H⁺)⁺); - UV (H₂O): λ/ [nm] (rel. [a.u.]) = 366 (1), 558 (0,22), 626 (0,37); |
| II | F-77a 658,8 g/mol 66 mg | 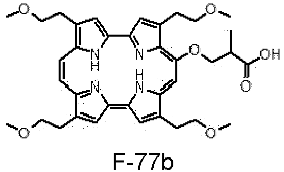 | 51 mg 79 % d.Th. Blaugrünes Pulver | 644,77 g/mol C₃₆H₄₄N₄O₇ | **MS** (ESI-MS, 95% H₂O + 0,1% HCOOH / 5% MeCN + 0,1% HCOOH): e/z (%) = 645,33 (100%, MH⁺), 1289,65 (27%, (2M+H⁺)⁺); - UV (H₂O): λ/ [nm] (rel. [a.u.]) = 366 (1), 556 (0,21), 628 (0,32); |
| III | F-78a Methylester 773,9 g/mol 77 mg | 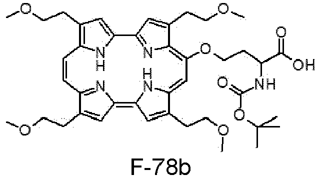 | 55 mg 72 % d.Th. Blaugrünes Pulver | 759,90 g/mol C₄₁H₅₃N₅O₉ | **¹H-NMR** (600 MHz, CDCl₃): δ [ppm] = 9,48 (d, J = 10,2 Hz, 1H), 9,25 (d, J = 11,4 Hz, 1H), 9,13 (s, 3H), 8,80 (m, 1H), 6,98 (s, 1H), 6,03 (s, 1H), 4,94 (m, 2H), 4,27 - 4,12 (m, 16H), 3,66 (s, 3H), 3,62 (s, 3H), 3,61 (s, 3H), 3,58 (s, 3H), 3,55 (m, 1H), 2,77 (m, 2H), 1,43 (s, 9H); - **MS** (ESI-MS, 95% H₂O + 0,1% HCOOH / 5% MeCN + 0,1% HCOOH): e/z (%) = 760,39 (100%, MH⁺), 1519,78 (69%, (2M+H⁺)⁺); - **UV** (H₂O): λ/ [nm] (rel. [a.u.]) = 372 (1), 561 (0,22), 626 (0,37); |

**Tabelle 31. Mit allgemeiner Vorschrift x) hergestellte Verbindungen**

| No | Reaktant Molmasse Einwaage | Zielverbindung / Nummer | Ausbeute / Aussehen | Molekülmasse / Summenformel | Analytik |
|---|---|---|---|---|---|
| I | F-71 Boc 715,89 g/mol 72 mg | 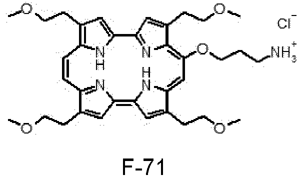 | 65 mg 83 % d.Th. Blaues feines Pulver | 652,24 g/mol C₃₅H₄₆N₅O₅Cl | **¹H-NMR** (600 MHz, CD₃OD): δ[ppm] = 8,84 (d, J = 7,8 Hz, 1H), 8,77 (d, J = 7,8 Hz, 1H), 8,52 - 8,68 (m, 2H), 8,47 (s, 1H), 8,04 (s, 1H), 4,27 (m, 2H), 4,12 (m, 4H), 4,01 (m, 4H), 3,81 - 3,94 (m, 4H), 3,61 - 3,67 (m, 2H), 3,61 (s, 3H), 3,57 (s, 6H), 3,53 (s, 3H), 3,51 (m, 2H), 3,32 (m, 2H), 2,38 (m, 2H); - **MS** (ESI-MS, 95% H₂O + 0,1% HCOOH / 5% MeCN + 0,1% HCOOH): e/z (%) = 309,18 (48%, (M+2H⁺)²⁺), 617,36 (100%, MH⁺); |
| II | F-78b 759,9 g/mol 76 mg | 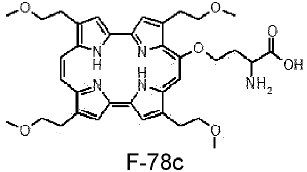 | 45 mg 68 % d.Th. Blaues Glas | 659,79 g/mol C₃₆H₄₅N₅O₇ | **MS** (ESI-MS, 95% H₂O + 0,1% HCOOH / 5% MeCN + 0,1% HCOOH): e/z (%) = 330,67 (94%, (M⁺+H⁺)²⁺), 660,34 (100%, MH⁺); - **UV** (H₂O): λ/ [nm] (rel. [a.u.]) = 366 (1), 562 (0,21), 625 (0,31); |
| III | F-85a 844,03 g/mol 84 mg | 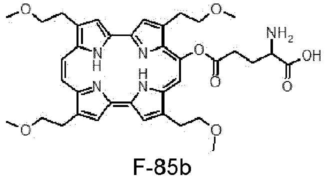 | 43 mg 62 % d.Th. Blaues Glas | 687,80 g/mol C₃₇H₄₅N₅O₈ | **MS** (ESI-MS, 95% H₂O + 0,1% HCOOH / 5% MeCN + 0,1% HCOOH): e/z (%) = 344,67 (100%, (M⁺+H⁺)²⁺), 688,33 (34%, MH⁺); - UV (H₂O): λ/ [nm] (rel. [a.u.]) = 365 (1), 390 (0,75), 570 (0,17), 608 (0,19), 633 (0,22); |
| IV | F-86a 901,12 g/mol 90 mg | 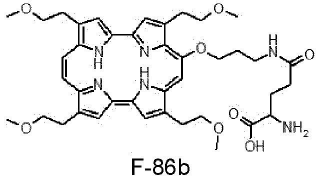 | 42 mg 57 % d.Th. Blaues Glas | 744,90 g/mol C₄₀H₅₂N₆O₈ | **MS** (ESI-MS, 95% H₂O + 0,1% HCOOH / 5% MeCN + 0,1% HCOOH): e/z (%) = 373,20 (30%, (M⁺+H⁺)²⁺), 745,39 (100%, MH⁺); - **UV** (H₂O): λ/ [nm] (rel. [a.u.]) = 368 (1), 391 (0,68), 562 (0,21), 626 (0,34); |

### Beispiel 2: Herstellung und Anwendung wasserbasierter Formulierungen

Die nachfolgenden Beispiele zeigen die Herstellung und Anwendung wasserbasierter Formulierungen, mit denen Photokatalysatoren nachträglich in Kunststofffolien eingebracht wurden ("Imprägnieren"). Ziel war es die Folien mit einem antimikrobiellen Oberflächeneffekt, zur Konservierung oder Geruchsbekämpfung ausstatten.

Zunächst sind verschiedene Herstellvorschriften der Imprägnierlösungen sowie mögliche Rezepturen beschrieben. Mit den so erzeugten Lösungen wurden verschiedene Kunststofffolien imprägniert und so festgestellt, welche Lösung für welchen Kunststoff geeignet waren. Anschließend wurden verschiedene Photokatalysatorklassen getestet und ebenfalls festgestellt, welcher Photokatalysator für welchen Kunststoff geeignet war. Für ausgewählte Beispiele wurden Auswaschversuche und weitere Belastungstests durchgeführt um die Robustheit des Verfahrens zu zeigen. Ebenso wurden für ausgewählte Bespiele mikrobiologische Tests auf eine antimikrobielle Wirkung durchgeführt, sowohl mit als auch ohne zusätzliche Belastungen (künstliche Alterung, Abrieb u.ä.).

Folgende Substanzen wurden neben den Photokatalysatoren und Wasser für die Bereitstellung der untersuchten Formulierungen verwendet:

**Tabelle 32. Verwendete Chemikalien und Abkürzungen; "reinst" bedeutet je nach Herstellerspezifikation > 96 - 98%; nicht näher angegeben**

| Kurzbezeichnung / Trivialname | Systematischer Name | CAS No | Gehalt |
|---|---|---|---|
| DMS | Dimethylsuccinat | 106-65-0 | > 98% |
| EA | Ethylacetat | 141-78-6 | > 99,8% |
| IPA | 2-Propanol / Isopropanol | 67-63-0 | > 99,5% |
| SDS | Natriumdodecylsulfat | 151-21-3 | > 97% |
| EDTA (Natriumsalz) | Dinatriumethylendiamintetraessigsäure | 6381-92-6 | reinst |
| MGDA (Natriumsalz) | Methylglycindiessigsäure-Trinatriumsalz | 164462-16-2 | reinst |
| MIT | Methylisothiazolinon | 2682-20-4 | reinst |
| MPA | 1-Methoxy-2-propylacetat | 108-65-6 | > 99% |
| MEK | Butanon | 78-93-3 | 99,5% |
| Aceton | Propan-2-on | 67-64-1 | > 99,5% |
| Heptanon | 3-Heptanon | 123-19-3 | reinst |
| DCA | Diacetonalkohol | 123-42-2 | reinst |
| Texanol | 2,2,4-Trimethyl-1,3-pentandiolmonoisobutyrat | 25265-77-4 | reinst |
| Adipinketon | Cyclopentanon | 120-92-3 | reinst |
| Pimelinketon | Cyclohexanon | 108-94-1 | > 99,5% |
| Tween 20 | Polyoxyethylen(20)-sorbitanmonolaurat | 9005-64-5 | reinst |
| Tween 60 | Polyoxyethylen(60)-sorbitanmonolaurat | 9005-67-8 | reinst |
| Docusat Natrium | Natriumdioctylsulfosuccinat | 577-11-7 | reinst |
| Brij35 | Polyethylenglycollaurylether | 9002-92-0 | reinst |
| Brij58 | Polyoxyethylen-(20)-cetylether | 9004-95-9 | reinst |
| Laureth-22 | Natriumdodecyl-polyoxyethylen-(22)-sulfat | 68439-50-9 | reinst |
| Butylenglycol | 1,2-Butandiol | 584-03-2 | > 98% |
| 1,4-Butandiol | 1,4-Butandiol | 110-63-4 | reinst |
| Propylenglykol | 1,2-Propandiol | 57-55-6 | > 99,5% |
| PDO | 1,3-Propandiol | 504-63-2 | > 98% |
| Diglycerin | Diglycerin | 59113-36-9 | reinst |
| Pentylenglycol | 1,2-Pentandiol | 5343-92-0 | > 98% |
| Pentamethylenglycol | 1,5-Pentandiol | 111-29-5 | > 97% |
| PrOH | n-Propanol | 71-23-8 | reinst |
| Isobutanol | 2-Methyl-1-propanol | 78-83-1 | reinst |
| BuOH | n-Butanol | 71-36-3 | 99,5% |
| sec-Butanol | 2-Butanol | 78-92-2 | reinst |
| sec-Butylcarbinol | 2-Methyl-1-butanol | 137-32-6 | reinst |
| Isoamylalkohol | 3-Methyl-1-butanol | 123-51-3 | > 97% |
| α-Ethylbenzylalkohol | 1-Phenyl-1-propanol | 93-54-9 | reinst |
| Methoxypropanol | 1-Methoxy-2-propanol | 107-98-2 | > 99% |
| Ethoxypropanol | 1-Ethoxy-2-propanol | 1569-02-4 | > 95% |
| MMB | 3-Methoxy-3-methyl-butanol | 56539-66-3 | > 98% |
| 3-Methoxybutanol | 3-Methoxy-1-butanol | 2517-43-3 | reinst |
| EHG | Ethylhexylglycerin | 70445-33-9 | reinst |
| IPE | 2-Isopropoxyethanol | 109-59-1 | > 99% |
| 2-Propoxyethanol | 2-Propoxyethanol | 2807-30-9 | reinst |
| Butylglykol | 2-Buthoxyethanol | 111-76-2 | > 99% |
| Butyldiglykol | 2-(2-Butoxyethoxy)ethanol | 112-34-5 | > 98 % |
| Propylenglykolmonobutylether | 1-Butoxy-2-propanol | 5131-66-8 | reinst |
| DES | Diethylsuccinat | 123-25-1 | > 99% |
| DBE | Dibasischer Ester | - | > 99% |
| DMG | Dimethylglutarat | 1119-40-0 | reinst |
| Isopropylacetat | 2-Propylacetat | 108-21-4 | > 99% |
| PA | n-Propylacetat | 109-60-4 | > 99% |
| PGDA | 1,2-Propylenglykoldiacetat | 623-84-7 | > 99% |
| β-Citronellol | 3,7-Dimethyloct-6-en-1-ol | 106-22-9 | > 99% |

### Herstellvorschriften der Imprägnierlösungen:

### Beispiel 2.1 Allgemeine Herstellvorschriften

Es wurden ein Keton und/oder ein Ester zusammen mit einem ein- oder mehrwertigen Alkohol in einem festen Mischungsverhältnis vermengt, mit deionisiertem Wasser auf die Anwendungsverdünnung eingestellt und bis zur Homogenität gerührt. Der Wasseranteil der anwendungsfertigen Lösung lag bei mindestens 70 Gewichts-%. Die so erhaltenen Basisrezeptur konnten optional Lösungsvermittler, Konservierungsstoffe, Puffer und/oder Komplexbildner zugemischt werden.

Anschließend wurde die entsprechende Menge Photokatalysator (üblicherweise werden Anwendungskonzentrationen von 0,1 bis 2 mM eingestellt) in dieser Basisformulierung gelöst. Die verwendeten Rezepturen sind in den nachfolgenden Tabelle 33 bis 34 dargestellt.

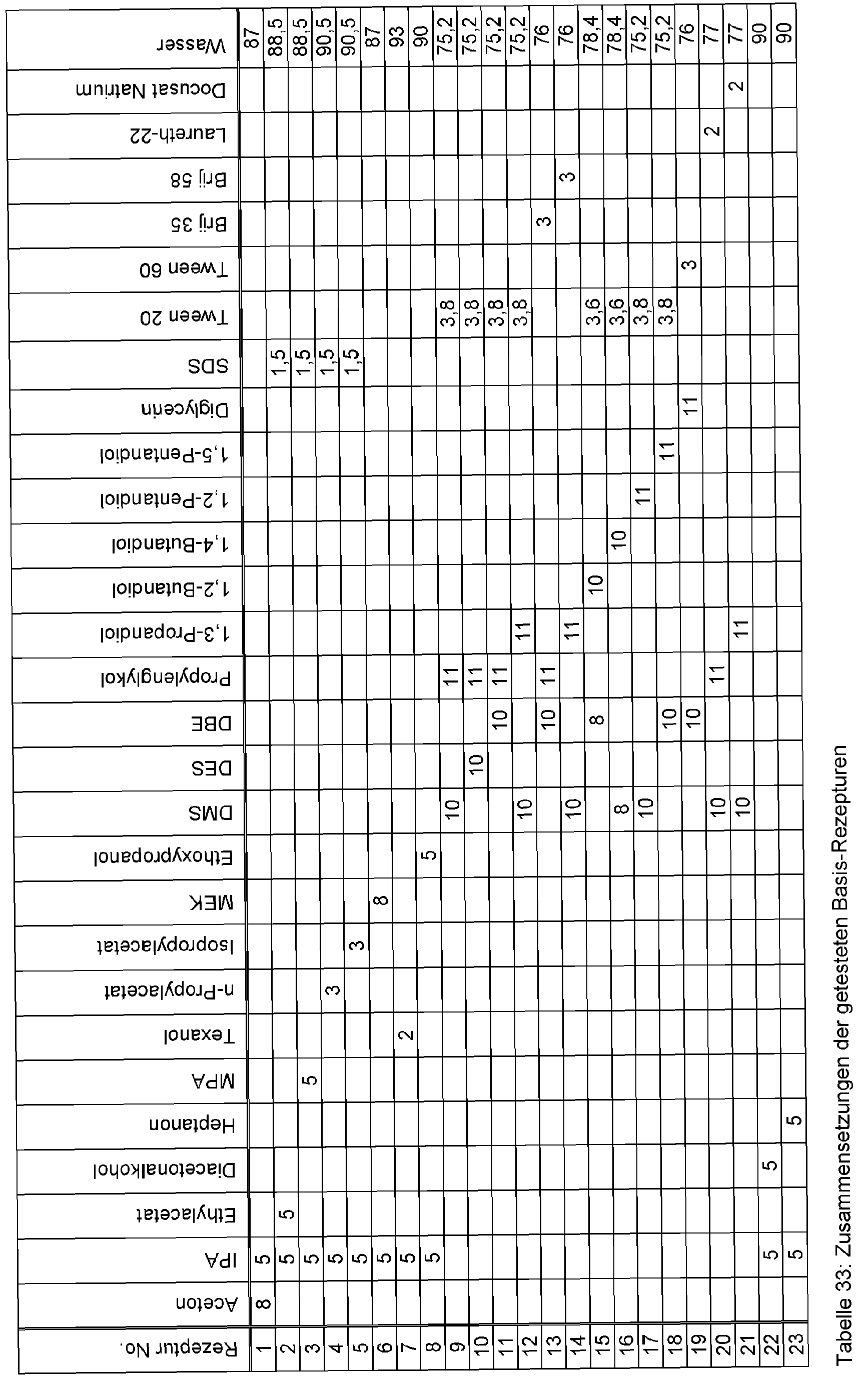

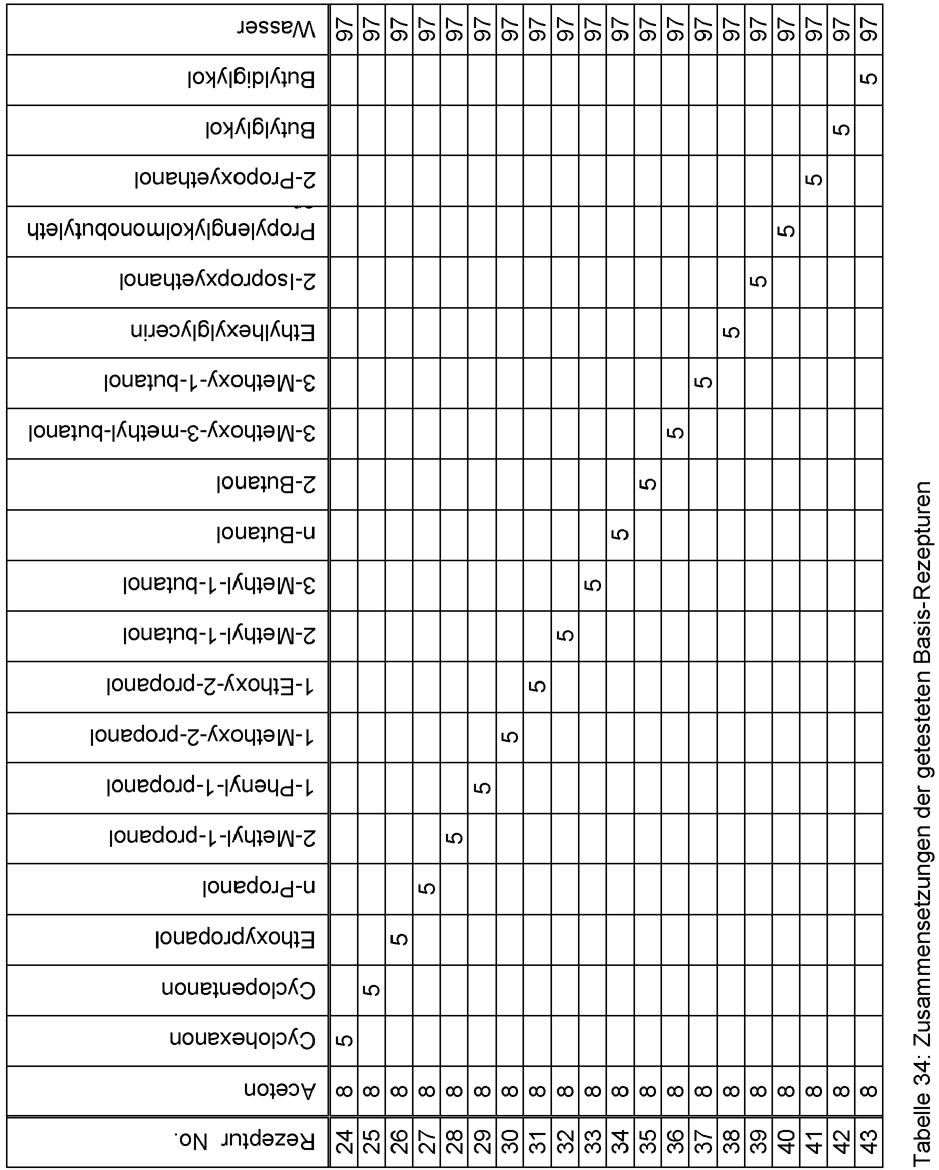

Bevorzugt verwendet für weitere Versuche wurden folgende Basis Rezepturen.:

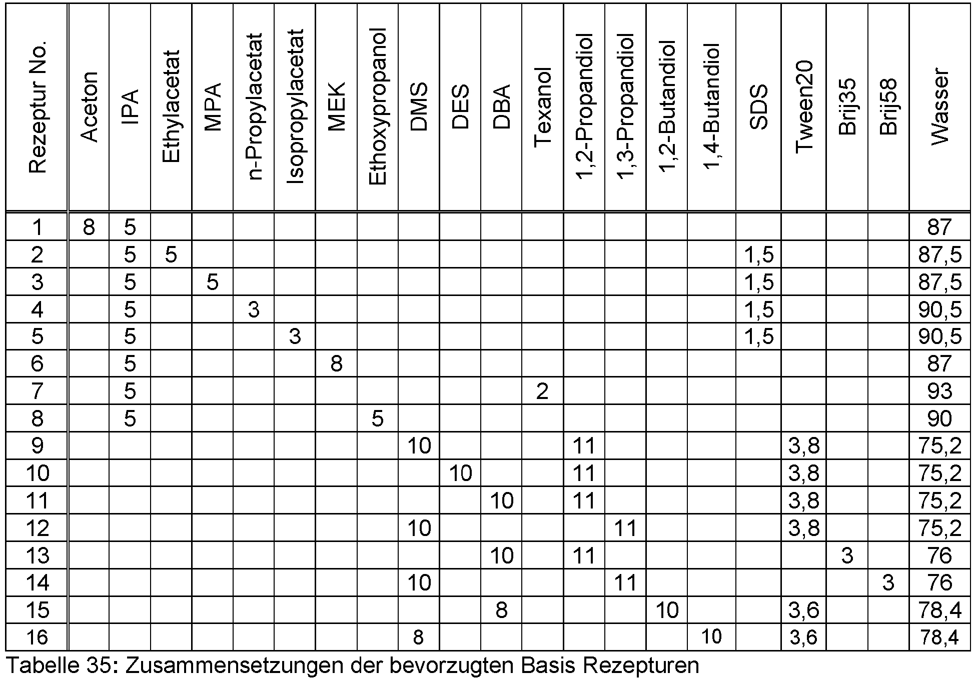

### Beispiel 2.2 Ketonbasierte Formulierung

Es wurden 8,0 g Aceton und 5,0 g Isopropanol mit deionisiertem Wasser auf 100,0 g aufgefüllt und kurz gerührt. Dabei wurde die entsprechende Menge Photokatalysator (üblicherweise werden Anwendungskonzentrationen von 0,1 bis 2 mM eingestellt) eingetragen. Die Lösung war sofort gebrauchsfertig.

Analog wurden mit anderen Komponenten Mischungen hergestellt, deren Zusammensetzung in Tabelle 34, Rezeptur No. 24 - 43 dargestellt sind.

### Beispiel 2.3 Esterbasierte Formulierung

Es wurden 5,0 g Ethylacetat und 5,0 g Isopropanol mit 0,5 g Natriumdodecylsulfat gemischt. Anschließend wurde mit deionisiertem Wasser auf 100,0 g aufgefüllt. Es wurde bis zur homogenen Verteilung gerührt, wobei darauf geachtet wurde, dass sich das Natriumdodecylsulfat vollständig gelöst hatte. Anschließend wurde die entsprechende Menge Photokatalysator, in einer Anwendungskonzentrationen von 0,1 bis 2 mM, in der Basisformulierung gelöst. Die Lösung war sofort gebrauchsfertig.

Analog wurden mit anderen Komponenten Mischungen hergestellt, deren Zusammensetzung in Tabelle 35, Rezeptur No. 46 - 48 dargestellt sind.

### Beispiel 2.4 Esterbasierte Formulierung mit Lösungsvermittler

Es wurden 10,0 g Dimethylsuccinat, 11,0 g 1,2-Propandiol und 3,8 g Tween 20 gemischt.

Anschließend wurde mit deionisiertem Wasser auf 100,0 g aufgefüllt und zur homogenen Verteilung gerührt. Dabei wurde die entsprechende Menge Photokatalysator, in einer Anwendungskonzentrationen von 0,1 bis 2 mM, eingetragen. Die Lösung war sofort gebrauchsfertig.

Analog wurden mit anderen Komponenten Mischungen hergestellt, deren Zusammensetzung in Tabelle 33, Rezeptur No. 9 bis 21dargestellt sind.

### Beispiel 2.5 Zusatzstoffe

Die Rezepturen 1 bis 7 wurden weiterhin mit 0,5% w/w EDTA (Natriumsalz) versetzt und bis zur homogenen Verteilung gerührt. Die Rezepturen 8 bis 16 wurden mit 0,5% w/w Phenoxyethanol und 0,5% w/w EDTA (Natriumsalz) versetzt und zur homogenen Verteilung gerührt.

Zum Vergleich wurden Rezepturen 1 bis 3 mit 0,05% w/w Methylisothiazolinon und 0,5% w/w MGDA (Natriumsalz) versetzt und zur homogenen Verteilung gerührt.

In allen Fällen wurden homogene, klare, gelblich gefärbte Lösungen erhalten. Diese waren über > 6 Monate bei Raumtemperatur im dicht verschlossenen Behälter stabil.

### Beispiel 3: Imprägnier-Verfahren

Im nachfolgenden Beispiel 3 wurde mit den in Beispiel 2 bereitgestellten Lösungen Folien mit Photokatalysatoren imprägniert. Dabei wurde der Photokatalysator durch einen reversiblen Quellungsprozess oberflächennah ins Material eingebracht.

Es wurden ca. 10 cm x 10 cm große Folienstücke zugeschnitten und gründlich mit deionisiertem Wasser und/oder 5%iger Neutralseifelösung gereinigt, um sie fett- und staubfrei zu hinterlassen. Die oben beschriebene Formulierung wurde auf eine Temperatur von 40 bis 60°C in einem Glas- oder Metallgefäß vorgewärmt. Die Folienstücke wurden in die warme Imprägnierlösung eingelegt, so dass diese vollständig benetzt wurden und beide Seiten der Folie mit der Lösung in Kontakt stehen. Je nach Material und gewünschtem Eintrag an Photokatalysator wurde das Verfahren des Imprägnierens für 1 bis 30 min durchgeführt. Nach dem Prozess wurden die Folienstücke entnommen und mit deionisiertem Wasser gründlich abgewaschen. Die Folien wurden an der Luft getrocknet.

Die Folien wirkten nach dem Prozess etwas weicher, waren teilweise leicht gequollen und schwach getrübt. Dieser Effekt war reversibel und verschwand nach einer gewissen Zeit vollständig ("Ablüftzeit"). Die Ablüftzeit diente auch dazu Lösemittelrückstände in den Folien zu entfernen.

Je nach Verwendung und eingesetzter Lösung wurden die Folienstücke bei Raumtemperatur (RT) getrocknet oder einer forcierten Trocknung unter einer IR-Strecke oder im Ofen bei 60°C unterzogen, um Lösemittelrückstände zu entfernen.

Für die Prüfungen wurde der Photokatalysator F-24a in einer Konzentration von 1 mM eingesetzt. Die Folie wurde behandelt bis durch UV/Vis Spektroskopie ein relativer Absorptionswert von > 0,05 bei 400nm messbar war bzw. eine erkennbare Gelbfärbung sichtbar wurde.

400nm entspricht dem Hauptabsorptionsmaximum des verwendeten Photokatalysators. Der Imprägnierprozess wurde mit unbehandelten Folienstücken wiederholt, um die Anzahl der Zyklen zu bestimmen, nach der die verwendete Imprägnierlösung erneuert werden musste, beispielsweise durch Zugabe des verwendeten Photokatalysators. Dabei wurde die Anzahl der durchgeführten Imprägnierungen bestimmt, die bei den in Tabelle 36 angegebenen Prozessbedingungen (Temperatur der Imprägnierlösung und Inkubationszeit) eine ausreichende Zunahme des Absorptionswert von > 0,05 bei 400nm, bestimmt durch UV/Vis Spektroskopie, erzielte.

Die in Tabelle 36 angegebene Anzahl an Wiederholungen (Prozesszyklen) entspricht der Mindestanzahl an durchgeführten Imprägnierprozessen, die bei den in Tabelle 36 angegebenen Prozessbedingungen (Temperatur der Imprägnierlösung und Inkubationszeit) eine ausreichende Imprägnierung bestimmt durch UV/Vis Spektroskopie unter Verwendung eines relativen Absorptionswert von > 0,05 bei 400nm, noch erreicht wurde.

Die Abschätzung des Flammpunktes erfolgte über die gemittelten Siedepunkte der beiden flüchtigsten Komponenten im wässrigen Gemischanteil.

**Tabelle 36: Eigenschaften der Rezepturen**

| Rezept No. | Flammpunkt (abgeschätzt) | Verwendbarkeit bis [°C] | Typische Prozesszeiten | Ablüftzeit nach dem Prozess | Anzahl der Zyklen |
|---|---|---|---|---|---|
| 1 | >5°C | 60°C | TPU, PVC, Nitril: < 5 min | 1h bei 40°C | >20 |
| 2 | >5°C | 60°C | Silikon: < 10 min | 1h bei 40°C | >20 |
| 3 | >20°C | 70°C | SEPS, EVA: < 20 min | 1h bei 40°C | >30 |
| | | | PET-G, Surlyn: < 30 min | | |
| 4 | >15°C | 70°C | TPU, PVC, Nitril: < 10 min | 1h bei 40°C | >30 |
| 5 | >8°C | 70°C | Silikon: < 15 min | 1h bei 40°C | >20 |
| | | | SEPS, EVA: < 30 min | | |
| | | | PET-G, Surlyn: < 30 min | | |
| 6 | >5°C | 60°C | TPU, PVC, Nitril: < 5 min | 1h bei 40°C | >20 |
| | | | Silikon: < 10 min | | |
| | | | SEPS, EVA: < 20 min | | |
| | | | PET-G, Surlyn: < 30 min | | |
| 7 | >20°C | 70°C | TPU, PVC, Nitril: < 10 min | 1h bei 40°C | >30 |
| 8 | >20°C | 70°C | Silikon: < 15 min | Über Nacht | >30 |
| | | | SEPS, EVA: < 30 min | | |
| | | | PET-G, Surlyn: < 30 min | | |
| 9 | >100°C | 70°C | TPU, PVC, Nitril und Silikon: | Über Nacht | >50 |
| 10 | >100°C | 70°C | < 10 min | Über Nacht | >50 |
| 11 | >100°C | 70°C | SEPS, EVA: < 20 min | Über Nacht | >50 |
| 12 | >100°C | 70°C | PET-G, Surlyn: < 30 min | Über Nacht | >50 |
| 13 | >100°C | 70°C | TPU, PVC, Nitril: < 10 min | Über Nacht | >50 |
| 14 | >100°C | 70°C | Silikon: < 15 min | Über Nacht | >50 |
| | | | SEPS, EVA: < 30 min | | |
| | | | PET-G, Surlyn: < 30 min | | |
| 15 | >100°C | 70°C | TPU, PVC, Nitril und Silikon: | Über Nacht | >50 |
| 16 | >100°C | 70°C | < 10 min | Über Nacht | >50 |
| | | | SEPS, EVA: < 20 min | | |
| | | | PET-G, Surlyn: < 30 min | | |

### Beispiel 4: Anwendung auf verschiedene Kunststoffoberflächen und Formkörper

Nachfolgend wurden verschiedene Folien und Schläuche behandelt, um die hergestellten Imprägnierungen und deren Einfluss auf die physikalischen Eigenschaften der verwendeten Kunststoffe und Formkörper zu bestimmen.

Dabei wurden ca. 4 cm x 4 cm große Folienstücke oder 4 cm lange Schlauchabschnitte (12 mm Außendurchmesser, 1 mm Wandstärke) zugeschnitten und gründlich mit deionisiertem Wasser und/oder 5%iger Neutralseifelösung gereinigt, um sie fett- und staubfrei zu hinterlassen. Die beschriebene Formulierung wurde auf eine Temperatur von 50°C in einem PE Becher erwärmt.

Die Proben wurden in die Färbelösung gegeben und bei 50°C getempert. Nach 2, 10 und 30 min wurde je eine Folie entnommen. Nach 30min wurden die Schläuche entnommen. Die Proben wurden mit deionisiertem Wasser gründlich abgewaschen, abgetrocknet und optisch auf Färbung kontrolliert.

Die Folien wirkten nach dem Prozess etwas weicher, waren teilweise leicht gequollen und schwach getrübt. Dieser Effekt war reversibel und verschwand nach einer gewissen Zeit vollständig ("Ablüftzeit"). Die Ablüftzeit diente auch dazu Lösemittelrückstände in den Folien zu entfernen.

Bevorzugt verwendet für weitere Versuche wurden die in Tabelle 37a dargestellten Basis-Rezepturen:

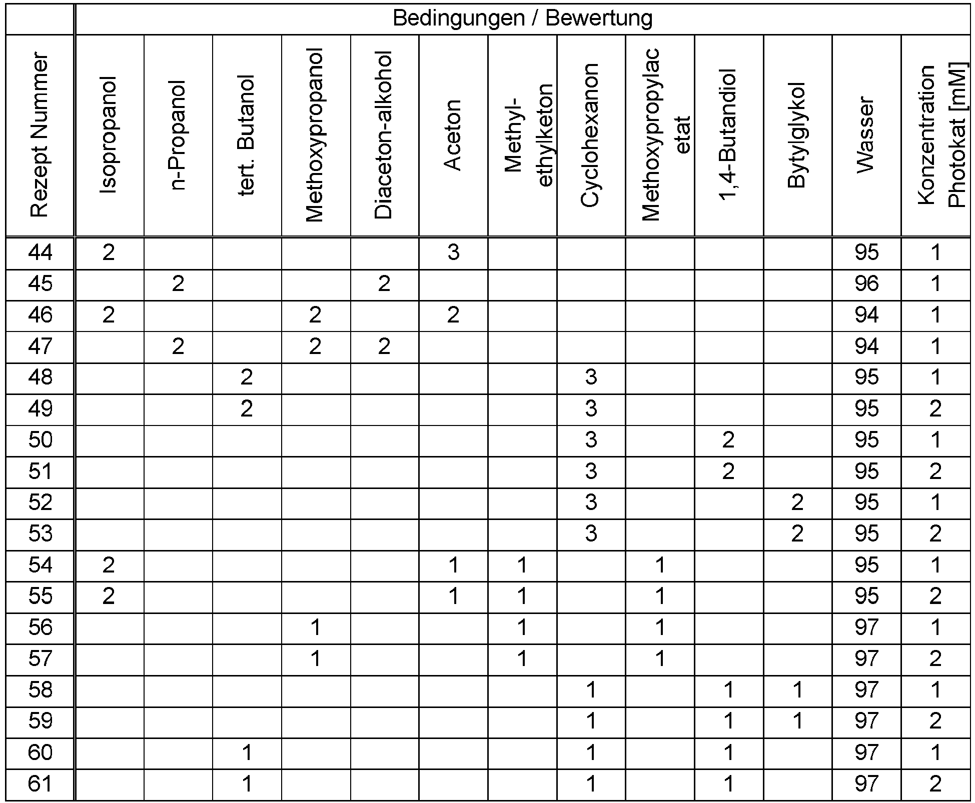

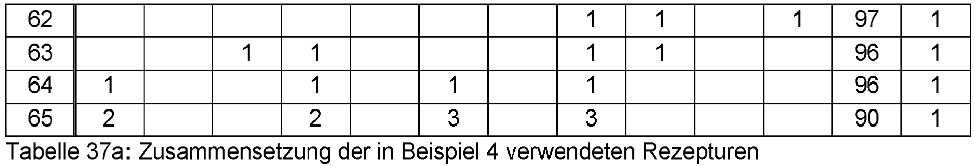

Die entsprechend verwendeten Imprägnier-Bedingungen sowie die jeweils bestimmten optischen und physikalischen Eigenschaften, insbesondere Formstabilität, der hergestellten Folien sind in Tabelle 37b dargestellt.

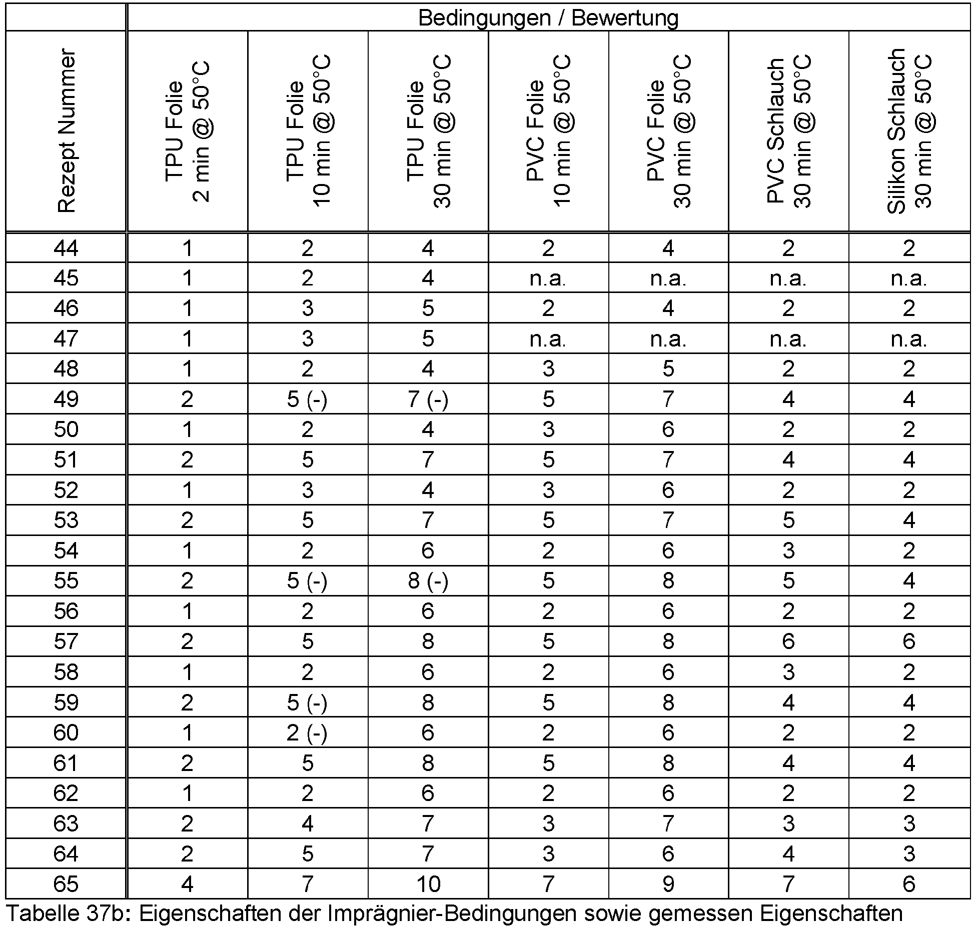

Dabei bedeutet (-) in Tabelle 37b, dass die verwendeten Folien sich leicht verzogen hatten.

Die optischen Eigenschaften wurden nach folgenden Kriterien bewertet.

**Tabelle 38: Kriterien für die optische Bewertung**

| Bewertung | Farbintensität | Praxisrelevanz |
|---|---|---|
| 0 | Keine | Keine |
| 1 | Gering, kaum sichtbar | Verwendbar mit Einschränkungen |
| 2-3 | Schwache Gelbfärbung | Gut verwendbar |
| 4 - 5 | Gelbfärbung | |
| 6 - 7 | Deutliche Gelbfärbung | Verwendbar, unwirtschaftlich |
| 8 | Sehr starke Färbung | |
| 9 - 10 | Tiefgelb, zu viel Farbeintrag | Nicht verwendbar |

Wie die mikrobiologischen Tests zeigten, wurde eine biozide Wirkung nach Bestrahlung erreicht, sobald eine schwache Eigenfarbe des Photokatalysators in der Folie erkennbar war. Die mikrobiologischen Tests sind in den Beispielen 11 bis 17 dargestellt.

Es zeigte sich, dass eine längere Einwirkzeit zu einem stärkeren Photokatalysatoreintrag führte. Bei Folien war eine Einwirkzeit von 2 min. bis 10 min. bei 50°C je nach Material und Formulierung ausreichend, um eine biozide Wirkung nach Bestrahlung zu erzielen.

Bei Schläuchen war eine Einwirkzeit von 10 min. bis 30 min. bei 50°C je nach Material und Formulierung ausreichend um eine biozide Wirkung nach Bestrahlung zu erzielen.

Darüber hinaus konnten gute Ergebnisse trotz eines Wassergehalts der verwendeten Imprägnierlösung von mehr als 90 Gew.-%, bezogen auf das Gesamtgewicht der verwendeten Lösung, erreicht werden.

Alle oben aufgeführten Lösungsmittel waren in verschiedenen Kombinationen verwendbar, wobei weiter bevorzugt Isopropanol, tert.-Butanol, 1,4-Butandiol, Aceton, Cyclohexanon, Methoxypropanol und Methoxypropylacetat verwendet wurden.

Je nach Lösungsmittelgemisch und geplanter Einwirkzeit können die oben aufgeführten Lösungsmittel in der Imprägnier-Lösung in einem Gesamtgehalt von 5 Gew.-% bis höchstens 15 Gew.-%, jeweils bezogen auf das Gesamtgewicht der Imprägnier-Lösung, verwendet werden, ohne dass eine signifikante Beeinträchtigung der Formstabilität der behandelten Folien während oder nach dem Verfahren auftritt.

### Beispiel 5: Anwendung auf größeren Kunststofffolien

Für ein Imprägnieren größerer Kunststofffolien wurden die anwendungsfertigen Lösungen Rezeptur No. 1 (sh. Tabelle 35) bzw. Rezeptur No. 60 (sh. Tabelle 37a) aus Beispielen 2 bis 4 in einer flachen Edelstahlwanne bei 40 bis 60°C vorgelegt. Als Prüfmuster wurden aromatisches TPU, aliphatisches TPU und Weich PVC eingesetzt. Der Photokatalysator F-24a wurde in einer Konzentration von 1 mM verwendet.

Dabei wurde entweder ein diskontinuierlicher Prozess oder ein kontinuierlicher Prozess angewendet.

### Beispiel 5.1 Diskontinuierlicher Prozess

Es wurde eine flache Wanne mit Abmessungen 0,8 m x 1,5 m verwendet. Ein großes Folienstück von 0,7 m x 1,2 m wurde wie oben beschrieben gereinigt, um es staub- und fettfrei zu hinterlassen. Die Folie wurde in die Lösung eingelegt, sodass sie vollständig von Flüssigkeit bedeckt war. Für den Prozess wird die Wanne abgedeckt, um das Verdampfen der Lösemittelbestände zu verhindern. Nach 5 min Behandlungszeit wurde die Folie entnommen, mit Wasser gewaschen und über Nacht ausgelüftet. Anschließend konnte sie direkt verwendet werden.

### Beispiel 5.2 Kontinuierlicher Prozess

Die Folie wurde über eine Walze langsam durch die Wanne geführt, so dass die Folie vollständig benetzt wurde und eine Kontaktzeit von 2 min an jeder Stelle des Folienstücks sichergestellt war. Das behandelte Material wurde mit Wasser gewaschen und über Nacht ausgelüftet. Anschließend konnte sie direkt verwendet werden.

### Beispiel 5.3 Messergebnisse

Am nächsten Tag wurden die Folien durch Absorptionsspektroskopie direkt vermessen (Maximum bei 400 nm). Dabei ergaben sich folgende Werte, die in Tabelle 39a und 39b zusammengefasst sind.

**Tabelle 39a: Ergebnisse der Prozesse (Rezeptur No.1)**

| Verfahren | Material | | |
|---|---|---|---|
| | Aliphatisches TPU | Aromatisches TPU | Weich PVC |
| Tauchprozess | 0,051 | 0,086 | 0,063 |
| Kontinuierlicher Prozess | 0,013 | 0,009 | 0,040 |
| Unbehandelte Folie | < 0,001 | < 0,001 | < 0,001 |

**Tabelle 39b: Ergebnisse der Prozesse (Rezeptur No. 60)**

| Verfahren | Material | | |
|---|---|---|---|
| | Aliphatisches TPU | Aromatisches TPU | Weich PVC |
| Tauchprozess | 0,065 | 0,091 | 0,072 |
| Kontinuierlicher Prozess | 0,017 | 0,012 | 0,048 |
| Unbehandelte Folie | < 0,001 | < 0,001 | < 0,001 |

Bei allen Materialien wurde ein deutlicher Anstieg des Absorptionswertes im relevanten Bereich des Photokatalysators im Vergleich zur unbehandelten Folie gemessen. Beide Prozessführungen lieferten gute Ergebnisse.

### Beispiel 6a: Mehrkomponenten-System (2K-System, "Kits of Parts")

Um eine Mehrkomponenten-System bereitzustellen, bei dem vorzugsweise flüchtigere Hilfskomponenten, die gegebenenfalls regelmäßiger im Prozess ergänzt werden müssen, in einer separaten Komponente bereitgestellt werden können, wurde zunächst folgendes 2K-System getestet.

Grundkomponente (A): Eine Mischung aus 40,0% w/w Aceton, 30,0% w/w Isopropanol und 20,0% w/w Wasser wurden homogen vermengt. Die Mischung war dicht verschlossen > 12 Monate bei Raumtemperatur lagerbar.

Zusatzkomponente (B): Eine Mischung aus 3,0% w/w Phenoxyethanol, 3,0% w/w EDTA und 0,1 bis 2,0 % Photokatalysator in Wasser wurden homogen verrührt. Die Mischung war dicht verschlossen in dunklen Flaschen > 6 Monate bei Raumtemperatur lagerbar.

Beide Lösungen wurden vor dem Prozess zu gleichen Teilen gemischt, gefolgt von einer 1:5 Verdünnung mit Wasser. Nach guter Durchmischung war die Lösung gebrauchsfertig.

Nach der Vorschrift in Beispiel 3 wurden so TPU Folien mit den Photokatalysatoren F-24a, F-24b, F-64a und F-73 Imprägniert. Die Ergebnisse zu F-24a und F-24b waren mit den vorherigen Daten vergleichbar. Bei F-64a und F-73 war eine schwache Gelb- bzw. Blaufärbung der Folie zu beobachten, die sich weder mit einem feuchten Schwamm abreiben noch mit Wasser auswaschen ließ.

### Beispiel 6b: Mehrkomponenten-System (2K-System, "Kits of Parts")

Grundkomponente (A) enthielt eine Mischung aus 30,0% w/w Dimethylsuccinat, 33,0% w/w 1,2-Propandiol, 12,0% w/w Tween 20 und 25,0 % Wasser.

Die Mischung war dicht verschlossen > 6 Monate bei Raumtemperatur lagerbar. Ein Zusatz von 0,05% MIT steigert die Lagerfähigkeit auf > 12 Monate.

Zusatzkomponente (B) enthielt eine Mischung aus 2,0 Gew.-% Phenoxyethanol, 2,0 Gew.-% EDTA und 0,1 Gew.-% bis 1,6 Gew.-% Photokatalysator, jeweils bezogen auf das Gesamtgewicht der Zusatzkomponente (B), wobei die entsprechenden Bestandteile in Wasser gelöst wurden und anschließend homogen verrührt wurden.

Die Mischung war dicht verschlossen in dunklen Flaschen > 6 Monate bei Raumtemperatur lagerbar.

Die Komponenten wurden vor Gebrauch im Verhältnis A zu B 2:1 gemischt, gefolgt von einer 1:1 Verdünnung mit Wasser. Nach guter Durchmischung war die Lösung gebrauchsfertig.

Nach der Vorschrift in Beispiel 4 wurden so TPU Folien mit den Photokatalysatoren F-24a, F-64a und F-73 Imprägniert. Die Ergebnisse zu F-24a waren mit den vorherigen Daten vergleichbar. Bei F-64a und F-73 war eine schwache Gelb- bzw. Blaufärbung der Folie zu beobachten, die sich weder mit einem feuchten Schwamm abreiben noch mit Wasser auswaschen ließ.

### Beispiele 7a bis 9b Verwendung verschiedener Photokatalysatoren

Im nächsten Schritt wurde die Verwendbarkeit der verschiedene Photokatalysatorklassen, ausgewählt aus Phenalen-1-on-Derivaten, Curcumin-Derivaten und Porphycen-Derivaten, mit unterschiedlichen Eigenschaften - von neutral unpolar bis ionisch polar - auf Ihre Verwendbarkeit und Wirkung im Verfahren untersucht.

Zur Beurteilung der optimalen Prozesszeit bei Einsatz der Photokatalysatoren, wurden TPU Folienstücke nach Beschreibung in Beispiel 4 zu je 1, 2, 5 und 10 min in den Imprägnierlösungen behandelt, gegebenenfalls auch 30 und 60 min bei undeutlichen Ergebnissen.

Als Basisformulierung wurde Rezeptur 1 nach Beispiel 2 verwendet.

Nach der angegebenen Prozesszeit wurden die Folie entnommen, mit Wasser gewaschen und für 1h bei 40°C getrocknet/abgelüftet.

Die Folien wurden durch Absorptionsspektroskopie gegen ein Blank-Folienstück direkt vermessen, beim Maximum der Absorption des Photokatalysators. Dabei ergaben sich in Abhängigkeit der Prozesszeit folgende Werte, die in den Tabellen 40 bis

### Beispiel 7a: Neutrale Phenalen-1-one

**Tabelle 40: Einbindung von Photokatalysatoren in Folien**

| Verbindung | Abs. 1 min | Abs. 2 min | Abs. 5 min | Abs. 10 min | Abs. 30 min | Abs. 60 min |
|---|---|---|---|---|---|---|
| F-07a | n.d. | n.d. | n.d. | 0,035 | 0,076 | 0,099 |
| F-08 | 0,003 | 0,015 | 0,016 | 0,023 | n.d. | n.d. |
| F-09 | 0,227 | 0,158 | 0,123 | 0,263 | n.d. | n.d. |
| F-10 | 0,044 | 0,011 | 0,062 | 0,087 | n.d. | n.d. |
| F-11a | 0,012 | 0,020 | 0,048 | 0,060 | n.d. | n.d. |
| F-12 | 0,108 | 0,349 | 0,411 | >1 | n.d. | n.d. |
| F-13a | n.d. | n.d. | n.d. | 0,038 | n.d. | n.d. |
| F-14a | 0,015 | 0,007 | 0,019 | 0,035 | n.d. | n.d. |
| F-14b | 0 | 0,009 | 0,032 | 0,151 | n.d. | n.d. |
| F-16a | 0,083 | 0,126 | 0,192 | 0,536 | n.d. | n.d. |
| F-17a | 0,138 | 0,046 | 0,072 | 0,176 | n.d. | n.d. |
| F-19a | >1 | >1 | >1 | >1 | n.d. | n.d. |
| F-20a | 0,020 | 0,014 | 0,020 | 0,035 | n.d. | n.d. |

### Beispiel 7b: Kationische Phenalen-1-one mit Hydroxyfunktion

**Tabelle 41: Einbindung von Photokatalysatoren in Folien**

| Verbindung | Abs. 1 min | Abs. 2 min | Abs. 5 min | Abs. 10 min |
|---|---|---|---|---|
| F-24b | 0,185 | 0,198 | 0,257 | 0,456 |
| F-25 | n.d. | n.d. | n.d. | Nicht messbar, schwache Gelbfärbung |
| F-26 | 0,025 | 0,015 | 0,028 | 0,047 |
| F-27 | n.d. | n.d. | n.d. | Nicht messbar, schwache Gelbfärbung |
| F-28* | 0,032 | 0,021 | 0,026 | 0,011 |
| F-29 | 0,005 | 0,020 | 0,024 | 0,042 |

| | | | | |
|---|---|---|---|---|
| *mit MEK statt Aceton | | | | |

### Beispiel 7c: Kationische Phenalen-1-one

**Tabelle 42: Einbindung von Photokatalysatoren in Folien**

| Verbindung | Abs. 1 min | Abs. 2 min | Abs. 5 min | Abs. 10 min | Abs. 30 min | Abs. 60 min |
|---|---|---|---|---|---|---|
| F-22a | n.d. | n.d. | n.d. | 0,035 | n.d. | n.d. |
| F-22b | 0,007 | 0,009 | 0,076 | 0,340 | n.d. | n.d. |
| F-22c | n.d. | n.d. | n.d. | 0,307 | n.d. | n.d. |
| F-30 | n.d. | n.d. | n.d. | 0,019 | 0,078 | 0,109 |
| F-31 | n.d. | n.d. | n.d. | 0,038 | 0,074 | 0,122 |
| F-32 | n.d. | n.d. | n.d. | 0,050 | 0,126 | 0,188 |
| F-33 | n.d. | n.d. | n.d. | 0,027 | 0,088 | 0,092 |
| F-34 | n.d. | n.d. | n.d. | 0,033 | 0,075 | 0,098 |
| F-35 | n.d. | n.d. | n.d. | 0,056 | 0,209 | 0,240 |
| F-36 | 0,031 | 0,036 | 0,057 | 0,055 | n.d. | n.d. |
| F-37 | n.d. | n.d. | n.d. | 0,007 | 0,022 | 0,078 |
| F-38 | n.d. | n.d. | n.d. | 0,012 | 0,090 | 0,102 |
| F-39 | n.d. | n.d. | n.d. | 0,019 | 0,072 | 0,082 |
| F-40 | n.d. | n.d. | n.d. | 0,027 | 0,101 | 0,134 |
| F-41 | n.d. | n.d. | n.d. | 0,014 | 0,058 | 0,062 |
| F-42 | 0,147 | 0,149 | 0,210 | 0,252 | n.d. | n.d. |
| F-43 | n.d. | n.d. | n.d. | 0,028 | 0,067 | 0,131 |
| F-44 | n.d. | n.d. | n.d. | 0,038 | 0,145 | 0,150 |
| F-46 | n.d. | n.d. | n.d. | 0,048 | 0,114 | 0,150 |
| F-47 | n.d. | n.d. | n.d. | 0,066 | 0,145 | 0,178 |
| F-48 | n.d. | n.d. | n.d. | ∼0,01 | 0,029 | 0,052 |
| F-49 | 0,579 | >1 | >1 | >1 | n.d. | n.d. |
| F-51a | n.d. | n.d. | n.d. | ∼0,01 | 0,024 | 0,041 |
| F-51b | 0,067 | 0,072 | 0,118 | 0,153 | n.d. | n.d. |

### Beispiel 7d: Anionische Phenalen-1-one

**Tabelle 43: Einbindung von Photokatalysatoren in Folien**

| Verbindung | Abs. 1 min | Abs. 2 min | Abs. 5 min | Abs. 10min | Abs. 30 min | Abs. 60 min |
|---|---|---|---|---|---|---|
| F-01b | n.d. | n.d. | n.d. | 0,008 | 0,011 | 0,065 |
| F-02 | 0,125 | 0,229 | 0,475 | 0,590 | n.d. | n.d. |

### Beipiel 8a: Kationische Curcuminderivate

**Tabelle 44: Einbindung von Photokatalysatoren in Folien**

| Verbindung | Abs. 1 min | Abs. 2 min | Abs. 5 min | Abs. 10 min |
|---|---|---|---|---|
| F-64a | n.d. | n.d. | n.d. | 0,55 |
| F-64c | n.d. | n.d. | 0,61 | >1 |
| F-65 | 0,08 | n.d. | n.d. | 0,87 |

### Beispiel 8b: Neutrale Curcuminderivate

**Tabelle 45: Einbindung von Photokatalysatoren in Folien**

| Verbindung | Abs. 1 min | Abs. 2 min | Abs. 5 min | Abs. 10 min |
|---|---|---|---|---|
| F-52a | >1 | >1 | >1 | >1 |
| F-52b | n.d. | 0,005 | 0,011 | 0,025 |
| F-55a | n.d. | n.d. | n.d. | 0,161 |
| F-55b | n.d. | n.d. | n.d. | 0,079 |
| F-56 | n.d. | n.d. | n.d. | 0,177 |
| F-57 | 0,019 | 0,021 | 0,044 | 0,063 |
| F-62 | n.d. | n.d. | n.d. | >1 |

### Beispiel 8c: Curcuminderivate mit Hydroxyfunktion

**Tabelle 46: Einbindung von Photokatalysatoren in Folien**

| Verbindung | Abs. 1 min | Abs. 2 min | Abs. 5 min | Abs. 10 min |
|---|---|---|---|---|
| F-58a | 0,441 | 0,520 | 0,554 | >1 |
| F-58b | >1 | >1 | >1 | >1 |
| F-60 | n.d. | n.d. | n.d. | 0,095 |
| F-61b | n.d. | n.d. | n.d. | 0,103 |

### Beispiel 8d: Curcuminderivate mit Silanfunktion

**Tabelle 47: Einbindung von Photokatalysatoren in Folien**

| Verbindung | Abs. 1 min | Abs. 2 min | Abs. 5 min | Abs. 10 min |
|---|---|---|---|---|
| F-63b | 0,123 | 0,166 | 0,268 | 0,331 |

### Beispiel 9a: Neutrale Porphycenderivate mit Hydroxyfunktion

**Tabelle 48: Einbindung von Photokatalysatoren in Folien**

| Verbindung | Abs. 1 min | Abs. 2 min | Abs. 5 min | Abs. 10 min | Abs. 30 min | Abs. 60 min |
|---|---|---|---|---|---|---|
| F-66* | 0,008 | 0,020 | 0,044 | 0,058 | n.d. | n.d. |
| F-67b | n.d. | n.d. | n.d. | 0,072 | n.d. | n.d. |
| F-68 | n.d. | n.d. | n.d. | 1,01 | >1 | >1 |
| F-69 | 0,016 | 0,039 | 0,058 | 0,193 | n.d. | n.d. |
| F-70 | n.d. | n.d. | n.d. | >1 | n.d. | n.d. |
| F-71 | 0 | 0 | 0,032 | 0,04 | n.d. | n.d. |
| F-72 | 0,340 | 0,784 | 0,845 | >1 | n.d. | n.d. |
| F-74a | 0,974 | 0,974 | >1 | >1 | n.d. | n.d. |
| F-75a | n.d. | n.d. | n.d. | >1 | n.d. | n.d. |
| F-76b | n.d. | n.d. | n.d. | >1 | n.d. | n.d. |
| F-78a | n.d. | n.d. | n.d. | 0,745 | n.d. | n.d. |
| F-78b | n.d. | n.d. | n.d. | 0,670 | n.d. | n.d. |
| F-79a | n.d. | n.d. | n.d. | 0,334 | n.d. | n.d. |
| F-80a | n.d. | n.d. | n.d. | >1 | n.d. | n.d. |
| F-80b | n.d. | n.d. | n.d. | >1 | n.d. | n.d. |
| F-81a | n.d. | n.d. | n.d. | 0,346 | n.d. | n.d. |
| F-81b | n.d. | n.d. | n.d. | >1 | n.d. | n.d. |
| F-82 | 0,164 | 0,152 | 0,432 | >1 | n.d. | n.d. |
| F-84 | n.d. | n.d. | n.d. | >1 | n.d. | n.d. |
| F-85a | n.d. | n.d. | n.d. | >1 | n.d. | n.d. |

| | | | | | | |
|---|---|---|---|---|---|---|
| * verwendet als 0,1 mM Lösung | | | | | | |

### Beispiel 9b: Kationische Porphycenderivate

**Tabelle 49: Einbindung von Photokatalysatoren in Folien**

| Verbindung | Abs. 10 min | Abs. 30 min | Abs. 60 min |
|---|---|---|---|
| F-71 | 0,023 | 0,062 | 0,114 |
| F-72 | 0,119 | n.d. | n.d. |
| F-73 | 0,001 | 0,043 | 0,068 |

Bei der Auswertung konnte festgestellt werden, dass mit steigender Prozesszeit mehr Farbstoff in die Folie eingebracht wurde.

Dieser Untersuchungen dienten dazu, die Prozesszeit zu optimieren und abzuschätzen, welche Prozesszeiten abhängig von der Farbstoffstruktur welchen Beladungsgrad erreichen konnten. Weiter sollte das dabei helfen, verschiedene Farbstoffklassen oder verschiedene Farbstoffderivate einer Farbstoffklasse bezüglich dieser Charakteristik miteinander zu vergleichen.

Die Prüfung diente nur zum Vergleich von verschiedenen Farbstoffen und zur Prozessoptimierung und liefert keine absoluten quantitativen Aussagen.

### Beispiel 10: Messung des Auswaschverhaltens der Farbstoffe

### A) Screening Test - Immersion

Um das Auswaschen der verschiedenen Farbstoffe in den Kunststofffolien zu beurteilen, wurde ein Immersionstest in deionisiertem Wasser mit einem pH-Wert von (6 ± 1) (oder gegebenenfalls anderen Lösungsmitteln) und einer Wassertemperatur von (22 ± 3) °C für 24 Stunden durchgeführt.

Dazu wurde ein Folienstück definierter Größe (3 cm x 3 cm) imprägniert (nach Rezeptur 1, Beispiel 1) und anschließend für 24 Stunden in eine definierte Menge deionisiertem Wasser (12,0 mL) gegeben und durch eine Schüttelplatte in Bewegung gehalten. Nach der angegeben Immersionszeit wurde die Folie entnommen und der Überschuss im UV-VIS Spektrometer vermessen, um die ausgewaschene Menge an Farbstoff zu bestimmen. Die Menge wird durch die definierte Größe der Folie und definierte Flüssigkeitsmenge auf einen Quadratzentimeter zurückgerechnet.

Dieser Immersionstest diente dazu, das Auswaschverhalten verschiedener Farbstoffe aus verschiedenen Kunststofffolien zu beurteilen. Weiter sollte er dabei helfen, verschiedene Farbstoffklassen oder verschiedene Farbstoffderivate einer Farbstoffklasse bezüglich dieser Charakteristik miteinander zu vergleichen.

Die zugeschnittenen Folien werden in einem geeigneten Behälter vollständig mit dem Prüfmedium (typischerweise deionisiertes Wasser) bedeckt. Anschließend wird der Behälter zugedeckt und auf die Schüttelplatte aufgebracht. Bei geringer Geschwindigkeit der Platte wird für 24 Stunden gewartet. Nach der Immersion wurden 3 mL des Überstandes durch Absorptionsspektroskopie vermessen.

Die Auswertung erfolgte über das Verhältnis der max. Absorptionswerte einer reinen Farbstofflösung definierter Konzentration und des vermessenen Überstandes. Die Ergebnisse sind in Tabelle 50 dargestellt.

**Tabelle 50: Auswaschverhalten verschiedener Photokatalysatoren nach Immersionstest A**

| Farbstoff | Rezeptur | Nach Beispiel | Auswaschmedium | Kunststoff | Abs. nach Auswaschen | Berechneter Verlust in % |
|---|---|---|---|---|---|---|
| F-14a | 1 | 1 | Wasser | TPU | 0,019 | 20,0 |
| F-14b | 1 | 1 | Wasser | TPU | 0,001 | 1,6 |
| F-16a | 1 | 1 | Wasser | TPU | 0,005 | 6,5 |
| F-11a | 1 | 1 | Wasser | TPU | 0,005 | 14,2 |
| F-02 | 1 | 1 | Wasser | Surlyn | 0,009 | 2,7 |
| F-66 | 1 | 1 | Wasser | TPU | 0,021 | 2,0 |
| F-24a | 1 | 1 | Wasser | TPU | 0,004 | 0,6 |
| F-24b | 1 | 1 | Wasser | TPU | 0,008 | 1,4 |
| F-24b | 1 | 1 | Wasser | EVA | 0,015 | 2,6 |
| F-24b | 1 | 1 | Wasser | SEPS | 0,027 | 4,7 |
| F-24b | 1 | 1 | 70% IPA | TPU | 0,194 | 34,0 |
| F-24b | 1 | 1 | 70% IPA | PVC | 0,160 | 25,4 |
| F-24b | 1 | 1 | 5% Neutralseife | TPU | 0,061 | 10,7 |
| F-24b | 1 | 1 | 5% Neutralseife | PVC | 0,024 | 3,8 |

### B) Belastungstest - Auswaschverhalten

Der Test wurde in Anlehnung an die DIN EN 16105:2011-12 ("Beschichtungsstoffe - Laborverfahren zur Bestimmung der Freisetzung von Substanzen aus Beschichtungen in intermittierendem Kontakt mit Wasser", Ausgabedatum: 2011-12) durchgeführt.

Die Prüfkörper wurden wie unter A) beschrieben erzeugt.

Es wurde vollentsalztes Wasser mit einem pH-Wert von (6 ± 1) und einer Wassertemperatur von (22 ± 3) °C verwendet. Beim Test wurden jeweils neun Tauchzyklen durchgeführt. Jeder Eintauchzyklus bestand dabei aus den folgenden Schritten:
- (1 ± 0,03) h Eintauchen,
- (4 ± 0,1) h Trocknung bei (22 ± 3) °C und (60 ± 10) % relativer Luftfeuchtigkeit und
- (1 ± 0,03) h Eintauchen.

Das Verhältnis zwischen dem Volumen des Auslaugmittels und der exponierten Oberfläche (Verhältnis von Flüssigkeit zu Oberfläche L/A) wurde auf 2,5 festgelegt. Die erforderliche Wassermenge (z. B. 250 mL pro 100 cm2) musste hinzugefügt werden. Für jedes Eintauchen wurde frisches deionisiertes Wasser verwendet.

Jeder Probekörper wurde in einem separaten Behälter angeordnet. Die Probekörper wurden in den Behältern so positioniert, dass ein freier Kontakt von Wasser mit der untersuchten Oberfläche möglich war. Der Tauchbehälter wurde dabei abgedeckt, um ein Verdunsten von Wasser während des Eintauchens zu vermeiden.

Der Überschuss wird direkt im UV-VIS-Spektrometer vermessen, vor dem ersten Immersionszyklus und nach Zyklen 1, 3, 5, 7 und 9.

Die Ausgangswerte (Absorptionsmaximum des jeweiligen Farbstoffs) lagen dabei zwischen 0,05 A.U. und 1,00 A.U. liegen. Die Ergebnisse sind in Tabelle 51 dargestellt.

**Tabelle 51: Auswaschverhalten verschiedener Photokatalysatoren nach Immersionstest B**

| Farbstoff | Rezeptur | Nach Beispiel | Auswaschmedium | Kunststoff | Abs. nach Auswaschen | Berechneter Verlust in % |
|---|---|---|---|---|---|---|
| F-24a | 1 | 1 | Wasser | TPU | 0,003 | 2,3 |
| F-24b | 1 | 1 | Wasser | TPU | 0,006 | 4,9 |

### C) Auswaschverhalten der Farbstoffe (Washability)

Der Test wurde in Anlehnung an die DIN EN ISO 11998:2006-10 ("Beschichtungsstoffe - Bestimmung der Nassabriebbeständigkeit und der Reinigungsfähigkeit von Beschichtungen (ISO 11998:2006)"; Ausgabedatum: 2006-10) durchgeführt, um das Auswaschen der verschiedenen Farbstoffe in den Kunststofffolien nach mechanischer Belastung zu beurteilen.

Hierzu wurden 200 Zyklen mit einem Anpressdruck von 135g angewendet. Es wurde ein Schwamm mit 4,0 mL Probelösung vorbereitet, die zu testende Folie eingespannt und der Schwamm aufgebracht. 200 Zyklen entsprachen dabei circa 5 min Kontaktzeit des Schwamms und der Oberfläche. Nach dem Test wurde die Folie entnommen und im UV-VIS Spektrometer vermessen, um die ausgewaschene Menge an Farbstoff zu bestimmen.

Der jeweils gemessene Wert vor dem Test und nach Abschluss der 200 Zyklen wurde verglichen und daraus ein prozentualer Verlust ermittelt.

Vorbereitet wurde die Folie nach Beispiel 2. Die zu verwendete Probelösung entsprach 5 Gew.-% Incidin Plus Lösung, bezogen auf das Gesamtgewicht der Probelösung.

Incidin Pro, der Firma ECOLAB Deutschland GmbH (Monheim), ist ein flüssiges Konzentrat, das sich zur Reinigung und Desinfektion medizinischer Geräte und Flächen in Einrichtungen des Gesundheitswesens angewendet wird.

Der Test ermittelt somit den nach wiederholtem flächigem Abwischen mit einem Desinfektionsmittel aus einer behandelten Oberfläche ausgetragenen Photokatalysator.

Die Ergebnisse sind in Tabelle 52 dargestellt.

**Tabelle 52: Ergebnisse nach Auswaschen unter simulierter Reinigung/mechanischer Belastung**

| Farbstoff | Rezeptur | Nach Beispiel | Kunststoff | Abs. vor Auswaschen | Abs. nach Auswaschen | Berechneter Verlust in % |
|---|---|---|---|---|---|---|
| F-24b | 1 | 2 | TPU | 0,121 | 0,119 | 1,7 |

### Beispiele 11 bis 17 Mikrobiologische Testungen

### Beispiel 11: Wirksamkeitsprüfung gegen Bakterien

Die Bestimmung der antimikrobiellen Aktivität erfolgte nach einer modifizierten Version der ISO 22196:2011-08 ("Messung von antibakterieller Aktivität auf Kunststoff- und anderen porenfreien Oberflächen", Ausgabedatum: 2011-08).

Bei der Testdurchführung wurde ein dünner Flüssigkeitsfilm, in dem die Bakterien enthalten sind (1,25 x 104 /cm²), direkt auf die Probenkörper (3 x 3 cm) aufgebracht. Direkt nach Beimpfung wurden von der Nullprobe die Bakterien von den Probenkörperoberflächen mittels Ultraschall und Vortexen abgelöst und die Keimzahl (KBE (engl. CFU), Kolonie-bildende Einheit) bestimmt (t0-Wert).

Ein weiterer Satz Nullproben und antimikrobiell ausgestatteter Proben wurden mit Bakterien im Flüssigkeitsfilm bei 37°C für 1-2 Stunden im Dunkeln bis zur sichtbaren Trocknung der Flüssigkeit inkubiert. Nach 4h wurden überlebende Bakterien von den Probenkörperoberflächen mittels Ultraschall und Vortexen abgelöst und die Keimzahl bestimmt (t4-Wert).

Im Unterschied zu der in der ISO 22196:2011-08 beschreibenen Methode wurde das Inokulum nicht mit einer Folie abgedeckt und die Inkubationszeit wurde von 24 auf 4 Stunden reduziert. Weiterhin wurden die Probenkörper der Proben 3 und 4 mit Licht der Intensität 20mW/cm2 für 10min belichtet, um eine biozide Wirkung nach Bestrahlung mit elektromagnetischer Strahlung zu bestimmen.

### Beispiel 12: Bakterizide Wirkung gegen verschiedene Bakterienstämme

Verwendet wurden TPU Folienstücke, die nach dem in Beispiel 4 beschriebenen Verfahren mit Rezeptur 1 behandelt wurden. Rezeptur 1 wurde hergestellt nach Beispiel 2. Die Farbstoffkonzentration in der Imprägnierlösung betrug 1 mM F-24b. Der Test wurde in Anlehnung an ISO 22196 nach oben beschriebener Methode durchgeführt.

Die Ergebnisse sind Tabelle 53 dargestellt.

**Tabelle 53: Photodynamische Wirkung in Iog10 Keimreduzierung einer imprägnierten Folie gegen verschiedene Bakterienstämme**

| Keim | | Log10 Reduktion Hauptprobe |
|---|---|---|
| S.aureus ATCC 6538 | Gram-positiv | 4,0 ± 0,3 |
| E.coli 10536 | Gram-negativ | 4,8 ± 0,4 |
| S.enterica | Gram-negativ | 4,2 ± 0,6 |
| Acinetobacter pitii | Gram-negativ | 3,0 ± 0,7 |

### Beispiel 13: Bakterizide Wirkung - verschiedene Imprägnierlösungen

TPU-Folienstücke wurden ebenfalls nach dem in Beispiel 4 beschriebenen Verfahren mit verschiedenen Rezepturen behandelt, die gemäß Beispiel 2 hergestellt wurden. Die Farbstoffkonzentration in der Imprägnierlösung betrug jeweils 1 mM F-24b. Der Test wurde in Anlehnung an ISO 22196 nach oben beschriebener Methode durchgeführt.

Die Ergebnisse sind Tabelle 54 dargestellt.

**Tabelle 54: Photodynamische Wirkung in log10 Keimreduzierung gegen S. aureus**

| Rezeptur | Nach Beispiel | Log10 Reduktion Hauptprobe |
|---|---|---|
| 1 | 2 | 4,0 ± 0,3 |
| 2 | 2 | 1,8 ± 1,3 |
| 3 | 2 | 4,2 ± 0,8 |
| 8 | 3 | 3,5 ± 0,5 |

### Beispiel 14: Bakterizide Wirkung - verschiedene Photokatalysatoren

TPU-Folienstücke wurden nach dem in Beispiel 6 beschriebenen Verfahren mit Rezeptur 1 behandelt (hergestellt nach Beispiel 1). Die Farbstoffkonzentration in der Imprägnierlösung betrug jeweils 1 mM.

Der Bakterientest wurde in Anlehnung an ISO 22196 nach oben beschriebener Methode durchgeführt. Die Ergebnisse sind Tabelle 55 dargestellt.

**Tabelle 55: Photodynamische Wirkung in log10 Keimreduzierung gegen S. aureus**

| Photokatalysator | Log10 Reduktion Hauptprobe |
|---|---|
| F-24b | 4,0 ± 0,3 |
| F-01a | 1,4 ± 0,4 |
| F-24a | 3,7 ± 0,3 |
| F-08 | 4,0 ± 0,3 |
| F-27 | 4,0 ± 0,3 |
| F-14 | 3,0 ± 0,6 |
| F-65 | 1,3 ± 0,1 |
| F-64a | 2,6 ± 1,7 |
| F-67 | 1,8 ± 0,3 |
| F-73 | 1,1 ± 0,1 |

### Beispiel 15: Bakterizide Wirkung - Einfluss verschiedener Photokatalysatorkonzentrationen

TPU-Folienstücke wurden nach dem in Beispiel 6 beschriebenen Verfahren mit Rezeptur 1 behandelt (hergestellt nach Beispiel 1). Die Photokatalysator-Konzentration von F-24b wurde variiert.

Der Bakterientest wurde in Anlehnung an ISO 22196 nach oben beschriebener Methode durchgeführt. Die Ergebnisse sind Tabelle 55 dargestellt.

**Tabelle 55: Photodynamische Wirkung in log10 Keimreduzierung gegen S. aureus**

| Photokatalysatorkonzentration (w/w) | Log10 Reduktion Hauptprobe |
|---|---|
| 0,25 mM | 2,8 ± 1,3 |
| 0,5 mM | 2,5 ± 1,4 |
| 1 mM | 3,5 ± 0,5 |

### Beispiel 16: Bakterizide Wirkung - Verschiedene Folienmaterialien

Verschiedene Folienmaterialien wurden nach dem in Beispiel 6 beschriebenen Verfahren mit Rezeptur 1 behandelt (hergestellt nach Beispiel 1). Die Farbstoffkonzentration in der Imprägnierlösung betrug 1 mM F-24b.

Der Bakterientest wurde in Anlehnung an ISO 22196 nach oben beschriebener Methode durchgeführt. Die Ergebnisse sind Tabelle 56 dargestellt.

**Tabelle 56: Photodynamische Wirkung in log10 Keimreduzierung gegen S. aureus**

| Folienmaterial | Log10 Reduktion Hauptprobe |
|---|---|
| TPU | 4,0 ± 0,3 |
| PVC | 3,9 ± 0,8 |
| Nitril | 3,8 ± 0,3 |
| PET-G | 4,0 ± 0,1 |
| Surlyn | 3,6 ± 0,5 |
| Silikon | 3,4 ± 0,1 |
| CPI (Polyimid) | 3,0 ± 0,1 |

### Beispiel 17 Wirksamkeitsprüfung gegen Viren

Eine antivirale Wirksamkeit wurde in Anlehnung an die in der ISO 21702:2019-05 ("Messung der antiviralen Aktivität an Kunststoffen und anderen nicht-porösen Oberflächen, Ausgabedatum: 2019-05) beschriebenen Methode durchgeführt.

Die imprägnierten Prüfkörper (mit und ohne Photosensitizer; PS) wurden mit 50µL einer Virussuspension beimpft. Die Suspension wurde im Dunkeln bis zur sichtbaren Trocknung der Flüssigkeit inkubiert. Die Hauptprobe (Imprägnierter Prüfkörper: mit PS) und die Lichtkontrolle (Imprägnierter Prüfkörper: ohne PS) wurden anschließend mit einer Lichtintensität von 20mW/cm² (LED Customer Device) für 83 min belichtet, wohingegen die Referenzkontrolle (Imprägnierter Prüfkörper: ohne PS) und die Dunkelkontrolle (Imprägnierter Prüfkörper: mit PS) solange im Dunkeln inkubiert wurden.

Nach Rückgewinnung der Viren vom Prüfkörper wurden die Titer der Lichtkontrolle, der Dunkelkontrolle, sowie der Hauptprobe gegenüber des Titer der Referenzkontrolle gemessen (Titration mittel Endpunktverdünnung bzw. Large Volume Plating) und in Log10-Stufen Reduktionen berechnet.

TPU-Folienstücke wurden nach dem in Beispiel 6 beschriebenen Verfahren mit Rezeptur 1 behandelt (hergestellt nach Beispiel 1) und gegenüber verschiedenen Viren getestet. Die Farbstoffkonzentration in der Imprägnierlösung betrug 1 mM F-24b.

Der Virentest wurde in Anlehnung an ISO 21702 nach oben beschriebener Methode durchgeführt. Die Ergebnisse sind Tabelle 57 dargestellt.

**Tabelle 57: Photodynamische Wirkung in log10 Keimreduzierung einer imprägnierten Folie gegen verschiedene Virusstämme**

| Virus | Log10 Reduktion Hauptprobe |
|---|---|
| Influenza A Virus (H1N1) | 2,5 ± 0,5 |
| Modified Vaccinia Virus Ankara (MVA) | 4,6 ± 0,6 |

### Beispiele 18 bis 21 Haltbarkeitstest via mikrobiologischer Untersuchungen

### Beispiel 18: Bakterizide Wirkung - Einfluss Auswaschen auf Wirksamkeit

Um die Haltbarkeit des Photokatalysators innerhalb der Folie bzw. dessen Auswaschen gegen gängige Reinigungsmittel zu untersuchen, wurden damit Immersionstest in Wasser und 70% Isopropanol durchgeführt.

TPU-Folienstücke wurden nach dem in Beispiel 6 beschriebenen Verfahren mit Rezeptur 1 behandelt (hergestellt nach Beispiel 1). Die Farbstoffkonzentration in der Imprägnierlösung betrug 1 mM F-24b. Die Folien wurden anschließend mit dem unter B) und C) beschrieben Belastungsverfahren mit den in den Tabellen gezeigten Medien behandelt und in ihrer Wirksamkeit vor/nach Belastung verglichen.

Der Bakterientest wurde in Anlehnung an ISO 22196 nach oben beschriebener Methode durchgeführt. Die Ergebnisse sind in der Tabelle 58b dargestellt.

**Tabelle 58: Photodynamische Wirkung nach Belastung in Iog10 Keimreduzierung gegen S. aureus**

| Testbedingungen | Verfahren | Immersionsmedium / Reiniger | Log10 Reduktion Hauptprobe |
|---|---|---|---|
| Screening Test - Immersion | A) | Wasser (dest.) | 3,3 ± 0,7 |
| | A) | 70% Isopropanol | 1,9 ± 0,6 |
| Auswaschen nach DIN EN 16105 | B) | Wasser (dest.) | 1,9 ± 0,5 |
| Washability nach DIN EN ISO 11998 | C) | Sauer-oxidierend | 4,8 ± 0,3 |
| | C) | Neutraldesinfizierend | 4,5 ± 0,6 |

Weiterhin wurde die Belastbarkeit des Photokatalysators in der Folie mit zusätzlichen Tests wie Tempern bei 80°C und Dampfsterilisation bei 121°C untersucht.

Für alle Prüfungen wurden TPU Folienstücke nach dem in Beispiel 6 beschriebenen Verfahren mit Rezeptur 1 behandelt (hergestellt nach Beispiel 1). Die Farbstoffkonzentration in der Imprägnierlösung betrug 1 mM F-24b.

### Beispiel 19: Bakterizide Wirkung - Einfluss Tempern auf die Wirksamkeit

Die Imprägnierten Folien wurden unter definierten Temperaturbedingungen für unterschiedliche Zeiten gelagert und anschließend in ihrer Wirksamkeit verglichen.

Der Bakterientest wurde in Anlehnung an ISO 22196 nach oben beschriebener Methode durchgeführt. Die Ergebnisse sind in Tabelle 59 dargestellt.

**Tabelle 59: Photodynamische Wirkung in log10 Keimreduzierung gegen S. aureus**

| | Log10 Reduktion Hauptprobe |
|---|---|
| 1 h 80 °C Tempern | 4,1 ± 0,1 |
| 1 Woche 80°C Tempern | 3,0 ± 1,1 |

### Beispiel 20: Bakterizide Wirkung - Einfluss Dampfsterilisation auf die Wirksamkeit

Die Imprägnierten Folien wurden bei 120°C für 2h dampfsterilisiert und anschließend in ihrer Wirksamkeit verglichen. Der Bakterientest wurde in Anlehnung an ISO 22196 nach oben beschriebener Methode durchgeführt. Die Ergebnisse sind in Tabelle 60 dargestellt.

**Tabelle 60: Photodynamische Wirkung in log10 Keimreduzierung gegen S. aureus**

| Dampfsterilisation 121°C | Log10 Reduktion Hauptprobe |
|---|---|
| 2 h | 1,5 ± 0,3 |

### Beispiel 21: Bakterizide Wirkung - Einfluss Langzeitbelichtung / Photostabilität

Zur Bewertung der Photostabilität des Photokatalysators in der Folie wurden imprägnierte Folienstücke mit 18 mW/cm² (405 nm, ca. 200-fache Raumlichtintensität, Zeitrafferversuch wobei 1h Belichtung ca. einer Belichtungszeit von 30 Arbeitstagen entspricht) belichtet.

Der Bakterientest wurde in Anlehnung an ISO 22196 nach oben beschriebener Methode durchgeführt. Die Ergebnisse sind in Tabelle 61 dargestellt.

**Tabelle 61: Photodynamische Wirkung in log10 Keimreduzierung gegen S. aureus**

| PN-B | 0 h | 2 h | 4 h | 8 h | 16 h | 48 h |
|---|---|---|---|---|---|---|
| Hauptprobe | 3,2 ± 0,9 | 2,5 ± 0,6 | 2,2 ± 0,2 | 1,9 ± 0,5 | 1,5 ± 0,6 | 1,5 ± 0,2 |

### Beispiel 22: Geruchsneutralisierung

Zusätzlich zur antimikrobiellen Wirkung wurde untersucht, in wie weit per Imprägnierung mit Photokatalysator ausgerüstete Folien zur Geruchsneutralisierung geeignet waren.

Hierzu wurde ein rundes Stück (Durchmesser = 9 cm, passgenau für eine große Petrischale) TPU Folie nach dem in Beispiel 6 beschriebenen Verfahren mit Rezeptur 1 behandelt (hergestellt nach Beispiel 1). Die Farbstoffkonzentration in der Imprägnierlösung betrug 1 mM F-24b.

Anschließend wurde das geruchstragende Molekül β-Citronellol in Wasser gelöst. Es wurden drei verschiedene Konzentrationen eingestellt (85 mmol/L ca. Löslichkeitsgrenze, 8,5 mmol/L, 0,85 mmol/L). Das runde Stück TPU Folie wird vollständig mit dieser Lösung bedeckt, so dass der Duftstoff gleichmäßig in der Folie verteilt wird, und abgedeckt bei 40°C für 30 min eingetaucht gelassen.

Nach der vergangenen Zeit wird das Folienstück mit dest. Wasser abgewaschen und getrocknet. Der Duft ist bei allen drei Konzentrationen olfaktorisch deutlich zu erkennen. Die Folienstücke wurden mit 25 mW/cm² (405 nm) belichtet. Die Auswertung erfolgte olfaktorisch. Die Ergebnisse sind in Tabelle 62 dargestellt.

**Tabelle 62: Ergebnisse zur photooxidativen Zerstörung von Gerüchen**

| Konzentration β-Citronellol bei Behandlung | Belichtungszeit der behandelten Folie | Auswertung |
|---|---|---|
| 85 mmol/L | 180 min | immer noch starker Geruch |
| 8,5 mmol/L | 60 min | Kaum wahrnehmbarer Geruch |
| 0,85 mmol/L | 60 min | kein Geruch mehr zu erkennen |

Bestimmte geruchstragende Moleküle könnten mittels Oxidation bei Belichtung des Photokatalysators, der in TPU-Folie imprägniert wurde, abgebaut werden. Der Prozess war abhängig von der Menge des Geruchsträgers und der Belichtungszeit der Folie.

### Vergleichsbeispiele 23 bis 26

Die im Folgenden untersuchten Folienstücke wurden nach dem in Beispiel 6 beschriebenen Verfahren mit Rezeptur 1 behandelt (hergestellt nach Beispiel 1). Die Farbstoffkonzentration in der Imprägnierlösung betrug jeweils 1 mM bei Phenalen-1-on- und Curcuminderivaten, 0,1 mM bei Prophycenderivaten.

### Beispiel 23: Ungeeigneter Kunststoff - Beschädigung

Es wurden 0,5 mm starke PMMA Folien eingesetzt; die Imprägnierlösung enthielt den Photokatalysator F-24a. Es kann eine deutliche Farbveränderung beobachtet werden, allerdings verformt sich das Material durch den Prozess irreversibel.

### Beispiel 24: Ungeeigneter Kunststoff - Keine Umsetzung

Es wurden 0,2 mm starke Polycarbonatfolien eingesetzt und mit Imprägnierlösungen enthaltend die Photokatalysatoren F-24b, F-64a oder F-73 behandelt. Auch nach 60 min Einwirkzeit kann keine Verfärbung beobachtet werden.

### Beispiel 25: Färbung mit Fluoreszenzfarbstoff - keine Wirkung

Es wurden TPU Folien eingesetzt und mit einer Imprägnierlösung enthaltend den Farbstoff 2-Chloro-6-(3-hydroxypropylamino)-1-phenalenon (CAS 113722-81-9) behandelt. Nach 30 min Einwirkzeit wurden homogen pink gefärbte Folien erhalten. Die Färbung konnte weder durch Abreiben mit einem feuchten Tuch noch durch Waschen mit Wasser entfernt werden.

Es wurde ein Bakterientest in Anlehnung an ISO 22196 nach oben beschriebener Methode durchgeführt. Dabei zeigte sich keine photodynamische Inaktivierung von S.aureus gegenüber den Kontrollen.

### Beispiel 26: Färbung mit hochpolaren Photokatalysatoren - starkes Auswaschen

Es wurden TPU Folien eingesetzt und mit Imprägnierlösungen enthaltend die folgenden Farbstoffe behandelt. Nach 30 min Einwirkzeit wurden homogen gefärbte Folien erhalten.

Die Ablüftzeit nach Behandlung betrug 20h.

Anschließend wurde ein Belastungstest Immersion mit destilliertem Wasser über 24h durchgeführt (siehe oben beschriebenes Verfahren A) ). Die Folien wurden durch Absorptionsspektroskopie direkt vermessen und die Maximum der Absorption vor/nach Auswaschen verglichen. Es ergaben sich folgende relative Werte, die auf einen starken Austrag der Photokatalysatoren schließen lassen. Die Ergebnisse sind in der Tabelle 63 dargestellt.

**Tabelle 63: Einbindung von Photokatalysatoren in Kunsttofffolien**

| Verbindungen | Stoffklasse | Verlust durch Auswaschen | Fehler |
|---|---|---|---|
| Phenalen-1-on (Verb.Bsp. 48); drei Ammoniumgruppen (a) (trikationisch) | Phenalen-1-one | > 50% | +/- 10% |
| Curcumin 05 Hydrochlorid / SACUR-05 (Verb. Bsp.50) (b) (hexakationisch) | Curcumine | > 70% | +/- 20% |
| 2,7,12-Tris(pyridinio-p-tolyl)-17-(p-(methoxymethyl)phenyl) porphycen bromid (c) | Porphycene | > 66% | +/- 25 % |
| (trikationisch) | | | |

| | | | |
|---|---|---|---|
| (a) hergestellt nach: WO2012113860A3. Dazu wurde 2-Chlormethyl-1H-phenalen-1-on wurde mit dem zweifach Boc-geschütztem Diethylentriamin umgesetzt und nach chromatographischer Aufreinigung mit HCl in Diethylether entschützt. Das Produkt wurde mit Diethylether gefällt und getrocknet. - MS (ESI-MS, CH₂Cl₂/MeOH + 10 mmol NH4OAc): e/z (%) = 310,1 (100, M⁺) (b) hergestellt nach, EP3135110A1. Dazu wurde 3,4,5-tris-(2-N-tert-butyloxycarbonyl-aminoethoxy)benzaldehyd wurde in Ethylacetat mit Acetylaceton in Gegenwart von Boroxid und n-Butylamin kondensiert. Das erhaltene Curcumin wurde chromatographisch aufgereinigt und anschließend mit Trifluoressigsäure in Dichlormethan entschützt. Durch lonenaustauschchromatographie und Gefriertrockung wurde anschließend das Hydrochlorid der Verbindung erhalten. - MS (ESI, MeCN/H₂O + 0.06 % TFA): 158.6 (47%, (M+4H⁺)⁴⁺), 211.1 (100%, (M+3H⁺)³⁺), 273.1 (6%, (M+2H⁺)²⁺ -C2H5N), 316.2 (24%, (M+2H⁺)²⁺), 631.3 (12%, MH⁺) (c) hergestellt nach: Ragàs, X.; et al.: "Cationic porphycenes as potential photosensitizers for antimicrobial photodynamic therapy". J. Med. Chem. 2010, 53, 7796-7803. | | | |

2,7,12,17-Tetra(p-(methoxymethyl)phenyl)porphycen wurde mit Bromwasserstoff in Eisessig entschützt und das Produkt chromatographisch gereinigt. Anschließend wurde mit Pyridin bei 80°C für 2h quaternisiert und das Produkt mit tert-Butylmethylether gefällt. - MS (ESI, MeCN/H₂O + 0.06 % TFA): 333,15 (100%, (M³⁺).

## Patentansprüche

1. Verfahren zur Herstellung eines Gegenstandes umfassend wenigstens einen photokatalytisch aktiven, vorzugsweise photodynamisch aktiven, Teilbereich wenigstens einer Oberfläche,
**dadurch gekennzeichnet,**
**dass** das Verfahren folgende Schritte umfasst
(a) Einbringen wenigstens eines Photosensibilisators in wenigstens einen Teilbereich wenigstens einer Oberfläche des Gegenstandes, wobei zumindest der Teilbereich wenigstens ein thermoplastisches Polymer, wenigstens ein elastomeres Polymer, Copolymere davon oder Mischungen davon umfasst, durch Kontaktieren des wenigstens eines Teilbereichs mit einer Photosensibilisator-haltigen Zusammensetzung umfassend:
• Wasser, vorzugsweise in einem Anteil von mindestens 50 Gew.-%, bezogen auf das Gesamtgewicht der Photosensibilisator-haltigen Zusammensetzung,
• wenigstens einen Photosensibilisator,
• wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, und
• optional, wenigstens einen Alkohol, der vorzugsweise 2 bis 10 C-Atome, die gradkettig oder verzweigt sind, und 1 bis 4 OH-Gruppen, umfasst,
bei einer Temperatur aus einem Bereich von 5°C bis 95°C, vorzugsweise für einen Zeitraum von wenigstens 0,1 Minuten, unter Erhalt wenigstens eines behandelten Teilbereichs, und
(b) Trocknen zumindest des in Schritt (a) erhaltenen behandelten Teilbereichs der Oberfläche,
wobei zwischen Schritt (a) und Schritt (b) optional der wenigstens eine behandelte Teilbereich der wenigstens einen Oberfläche gespült wird, vorzugsweise mit Wasser.

2. Verfahren nach Anspruch 1,
**dadurch gekennzeichnet,**
**dass** das Kontaktieren in Schritt (a) bei einer Temperatur aus einem Bereich von 15°C bis 85°C, vorzugsweise aus einem Bereich von 20°C bis 80°C, weiter bevorzugt aus einem Bereich von 35°C bis 70°C, vorzugsweise für einen Zeitraum von 0,5 Minuten bis höchstens 360 Minuten, erfolgt.

3. Verfahren nach einem der Ansprüche 1 oder 2,
**dadurch gekennzeichnet,**
**dass** der wenigstens eine Teilbereich der wenigstens einen Oberfläche wenigstens ein thermoplastisches Polymer, das aus der Gruppe ausgewählt ist, die aus thermoplastischen aliphatischen Polyurethanen (TPU), thermoplastischen aromatischen Polyurethanen (TPU), aliphatischen Polyamiden (PA), Polyimide (PI), Polyvinylchlorid (PVC), Polyethylenterephthalaten (PET), Polylactiden (PLA), Polymethacrylaten und Estern davon, Polyacrylaten und Estern davon, Copolymere von Polystyrol mit Ethylen, Propylen, Acrylnitril oder Vinylacetat, Copolymere aus Ethylen und Methacrylsäure, und Mischungen davon besteht, wenigstens ein elastomeres Polymer, das aus der Gruppe ausgewählt ist, die aus Kautschuk, Acrylnitril-Butadien-Kautschuk, Poly(organo)siloxanen, Styren-Ethylen-Propylen-Styren-Copolymere (SEPS), Ethylen-Vinylacetat-Copolymere (EVA), und Mischungen davon besteht, umfasst oder daraus besteht.

4. Verfahren nach einem der Ansprüche 1 bis 3,
**dadurch gekennzeichnet,**
**dass** der wenigstens eine Photosensibilisator aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on-Derivaten, besteht, ausgewählt ist.

5. Verfahren nach einem der Ansprüche 1 bis 4,
**dadurch gekennzeichnet,**
**dass** der Gegenstand in Form eines selbsttragenden Films, eines Verbunds aus Fasern oder eines Formgegenstandes vorliegt.

6. Photosensibilisator-haltige Zusammensetzung, vorzugsweise zur Verwendung in einem Verfahren nach einem der Ansprüche 1 bis 5, umfassend
• wenigstens einen Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, weiter bevorzugt Phenalen-1-on Derivaten, besteht,
• wenigstens ein Co-Solvens, das ausgewählt ist aus aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon, und,
• optional, wenigstens ein Alkohol, der vorzugsweise 2 bis 10 C-Atome, die gradkettig oder verzweigt sein können, und 1 bis 4 OH-Gruppen aufweist.

7. Photosensibilisator-haltige Zusammensetzung nach Anspruch 6, wobei die Photosensibilisator-haltige Zusammensetzung weiterhin Wasser enthält, vorzugsweise in einem Anteil von wenigstens 10 Gew.-%, jeweils bezogen auf das Gesamtgewicht der Zusammensetzung.

8. Photosensibilisator-haltige Zusammensetzung nach einem der Ansprüche 6 oder 7, wobei das wenigstens eine Phenalen-1-on-Derivat ein Phenalen-1-on-Derivat der allgemeinen Formel (1) ist: 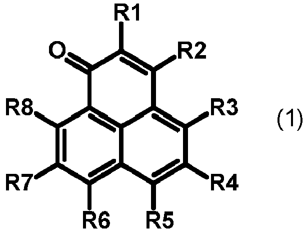
wobei die Reste R1 bis R8, die jeweils unabhängig voneinander gleich oder voneinander verschieden sein können, jeweils Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, oder einen organischen Rest W1a, der:
a1) ein ungeladener organischer Rest, oder
a2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist, oder,
a3) ein organischer Rest mit wenigstens einer Säuregruppe, vorzugsweise 1 oder 2 Säuregruppen, oder
a4) ein amphoterer organischer Rest, der ein permanent positiv geladenes Stickstoffatom oder ein protonierbares Stickstoffatom, das jeweils nicht direkt am aromatischen Ring angeordnet ist, und eine Säuregruppe umfasst,
bedeutet, mit der Maßgabe, dass einer der Reste R1 bis R8, vorzugsweise einer der Reste R1 bis R6, vorzugsweise einer der Reste R1, R2, R4, R6 oder R8, weiter bevorzugt einer der Reste R1, R2, R4 oder R6, weiter bevorzugt einer der Reste R1 oder R2, weiter bevorzugt der Rest R1, ein organischer Rest W1a bedeutet,
wobei der Rest W1a in Variante a1) ein Rest mit der allgemeinen Formel (10a) bis (16b) bedeutet:
* - (C(D)(E))ₕ - T (10a)
* - Q - (C(D)(E))ₕ - T (10b)
* - [(C(D)(E))_{b} - A]ₐ - (C(D)(E))_{g} - T (11a)
* - Q - [(C(D)(E))_{b} - A]ₐ - (C(D)(E))_{g} - T (11b)
* - [(C(D)(E))_{b} - A]ₐ - (C(D)(E))_{y}- Ar - (C(D)(E))_{g} - T (12a)
* - Q - [(C(D)(E))_{b} - A]ₐ - (C(D)(E))_{y}- Ar - (C(D)(E))_{g} - T (12b)
* - (C(D)(E))ₕ - Ar - (C(D)(E))_{g} - T (13a)
* - Q - (C(D)(E))ₕ - Ar - (C(D)(E))_{g} - T (13b)
* - (C(D)(E))_{y} - Ar - B - (C(D)(E))_{g} - T (14a)
* - Q - (C(D)(E))_{y} - Ar - B - (C(D)(E))_{g} - T (14b)
* - (C(D)(E))_{y} - Ar - B - [(C(D)(E))_{b} - A]_{c} - (C(D)(E))_{y} - T (15a)
* - Q - (C(D)(E))_{y} - Ar - B - [(C(D)(E))_{b} - A]_{c} - (C(D)(E))_{y} - T (15b)
* - [(C(D)(E))_{b} - A]ₐ - (C(D)(E)_{y} - Ar - B - [(C(D)(E))_{b} - A]_{c} - (C(D)(E))_{y} - T (16a)
* - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Hydroxyl, Nitro, Alkyl, das gradkettig oder verzweigt sein kann, mit 1 bis 15 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
und wobei zwei oder mehr der Reste D und/oder E jeweils verbrückt sein können und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkanonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei wenigstens einer der Reste D und/oder E mit wenigstens einem Ringatom des Rests Ar verbrückt sein kann und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei C-Atome des Cycloalkylrestes, cyklischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, Nitro, Nitril, einem Rest der allgemeinen Formel * - C(=O)NH₂ und Kombinationen davon, besteht, ausgewählt werden, substituiert ist, bedeutet, und
wobei der Rest W1a in Variante a2) ein Rest mit der allgemeinen Formel (21a) bis (24b) bedeutet:
* - [(C(D)(E))_{b} - A]_{c} - (C(D)(E))_{g} - X (21a)
* - Q - [(C(D)(E))_{b} - A]_{c} - (C(D)(E))_{g} - X (21b)
* - [(C(D)(E))_{b} - A]ₐ - (C(D)(E))_{y} - Ar - (C(D)(E))_{f} - X (22a)
* - Q - [(C(D)(E))_{b} - A]ₐ - (C(D)(E))_{y} - Ar - (C(D)(E))_{f} - X (22b)
* - (C(D)(E))_{d} - Ar - (C(D)(E))_{f} - X (23a)
* - Q - (C(D)(E))_{d} - Ar - (C(D)(E))_{f} - X (23b)
*- (C(D)(E))_{d} - Ar - [A - (C(D)(E))_{b}]ₐ - X (24a)
* - Q - (C(D)(E))_{d} - Ar - [A - (C(D)(E))_{b}]ₐ - X (24b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise 1 bis 6, vorzugsweise von 2 bis 4, weiter bevorzugt 1 bis 2, bedeutet, und
wobei der Index b jeweils unabhängig voneinander eine ganze Zahl von 1 bis 4, vorzugsweise von 1 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices d, und f jeweils unabhängig voneinander eine ganze Zahl von 1 bis 10, vorzugsweise 1 bis 8, vorzugsweise von 2 bis 4, bedeuten, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar einen unsubstituierten Phenylen-Rest oder einen substituierten Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert ist, bedeutet, und
wobei der Rest X jeweils unabhängig voneinander ein Rest der Formel (15a), (15b), (16), (17a), (17b), (17c), oder (17d), vorzugsweise ein Rest der Formel (15a), (15b), (17a) oder (17b), bedeutet: 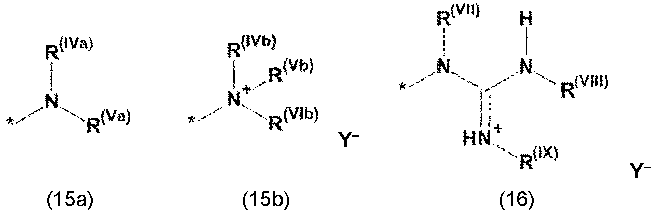 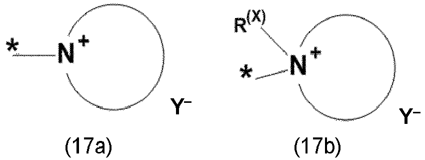 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)} und R^{(X)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (17a): 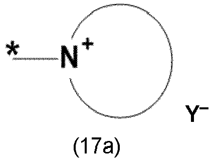 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, bedeutet, wobei der heterocyclische Rest mit 5 bis 10 Ringatomen unsubstituiert oder mit einem oder mehrerer Reste substituiert ist, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden, und
wobei der Rest mit der Formel (17b): 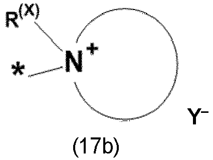 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung mit einem Ringatom, vorzugsweise Kohlenstoffatom, ausbildet, bedeuten, wobei der heterocyclische Rest mit 5 bis 7 Ringatomen, unsubstituiert oder mit einem oder mehrerer Reste substituiert ist, die aus der Gruppe, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, und Mischungen davon besteht, ausgewählt ist, und
wobei der Rest W1a in Variante a3) ein Rest mit der allgemeinen Formel (30) bis (34b) bedeutet:
- Z (30)
* - [(C(D)(E))_{b} - A]_{c} - (C(D)(E))_{g} - Z (31a)
_{*} - Q - [(C(D)(E))_{b} - A]_{c} - (C(D)(E))_{g} - Z (31b)
* - [(C(D)(E))_{b} - A]ₐ - (C(D)(E))_{y} - Ar - (C(D)(E))ₑ - Z (32a)
_{*} - Q - [(C(D)(E))_{b} - A]ₐ - (C(D)(E))_{y} - Ar - (C(D)(E))ₑ - Z (32b)
* - (C(D)(E))_{d} - Ar - (C(D)(E))ₑ - Z (33a)
* - Q - (C(D)(E))_{d} - Ar - (C(D)(E))ₑ - Z (33b)
*- (C(D)(E))_{d} - Ar - [A - (C(D)(E))_{b}]ₐ - Z (34a)
* - Q - (C(D)(E))_{d} - Ar - [A - (C(D)(E))_{b}]ₐ - Z (34b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise 1 bis 6, vorzugsweise von 2 bis 4, weiter bevorzugt 1 bis 2, bedeutet, und
wobei der Index b jeweils unabhängig voneinander eine ganze Zahl von 1 bis 4, vorzugsweise von 1 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index d jeweils unabhängig voneinander eine ganze Zahl von 1 bis 10, vorzugsweise von 2 bis 4, bedeutet, und
wobei der Index e jeweils unabhängig voneinander eine ganze Zahl von 0 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Rest W1a in Variante a4) ein Rest mit der allgemeinen Formel (41a) oder (41b) bedeutet:
* - (CH₂)_{g} - Het - (CH₂)ᵢ - Z (41a)
* - (CH₂)_{g} -Het - [B - (CH₂)ₖ]ₗ - Z (41b)
wobei der Rest B jeweils unabhängig voneinander Sauerstoff, ein Rest der Formel * - C(=O) - *, ein Rest der Formel * - C(=O)O - *, ein Rest der Formel * - OC(=O) - *, oder ein Rest der Formel * - C(=O)N(R⁽⁶⁾) - * bedeutet, wobei der Rest R⁽⁶⁾ jeweils unabhängig voneinander Wasserstoff oder Alkyl, das gradkettig oder verzweigt sein kann, mit 1 bis 4 Kohlenstoffatomen bedeutet,
wobei der Rest Het jeweils einen heterocyclischen Rest mit 5 bis 10 Ringatomen, die mindestens 1 Kohlenstoffatom und mindestens 1 Stickstoffatom sowie optional 1 oder 2 Sauerstoffatome umfassen, bedeutet, der vorzugsweise ein Rest mit der Formel (17a) oder ein Rest mit der Formel (17c) ist: 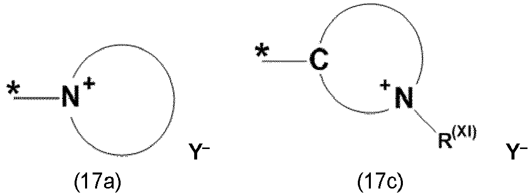
wobei der heterocyclisch Rest 5 bis 10 Ringatome aufweist, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, wobei der Rest R^{(XI)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices i, und k jeweils unabhängig voneinander eine ganze Zahl von 1 bis 4, vorzugsweise 2 bis 3, bedeutet, und
wobei die Index I jeweils unabhängig voneinander eine ganze Zahl von 0 bis 4, vorzugsweise 0 bis 3, bedeuten, und
wobei der Rest Z jeweils unabhängig voneinander ein Säure-Rest ist, der aus der Gruppe, die aus Carboxygruppe und Sulfonsäuregruppe besteht, ausgewählt wird.

9. Photosensibilisator-haltige Zusammensetzung nach einem der Ansprüche 6 bis 8, wobei das wenigstens eine Curcumin-Derivat ein Curcumin-Derivat der allgemeinen Formel (2a) oder Formel (2b) ist: 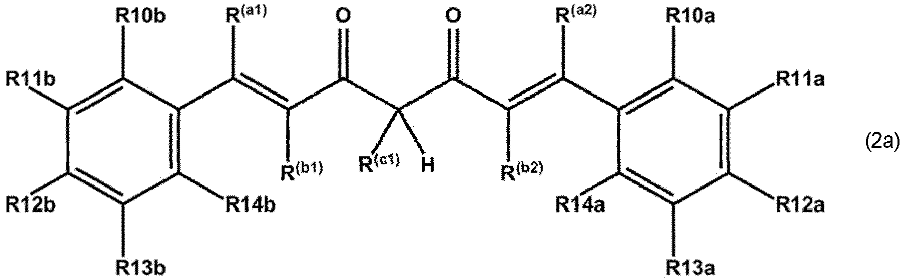 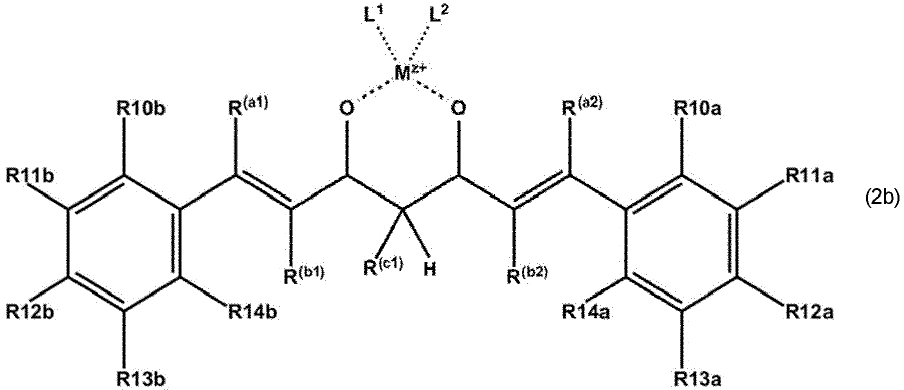
wobei M^{z+} das Kation eines Metalls oder Halbmetalls M, das vorzugsweise aus der Gruppe, die aus Bor, Zink, Aluminium und Eisen besteht, ausgewählt ist, bedeutet, wobei z die formale Oxidationszahl des Metalls oder Halbmetalls M ist und wobei z eine ganze Zahl von 1 bis 7, vorzugsweise von 2 bis 3, ist, und wobei L¹ und L² jeweils unabhängig voneinander Wasser, Fluorid, Chlorid, Bromid, lodid, Phosphat, Hydrogenphosphat, Dihydrogenphosphat, Sulfat, Hydrogensulfat, Tosylat, Mesylat oder wenigstens ein Carboxylat-Ion einer Carbonsäure mit 1 bis 15 Kohlenstoffatomen und/oder Mischungen davon bedeuten,
wobei die Reste R^{(a1)}, R^{(a2)}, R^{(b1)} und R^{(b2)}, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können,
wobei der Rest R^{(c1)} jeweils unabhängig voneinander Wasserstoff, Halogen, Alkyl, das gradkettig oder verzweigt sein kann, mit 1 bis 4 C-Atomen, vorzugsweise Methyl, Ethyl, n-Propyl oder iso-Propyl, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
und wobei und wobei einer der Reste R^{(b1)} oder R^{(b2)} und der Rest R^{(c1)} jeweils verbrückt sein können und einen Cycloalkylrest mit 4 bis 8 C-Atomen und/oder einen Cycloalkenylrest mit 4 bis 8 C-Atomen bilden können, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können,
und wobei die Reste R10a bis R14b, die jeweils unabhängig voneinander gleich oder voneinander verschieden sein können, jeweils Wasserstoff, Halogen, vorzugsweise Brom, oder lod,, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, oder einen organischen Rest W1b bedeuten, und
wobei der organische Rest W1b, die jeweils unabhängig voneinander gleich oder voneinander verschieden sein können,:
b1) ein ungeladener organischer Rest, oder
b2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder,
b3) ein organischer Rest mit wenigstens einer Säuregruppe, vorzugsweise 1 oder 2 Säuregruppen, oder
b4) ein amphoterer organischer Rest, der ein permanent positiv geladenes Stickstoffatom oder ein protonierbares Stickstoffatom, das jeweils nicht direkt am aromatischen Ring angeordnet ist, und eine Säuregruppe umfasst,
bedeutet, mit der Maßgabe, dass wenigstens einer der Reste R10a bis R14a und/oder wenigstens einer der Reste R10b bis R14b ein organischer Rest W1b bedeutet,
wobei der Rest W1b in Variante b1) ein Rest mit der allgemeinen Formel (10a) bis (16b) bedeutet:
* - (C(D)(E))ₕ - T (10a)
* - Q - (C(D)(E))ₕ - T (10b)
* - [(C(D)(E))_{b} - A]ₐ - (C(D)(E))_{g} - T (11a)
* - Q - [(C(D)(E))_{b} - A]ₐ - (C(D)(E))_{g} - T (11b)
* - [(C(D)(E))_{b} - A]ₐ - (C(D)(E))_{y}- Ar - (C(D)(E))_{g} - T (12a)
* - Q - [(C(D)(E))_{b} - A]ₐ - (C(D)(E))_{y}- Ar - (C(D)(E))_{g} - T (12b)
* - (C(D)(E))ₕ -Ar - (C(D)(E))_{g} - T (13a)
* - Q - (C(D)(E))ₕ - Ar - (C(D)(E))_{g} - T (13b)
* - (C(D)(E))_{y} - Ar - B - (C(D)(E))_{g} - T (14a)
* - Q - (C(D)(E))_{y} - Ar - B - (C(D)(E))_{g} - T (14b)
* - (C(D)(E))_{y} - Ar - B - [(C(D)(E))_{b} - A]_{c} - (C(D)(E))_{y} - T (15a)
* - Q - (C(D)(E))_{y} - Ar - B - [(C(D)(E))_{b} - A]_{c} - (C(D)(E))_{y} - T (15b)
* - [(C(D)(E))_{b} - A]ₐ - (C(D)(E)_{y} - Ar - B - [(C(D)(E))_{b} - A]_{c} - (C(D)(E))_{y} - T (16a)
* - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - O S(=O)₂ - *, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet,
und wobei zwei oder mehr der Reste D und/oder E jeweils verbrückt sein können und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkanonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyklischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet und
wobei der Rest W1b in Variante b2) ein Rest mit der allgemeinen Formel (21a) bis (24b) bedeutet:
* - [(C(D)(E))_{b} - A]_{c} - (C(D)(E))_{g} - X (21a)
_{*} - Q - [(C(D)(E))_{b} - A]_{c} - (C(D)(E))_{g} - X (21b)
* - [(C(D)(E))_{b} - A]ₐ - (C(D)(E))_{y} - Ar - (C(D)(E))_{f} - X (22a)
* - Q - [(C(D)(E))_{b} - A]ₐ - (C(D)(E))_{y} - Ar - (C(D)(E))_{f} - X (22b)
* - (C(D)(E))_{d} - Ar - (C(D)(E))_{f} - X (23a)
* - Q - (C(D)(E))_{d} - Ar - (C(D)(E))_{f} - X (23b)
*- (C(D)(E))_{d} - Ar - [A - (C(D)(E))_{b}]ₐ - X (24a)
* - Q - (C(D)(E))_{d} - Ar - [A - (C(D)(E))_{b}]ₐ - X (24b)
wobei die Reste Q und A jeweils unabhängig voneinander Sauerstoff, , ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxylalkyl, das gradkettig oder verzweigt sein kann, mit 1 bis 5 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert sein können, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices d, und f jeweils unabhängig voneinander eine ganze Zahl von 1 bis 10, vorzugsweise 1 bis 8, vorzugsweise von 2 bis 4, bedeuten, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar einen unsubstituierten Phenylen-Rest oder einen substituierten Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert ist, bedeutet,
wobei der Rest X jeweils unabhängig voneinander ein Rest der Formel (15a), (15b), (16), (17a), (17b), (17c), oder (17d), vorzugsweise ein Rest der Formel (15a), (15b), (17a) oder (17b), bedeutet: 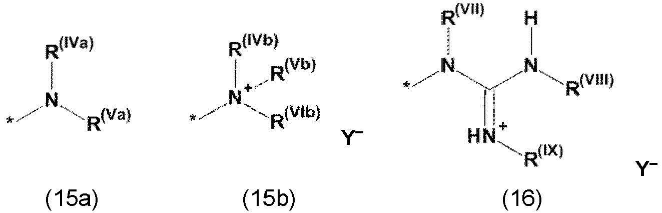 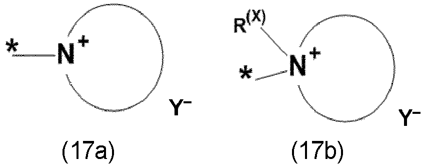 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)} und R^{(X)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (17a): 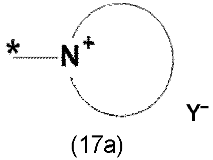 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, bedeutet, wobei der heterocyclische Rest mit 5 bis 10 Ringatomen unsubstituiert oder mit einem oder mehrerer Reste substituiert ist, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden, und
wobei der Rest mit der Formel (17b): 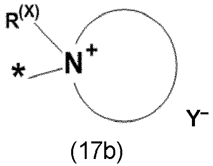 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung mit einem Ringatom, vorzugsweise C-Atom, ausbildet, bedeuten, wobei der substituierte heterocyclische Rest mit 5 bis 7 Ringatomen, unsubstituiert oder mit einem oder mehrerer Reste substituiert ist, die aus der Gruppe, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, und Mischungen davon besteht, ausgewählt ist, und
wobei der Rest W1b in Variante b3) ein Rest mit der allgemeinen Formel (30) bis (24b) bedeutet:
- Z (30)
* - [(C(D)(E))_{b} - A]_{c} - (C(D)(E))_{g} - Z (31a)
_{*} - Q - [(C(D)(E))_{b} - A]_{c} - (C(D)(E))_{g} - Z (31b)
* - [(C(D)(E))_{b} - A]ₐ - (C(D)(E))_{y} - Ar - (C(D)(E))ₑ - Z (32a)
_{*} - Q - [(C(D)(E))_{b} - A]ₐ - (C(D)(E))_{y} - Ar - (C(D)(E))ₑ - Z (32b)
* - (C(D)(E))_{d} - Ar - (C(D)(E))ₑ - Z (33a)
* - Q - (C(D)(E))_{d} - Ar - (C(D)(E))ₑ - Z (33b)
*- (C(D)(E))_{d} - Ar - [A - (C(D)(E))_{b}]ₐ - Z (34a)
* - Q - (C(D)(E))_{d} - Ar - [A - (C(D)(E))_{b}]ₐ - Z (34b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index d jeweils unabhängig voneinander eine ganze Zahl von 1 bis 10, vorzugsweise 2 bis 4, bedeutet, und
wobei der Index e jeweils unabhängig voneinander eine ganze Zahl von 0 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Rest W1b in Variante b4) ein Rest mit der allgemeinen Formel (41a) oder (41b) bedeutet:
* - (CH₂)_{g} - Het - (CH₂)ᵢ - Z (41a)
* - (CH₂)_{g} -Het - [B - (CH₂)ₖ]ₗ - Z (41b)
wobei der Rest B jeweils unabhängig voneinander Sauerstoff, ein Rest der Formel * - C(=O) - *, ein Rest der Formel * - C(=O)O - *, ein Rest der Formel * - OC(=O) - *, oder ein Rest der Formel * - C(=O)N(R⁽⁶⁾) - * bedeutet, wobei der Rest R⁽⁶⁾ jeweils unabhängig voneinander Wasserstoff oder Alkyl, das gradkettig oder verzweigt sein kann, mit 1 bis 4 Kohlenstoffatomen bedeutet,
wobei der Rest Het jeweils einen heterocyclischen Rest mit 5 bis 10 Ringatomen, die mindestens 1 Kohlenstoffatom und mindestens 1 Stickstoffatom sowie optional 1 oder 2 Sauerstoffatome umfassen, bedeutet, der vorzugsweise ein Rest mit der Formel (17a) oder ein Rest mit der Formel (17c) ist: 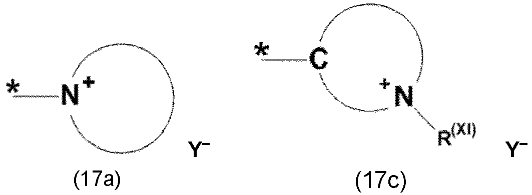
wobei der heterocyclisch Rest 5 bis 10 Ringatome aufweist, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, wobei der Rest R^{(XI)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist, und
wobei der Index g jeweils unabhängig voneinander eine ganze Zahl von 1 bis 35, vorzugsweise von 1 bis 30, bedeutet, und
wobei die Indices i, und k jeweils unabhängig voneinander eine ganze Zahl von 1 bis 4, bedeuten, und
wobei die Index l jeweils unabhängig voneinander eine ganze Zahl von 0 bis 4, vorzugsweise 1 bis 3, bedeutet, und
wobei der Rest Z jeweils unabhängig voneinander ein Säure-Rest ist, der aus der Gruppe, die aus Carboxygruppe und Sulfonsäuregruppe besteht, ausgewählt wird.

10. Photosensibilisator-haltige Zusammensetzung nach einem der Ansprüche 6 bis 9, wobei das wenigstens eine Porphycen-Derivat ein Porphycen-Derivat der allgemeinen Formel (3) ist: 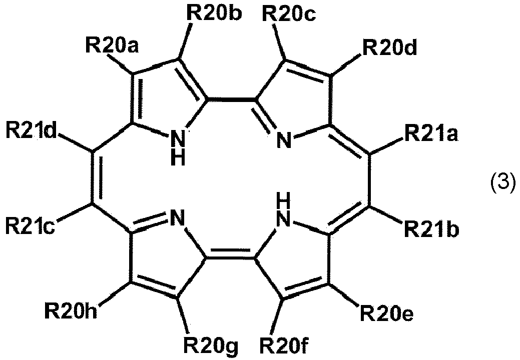
wobei die Reste R20a, R20b, R20c, R20d, R20e, R20f, R20g, und R20h, die jeweils unabhängig voneinander gleich oder voneinander verschieden sein können, jeweils Wasserstoff, Halogen, vorzugsweise Chlor, Brom, oder lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - [(C(D)(E))ₘ - O)ₙ - W oder ein Rest der allgemeinen Formel * - O [(C(D)(E))ₘ - O)ₙ - W bedeuten,
wobei der Index m jeweils unabhängig voneinander eine ganze Zahl von 1 bis 8, vorzugsweise von 2 bis 4, bedeutet, und wobei der Index n jeweils unabhängig voneinander eine ganze Zahl von 1 bis 6 bedeutet,
wobei der Rest W jeweils unabhängig voneinander Alkyl, das gradkettig oder verzweigt sein kann, mit 1 bis 6 Kohlenstoffatomen, Phenyl oder Benzyl bedeutet, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können,
wobei die Reste R21a, R21b, R21c, und R21d, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Aryloxy mit 6 bis 10 Kohlenstoffatomen, vorzugsweise Phenoxy oder Benzyloxy, Aryl, vorzugsweise Phenyl oder Benzyl, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, oder einen organischen Rest W1c, der:
c1) ein ungeladener organischer Rest, oder
c2) ein organischer Rest mit wenigstens einem permanent positiv geladenen Stickstoffatom oder wenigstens einem protonierbaren Stickstoffatom, vorzugsweise 1 oder 2 permanent positiv geladenen Stickstoffatomen oder 1 oder 2 protonierbaren Stickstoffatomen, das/die jeweils nicht direkt am aromatischen Ring angeordnet ist/sind, oder,
c3) ein organischer Rest mit wenigstens einer Säuregruppe, vorzugsweise 1 oder 2 Säuregruppen, oder
c4) ein amphoterer organischer Rest, der ein permanent positiv geladenes Stickstoffatom oder ein protonierbares Stickstoffatom, das jeweils nicht direkt am aromatischen Ring angeordnet ist, und eine Säuregruppe umfasst,
bedeutet, mit der Maßgabe, dass wenigstens einer der Reste R21a, R21b, R21c, und R21d ein organischer Rest W1c bedeutet,
wobei der Rest W1c in Variante c1) ein Rest mit der allgemeinen Formel (10a) bis (16b) bedeutet:
* - (C(D)(E))ₕ - T (10a)
* - Q - (C(D)(E))ₕ - T (10b)
* - [(C(D)(E))_{b} - A]ₐ - (C(D)(E))_{g} - T (11a)
_{*} - Q - [(C(D)(E))_{b} - A]ₐ - (C(D)(E))_{g} - T (11b)
* - [(C(D)(E))_{b} - A]ₐ - (C(D)(E))_{y}- Ar - (C(D)(E))_{g} - T (12a)
* - Q - [(C(D)(E))_{b} - A]ₐ - (C(D)(E))_{y}- Ar - (C(D)(E))_{g} - T (12b)
* - (C(D)(E))ₕ - Ar - (C(D)(E))_{g} - T (13a)
* - Q - (C(D)(E))ₕ - Ar - (C(D)(E))_{g} - T (13b)
* - (C(D)(E))_{y} - Ar - B - (C(D)(E))_{g} - T (14a)
* - Q - (C(D)(E))_{y} - Ar - B - (C(D)(E))_{g} - T (14b)
* - (C(D)(E))_{y} - Ar - B - [(C(D)(E))_{b} - A]_{c} - (C(D)(E))_{y} - T (15a)
* - Q - (C(D)(E))_{y} - Ar - B - [(C(D)(E))_{b} - A]_{c} - (C(D)(E))_{y} - T (15b)
* - [(C(D)(E))_{b} - A]ₐ - (C(D)(E)_{y} - Ar - B - [(C(D)(E))_{b} - A]_{c} - (C(D)(E))_{y} - T (16a)
_{*} - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet,
und wobei zwei oder mehr der Reste D und/oder E jeweils verbrückt sein können und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkanonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeute, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet, und
wobei der Rest W1c in Variante c2) ein Rest mit der allgemeinen Formel (21a) bis (24b) bedeutet:
* - [(C(D)(E))_{b} - A]_{c} - (C(D)(E))_{g} - X (21a)
_{*} - Q - [(C(D)(E))_{b} - A]_{c} - (C(D)(E))_{g} - X (21b)
* - [(C(D)(E))_{b} - A]ₐ - (C(D)(E))_{y} - Ar - (C(D)(E))_{f} - X (22a)
* - Q - [(C(D)(E))_{b} - A]ₐ - (C(D)(E))_{y} - Ar - (C(D)(E))_{f} - X (22b)
* - (C(D)(E))_{d} - Ar - (C(D)(E))_{f} - X (23a)
* - Q - (C(D)(E))_{d} - Ar - (C(D)(E))_{f} - X (23b)
*- (C(D)(E))_{d} - Ar - [A - (C(D)(E))_{b}]ₐ - X (24a)
* - Q - (C(D)(E))_{d} - Ar - [A - (C(D)(E))_{b}]ₐ - X (24b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Hydroxylalkyl, das gradkettig oder verzweigt sein kann, mit 1 bis 5 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon besteht, ausgewählt werden, substituiert sein können, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices d, und f jeweils unabhängig voneinander eine ganze Zahl von 1 bis 10, vorzugsweise 1 bis 8, vorzugsweise von 2 bis 4, bedeuten, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar einen unsubstituierten Phenylen-Rest oder einen substituierten Phenylen-Rest, der mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon besteht, ausgewählt werden, substituiert ist, bedeutet,
wobei der Rest X jeweils unabhängig voneinander ein Rest der Formel (15a), (15b), (16), (17a), (17b), (17c), oder (17d), vorzugsweise ein Rest der Formel (15a), (15b), (17a) oder (17b), bedeutet: 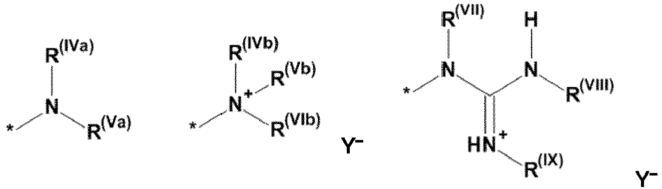 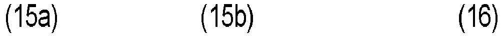 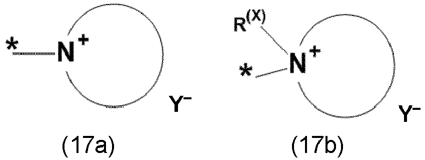 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)} und R^{(X)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (17a): 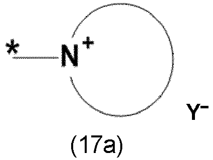 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, bedeutet, wobei der heterocyclische Rest mit 5 bis 10 Ringatomen unsubstituiert oder mit einem oder mehrerer Reste substituiert ist, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden, und
wobei der Rest mit der Formel (17b): 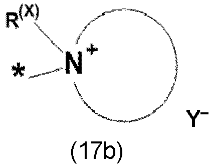 jeweils einen unsubstituierten oder substituierten heterocyclischen Rest mit 5 bis 10 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung mit einem Ringatom, vorzugsweise Kohlenstoffatom, ausbildet, bedeuten, wobei der heterocyclische Rest mit 5 bis 7 Ringatomen unsubstituiert oder mit einem oder mehrerer Reste substituiert ist, die aus der Gruppe, die aus der Gruppe, die aus Hydroxyl, Chlor, Brom, Fluor, Nitro, Cyano, Alkyl, das gradkettig oder verzweigt sein kann, mit 1 bis 15 Kohlenstoffatomen, vorzugsweise Methyl, Ethyl, n-Propyl, iso-Propyl, n-Butyl, tert-Butyl, n-Pentyl, Pent-3-yl, n-Nonyl, Non-5-yl, Aryl mit 5 bis 10 C-Atomen, vorzugsweise Phenyl, Naphth-1-yl oder Naphth-2-yl, Alkylaryl, das geradkettig oder verzweigt sein kann, mit 5 bis 15 C-Atomen, vorzugsweise Benzyl oder 3-Phenyl-prop-1-yl, Alkoxy, das gradkettig oder verzweigt sein kann, mit 1 bis 15 Kohlenstoffatomen, vorzugsweise Methoxy, Ethoxy, n-Propoxy oder iso-Propoxy, Aryloxy mit 5 bis 10 C-Atomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl, mit 6 bis 15 Kohlenstoffatomen und 1 bis 5 OH-Gruppen, Hydroxyalkylaryl, mit 7 bis 20 Kohlenstoffatomen und 1 bis 5 OH-Gruppen, Carbonsäurester, der geradkettig oder verzweigt sein kann, mit 1 bis 20 C-Atomen, Keton, das geradkettig oder verzweigt sein kann, mit 2 bis 20 C-Atomen, Ether, der geradkettig oder verzweigt sein kann, mit 2 bis 20 C-Atomen und Kombinationen davon besteht, ausgewählt werden,
und wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat, Tosylat und Mischungen davon besteht, ausgewählt ist, und
wobei der Rest W1c in Variante c3) ein Rest mit der allgemeinen Formel (20) bis (24b) bedeutet:
- Z (30)
* - [(C(D)(E))_{b} - A]_{c} - (C(D)(E))_{g} - Z (31a)
* - Q - [(C(D)(E))_{b} - A]_{c} - (C(D)(E))_{g} - Z (31b)
* - [(C(D)(E))_{b} - A]_{c} - (C(D)(E))_{y} - Ar - (C(D)(E))ₑ - Z (32a)
_{*} - Q - [(C(D)(E))_{b} - A]_{c} - (C(D)(E))_{y} - Ar - (C(D)(E))ₑ - Z (32b)
* - (C(D)(E))_{d} - Ar - (C(D)(E))ₑ - Z (33a)
* - Q - (C(D)(E))_{d} - Ar - (C(D)(E))ₑ - Z (33b)
*- (C(D)(E))_{d} - Ar - [A - (C(D)(E))_{b}]ₐ - Z (34a)
* - Q - (C(D)(E))_{d} - Ar - [A - (C(D)(E))_{b}]ₐ - Z (34b)
wobei die Reste Q und A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet,
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Index a jeweils unabhängig voneinander eine ganze Zahl von 1 bis 10, vorzugsweise von 1 bis 6, vorzugsweise 2 bis 4, weiter bevorzugt 1 bis 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis107, bedeutet, und
wobei der Index d jeweils unabhängig voneinander eine ganze Zahl von 1 bis 10, vorzugsweise von 1 bis 4 bedeutet, und
wobei der Index e jeweils unabhängig voneinander eine ganze Zahl von 0 bis 10, vorzugsweise 1 bis 8, vorzugsweise von 2 bis 4, bedeutet, und
wobei die Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert ist, bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Rest W1c in Variante c4) ein Rest mit der allgemeinen Formel (41a) oder (41b) bedeutet:
* - (CH₂)_{g} - Het - (CH₂)ᵢ - Z (41a)
* - (CH₂)_{g} -Het - [B - (CH₂)ₖ]ₗ - Z (41b)
wobei der Rest B jeweils unabhängig voneinander Sauerstoff, ein Rest der Formel * - C(=O) - *, ein Rest der Formel * - C(=O)O - *, ein Rest der Formel * - OC(=O) - *, oder ein Rest der Formel * - C(=O)N(R⁽⁶⁾) - * bedeutet, wobei der Rest R⁽⁶⁾ jeweils unabhängig voneinander Wasserstoff oder Alkyl, das gradkettig oder verzweigt sein kann, mit 1 bis 4 Kohlenstoffatomen bedeutet,
wobei der Rest Het jeweils einen heterocyclischen Rest mit 5 bis 10 Ringatomen, die mindestens 1 Kohlenstoffatom und mindestens 1 Stickstoffatom sowie optional 1 oder 2 Sauerstoffatome umfassen, bedeutet, der vorzugsweise ein Rest mit der Formel (17a) oder ein Rest mit der Formel (17c) ist: 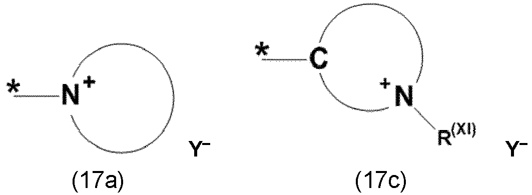
wobei der heterocyclisch Rest 5 bis 10 Ringatome aufweist, die mindestens 1 Kohlenstoffatom und mindestens 1 Stickstoffatom sowie optional 1 oder 2 Sauerstoffatome umfassen, wobei 1 Stickstoffatom eine Doppelbindung mit einem Ringatom, vorzugsweise Kohlenstoffatom, ausbildet, wobei der Rest R^{(XI)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest Y⁻ ein Anion bedeutet, das jeweils unabhängig voneinander aus der Gruppe, die aus Chlorid, Bromid, lodid, Sulfat, Hydrogensulfat, Phosphat, Hydrogenphosphat, Dihydrogenphosphat, Hexafluorophosphat, wenigstens ein Carboxylat-Anion einer Carbonsäure mit 1 bis 15 Kohlenstoffatomen und wenigstens einer Carboxylgruppe, wenigstens ein Sulfonat-Anion einer Sulfonsäure mit 1 bis 25 C-Atomen und wenigstens einer Sulfonsäuregruppe, Tetrafluoroborat, Tetraphenylborat, Tetrabutylborat und Mischungen davon besteht, ausgewählt ist, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei die Indices i, und k jeweils unabhängig voneinander eine ganze Zahl von 1 bis 4, vorzugsweise 2 bis 3, bedeuten, und
wobei der Index l jeweils unabhängig voneinander eine ganze Zahl von 0 bis 4, vorzugsweise 1 bis 3, bedeutet, und
wobei der Rest Z jeweils unabhängig voneinander ein Säure-Rest ist, der aus der Gruppe, die aus Carboxygruppe und Sulfonsäuregruppe besteht, ausgewählt wird.

11. Photosensibilisator-haltige Zusammensetzung nach einem der Ansprüche 6 bis 10, ferner umfassend wenigstens einen Hilfsstoff, vorzugsweise wenigstens ein Komplexbildner, wenigstens ein Konservierungsmittel, und/oder wenigstens ein Pufferungsmittel.

12. Phenalen-1-on Derivat der allgemeinen Formel (1): 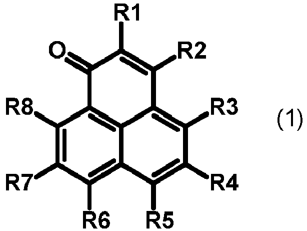
wobei die Reste R1 bis R8, die jeweils unabhängig voneinander gleich oder voneinander verschieden sein können, jeweils Wasserstoff, Chlor, Brom, lod, Alkyl mit 1 bis 4 C-Atomen, vorzugsweise Methyl, Ethyl, n-Propyl oder iso-Propyl, *-O-Alkyl mit 1 bis 4 C-Atomen, vorzugsweise Methoxy oder Ethoxy, oder einen organischen Rest W1a, der ein ungeladener organischer Rest ist, bedeuten,
mit der Maßgabe, dass wenigstens einer der Reste R1 bis R8, vorzugsweise wenigstens einer der Reste R1 bis R6, vorzugsweise wenigstens einer der Reste R1, R2, R4, R6 oder R8, weiter bevorzugt wenigstens einer der Reste R1, R2, R4 oder R6, weiter bevorzugt wenigstens einer der Reste R1 oder R2, ein organischer Rest W1a bedeutet,
wobei der Rest W1a ein Rest mit der allgemeinen Formel (10a) bis (12b), (13b), (14b), oder (15b) bis (16b) vorzugsweise ein Rest mit der allgemeinen Formel (10b) bis (12b), (13b), (14b), oder (15b) bis (16b), bedeutet:
* - (C(D)(E))ₕ - T (10a)
* - Q - (C(D)(E))ₕ - T (10b)
* - [(C(D)(E))_{b} - A]ₐ - (C(D)(E))_{g} - T (11a)
_{*} - Q - [(C(D)(E))_{b} - A]ₐ - (C(D)(E))_{g} - T (11b)
* - [(C(D)(E))_{b} - A]ₐ - (C(D)(E))_{y}- Ar - (C(D)(E))_{g} - T (12a)
* - Q - [(C(D)(E))_{b} - A]ₐ - (C(D)(E))_{y}- Ar - (C(D)(E))_{g} - T (12b)
* - Q - (C(D)(E))ₕ - Ar - (C(D)(E))_{g} - T (13b)
* - Q - (C(D)(E))_{y} - Ar - B - (C(D)(E))_{g} - T (14b)
* - Q - (C(D)(E))_{y} - Ar - B - [(C(D)(E))_{b} - A]_{c} - (C(D)(E))_{y} - T (15b)
* - [(C(D)(E))_{b} - A]ₐ - (C(D)(E)_{y} - Ar - B - [(C(D)(E))_{b} - A]_{c} - (C(D)(E))_{y} - T (16a)
_{*} - Q - [(C(D)(E))_{b} - A]ₐ - (C(D)(E)_{y} - Ar - B - [(C(D)(E))_{b} - A]_{c} - (C(D)(E))_{y} - T (16b)
wobei der Rest Q jeweils unabhängig voneinander Sauerstoff, ein Rest der Formel * - C(=O)O - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest B jeweils unabhängig voneinander Sauerstoff, ein Rest der Formel * - C(=O) - *, ein Rest der Formel * - C(=O)O - *, ein Rest der Formel * - OC(=O) - *, ein Rest der Formel * - NR⁽²⁾(=O)C - *, oder ein Rest der Formel * - C(=O)NR⁽²⁾ - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest T jeweils unabhängig voneinander ein Rest der allgemeinen Formel * - (C(D)(E)H, oder * - Ph bedeutet,
wobei die Reste D und E jeweils unabhängig voneinander Wasserstoff, Halogen, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, ein Rest der allgemeinen Formel * - N(R⁽²⁾)C(=O) - R⁽³⁾, oder ein Rest der allgemeinen Formel * - C(=O)O - R⁽⁴⁾ bedeuten,
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽²⁾ jeweils unabhängig voneinander Wasserstoff oder Alkyl, das gradkettig oder verzweigt sein kann, mit 1 bis 4 Kohlenstoffatomen bedeutet, und
wobei der Rest R⁽³⁾ jeweils unabhängig voneinander Alkyl, das gradkettig oder verzweigt sein kann, mit 1 bis 12 Kohlenstoffatomen, Alkoxy, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Ar ein aromatischer Rest mit 6 Ringatomen bedeutet, wobei Rest Ar ein unsubstituierter Phenylen-Rest oder ein substituierter Phenylen-Rest, der mit einem oder mehrerer Reste, die aus der Gruppe, die aus Hydroxyl, Chlor, Brom, Fluor, Nitro, Methyl, Ethyl, n-Propyl, iso-Propyl, Methoxy, Ethoxy, und Kombinationen davon, besteht, ausgewählt werden, substituiert ist, bedeutet, und
wobei zwei oder mehr der Reste D und/oder E jeweils verbrückt sein können und jeweils einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, cyclischer Ether-Rest mit 4 bis 25 C-Atomen und 1 oder 2 O-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei die C-Atome des Cycloalkylrestes, cyclischen Ether-Restes, Cycloalkenylrestes, Cycloalkanonrestes, und Cycloalkanonrestes jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei wenigstens einer der Reste D und/oder E mit wenigstens einem Ringatom des Rests Ar verbrückt sein kann und einen oder mehrere ringförmige monocyclische oder polycyclische organische Reste mit 4 bis 25 C-Atomen, die vorzugsweise aus der Gruppe, die aus Cycloalkylresten mit 4 bis 25 C-Atomen, Cycloalkenylresten mit 4 bis 25 C-Atomen und wenigstens einer Doppelbindung, Cycloalkanonresten mit 4 bis 25 C-Atomen und wenigstens einer Ketogruppe, Cycloalkenonresten mit 4 bis 25 C-Atomen, wenigstens einer Doppelbindung und wenigstens einer Ketogruppe und Kombinationen davon besteht, ausgewählt werden, bilden können, wobei C-Atome der Cycloalkylreste, Cycloalkenylreste, Cycloalkanonreste, und Cycloalkanonreste jeweils unsubstituiert oder mit einem oder mehrerer Reste, die aus der Gruppe, die aus - OH, Chlor, Brom, Fluor, Nitro, Methyl, Ethyl, n-Propyl, iso-Propyl, Methoxy, Ethoxy, und Kombinationen davon, besteht, ausgewählt werden, substituiert sein können, und
wobei der Index h jeweils unabhängig voneinander eine ganze Zahl von 4 bis 30, vorzugsweise von 5 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 1 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 bis 2, bedeutet, und
wobei der Index c jeweils unabhängig voneinander eine ganze Zahl von 0 bis 10, vorzugsweise 0 bis 6, vorzugsweise 0 bis 4, weiter bevorzugt 1 bis 2, bedeutet, und
wobei der Index y jeweils unabhängig voneinander eine ganze Zahl von 0 bis 6, vorzugsweise 0 bis 4, vorzugsweise 1 bis 2, bedeutet, und
wobei in Rest (11a) Index g eine ganze Zahl von 2 bis 35 bedeutet, wenn Rest A ein Rest der Formel * - OC(=O) - ** und Rest T ein Rest der allgemeinen Formel * - C(D)(E)H bedeutet, und
wobei in Rest (15b) Index c eine ganze Zahl von 1 bis 35 bedeutet, wenn Rest B Sauerstoff und Rest T ein Rest der allgemeinen Formel * - C(D)(E)H bedeute, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet.

13. Curcumin-Derivat der allgemeinen Formel (2a): 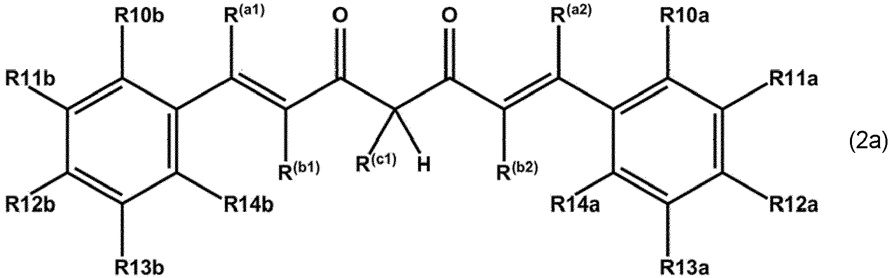
wobei die Reste R^{(a1)}, R^{(a2)}, R^{(b1)} und R^{(b2)}, die jeweils unabhängig voneinander gleich oder voneinander verschieden sein können, jeweils Wasserstoff, Halogen, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können,
wobei der Rest R^{(c1)} jeweils unabhängig voneinander Wasserstoff, Chlor, Brom, lod, Alkyl, das gradkettig oder verzweigt sein kann, mit 1 bis 4 C-Atomen, vorzugsweise Methyl, Ethyl, n-Propyl oder iso-Propyl, *-O-Alkyl, das gradkettig oder verzweigt sein kann, mit 1 bis 4 C-Atomen, vorzugsweise Methoxy oder Ethoxy, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
und wobei die Reste R10a bis R14b, die jeweils unabhängig voneinander gleich oder voneinander verschieden sein können, jeweils Wasserstoff, Halogen, vorzugsweise Chlor, Brom oder lod, Alkyl, das gradkettig oder verzweigt sein kann, mit 1 bis 8 C-Atomen, vorzugsweise Methyl, Ethyl, n-Propyl oder iso-Propyl, *-O-Alkyl, das gradkettig oder verzweigt sein kann, mit 1 bis 8 C-Atomen, vorzugsweise Methoxy oder Ethoxy, Aryl mit 5 bis 10 C-Atomen, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkylaryl mit 5 bis 10 C-Atomen, die jeweils unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, oder einen organischen Rest W1b bedeutet,
wobei in Variante b1) wenigstens einer der Reste R10a bis R14a und wenigstens einer der Reste R10b bis R14b ein organischer Rest W1b bedeutet und optional wenigstens einer der Reste R10a bis R14a und wenigstens einer der Reste R10b bis R14b, der nicht ein Rest W1b bedeutet, weiterhin Chlor, Brom oder lod bedeutet,
wobei der Rest W1b in Variante b1) ein Rest mit der allgemeinen Formel (10a) bis (11b) bedeutet:
* - (C(D)(E))ₕ - T (10a)
* - Q - (C(D)(E))ₕ - T (10b)
* - [(C(D)(E))_{b} - A]ₐ - (C(D)(E))_{g} - T (11a)
* - Q - [(C(D)(E))_{b} - A]ₐ - (C(D)(E))_{g} - T (11b)
wobei der Rest Q jeweils unabhängig voneinander Sauerstoff, ein Rest der Formel * - C(=O)O - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, weiter bevorzugt Sauerstoff, bedeutet, und
wobei der Rest T jeweils unabhängig voneinander ein Rest der allgemeinen Formel * - (C(D)(E)H, oder * - Ph, vorzugsweise ein Rest der allgemeinen Formel * - (C(D)(E)H, bedeutet
wobei die Reste D und E jeweils unabhängig voneinander Wasserstoff, Halogen, Hydroxyl, Nitro, Alkyl, das gradkettig oder verzweigt sein kann, mit 1 bis 5 Kohlenstoffatomen, Phenyl, Benzyl, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, oder ein Rest der allgemeinen Formel * - C(=O)O - R⁽⁴⁾ bedeuten,
wobei der Rest R⁽¹⁾, jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeutet, und
wobei der Reste R⁽⁴⁾ jeweils unabhängig voneinander Alkyl, das gradkettig oder verzweigt sein kann, mit 1 bis 4 Kohlenstoffatomen bedeutet,
wobei der Index h jeweils unabhängig voneinander eine ganze Zahl von 1 bis 30, vorzugsweise von 4 bis 28, vorzugsweise von 6 bis 15, vorzugsweise von 7 bis 10, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet, und
wobei der Index b jeweils unabhängig voneinander eine ganze Zahl von 1 bis 4, vorzugsweise von 2 bis 3, weiter bevorzugt 1, bedeutet, und
wobei der Index a jeweils unabhängig voneinander eine ganze Zahl von 1 bis 10, vorzugsweise 1 bis 6, vorzugsweise 2 bis 4, weiter bevorzugt 1 oder 2, bedeutet, und
wobei der Rest Ph ein unsubstituierter Phenyl-Rest oder ein substituierter Phenyl-Rest, der mit einem oder mehrerer Reste, die aus der Gruppe, die aus Chlor, Brom, Fluor, Methyl, Ethyl, n-Propyl, iso-Propyl, Methoxy, Ethoxy, einem Rest der allgemeinen Formel * - C(=O)NH₂ und Kombinationen davon, besteht, ausgewählt werden, substituiert ist, bedeutet oder
wobei in Variante b3) wenigstens einer der Reste R10a bis R14a und wenigstens einer der Reste R10b bis R14b ein organischer Rest W1b bedeutet und optional wenigstens einer der Reste einer der Reste R10a bis R14a und wenigstens einer der Reste R10b bis R14b, der nicht ein Rest W1b bedeutet, weiterhin Chlor, Brom oder Iod bedeutet,
wobei der Rest W1b in Variante b3) ein Rest mit der allgemeinen Formel (30) bis (31b) bedeutet:
- Z (30)
* - [(C(D)(E))_{b} - A]_{c} - (C(D)(E))_{g} - Z (31a)
* - Q - [(C(D)(E))_{b} - A]_{c} - (C(D)(E))_{g} - Z (31b)
wobei der Rest Q jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei der Rest A jeweils unabhängig voneinander Sauerstoff, ein Rest der Formel * - C(=O) - *, ein Rest der Formel * - C(=O)O - *, oder ein Rest der Formel * - OC(=O) - *, vorzugsweise Sauerstoff, ein Rest der Formel * - C(=O)O - *, oder ein Rest der Formel * - OC(=O) - *, vorzugsweise Sauerstoff oder ein Rest der Formel * - OC(=O) - *, bedeutet, und
wobei die Reste D und E jeweils unabhängig voneinander Wasserstoff, Halogen, Hydroxyl, Alkyl, das gradkettig oder verzweigt sein kann, mit 1 bis 5 Kohlenstoffatomen, Phenyl, oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Methyl, Ethyl, n-Propyl, iso-Propyl, Methoxy, Ethoxy, und Kombinationen davon, besteht, ausgewählt werden, substituiert sein können, Alkoxy, das gradkettig oder verzweigt sein kann, mit 1 bis 5 Kohlenstoffatomen, Aryloxy mit 6 bis 10 Kohlenstoffatomen, vorzugsweise Phenoxy oder Benzyloxy, Hydroxylalkyl, das gradkettig oder verzweigt sein kann, mit 1 bis 5 Kohlenstoffatomen und 1 bis 5 OH-Gruppen, Hydroxylaryl mit 6 bis 10 Kohlenstoffatomen und 1 bis 5 OH-Gruppen, Halogenalkyl, das gradkettig oder verzweigt sein kann, mit 1 bis 5 Kohlenstoffatomen und 1 bis 5 Halogen-Gruppen, Halogenaryl mit 6 bis 10 Kohlenstoffatomen und 1 bis 5 Halogen-Gruppen, ein Rest der allgemeinen Formel * - OC(=O) - R⁽¹⁾, oder ein Rest der allgemeinen Formel * - C(=O)O - R⁽⁴⁾ bedeuten, und
wobei der Rest R⁽¹⁾ jeweils unabhängig voneinander Alkyl, das gradkettig oder verzweigt sein kann, mit 1 bis 4 Kohlenstoffatomen, Phenyl oder Benzyl bedeuten, wobei Phenyl und Benzyl jeweils unabhängig voneinander unsubstituiert oder mit einem oder mehrerer Reste, die aus der Gruppe, die aus Chlor, Brom, Fluor, Nitro, Methyl, Ethyl, n-Propyl, iso-Propyl, Methoxy, Ethoxy, und Kombinationen davon, besteht, ausgewählt werden, substituiert sein können, bedeuten, und
wobei der Rest R⁽⁴⁾ jeweils unabhängig voneinander Alkyl, das gradkettig oder verzweigt sein kann, mit 1 bis 4 Kohlenstoffatomen bedeutet, und
wobei der Rest Z jeweils unabhängig voneinander ein Säure-Rest ist, der aus der Gruppe, die aus Carboxygruppe und Sulfonsäuregruppe besteht, ausgewählt wird,
wobei der Index c jeweils unabhängig voneinander eine ganze Zahl von 0 bis 10, vorzugsweise von 0 bis 6, vorzugsweise 0 bis 4, weiter bevorzugt 1 oder 2, bedeutet, und
wobei der Index b jeweils unabhängig voneinander eine ganze Zahl von 1 bis 4, vorzugsweise von 2 bis 3, weiter bevorzugt 1, bedeutet, und
wobei der Index g jeweils unabhängig voneinander eine ganze Zahl von 1 bis 30, vorzugsweise von 2 bis 28, vorzugsweise von 3 bis 15, vorzugsweise von 4 bis 10, bedeutet.

14. Photosensibilisator-haltige Zusammensetzung nach einem der Ansprüche 6 bis 11, wobei der wenigstens einen Photosensibilisator aus der Gruppe ausgewählt ist, die aus wenigstens einem Phenalen-1-on-Derivat gemäß Anspruch 13, wenigstens einem Curcumin-Derivat gemäß Anspruch 14, und Mischungen davon besteht.

15. Verfahren nach einem der Ansprüche 1 bis 5,
**dadurch gekennzeichnet,**
**dass** in Schritt (a) eine Photosensibilisator-haltige Zusammensetzung gemäß einem der Ansprüche 6 bis 11 oder 14 verwendet wird, wobei vorzugsweise vor Verwendung in Schritt (a) der Wassergehalt der Zusammensetzung auf einen Anteil von mindestens 50 Gew.-%, bezogen auf das Gesamtgewicht der Photosensibilisator-haltigen Zusammensetzung, eingestellt wird.

16. Mehrkomponentenzusammensetzung zur gemeinsamen Verwendung in einem Verfahren nach einem der Ansprüche 1 bis 5 oder 15, umfassend
A) eine erste Zusammensetzung, umfassend Wasser, und wenigstens ein Co-Solvens, das aus der Gruppe, die aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon besteht, ausgewählt ist, sowie optional wenigstens einen Alkohol, der vorzugsweise 2 bis 10 C-Atome und 1 bis 4 OH-Gruppen aufweist, und/oder wenigstens einen Hilfsstoff, und
B) eine zweite Zusammensetzung, umfassend wenigstens einen Photosensibilisator, der aus der Gruppe ausgewählt ist, die aus Phenalen-1-on-Derivaten, Curcumin-Derivaten, Porphycen-Derivaten und Mischungen davon, vorzugsweise Phenalen-1-on-Derivaten, Curcumin-Derivaten, und Mischungen davon, besteht, wenigstens ein Co-Solvens, das aus der Gruppe, die aliphatischen Estern mit vorzugsweise 4 bis 20 C-Atomen, die gradkettig oder verzweigt sein können, aliphatischen Ketonen mit vorzugsweise 3 bis 10 C-Atomen, die gradkettig oder verzweigt sein können, und Mischungen davon besteht, ausgewählt ist, sowie optional Wasser und/oder wenigstens einen Hilfsstoff.

17. Verfahren nach einem der Ansprüche 1 bis 5 oder 15,
**dadurch gekennzeichnet,**
**dass** im Verfahren eine Mehrkomponentenzusammensetzung nach Anspruch 16 verwendet wird, wobei vor und/oder während Schritt (a) die erste Zusammensetzung und die zweite Zusammensetzung der Mehrkomponentenzusammensetzung in einem Verhältnis von 10:1 bis 1:10 unter Erhalt einer als Photosensibilisator-haltigen Zusammensetzung gemischt werden, wobei optional die erhaltene Photosensibilisator-haltige Zusammensetzung nach dem Mischen mit Wasser verdünnt wird und/oder die erste Zusammensetzung und/oder die zweite Zusammensetzung der Mehrkomponentenzusammensetzung vor dem Mischen mit Wasser verdünnt wird.

18. Photokatalytisch aktiver Gegenstand, vorzugsweise erhältlich nach einem Verfahren gemäß einem der Ansprüche 1 bis 5, 15 oder 17,
**dadurch gekennzeichnet,**
**dass** in wenigstens einem Teilbereich wenigstens einer Oberfläche des Gegenstandes, umfassend wenigstens ein thermoplastisches Polymer, wenigstens ein elastomeres Polymer, Copolymere davon und Mischungen davon, wenigstens ein Photosensibilisator, der aus der Gruppe ausgewählt ist, die aus Phenalen-1-on-Derivaten, Curcumin-Derivaten, Porphycen-Derivaten und Mischungen davon besteht, eingebracht ist.

19. Photokatalytisch aktiver Gegenstand nach Anspruch 18, wobei der Gegenstand in Form eines selbsttragenden Films, eines Verbunds aus Fasern oder eines Formgegenstandes vorliegt.
